# EUROPÄISCHE PATENTSCHRIFT

(11) **EP 1 571 140 B1**
(45) Veröffentlichungstag und Bekanntmachung des Hinweises auf die Patenterteilung: **12.11.2008**
(21) Anmeldenummer: 05011581.5
(22) Anmeldetag: 28.09.2001
(51) Int. Cl.: C07C 217/52, A61K 31/133, A61P 23/00, C07C 233/41, A61K 31/33

(54) **Substituierte C-Cyclohexylmethylamin-Derivative**
C-substituted cyclohexylmethylamine derivatives
Dérivés de cyclohexylméthylamine C-substitués

(30) Priorität: 29.09.2000 DE 10049481
(43) Veröffentlichungstag der Anmeldung: 07.09.2005
(62) Teilanmeldung aus: 01982379.8
(73) Patentinhaber: Grünenthal GmbH, 52099 Aachen (DE)
(72) Erfinder: Sundermann, Bernd, Dr., 52074 Aachen (DE); Sundermann, Corinna, Dr., 52074 Aachen (DE); Buschmann, Helmut, Dr., 8950 Espluges de Llobregat (ES); Finkam, Michael, Dr., 52066 Aachen (DE); Kögel, Babett-Yvonne, Dr., 52379 Langerwehe-Hamich (DE)

(56) Entgegenhaltungen:
- EP-A- 0 780 369
- WO-A-01/57232
- CHEMICAL ABSTRACTS, Bd. 94, Nr. 21, 25. Mai 1981 (1981-05-25), Columbus, Ohio, US; abstract no.: 175268m, YRJAENHEIKKI, ERKKI ET AL.: "The reactions of certain pinenes with dimethylamine and formaldehyde." Seite 747 Spalte 2 XP002229778 & ACTA UNIV. OULUENSIS, SER. A, Bd. 103, 1980, Seite 88,
- GAIS H-J ET AL: "Enzymatic resolution of analgesics: delta-hydroxytramadol, ?-hydroxytramadol and O-desmethyltramadol" TETRAHEDRON: ASYMMETRY, ELSEVIER SCIENCE PUBLISHERS, AMSTERDAM, NL, Bd. 11, Nr. 4, März 2000 (2000-03), Seiten 917-928, XP004192904 ISSN: 0957-4166
- BENNO REICHERT ET AL.: "Aminomethylierungsprodukte alkylsubstituierter Cyclohexanone und ihre Umsetzung" ARZNEIMITTEL FORSCHUNG. DRUG RESEARCH., Bd. 13, Nr. 11, 1963, Seiten 991-999, XP001148954 EDITIO CANTOR. AULENDORF., DE ISSN: 0004-4172

## Beschreibung

Die vorliegende Erfindung betrifft substituierte C-Cyclohexylmethylamin-Derivate, Verfahren zu deren Herstellung, Arzneimittel enthaltend diese Verbindungen und die Verwendung von substituierten C-Cyclohexylmethylamin-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Eine der Erfindung zugrundeliegende Aufgabe bestand darin, neue analgetisch wirksame Substanzen zur Verfügung zu stellen, die sich zur Schmerztherapie - insbesondere auch chronischer und neuropathischer Schmerzen - eignen.

Gegenstand der Erfindung sind daher substituierte C-Cyclohexylmethylamin-Derivate der allgemeinen Formel I, 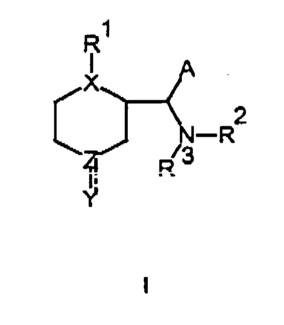 bei denen
A ausgewählt ist aus H Phenyl oder
R¹ ausgewählt ist aus C₁₋₁₀-Alkyl oder C₃₋₁₀-Cycloalkyl, jeweils gesättigt oder ungesättigt, verzweigt oder unverzweigt einfach oder mehrfach substituiert oder unsubstituiert; Phenyl, Naphtyl, Furyl oder Thiophenyl über C₁₋₃-Alkylen oder Ethinyl gebundenem Phenyl oder naphtyl, C₁₋₃-Alkylen oder Ethinyl gebundenem C₃₋₁₀-Cycloalkyl, oder über C₁₋₃-Alkylen oder Ethinyl gebundenem Furyl oder thiophenyl jeweils unsubstituiert oder einfach oder mehrfach substituiert mit Resten unabhängig voneinander ausgewählt aus
-F, Cl, Br, I, OR¹⁸, -SR¹⁸, SO₂R¹⁸, SO₂OR¹⁸, CN, COOR¹⁸, NR¹⁹R²⁰; C₁₋₁₀-Alkyl oder C₃₋₁₀-Cycloalkyl, jeweils unsubstituiert oder einfach oder mehrfach, gleich oder verschieden, substituiert mit F, Cl, Br, NH₂, SH oder OH, oder Silyl, mit R¹⁸ ausgewählt aus H; C₁₋₁₀-Alkyl oder C₃₋₁₀-Cycloalkyl, jeweils gesättigt oder ungesättigt, verzweigt oder unverzweigt,
R¹⁹ und R²⁰ unabhängig voneinander ausgewählt aus H; C₁₋₁₀-Alkyl oder C₃₋₁₀-Cycloalkyl, jeweils gesättigt oder ungesättigt, verzweigt oder unverzweigt,
oder R¹⁹ und R²⁰ zusammen CH₂CH₂OCH₂CH₂, CH₂CH₂NR²¹ CH₂CH₂ oder (CH₂)₃₋₆ bilden,
mit R²¹ ausgewählt aus H; Phenyl, C₁₋₁₀-Alkyl gesättigt oder ungesättigt, verzweigt oder unverzweigt,
R² und R³, unabhängig voneinander ausgewählt sind aus H; C₁₋₁₀-Alkyl oder C₃₋₁₀-Cycloalkyl, jeweils gesättigt oder ungesättigt, verzweigt oder unverzweigt,
oder die Reste R² und R³ zusammen CH₂CH₂OCH₂CH₂, CH₂CH₂NR⁶CH₂CH₂ oder (CH₂)₃₋₆ bedeuten,
mit R⁶ ausgewählt aus H; C₁₋₁₀-Alkyl oder C₃₋₁₀-Cycloalkyl, jeweils gesättigt oder ungesättigt, verzweigt oder unverzweigt,
X in der Formel I gemäß Teilformel la der Formel I 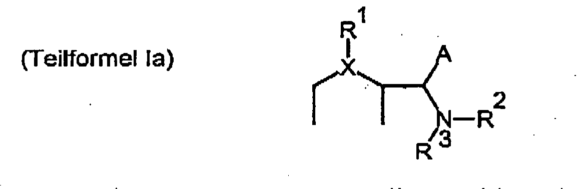 folgende Bedeutung hat 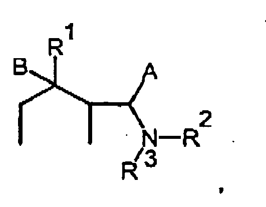 mit B ausgewählt aus OH, OR⁷, H, F, Cl oder NR⁸R⁹,
mit R⁷ ausgewählt aus C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH; unsubstituiert oder einfach oder mehrfach, gleich oder verschieden, substituiert mit F, Cl, Br, I, NH₂, SH oder OH;
R⁸ und R⁹, unabhängig voneinander ausgewählt sind aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH;
oder die Reste R⁸ und R⁹ zusammen CH₂CH₂OCH₂CH₂, CH₂CH₂NR¹⁰CH₂CH₂ oder (CH₂)₃₋₆ bilden
mit R¹⁰ ausgewählt aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH; 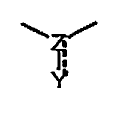 ausgewählt ist aus 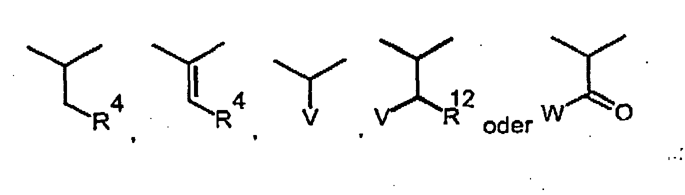 mit R⁴ ausgewählt aus COR⁵, SO₂R⁵; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Naphthyl, Phenyl, Furyl oder Thiophenyl, jeweils einfach oder mehrfach substituiert oder unsubstituiert; oder über C₁₋₃-Alkylen gebundenem Phenyl, Naphthyl, C₃₋₁₀-Cycloalkyl, Furyl oder Thiophenyl jeweils einfach oder mehrfach substituiert oder unsubstituiert,
mit R⁵ ausgewählt aus C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Phenyl, Naphthyl Furyl oder Thiophenyl, jeweils einfach oder mehrfach substituiert oder unsubstituiert; oder über C₁₋₃-Alkylen gebundenem Phenyl, Naphthyl, C₃₋₁₀-Cycloalkyl Furyl oder Thiophenyl, jeweils einfach oder mehrfach substituiert oder unsubstituiert,
mit V ausgewählt aus OR⁴ oder NR⁴R¹¹,
mit W ausgewählt aus R¹¹, OR¹² oder NR¹¹R¹²,
mit R¹¹ und R¹² unabhängig voneinander ausgewählt aus H, C₇₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Naphthyl Phenyl, Furyl oder Thiophenyl, jeweils einfach oder mehrfach substituiert oder unsubstituiert; oder über C₁₋₃-Alkylen gebundenem Phenyl, Naphthyl, C₃₋₁₀-Cycloalkyl, Furyl oder Thiophenyl, jeweils einfach oder mehrfach substituiert oder unsubstituiert,
wobei man im Zusammenhang mit Alkyl und Cycloalkyl von R⁴, R⁵, R¹¹ und R¹² unter substituiert die Substitution mit F, Cl, Br, I, NH₂, SH oder OH versteht, und
im Zusammenhang mit Naphthyl, Phenyl, Furyl und Thiophenyl von R⁴, R⁵, R¹¹ und R¹² unter substituiert die Substitution des Naphthyl, Phenyl, Furyl oder Thiophenyl mit R²³, OR²³, einem Halogen, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen versteht, wobei
der Rest R²³ für H, einen C₁₋₁₀-Alkyl-, einen Naphthyl-, Phenyl-, Furyl- oder Thiophenyl-Rest oder für einen über eine C₁₋₃-Alkylen-Gruppe gebundenen Naphthyl-, Phenyl-, Furyl-oder Thiophenyl-Rest steht, wobei diese Naphthyl-, Phenyl-, Furyl- und Thiophenyl-Reste nicht selbst mit Aryl- oder Heteroaryl-Resten substituiert sein dürfen,
die Reste R²⁴ und R²⁵, gleich oder verschieden, für H, einen C₁₋₁₀-Alkyl-, einen Naphthyl-, Phenyl-, Furyl- oder Thiophenyl-Rest oder einen über eine C₁₋₃-Alkylen-Gruppe gebundenen Naphthyl-, Phenyl-, Furyl- oder Thiophenyl-Rest stehen, wobei diese Naphthyl-, Phenyl-, Furyl- und Thiophenyl-Reste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, Naphthyl-, Phenyl-, Furyl- oder Thiophenyl-Rest oder für einen über eine C₁₋₃-Alkylen-Gruppe gebundenen Naphthyl-, Phenyl-, Furyl- oder Thiophenyl-Rest steht, wobei diese Naphthyl-, Phenyl-, Furyl- oder Thiophenyl-Reste nicht selbst mit Aryl- oder Heteroaryl-Resten substituiert sein dürfen gegebenenfalls In Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Die erfindungsgemäßen Verbindungen zeigen hervorragende analgetische Eigenschaften.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4-oder C5-Alkyl, C₁₋₆-Alkyl für C1-, C2-, C3-, C4-, C5- oder C6-Alkyl, C₁₋₇-Alkyl für C1-,
C2-, C3-. C4-, C5-, C6- oder C7-Alkyl, C₁₋₈-Alkyl für C1-, C2-, C3-, C4-, C5-, C6-, C7-oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8, C9-, C10-, C11-, C12-C13-, C14-, C15-, C16-, C17- oder C18-Alkyl. Weiter steht C₃₋₄-Cycloalkyl für C3-, oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl. C₄₋₅-Cycloalkyl für C4- oder C5-Cycloalkyl, C₄₋₆-Cycloalkyl für C4-, C5- oder C6-Cycloalkyl, C₄₋₇-cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen, unter (CH₂)₁₋₄ ist -CH₂-,-CH₂-CH₂-, -CH₂-CH₂-CH₂- und -CH₂-CH₂-CH₂-CH₂- zu verstehen, unter (CH₂)₄₋₅ ist - CH₂-CH₂-CH₂-CH₂- und -CH₂-CH₂-CH₂-CH₂-CH₂- zu verstehen, etc.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und lonen zu verstehen, insbesondere Komplexe, die über ionische Wechselwirkungen komplexiert sind. Insbesondere versteht man darunter physiologisch verträgliche Salze mit Kationen oder Basen und physiologisch verträgliche Salze mit Anionen oder Säuren.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Das Analgetikum Tramadol wird in vielen Ländern seit Jahren erfolgreich zur Therapie mäßig starker bis starker Schmerzen eingesetzt. Strukturell vom Codein abgeleitet und ursprünglich als Hustenmittel konzipiert, stellte man schon bei frühen Untersuchungen fest, das Tramadol eine ausgeprägte analgetische Wirksamkeit aufweist. Erklärt wird dies heute dadurch, das in vivo der Tramadol-Metabolit M1, der eine deutliche Affinität zum µ-Subtyp des Opiatrezeptors zeigt, zügig gebildet wird. Anders als bei Codein bzw. Morphin konnte aber die Beteiligung weiterer Mechanismen, der Hemmung der symaptosomalen Noradrenalin- und Serotonin-Wiederaufnahme, an der analgetischen Wirksamkeit von Tramadol nachgewiesen werden (M.C. Frink et al., Arzneim.-Forsch/Drug Res. 46 (1996) 1029-1036: K. Flick et al., Arzneim.-Forsch/Drug Res. 28 (1978) 107-113). Trotzdem ist nach heutigem Verständnis die Wirkung von M1 auf den µ-Subtyp des Opiatrezeptors maßgeblich für die analgetische Wirksamkeit verantwortlich. Die Wechselwirkung von M1 mit diesem Rezeptor ist dabei der von Morphin vergleichbar, d.h. neben dem basischen Stickstoffatom ist vor allem die phenolische OH-Gruppe, die im Codein bzw. Tramadol methyliert ist, für die Bindung an und die Aktivierung des µ-Opiatrezeptors entscheidend, die schließlich zur analgetischen Wirkung führt.

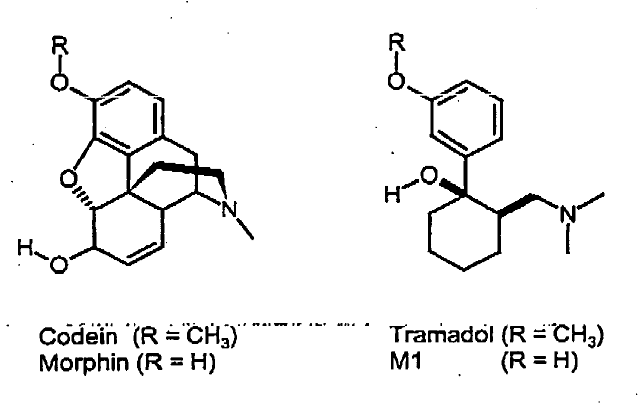

Mitte der neunziger Jahre wurde dann gefunden, daß ein Substituent in 4-Position des Cyclohexanrings von Tramadol die analgetische Wirksamkeit verstärkt. Dies trifft dabei nicht nur für die 3-Phenole, sondern auch für 3-Thiophenole und Prodrugs zu (I. Graudums et al., DE 19547766, Grünenthal GmbH, 1995).

Verbindungen gemäß der allgemeinen Formel I sind dann nicht Teil dieser Erfindung, wenn
A Wasserstoff ist,
R¹ ein einfach in 3-Position O- oder S-substituierter Phenylring ist, und
R² und R³ beide Methyl sind und
X in der Formel I gemäß Teilformel la der Formel I 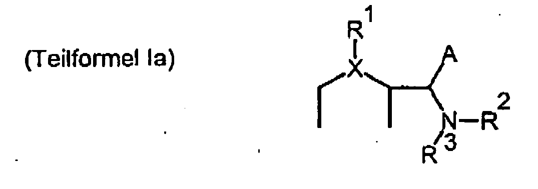 die folgende Bedeutung hat 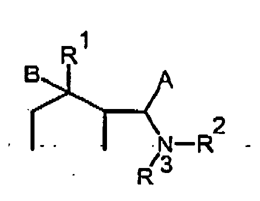 also X 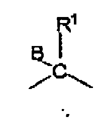 bedeutet, und B für OH steht
und gleichzeitig die Gruppierung 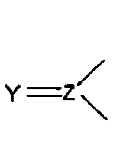 ausgewählt ist aus 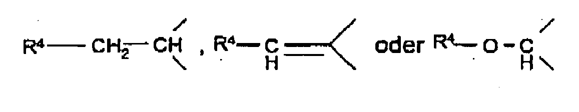 mit R⁴ ausgewählt aus C₁₋₆-Alkyl, C₂₋₆-Alkenyl, C₅₋₇-Cycloalkylmethyl, substituiertem oder unsubstituiertem Phenyl oder substituiertem oder unsubstituiertem Benzyl.

Solche Verbindungen sind bereits aus der DE 195 47 766 A1 (Graudums et al.) bekannt Aber wie an der dort gezeigte Formel I zu sehen ist, gingen die Erfinder auch dort davon aus, daß prinzipiell ein einfach in 3-Position O- oder S-substituierter Phenylring für die analgetische Wirkung, insbesondere die µ-Bindung, notwendig sei. Das ist auch in Übereinstimmung mit der herrschenden Lehre wie z.B. Chen. Z. R.; Irvine, R.J.; Somogyi, A. A., Bochner, F. "Mu receptor binding of some commonly used opioids and their metabolites". Life Sci. 1991, 48, 2165-2171 zu entnehmen.

Überraschenderweise wurde in dieser Erfindung nun gefunden, daß dies keine notwendige Voraussetzung für µ-Bindung und insbesondere Analgesie ist

Entsprechend sind ein Gegenstand dieser Erfindung erfindungsgemäße substituierte C-Cyclohexylmethylamin-Derivate, in denen R¹ kein einfach in 3-Position O- oder S-substituierter Phenylring ist.

Ebenfalls entsprechend bevorzugt sind erfindungsgemäße substituierte C-Cyclohexylmethylamin-Derivate, in denen
R¹ ausgewählt ist aus C₁₋₁₀-Alkyl oder C₃₋₁₀-Cycloalkyl, vorzugsweise C₁₋₆-Alkyl oder C₃₋₆-Cycloalkyl, jeweils gesättigt oder ungesättigt, verzweigt oder unverzweigt, einfach oder mehrfach substituiert oder unsubstituiert; Naphtyl, Furyl, Thiophenyl, über C₁₋₃-Alkylen oder Ethinyl gebundenem Phenyl, Naphthyl, über C₁₋₃-Alkylen oder Ethinyl gebundenem C₃₋₁₀-Cycloalkyl, vorzugsweise C₃₋₈-Cycloalkyl, oder über C₁₋₃-Alkylen oder Ethinyl gebundenem Furyl, Thiophenyl, jeweils unsubstituiert oder einfach oder mehrfach substituiert mit Resten unabhängig voneinander ausgewählt aus
F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, C₁₋₁₀-Cycloalkyl, vorzugsweise C₁₋₆-Alkyl oder C₃₋₆-Cycloalkyl , jeweils unsubstituiert oder einfach oder mehrfach, gleich oder verschieden, substituiert mit F, Cl, Br, NH₂, SH oder OH, oder Silyl, oder R¹ einer Verbindung gemäß der allgemeinen Formel II entspricht 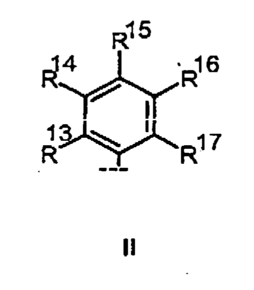 , worin
R¹³, R¹⁵ und R¹⁷ unabhängig voneinander ausgewählt sind aus H, F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, C₃₋₁₀-Cycloalkyl, vorzugsweise C₁₋₆-Alkyl oder C₃₋₆-Cycloalky, , jeweils unsubstituiert oder einfach oder mehrfach, gleich oder verschieden, substituiert mit F, Cl, Br, NH₂, SH oder OH, oder Silyl, gesättigt oder ungesättigt, verzweigt oder unverzweigt,
R¹⁴ und R¹⁶ unabhängig voneinander ausgewählt sind aus H, F, Cl, Br, I, SO₂R¹⁸, SO₂OR¹⁸, CN, COOR¹⁸, NR¹⁹R²⁰; C₁₋₁₀-Alkyl, C₃₋₁₀-Cycloalkyl; vorzugsweise C₁₋₆-Alkyl oder C₃₋₆-Cycloalkyl , jeweils unsubstituiert oder einfach oder mehrfach, gleich oder verschieden, substituiert mit F, Cl, Br, NH₂, SH oder OH, oder Silyl, gesättigt oder ungesättigt, verzweigt oder unverzweigt, oder die Reste R¹³ und R¹⁴ oder R¹⁴ und R¹⁵ zusammen jeweils die Gruppe OCH₂O, OCH₂CH₂O, CH=CHO, CH=C(CH₃)O oder CH=CHNH bilden und R¹⁵-R¹⁷ oder R¹³, R¹⁶ und R¹⁷ die oben genannte Bedeutung haben.
mit R¹⁸ ausgewählt aus H; C₁₋₁₀-Alkyl oder C₃₋₁₀-Cycloalkyl, vorzugsweise C₁₋₆-Alkyl oder C₃₋₆-Cycloalkyl, jeweils gesättigt oder ungesättigt, verzweigt oder unverzweigt,
R¹⁹ und R²⁰ unabhängig voneinander ausgewählt aus H; C₁₋₁₀-Alkyl oder C₃₋₁₀-Cycloalkyl, vorzugsweise C₁₋₆-Alkyl oder C₃₋₆-Cycloalkyl, jeweils gesättigt oder ungesättigt, verzweigt oder unverzweigt, oder R¹⁹ und R²⁰ zusammen CH₂CH₂OCH₂CH₂, CH₂CH₂NR²¹CH₂CH₂ oder (CH₂)₃₋₆ bilden,
mit R²¹ ausgewählt aus H, C₁₋₁₀-Alkyl, vorzugsweise C₁₋₆-Alkyl, gesättigt oder ungesättigt, verzweigt oder unverzweigt, , jeweils unsubstituiert oder einfach oder mehrfach, gleich oder verschieden, substituiert mit F, Cl, Br, NH₂, SH oder OH.

In der DE 195 47 766 A1 (Graudums et al.) ist ebenfalls davon ausgegangen worden, daß für eine analgetische Wirkung das Cyclohexanol notwendig sei. Überraschenderweise ist dies aber nicht notwendig, wie die analgetischen Daten einer Reihe von erfindungsgemäßen Verbindungen zeigen. Ebenfalls bevorzugt sind daher erfindungsgemäße substituierte C-Cyclohexylmethylamin-Derivate, in denen

X in der Formel I gemäß Teilformel la der Formel I 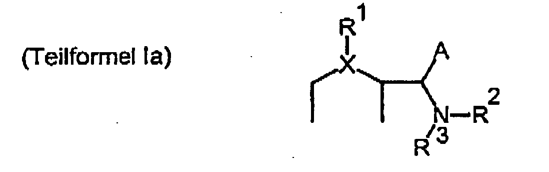 folgende Bedeutung hat 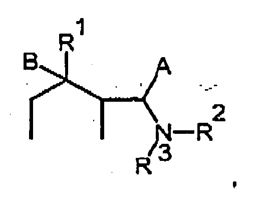 mit B ausgewählt aus OR⁷, H, F, Cl oder NR⁸R⁹,
mit R⁷ ausgewählt aus C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH;
R⁸ und R⁹, unabhängig voneinander ausgewählt sind aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH;
oder die Reste R⁸ und R⁹ zusammen CH₂CH₂OCH₂CH₂, CH₂CH₂NR¹⁰CH₂CH₂ oder (CH₂)₃₋₆ bilden
mit R¹⁰ ausgewählt aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH;

Überraschenderweise zeigen erfindungsgemäße Substanzen, in denen A gemäß der allgemeinen Formel Phenyl ist eine höhere Affinität zum α-Rezeptor als die beispielsweise aus der DE 195 47 766 A1 bekannten Verbindungen, auch wenn R¹ ein einfach in 3-Position O- oder S-substituierter Phenylring ist. Damit eröffnen die erfindungsgemäßen Verbindungen die Möglichkeit, die mit der Bindung an den µ-Rezeptor einhergehenden Nebenwirkungen zu mindern oder ganz zu vermeiden.

Ein entscheidendes Kriterium bleibt aber auch hier natürlich die analgetische Wirkung, insbesondere in vivo.

Ein weiterer bevorzugter Gegenstand sind erfindungsgemäße substituierte C-Cyclohexylmethylamin-Derivate, in denen A Wasserstoff ist. Auch dies sind neue Analgetika.

Andere Verbindungen gemäß der allgemeinen Formel I sind dann nicht Teil dieser Erfindung, wenn
R², R³ unabhängig voneinander ausgewählt sind aus
H, C₁-C₁₀-Alkyl, verzweigt oder unverzweigt, einfach oder mehrfach substituiert oder unsubstituiert, gesättigt oder ungesättigt: C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; jeweils einfach oder mehrfach substituiert oder unsubstituiert;
oder
R² und R³ zusammen einen Ring bilden und CH₂CH₂OCH₂CH₂, CH₂CH₂NR⁶CH₂CH₂ oder (CH₂)₃₋₆ bedeuten;
X in der Formel I gemäß Teilformel la der Formel I 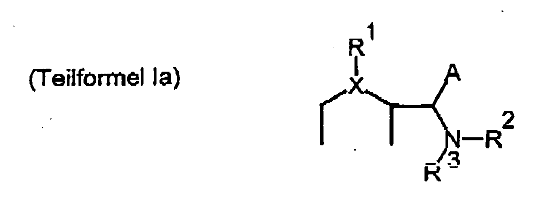 die folgende Bedeutung hat 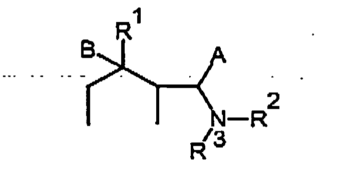 , also X 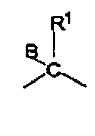 bedeutet,
A Wasserstoff ist,
R1 ein unsubstituierter oder ein- oder zweifach substituierter Phenylring ist,
B ausgewählt ist aus
H, F, Cl, OH oder OR⁷, wobei R⁷ ausgewählt ist aus C₁₋₁₀-Alkyl, verzweigt oder unverzweigt, einfach oder mehrfach substituiert oder unsubstituiert; gesättigt oder ungesättigt; C₃-C₇-Cycloalkyl, gesättigt oder ungesättigt, einfach oder mehrfach substituiert oder unsubstituiert, bzw. einem entsprechenden Heterocyclus, bei dem ein C-Atom im Ring durch N, S oder O ersetzt ist; jeweils einfach oder mehrfach substituiert oder unsubstituiert; und gleichzeitig die Gruppierung 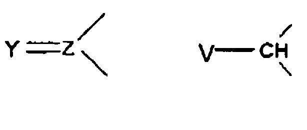 bedeutet mit V ausgewählt aus OR⁴ mit R⁴ ausgewählt aus COR⁵ und mit R⁵ ausgewählt aus C₁₋₆-Alkyl, unsubstituiert oder einfach oder mehrfach substituiert.

Verbindungen dieser Art sind in der WO01/57232 A1 beschrieben und finden sich z.T. auch in Gais H.J., Griebel C. und Buschmann H., Tetrahedron: Asymmetry 11, 917-928 (2000).

Es ist ebenso bevorzugt, wenn für erfindungsgemäße substituierte C-Cyclohexylmethylamin-Derivate gilt, daß
R¹ einer Verbindung gemäß der allgemeinen Formel II entspricht 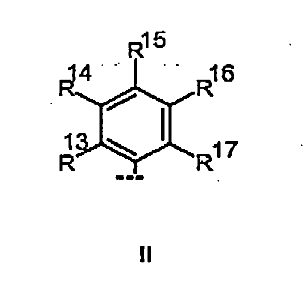 worin
R¹³, R¹⁵ und R¹⁷ unabhängig voneinander ausgewählt sind aus H, F, CI, Br, I, OR¹⁸, SR¹⁸, SO₂R¹⁸, SO₂OR¹⁸, CN, COOR¹⁸, NR¹⁹R²⁰; C₁₋₁₀-Alkyl. C₃₋₁₀-Cycloalkyl, vorzugsweise C₁₋₆-Alkyl oder C₃₋₆-Cycloalkylunsubstituiert oder einfach oder mehrfach, gleich oder verschieden, substituiert mit F, Cl, Br, I, NH₂, SH oder OH; oder Silyl, gesättigt oder ungesättigt, verzweigt oder unverzweigt,
R¹⁴ und E¹⁶ unabhängig voneinander ausgewählt sind aus H, F, Cl, Br, I, SO₂R¹⁸, SO₂OR¹⁸. CN, COOR¹⁸, NR¹⁹R²⁰; C₁₋₁₀-Alkyl, C₃₋₁₀-Cycloalkyl, vorzugsweise C₁₋₆-Alkyl oder C₃₋₆-Cycloalkyl jeweils unsubstituiert oder einfach oder mehrfach, gleich oder verschieden, substituiert mit F, Cl, Br, NH₂, SH oder OH, oder Silyl, gesättigt oder ungesättigt, verzweigt oder unverzweigt,
oder die Reste R¹³ und R¹⁴ oder R¹⁴ und R¹⁵ zusammen jeweils die Gruppe OCH₂O, OCH₂CH₂O, CH=CHO, CH=C(CH₃)O oder CH=CHNH bilden und R¹⁵-R¹⁷ oder R¹³, R¹⁶ und R¹⁷ die oben genannte Bedeutung haben,
mit R¹⁸ ausgewählt aus H; C₁₋₁₀-Alkyl oder C₃₋₁₀-Cycloalkyl, vorzugsweise C₁₋₆-Alkyl oder C₃₋₆-Cycloalkyl, jeweils gesättigt oder ungesättigt, verzweigt oder unverzweigt,
R¹⁹ und R²⁰ unabhängig voneinander ausgewählt aus H; C₁₋₁₀-Alkyl oder C₃₋₁₀-Cycloalkyl, vorzugsweise C₁₋₆-Alkyl oder C₃₋₆-Cycloalkyl, jeweils gesättigt oder ungesättigt, verzweigt oder unverzweigt,
oder R¹⁹ und R²⁰ zusammen CH₂CH₂OCH₂CH₂, CH₂CH₂NR²¹ CH₂CH₂ oder (CH₂)₃₋₆ bilden,
mit R²¹ ausgewählt aus H, C₁₋₁₀-Alkyl, vorzugsweise C₁₋₆-Alkyl, gesättigt oder ungesättigt, verzweigt oder unverzweigt, jeweils unsubstituiert oder einfach oder mehrfach, gleich oder verschieden, substituiert mit F, Cl, Br, NH₂, SH oder OH,
und 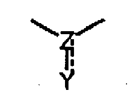 ausgewählt ist aus 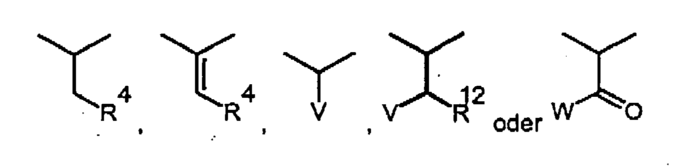 mit R⁴ ausgewählt aus H, COR^{5a}, SO₂R⁵; C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Phenyl, Naphthyl, Furyl oder Thiophenyl jeweils einfach oder mehrfach substituiert oder unsubstituiert; oder über C₁₋₃-Alkylen gebundenem Naphthyl, Phenyl, C₃₋₁₀-Cycloalkyl, Furyl oder Thiophenyl, jeweils einfach oder mehrfach substituiert oder unsubstituiert,
mit R⁵ ausgewählt aus C₁₋₁₀-Alkyl oder C₃₋₁₀-Cycloalkyl, jeweils gesättigt oder ungesättigt, verzweigt oder unverzweigt, einfach oder mehrfach substituiert oder unsubstituiert; Phenyl, Naphthyl Furyl oder Thiophenyl, jeweils einfach oder mehrfach substituiert oder unsubstituiert; oder über C₁₋₃-Alkylen gebundenem Phenyl, Naphthyl, C₃₋₁₀-Cycloalkyl, Furyl oder Thiophenyl, jeweils einfach oder mehrfach substituiert oder unsubstituiert,
mit R^{5a} ausgewählt aus C₃₋₁₀-Cycloalkyl, gesättigt oder ungesättigt, verzweigt oder unverzweigt einfach oder mehrfach substituiert oder unsubstituiert; Naphthyl, Phenyl, Furyl oder Thiophenyl jeweils einfach oder mehrfach substituiert oder unsubstituiert; oder über C₁₋₃-Alkylen gebundenem Phenyl, Naphthyl, C₃₋₁₀-Cycloalkyl Furyl oder Thiophenyl, jeweils einfach oder mehrfach substituiert oder unsubstituiert,
mit V ausgewählt aus OR⁴ oder NR⁴R¹¹,
mit W ausgewählt aus R¹¹, OR¹² oder NR¹¹R¹²,
mit R¹¹ und R¹² unabhängig voneinander ausgewählt aus H, C₇₋₁₀-Alkyl oder C₃₋₁₀-Cycloalkyl, jeweils gesättigt oder ungesättigt verzweigt oder unverzweigt, einfach oder mehrfach substituiert oder unsubstituiert; Phenyl, Naphthyl, Furyl oder Thiophenyl, jeweils einfach oder mehrfach substituiert oder unsubstituiert; oder über C₁₋₃-Alkylen gebundenem Naphtyl, Phenyl Aryl, C₃₋₁₀-Cycloalkyl Furyl oder Thiophenyl, jeweils einfach oder mehrfach substituiert oder unsubstituiert.

Besonders bevorzugt sind weiter erfindungsgemäße substituierte C-Cyclohexylmethylamin-Derivate, in denen A Wasserstoff ist.

Weiter bevorzugt sind erfindungsgemäße substituierte C-Cyclohexylmethylamin-Derivate, in denen
R² und R³ ausgewählt sind aus C₁₋₆-Alkyl, gesättigt oder ungesättigt, verzweigt oder unverzweigt, unsubstituiert oder ein oder mehrfach substituiert, vorzugsweise CH₃, insbesondere beide CH₃,
oder die Reste R² und R³ zusammen CH₂CH₂NR⁶CH₂CH₂ oder (CH₂)₃₋₆ bedeuten, mit R⁶ ausgewählt aus H oder C₁₋₆-Alkyl, gesättigt, verzweigt oder unverzweigt und unsubstituiert; insbesondere H oder CH₃.

Eine bevorzugte Ausführungsform der Erfindung sind erfindungsgemäße substituierte C-Cyclohexylmethylamin-Derivate, in denen
R¹ ausgewählt ist aus; C₁₋₆-Alkyl, gesättigt oder ungesättigt, verzweigt oder unverzweigt; C₃₋₆-Cycloalkyl, Naphthyl, Phenyl, Furyl, Thiophenyl, über C₁₋₃-Alkylen oder Ethinyl gebundenem Naphthyl, Phenyl, C₃₋₆-Cycloalkyl oder über C₁₋₃-Alkylen oder Ethinyl gebundenem Thiophenyl oder Furyl, die gemäß Anspruch 1 unsubstituiert oder ein- oder mehrfach unsubstituiert sein können.

Besonders bevorzugt sind weiter erfindungsgemäße substituierte C-Cyclohexylmethylamin-Derivate, in denen 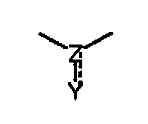 ausgewählt ist aus

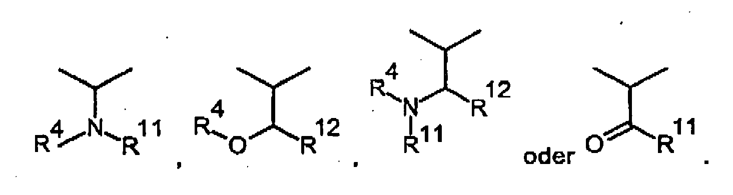

Ein ganz besonders bevorzugter Gegenstand der Erfindung sind substituierte C-Cyclohexylmethylamin-Derivate der allgemeinen Formel I. 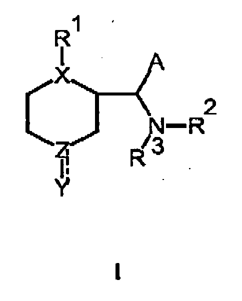 , worin
A H; oder Phenyl ist ;
R¹ ausgewählt ist aus Naphthyl oder über C₁₋₃-Alkylen oder Ethinyl gebundenem Naphthyl, jeweils unsubstituiert oder einfach oder mehrfach substituiert mit Resten unabhängig voneinander ausgewählt aus
F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl oder C₃₋₁₀-Cycloalkyl , jeweils unsubstituiert oder einfach oder mehrfach, gleich oder verschieden, substituiert mit F, Cl, Br, NH₂, SH oder OH, oder Silyl,
mit R¹⁸ ausgewählt aus H; C₁₋₁₀-Alkyl oder C₃₋₁₀-Cycloalkyl, jeweils gesättigt oder ungesättigt, verzweigt oder unverzweigt,
R¹⁹ und R²⁰ unabhängig voneinander ausgewählt aus H; C₁₋₁₀-Alkyl oder C₃₋₁₀-Cycloalkyl, jeweils gesättigt oder ungesättigt, verzweigt oder unverzweigt, oder R¹⁹ und R²⁰ zusammen CH₂CH₂OCH₂CH₂, CH₂CH₂NR²¹ CH₂CH₂ oder (CH₂)₃₋₆ bilden,
mit R²¹ ausgewählt aus H, C₁₋₁₀-Alkyl gesättigt oder ungesättigt, verzweigt oder unverzweigt;
R² und R³, unabhängig voneinander ausgewählt sind aus H; C₁₋₁₀-Alkyl oder C₃₋₁₀-Cycloalkyl, jeweils gesättigt oder ungesättigt, verzweigt oder unverzweigt,
oder die Reste R² und R³ zusammen CH₂CH₂OCH₂CH₂, CH₂CH₂NR⁶CH₂CH₂ oder (CH₂)₃₋₆ bedeuten,
mit R⁶ ausgewählt aus H; C₁₋₁₀-Alkyl oder C₃₋₁₀-Cycloalkyl, jeweils gesättigt oder ungesättigt, verzweigt oder unverzweigt,
X in der Formel I gemäß Teilformel la der Formel I 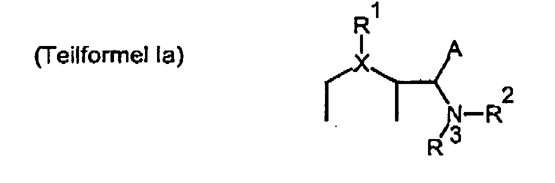 folgende Bedeutung hat 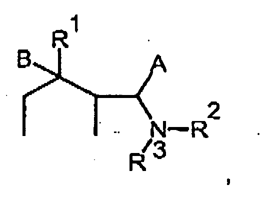 mit B ausgewählt aus OH, OR⁷, H, F, Cl oder NR⁸R⁹,
mit R⁷ ausgewählt aus C₁₋₁₀-Alkyl oder C₃₋₁₀-Cycloalkyl, jeweils gesättigt oder ungesättigt, verzweigt oder unverzweigt, jeweils unsubstituiert oder einfach oder mehrfach, gleich oder verschieden, substituiert mit F, Cl, Br, NH₂, SH oder OH,
R⁸ und R⁹, unabhängig voneinander ausgewählt sind aus H; C₁₋₁₀-Alkyl oder C₃₋₁₀-Cycloalkyl, jeweils gesättigt oder ungesättigt, verzweigt oder unverzweigt, oder die Reste R⁸ und R⁹ zusammen CH₂CH₂OCH₂CH₂, CH₂CH₂NR¹⁰CH₂CH₂ oder (CH₂)₃₋₆ bilden
mit R¹⁰ ausgewählt aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH; 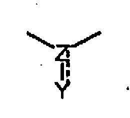 ausgewählt ist aus 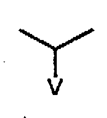 mit V ausgewählt aus OR⁴,
mit R⁴ ausgewählt aus COR⁵; C₁₋₁₀-Alkyl, gesättigt oder ungesättigt, verzweigt oder unverzweigt, einfach oder mehrfach substituiert oder unsubstituiert; C₃₋₁₀-Cycloalkyl, gesättigt oder ungesättigt, einfach oder mehrfach substituiert oder unsubstituiert; Phenyl, Naphthyl, Furyl, Thiophenyl, jeweils einfach oder mehrfach substituiert oder unsubstituiert; oder über C₁₋₃-Alkyl gebundenem Phenyl, Naphthyl, C₃₋₁₀-Cycloalkyl, Furyl oder Thiophenyl, jeweils einfach oder mehrfach substituiert oder unsubstituiert,
mit R⁵ ausgewählt aus C₁₋₁₀-Alkyl, gesättigt oder ungesättigt, verzweigt oder unverzweigt, einfach oder mehrfach substituiert oder unsubstituiert; C₃₋₁₀-Cycloalkyl, gesättigt oder ungesättigt, einfach oder mehrfach substituiert oder unsubstituiert; Naphthyl, Phenyl, Furyl oder Thiophenyl, jeweils einfach oder mehrfach substituiert oder unsubstituiert; oder über, C₁₋₃-Alkylen gebundenem Phenyl, Naphthyl C₃₋₁₀-Cycloalkyl, Furyl oder Thiophenyl, jeweils einfach oder mehrfach substituiert oder unsubstituiert,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.
Bezüglich dieser ganz besonders bevorzugten substituierten C-Cyclohexylmethylamin-Derivate gilt, daß in der bevorzugten Ausführungsform
A ausgewählt ist aus H oder Phenyl, jeweils einfach oder mehrfach substituiert oder unsubstituiert; vorzugsweise H
**und/oder**
R¹ ausgewählt ist aus Naphthyl, unsubstituiert oder einfach oder mehrfach substituiert mit Resten unabhängig voneinander ausgewählt aus
F, Cl, Br, I, OR¹⁸; C₁₋₄-Alkyl, verzweigt oder unverzweigt, unsubstitulert oder einfach oder mehrfach substituiert;
mit R¹⁸ ausgewählt aus H; C₁₋₄-Alkyl, verzweigt oder unverzweigt,
vorzugsweise R¹ ausgewählt ist aus Naphthyl, unsubstituiert oder substituiert mit OH oder OCH3,
**und/oder**
R2 und R3 ausgewählt sind aus C1-6-Alkyl, gesättigt oder ungesättigt, verzweigt oder unverzweigt, vorzugsweise CH3, insbesondere beide CH3,
oder die Reste R2 und R3 zusammen CH2CH2NR6CH2CH2 oder (CH2)4-5 bedeuten, insbesondere zusammen CH2CH2NR6CH2CH2 bedeuten, mit R⁶ ausgewählt aus H oder C₁₋₆-Alkyl, gesättigt, verzweigt oder unverzweigt und unsubstituiert; insbesondere H oder CH₃;
**und/oder**
X in der Formel I gemäß Teilformel la der Formel I 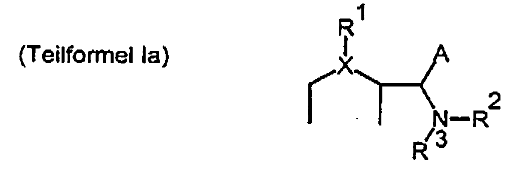 ausgewählt ist aus den folgenden Bedeutungen 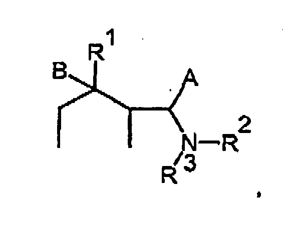 also X 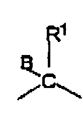 bedeutet, mit B ausgewählt aus OH, OR⁷, H, F, Cl, mit R⁷ ausgewählt aus C₁₋₄-Alkyl, verzweigt oder unverzweigt, unsubstituiert oder einfach oder mehrfach, gleich oder verschieden, substituiert mit F, Cl, Br, I, NH₂, SH oder OH; vorzugsweise mit B ausgewählt aus OH,
und/oder 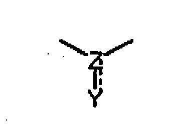 ausgewählt ist aus 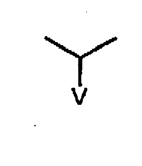 mit V ausgewählt aus OR⁴,
mit R⁴ ausgewählt aus COR⁵; Phenyl oder Benzoyl, einfach oder mehrfach substituiert oder unsubstituiert; , insbesondere COR⁵ oder Benzyl, einfach oder mehrfach substituiert oder unsubstituiert;,
mit R⁵ ausgewählt aus C₁₋₁₀-Alkyl, insbesondere C₁₋₆-Alkyl, gesättigt oder ungesättigt, verzweigt oder unverzweigt, einfach oder mehrfach substituiert oder unsubstituiert; oder Phenyl, einfach oder mehrfach substituiert oder unsubstituiert.

Weiter besonders bevorzugte erfindungsgemäße Verbindungen sind ausgewählt aus der folgenden Gruppe:
• 4-Benzyloxy-1-(4-brom-phenyl)-2-dimethylaminomethyl-cyclohexanol;
• 4-Benzyloxy-2-dimethylaminomethyl-1-(4-trifluormethyl-phenyl)-cyclohexanol;
• 4-Benzyloxy-1-(3.4-difluor-phenyl)-2-dimethylaminomethyl-cyclohexanol;
• 4-Benzyloxy-2-dimethylaminomethyl-1-(3-fluor-4-methyl-phenyl)-cyclohexanol;
• 4-Benzyloxy-1-(3,4-dichlor-phenyl)-2-dimethylaminomethyl-cyclohexanol;
• 4-Benzyloxy-2-dimethylaminomethyl-1-naphthalin-2-yl-cyclohexanol:
• 4-Benzyloxy-2-dimethylaminomethyl-1-(3,4-dimethyl-phenyl)-cyclohexanol;
• 4-Benzyloxy-2-dimethylaminomethyl-1-(4-fluor-3-methyl-phenyl)-cyclohexanol;
• 4-Benzyloxy-2-dimethylaminomethyl-1-(5-fluor-2-methyl-phenyl)-cyclohexanol;
• 4-Benzyloxy-1-(3-chlor-4-fluor-phenyl)-2-dimethylaminomethyl-cyclohexanol;
• 4-Benzyloxy-1-(5-chlor-2-methoxy-phenyl)-2-dimethylaminomethyl-cyclohexanol;
• 4-Benzyloxy-1-(2-brom-phenyl)-2-dimethylaminomethyl-cyclohexanol;
• 4-Benzyloxy-2-dimethylaminometyl-1-(2-methoxy-phenyl)-cyclohexanol;
• 4-Benzyloxy-2-dimethylaminomethyl-1-(2-methylsulfanyl-phenyl)-cyclohexanol;
• 4-Benzyloxy-1-(4-chlor-phenyl)-2-dimethylaminomethyl-yclohexanol;
• 4-Benzyloxy-1-(3-brom-phenyly)-2-dimethylaminomethyl-cyclohexanol;
• 4-Benzyloxy-1-(4-brom-3-fluorphenyl)-2-dimethylaminomethyl-cyclohexanol;
• 4-Benzyloxy-2-dimethylaminomethyl-1-(4-methoxy-phenyl)-cyclohexanol;
• 4-Benzyloxy-1-(2-chlor-phenyl)-2-dimethylaminomethyl-cyclohexanol;
• 4-Benzyloxy-2-dimethylaminomethyl-1-(3-trifluormethyl-phenyl)-cyclohexanol;
• 4-Benzyloxy-2-dimethylaminomethyl-1-(4-pentyl-phenyl)-cyclohexanol;
• 4-Benzyloxy-2-dimethylaminomethyl-1-(2,5-dimethyl-phenyl)-cyclohexanol;
• 4-Benzyloxy-2-dimethylaminomethyl-1-(4-isopropyl-phenyl)-cyclohexanol;
• 4-Benzyloxy-2-dimethylaminomethyl-1-(2,3-dimethyl-phenyl)-cyclohexanol;
• 4-Benzyloxy-2-dimethylaminomethyl-1-(3-fluor-phenyl)-cyclohexanol;
• 4-Benzyloxy-1-(3,5-bis-trifluormethyl-phenyl)-2-dimethylaminomethyl-cyclohexanol;
• 4-Benzyloxy-2-dimethylaminomethyl-1-(4-ethyl-phenyl)-cyclohexanol;
• 4-Benzyloxy-1-(3,5-dichlor-phenyl)-2-dimethylaminomethyl-cyclohexanol;
• 2-Dimethylaminomethyl-4-(4-fluor-benzyloxy)-1-o-tolyl-cyclohexanol;
• 2-Dimethylaminomethyl-4-(4-fluo-benzyloxy)-1-p-tolyl-cyclohexanol;
• 2-Dimethylaminomethyl-1-(2,5-dimethyl-phenyl)-4-(4-fluorbenzyloxy)-cyclohexanol;
• 2-Dimethylaminomethyl-1-(3,4-dimethyl-phenyl)-4-(4-fluor-benzyloxy)-cyclohexanol;
• 2-Dimethylaminomethyl-1-(3,5-dimethyl-phenyl)-4-(4-fluor-benzyloxy)-cyclohexanol;
• 2-Dimethylaminomethyl-4-(4-fluor-benzyloxy)-1-(4-isopropyl-phenyl)-cyclohexanol;
• 2-Dimethylaminomethyl-4-(4-flüor-benzyloxy)-1-(3-fluor-phenyl)-cyclohexanol;
• 2-Dimethylaminomethyl-4-(4-fluor-benzyloxy)-1-(4-fluor-phenyl)-cyclohexanol;
• 1-(3,4-Difluor-phenyl)-2-dimethylaminomethyl-4-(4-fluor-benzyloxy)-cyclohexanol;
• 2-Dimethylaminomethyl-4-(4-fluor-benzyloxy)-1-(3-fluor-4-methyl-phenyl)-cyclohexanol;
• 2-Dimethylaminomethyl-4-(4-fluorbenzyloxy)-1-(4-fluor3-methyl-phenyl)-cyclohexanol;
• 1-(2-Chlor-phenyl)-2-dimethylaminomethyl-4-(4-fluor-benzyloxy)-cyclohexanol;
• 1-(3-Chlor-phenyl)-2-dimethylaminomethyl-4-(4-fluor-benzyloxy)-cyclohexanol;
• 1-(4-Chlor-phenyl)-2-dimethylaminomethyl-4-(4-fluor-benzyloxy)-cyclohexanol;
• 1-(3,4-Dichlor-phenyl)-2-dimethylaminomethyl-4-(4-fluor-benzyloxy)-cyclohexanol;
• 1-(4-Chlor-3-fluor-phenyl)-2-dimethylaminomethyl-4-(4-fluor-benzyloxy)-cyclohexanol;
• 1-(3,5-Dichlor-phenyl)-2-dimethylaminomethyl-4-(4-fluor-benzyloxy)-cyclohexanol;
• 1-(4-Chlor2-methoxy-phenyl)-2-dimethylaminomethyl-4-(4-fluor-benzyloxy)-cyclohexanol;
• 2-Dimethylaminomethyl-4-(4-fluor-benzyloxy)-1-(4-trifluormethyl-phenyl)-cyclohexanol;
• 1-(3,5-Bis-trifluormethyl-phenyl)-2-dimethylaminomethyl-4-(4-fluor-benzyloxy)-cyclohexanol;
• 2-Dimethylaminomethyl-4-(4-fluor-benzyloxy)-1-(2-methoxy-phenyl)-cyclohexanol;
• 2-Dimethylaminomethyl-4-(4-fluor-benzyloxy)-1-(2-methylsulfanyl-phenyl)-cyclohexanol;
• 3,4-Dichlor-N-[3-dimethylaminomethyl-4-hydroxy-4-(3-methoxy-phenyl)-cyclohexyl]-benzamid;
• Naphthalin-2-carbonsäure [3-dimethylaminomethyl-4-hydroxy-4-(3-methoxyphenyl)-cycloheyl]-amid;
• N-[3-Dimethylaminomethyl-4-hydroxy-4-(3-methoxy-phenyl)-cyclohexyl]-3-phenyl-propionamid;
• N-[3-Dimethylaminomethyl-4-hydroxy-4-(3-methoxy-phenyl)-cyclohexyl]-4-nitro-benzamid;
• N-[3-Dimethylaminomethyl-4-hydroxy-4-(3-methoxy-phenyl)-cyclohexyl]-4-methyl-3-nitro-benzamid;
• N-[3-Dimethylaminomethyl-4-hydroxy-4-(3-methoxy-phenyl)-cyclohexyl]-3,4,5-trimethoxy-benzamid;
• 2-(4-Chlor-phenoxy)-N-[3-dimethylaminomethyl-4-hydroxy-4-(3-methoxyphenyl)-cyclohexyl]-acetamid;
• N-[3-Dimethylaminomethyl-4-hydroxy-4-(3-methoxy-phenyl)-cyclohexyl]-3-nitro-benzamid;
• Furan-2-carbonsäure [3-dimethylaminomethyl-4-hydroxy-4-(3-methoxyphenyl)-cyclohexyl]-amid;
• N-[3-Dimethylaminomethyl-4-hydroxy-4-(3-methoxy-phenyl)-cyclohexyl]-2-phenoxy-acetamid;
• N-[3-Dimethylaminomethyl-4-hydroxy-4-(3-methoxy-phenyl)-cyclohexyl]-4-trifluormethyl-benzamid;
• N-[3-Dimethylaminomethyl-4-hydroxy-4-(3-methoxy-phenyl)-cyclohexyl]-4-methoxy-benzamid;
• 2-Dimethylaminomethyl-1-(4-fluor-phenyl)-4-phenethyl-cyclohexanol-;
• 1-(4-Brom-phenyl)-2-dimethylaminomethyl-4-phenethyl-cyclohexanol;
• 2-Dimethylaminomethyl-1-(4-ethyl-phenyl)-4-phenethyl-cyclohexanol;
• 2-Dimethylaminomethyl-1-(4-isopropyl-phenyl)-4-phenethyl-cyclohexanol;
• 2-Dimethylaminomethyl-1-(4-methoxy-phenyl)-4-phenethyl-cyclohexanol;
• 2-Dimethylaminomethyl-1-(2,4-dimethyl-phenyl)-4-phenethyl-cyclohexanol;
• 2-Dimethylaminomethyl-1-(2-methylsulfanyl-phenyl)-4-phenethyl-cyclohexanol;
• 2-Dimethylaminomethyl-1-(5-fluor-2-methyl-phenyl)-4-phenethyl-cyclohexanol;
• 2-Dimethylaminomethyl-1-(2,5-dimethyl-phenyl)-4-phenethyl-cyclohexanol;
• 2-Dimethylaminomethyl-4-phenethyl-1-p-tolyl-cyclohexanol:
• 2-Dimethylaminomethyl-1-(3-fluor-phenyl)-4-phenethyl-cyclohexanol;
• 1-(3-Chlor-phenyl)-2-dimethylaminomethyl-4-phenethyl-cyclohexanol;
• 1-(4-Chlor-3-fluor-phenyl)-2-dimethylaminomethyl-4-phenethyl-cyclohexanol:
• 2-Dimethylaminomethyl-4-phenethyl-1-(3-trifluormethyl-phenyl)-cyclohexanol;
• 1-(3,5-Dichlor-phenyl)-2-dimethylaminomethyl-4-phenethyl-cyclohexanol;
• 2-Dimethylaminomethyl-1-(3,4-dimethyl-phenyl)-4-phenethyl-cyclohexanol;
• 1-(3,4-Dichlor-phenyl)-2-dimethylaminomethyl-4-phenethyl-cyclohexanol;
• 1-(4-Chlor-phenyl)-2-dimethylaminomethyl-4-phenethyl-cyclohexanol;
• 2-Dimethylaminomethyl-1-(3-fluor-4-methyl-phenyl)-4-phenethyl-cyclohexanol;
• 2-Dimethylaminomethyl-1-(3,5-dimethyl-phenyl)-4-phenethyl-cyclohexanol;
• 1-(4-Brom-3-fluor-phenyl)-2-dimethylaminomethyl-4-phenethyl-cyclohexanol;
• 1-(3,4-Difluor-phenyl)-2-dimethylaminomethyl-4-phenethyl-cyclohexanol;
• 2-Dimethylaminomethyl-1-(2,3-dimethyl-phenyl)-4-phenethyl-cyclohexanol;
• 2-Dimethylaminomethyl-1-naphthalin-1-yl-4-phenethyl-cyclohexanol;
• 1-(3,5-Bis-trifluormethyl-phenyl)-2-dimethylaminomethyl-4-phenethyl-cyclohexanol;
• 1-(3-Brom-phenyl)-2-dimethylaminomethyl-4-phenethyl-cyclohexanol;
• 2-Dimethylaminomethyl-1-(4-fluor-3-methyl-phenyl)-4-phenethyl-cyclohexanol;
• 2-Dimethylaminomethyl-4-phenethyl-1-m-tolyl-cyclohexanol;
• 4-Benzyl-2-dimethylaminomethyl-1-m-tolyl-cyclohexanol;
• 4-Benzyl-1-(3,4-difluor-phenyl)-2-dimethylaminomethyl-cyclohexanol;
• 4-Benzyl-2-dimethylaminomethyl-1-p-tolyl-cyclohexanol;
• 2-Dimethylaminomethyl-4-(3-methoxy-benzyl)-1-p-tolyl-cyclohexanol;
• 1-(4-Chlor-phenyl)-2-dimethylaminomethyl-4-(3-methoxy-benzyl)-cyclohexanol;
• 1-(4-Brom-3-fluor-phenyl)-2-dimethylaminomethyl-4-(3-methoxy-benzyl)-cyclohexanol;
• 2-Dimethylaminomethyl-4-(4-fluor-benzyl)-1-p-tolyl-cyclohexanol;
• 1-(4-Chlor-phenyl)-2-dimethylaminomethyl-4-(4-fluor-benzyl)-cyclohexanol;
• [2-(3-Chlor-4-fluor-phenyl)-2-fluor-5-phenethyl-cyclohexylmethyl]-dimethylamin;
• [2-Fluor-5-(4-fluor-benzyl)-2-p-tolyl-cyclohexylmethyl]-dimethyl-amin;
• Benzyl-2-(dimethylamino-phenyl-methyl)-4-phenyl-cyclohexanol
• 2-(Dimethylamino-phenyl-methyl)-4-phenyl-1-vinyl-cyclohexanol
• 1-(4-tert-Butyl-phenyl)-2-(dimethylamino-phenyl-methyl)-4-phenyl- , , cyclohexanol
• 2-(Dimethylamino-phenyl-methyl)-4-phenyl-1-m-tolyl-cyclohexanol
• 2-(Dimethylamino-phenyl-methyl)-1-phenethyl-4-phenyl-cyclohexanol
• 2-(Dimethylamino-phenyl-methyl)-4-phenyl-1-phenylethynyl-cyclohexanol
• 2-(Dimethylamino-phenyl-methyl)-1-(3-methoxy-phenyl)-4-phenyl-cyclohexanol
• 2-(Dimethylamino-phenyl-methyl)-4-phenyl-1-(3-phenyl-propyl)-cyclohexanol
• 2-(Dimethylamino-phenyl-methyl)-1-(4-methoxy-phenyl)-4-phenyl-cyclohexanol
• 2-(Dimethylamino-phenyl-methyl)-1-(2-methoxy-phenyl)-4-phenyl-cyclohexanol
• 4-Benzyloxy-2-dimethylaminomethyl-1-phenyl-cyclohexanol
• 1-Benzyl-4-benzyloxy-2-dimethylaminomethyl-cyclohexanol
• 4-Benzyloxy-2-dimethylaminomethyl-1-(4-fluor-3-methyl-phenyl)-cyclohexanol
• 4-Benzyloxy-2-dimethylaminomethyl-1-o-tolyl-cyclohexanol
• 4-Benzyloxy-2-dimethylaminomethyl-1-vinyl-cyclohexanol
• 4-Benzyloxy-1-cyclopentyl-2-dimethylaminomethyl-cyclohexanol
• 4-Benzyloxy-2-dimethylaminomethyl-1-m-tolyl-cyclohexanol
• 4-Benzyloxy-2-dimethylaminomethyl-bicyclohexyl-1-ol
• 4-Benzyloxy-2-dimethylaminomethyl-1-(4-fluor-phenyl)-cyclohexanol
• 4-Benzyloxy-2-dimethylaminomethyl-1-phenylethynyl-cyclohexanol
• 4-Benzyloxy-2-dimethylaminomethyl-1-thiophen-2-yl-cyclohexanol
• 4-Benzyloxy-2-dimethylaminomethyl-1-(3-methoxy-phenyl)-cyclohexanol
• 4-Benzyloxy-2-dimethylaminomethyl-1-(3-phenyl-propyl)-cyclohexanol
• 4-Benzyloxy-2-dimethylaminomethyl-1-p-tolyl-cyclohexanol
• 4-Benzyloxy-2-dimethylaminomethyl-1-(4-methoxy-phenyl)-cyclohexanol
• 4-Benzyloxy-2-dimethylaminomethyl-1-(3-fluor-phenyl)-cyclohexanol
• 4-Benzyloxy-1-(3-chlor-phenyl)-2-dimethylaminomethyl-cyclohexanol
• 4-Benzyloxy-2-dimethylaminomethyl-1-(3-methoxy-benzyl)-cyclohexanol
• 4-Benzyloxy-1-(4-chlor-3-trifluormethyl-phenyl)-2-dimethylaminomethyl-cyclohexanol
• 4-Benzyloxy-2-dimethylaminomethyl-1-(3-fluor-benzyl)-cyclohexanol
• 4-Benzyloxy-2-dimethylaminomethyl-1-(2-methyl-benzyl)-cyclohexanol
• 4-Benzyloxy-2-dimethylaminomethyl-1-(2,5-dimethyl-benzyl)-cyclohexanol
• 4-(4-Chlor-benzyl)-2-dimethylaminomethyl-1-(3-methoxy-phenyl)-cyclohexanol
• 4-(4-Chlor-benzyl)-2-dimethylaminomethyl-1-(3-phenyl-propyl)-cyclohexanol
• 4-(4-Chlor-benzyl)-1-(2,3-dichlor-phenyl)-2-dimethylaminomethyl-cyclohexanol
• 4-(4-Chlor-benzyl)-1-cyclohexylmethyl-2-dimethylaminomethyl-cyclohexanol
• 4-(4-Chlor-benzyl)-2-dimethylaminomethyl-1-(5-fluor-2-methoxy-phenyl)-cyclohexanol
• 4-(4-Chlor-benzyl)-2-dimethylaminomethyl-1-(3-fluor-phenyl)-cyclohexanol .
• 4-(4-Chlor-benzyl)-1-(3-chlor-phenyl)-2-dimethylaminomethyl-cyclohexanol
• 4-(4-Chlor-benzyl)-1-(3,5-dichlor-phenyl)-2-dimethylaminomethyl-cyclohexanol
• 4-(4-Chlor-benzyl)-1-(2-chlor-benzyl)-2-dimethylaminomethyl-cyclohexanol
• 4-(4-Chlor-benzyl)-2-dimethylaminomethyl-1-(4-fluor-benzyl)-cyclohexanol
• 4-(4-Chlor-benzyl)-2-dimethylaminomethyl-1-(3-fluor-benzyl)-cyclohexanol
• 4-(4-Chlor-benryl)-2-dimethylaminomethyl-1-(2-methoxy-phenylrcyctohexanot
• 4-(4-Chlor-benzyl)-2-dimethylaminomethyl-1-(2-methyl-benzyl)-cyclohexanol
• 4-(4-Chlor-benzyl)-2-dimethylaminomethyl-1-(3-methyl-benzyl)-cyclohexanol
• 1,4-Bis-(4-chlor-benzyl)-2-dimethylaminomethyl-cyclohexanol
• 4-(4-Chlor-benzyl)-1-(2-chlor-6-fluor-benzyl)-2-dimethylaminomethyl-cyclohexanol
• 4-(4-Chlor-benzyl)-2-dimethylaminomethyl-1-(2,5-dimethyl-benzyl)-cyclohexanol
• 4-(4-Chlor-benzyl)-1-(3-chlor-benzyl)-2-dimethylaminomethyl-cyclohexanol
• 4-(4-Chlor-benzyl)-1-(2,4-dichlor-benzyl)-2-dimethylaminomethyl-cyclohexanol
• 4-(4-Chlor-benzyl)-2-dimethylaminomethyl-1-phenyl-cyclohexanol
• 4-(4-Chlor-benzyl)-1-(4-chlor-phenyl)-2-dimethylaminomethyl-cyclohexanol
• 4-(4-Chlor-benzyl)-2-dimethylaminomethyl-1-(4-fluor-3-methyl-phenyl)-cyclohexanol
• 4-(4-Chlor-benzyl)-2-dimethylaminomethyl-1-o-tolyl-cyclohexanol
• 4-(4-Chlor-benzyl)-2-dimethylaminomethyl-1-vinyl-cyclohexanol
• 4-(4-Chlor-benzyl)-1-cyclopentyl-2-dimethylaminomethyl-cyclohexanol
• 4-(4-Chlor-benzyl)-2-dimethylaminomethyl-1-m-tolyl-cyclohexanol
• 4-(4-Chlor-benzyl)-2-dimethylaminomethyl-bicyclohexyl-1-ol
• 4-(4-Chlor-benzyl)-2-dimethylaminomethyl-1-(4-fluor-phenyl)-cyclohexanol
• 4-(4-Chlor-benzyl)-2-dimethylaminomethyl-1-phenethyl-cyclohexanol
• 4-(4-Chlor-benzyl)-2-dimethylaminomethyl-1-phenylethynyl-cyclohexanol
• 4-(4-Chlor-benzyl)-2-dimethylaminomethyl-1-thiophen-2-yl-cyclohexanol
• 4-(4-Chlor-benzyl)-2-dimethylaminomethyl-1-p-tolyl-cyclohexanol
• 4-(4-Chlor-benzyl)-2-dimethylaminomethyl-1-(4-methoxy-phenyl)-cyclohexanol
• 4-(4-Chlor-benzyl)-2-dimethylaminomethyl-1-trimethylsilanylethynyl-cyclohexanol
• 4-(4-Chlor-benzyl)-1-(4-chlor-3-trifluormethyl-phenyl)-2-dimethylaminomethyl-cyclohexanol
• 4-(4-Chlor-benzyl)-1-(3-chlor-4-fluor-phenyl)-2-dimethylaminomethyl-cyclohexanol
• 4-(4-Chlor-benzyl)-2-dimethylaminomethyl-1-(3-trifluormethyl-phenyl)-cyclohexanol
• 2-Dimethylaminomethyl-4-(4-fluor-benzyl)-1-phenyl-cyclohexanol
• 1-(4-chlor-phenyl)-2-dimethylaminomethyl-4-(4-fluor-benzyl)-cyclohexanol
• 1-Benzyl-2-dimethylaminomethyl-4-(4-fluor-benzyl)-cyclohexanol
• 2-Dimethylaminomethyl-4-(4-fluor-benzyl)-1-(4-fluor-3-methyl-phenyl)-cyclohexanol
• 2-Dimethylaminomethyl-4-(4-fluor-benzyl)-1-o-tolyl-cyclohexanol
• 2-Dimethylaminomethyl-4-(4-fluor-benzyl)-1-vinyl-cyclohexanol
• 1-(4-tert-Butyl-phenyl)-2-dimethylaminomethyl-4-(4-fluor-benzyl)-cyclohexanol
• 1-Cyclopentyl-2-dimethylaminomethyl-4-(4-fluor-benzyl)-cyclohexanol
• 2-Dimethylaminomethyl-4-(4-fluor-benzyl)-1-m-tolyl-cyclohexanol
• 2-Dimethylaminomethyl-4-(4-fluor-benzyl)-bicyclohexyl-1-ol
• 2-Dimethylaminomethyl-4-(4-fluor-benzyl)-1-(4-fluor-phenyl)-cyclohexanol
• 2-Dimethylaminomethyl-4-(4-fluor-benzyl)-1-phenethyl-cyclohexanol
• 2-Dimethylaminomethyl-4-(4-fluor-benzyl)-1-phenylethynyl-cyclohexanol
• 2-Dimethylaminomethyl-4-(4-fluor-benzyl)-1-thiophen-2-yl-cyclohexanol
• 2-Dimethylaminomethyl-4-(4-fluor-benzyl)-1-(3-methoxy-phenyl)-cyclohexanol
• 2-Dimethylaminomethyl-4-(4-fluor-benzyl)-1-(3-phenyl-propyl)-cyclohexanol
• 1-(2,3-Dichlor-phenyl)-2-dimethylaminomethyl-4-(4-fluor-benzyl)-cyclohexanol
• 2-Dimethylaminomethyl-4-(4-fluor-benzyl)-1-p-tolyl-cyclohexanol
• 2-Dimethylaminomethyl-4-(4-fluor-benzyl)-1-(4-methoxy-phenyl)-cyclohexanol
• 1-Cyclohexylmethyl-2-dimethylaminomethyl-4-(4-fluor-benzyl)-cyclohexanol
• 2-Dimethylaminomethyl-4-(4-fluor-benzyl)-(5-fluor-2-methoxy-phenyl)-cyclohexanol
• 2-Dimethylaminomethyl-4-(4-fluor-benzyl)-1-(3-fluor-phenyl)-cyclohexanol
• 1-(3-chlor-phenyl)-2-dimethylaminomethyl-4-(4-fluor-benezyl)-cyclohexanol
• 1-(3,5-Dichlor-phenyl)-2-dimethylaminomethyl-4-(4-fluor-benzyl)-cyclohexanol
• 1-(2-chlor-benzyl)-2-dimethylaminomethyl-4-(4-fluor-benzyl)-cyclohexanol
• 2-Dimethylaminomethyl-1,4-bis-(4-fluor-benzyl)-cyclohexanol
• 2-Dimethylaminomethyl-4-(4-fluor-benzyl)-1-(3-methoxy-benzyl)-cyclohexanol
• 1-(4-chlor-3-trifluormethyl-phenyl)-2-dimethylaminomethyl-4-(4-fluor-benzyl)-cyclohexanol
• 2-Dimethylaminomethyl-4-(4-fluor-benzyl)-1-(3-fluor-benzyl)-cyclohexanol
• 2-Dimethylaminomethyl-4-(4-fluor-benzyl)-1-(2-methoxy-phenyl)-cyclohexanol
• 2-Dimethylaminomathyl-4-(4-fluor-benzyl)-1-(2-methyl-benzyl)-cyclohexanol
• 1-(3-chlor-4-fluor-phenyl)-2-dimethylaminomethyl-4-(4-fluor-benzyl)-cyclohexanol
• 2-Dimethylaminomethyl-4-(4-fluor-benzyl)-1-(3-trifluormethyl-phenyl)-cyclohexanol
• 2-Dimethylaminomethyl-4-(4-fluor-benzyl)-1-(3-methyl-benzyl)-cyclohexanol
• 1-(4-chlor-benzyl)-2-dimethylaminomethyl-4-(4-fluor-benzyl)-cyclohexanol
• 1-(2-chlor6-fluor-benzyl)-2-dimethylaminomethyl-4-(4-fluor-benzyl)-cyclohexanol
• 2-Dimethylaminomethyl-1-(2,5-dimethyl-benzyl)-4-(4-fluor-benzyl)-cyclohexanol
• 1-(3-chlor-benzyl)-2-dimethylaminomethyl-4-(4-fluor-benzyl)-cyclohexanol
• 1-(2,4-Dichlor-benzyl)-2-dimethylaminomethyl-4-(4-fluor-benzyl)-cyclohexanol
• 2-Dimethylaminomethyl-4-(3-methoxy-benzyl)-1-phenyl-cyclohexanol
• 1-Benzyl-2-dimethylaminomethyl-4-(3-methoxy-benzyl)-cyclohexanol
• 2-Dimethylaminomethyl-1-(4-fluor-3-methyl-phenyl)-4-(3-methoxy-benzyl)-cyclohexanol
• 2-Dimethylaminomethyl-4-(3-methoxy-benzyl)-1-o-totyl-cyclohexanol
• 2-Dimethylaminomethyl-4-(3-methoxy-benzyl)-1-vinyl-cyclohexanol
• 1-(4-tert-Butyl-phenyl)-2-dimethylaminomethyl-4-(3-methoxy-benzyl)-cyclohexanol
• 1-Cyclopentyl-2-dimethylaminomethyl-4-(3-methoxy-benzyl)-cyclohexanol
• 2-Dimethylaminomethyl-4-(3-methoxy-benzyl)-1-m-tolyl-cyclohexanol
• 2-Dimethylaminomethyl-4-(3-methoxy-benzyl)-bicyclohexyl-1-ol
• 2-Dimethylaminomethyl-4-(3-methoxy-benzyl)-1-phenethyl-cyclohexanol
• 2-Dimethylaminomethyl-4-(3-methoxy-benzyl)-1-phenylethynyl-cyclohexanol
• 2-Dimethylaminomethyl-4-(3-methoxy-benzyl)-1-thiophen-2-yl-cyclohexanol
• 2-Dimethylaminomethyl-4-(3-methoxy-benzyl)-1-(3-methoxy-phenyl)-cyclohexanol
• 2-Dimethylaminomethyl-4-(3-methoxy-benzyl)-1-(3-phenyl-propyl)-cyclohexanol
• 1-(2,3-Dichlor-phenyl)-2-dimethylaminomethyl-4-(3-methoxy-benzyl)-cyclohexanol
• 2-Dimethylaminomethyl-4-(3-methoxy-benzyl)-1-p-tolyl-cyclohexanol
• 1-Cyclohexylmethyl-2-dimethylaminomethyl-4-(3-methoxy-benzyl)-cyclohexanol
• 2-Dimethylaminomethyl-1-(5-fluor-2-methoxy-phenyl)-4-(3-methoxy-benzyl)-cyclohexanol
• 1-(3-chlor-phenyl)-2-dimethylaminomethyl-4-(3-methoxy-benzyly-cyclohexanol
• 1-(3,5-Dichlor-phenyl)-2-dimethylaminomethyl-4-(3-methoxy-benzyl)-cyclohexanol
• 1-(4-chlor-3-trifluormethyl-phenyl)-2-dimethylaminomethyl-4-(3-methoxybenzyl)-cyclohexanol
• 2-Dimethylaminomethyl-1-(3-fluor-benzyl)-4-(3-methoxy-benzyl)-cyclohexanol
• 2-Dimethylaminomethyl-4-(3-methoxy-benzyl)-(2-methoxy-phenyl)-cyclohexanol
• 2-Dimethylaminomethyl-4-(3-methoxy-benzyl)-1-(3-trifluormethyl-phenyl)-cyclohexanol
• 1-(2-chlor-6-fluor-benzyl)-2-dimethylaminomethyl-4-(3-methoxy-benzyl)-cyclohexanol
• 2-Dimethylaminomethyl-1-(2,5-dimethyl-benzyl)-4-(3-methoxy-benzyl)-cyclohexanol
• 1-(3-chlor-benzyl)-2-dimethylaminomethyl-4-(3-methoxy-benzyl)-cyclohexanol
• 2-Dimethylaminomethyl-4-(4-fluor-benzyloxy)-1-phenyl-cyclohexanol
• 1-Benzyl-2-dimethylaminomethyl-4-(4-fluor-benryloxy)-cyclohexanol
• 2-Dimethylaminomethyl-4-(4-fluor-benzyloxy)-1-(4-fluor-3-methyl-phenyl)-cyclohexanol
• 2-Dimethylaminomethyl-4-(4-fluor-benzyloxy)-1-vinyl-cyclohexanol
• 1-(4-tert-Butyl-phenyl)-2-dimethylaminomethyl-4-(4-fluor-benzyloxy)-cyclohexanol
• 1-Cyclopentyl-2-dimethylaminomethyl-4-(4-fluor-benzyloxy)-cyclohexanol
• 2-Dimethylaminomethyl-4-(4--fluor-benzyloxy)-1-m-tolyl-cyclohexanol
• 2-Dimethylaminomethyl-4-(4-fluor-benzyloxy)-bicyclohexyl-1-ol
• 2-Dimethylaminomethyl-4-(4-fluor-benzyloxy)-1-phenethyl-cyclohexanol
• 2-Dimethylaminomethyl-4-(4-fluor-benzyloxy)-1-phenylethnyl-cyclohexanol
• 2-Dimethylaminomethyl-4-(4-fluor-benzyloxy)-1-thiophen-2-yl-cyclohexanol
• 2-Dimethylaminomethyl-4-(4-fluor-benzyloxy)-1 -(3-methoxy-phenyl)-cyclohexanol
• 2-Dimethylaminomethyl-4-(4-fluor-benzyloxy)-1-p-tolyl-cyclohexanol
• 1-Cyclohexylmethyl-2-dimethylaminomethyl-4-(4-fluor-benzylyloxy)-cyclohexanol
• 2-Dimethylaminomethyl-1-(3-fluor-benzyl)-4-(4-fluor-benzyloxy)-cyclohexanol
• 2-Dimethylaminomethyl-4-(4-fluor-benzyloxy)-1-(2-methoxy-phenyl)-cyclohexanol
• 2-Dimethylaminomethyl-4-(4-fluor-benzyloxy)-1-(3-methyl-benzyl)-cyclohexanol
• 2-Dimethylaminomethyl-1-(2,5-dimethyl-benzyl)-4-(4-fluor-benzyloxy)-cyclohexanol
• 1-(3-Chlor-benzyl)-2-dimethylaminomethyl-4-(4-fluor-benzyloxy)-cyclohexanol
• [5-Benzyloxy-2-(3-methoxy-phenyl)-cyclohexylmethyl]-dimethyl-amin;
• [5-(3-Chlor-benzyloxy)-2-(3-methoxy-phenyl)-cyclohexylmethyl]-dimethyl-amin;
• [2-(3-Methoxy-phenyl)-5-(naphthalin-2-ylmethoxy)-cyclohexylmethyl]-dimethylamin;
• [5-(3-Methoxy-benzyloxy)-2-(3-methoxy-phenyl)-cyclohexylmethyl]-dimethylamin;
• [5-(4-Chlor-benzyloxy)-2-(3-methoxy-phenyl)-cyclohexylmethyl]-dimethyl-amin;
• [5-(4-Methoxy-benzyloxy)-2-(3-methoxy-phenyl)-cyclohexylmethyl]-dimethylamin;
• 2-Dimethylaminomethyl-1-(3-methoxy-phenyl)-4-(naphthalin-2-ylmethoxy)-cyclohexanol;
• Buttersäure 3-dimetthylaminomethyl-4-hydroxy-4-(3-methoxy-phenyl)-cyclohexylester;
• 2-Dimethylaminomethyl-1-(3-methoxy-phenyl)-4-(naphthalin-2-ylmethoxy)-cyclohexanol
• 4-Methoxy-benzoesäure 3-dimethylaminomethyl-4-hydroxy-4-(3-methoxy-phenyl)-cyclohexylester;
• 3-Methoxy-benzoesäure 3-dimethylaminomethyl-4-hydroxy-4-(3-methoxyphenyl)-cyclohexylester;
• 2,2-Dimethyl-propionsäure 3-dimethylaminomethyl-4-hydroxy-4-(3-methoxyphenyl)-cyclohexylester;
• Buttersäure 3-dimethylaminomethyl-4-hydroxy-4-(3-methoxy-phenyl)-cyclohexylester;
• 4-Methoxy-benzoesäure 3-dimethylaminomethyl-4-hydroxy-4-(3-methoxyphenyl)-cyclohexylester;
• 3-Methoxy-benzoesäure 3-dimethylaminomethyl-4-hydroxy-4-(3-methoxyphenyl)-cyclohexylester;
• 2,2-Dimethyl-propionsäure 3-dimethylaminomethyl-4-hydroxy-4-(3-methoxyphenyl)-cyclohexylester;
• 3,4-Dimethoxy-benzoesäure 3-dimethylaminomethyl-4-hydroxy-4-(3-methoxyphenyl)-cyclohexylester;
• 3,4-Dimethoxy-benzoesäure 3-dimethylaminomethyl-4-hydroxy-4-(3-methoxyphenyl)-cyclohexylester,
• Naphthalin-2-carbonsäure 3-dimethylaminomethyl-4-hydroxy-4-(3-methoxy-phenyl)-cyclohexylester;
• 4-Methyl-benzoesäure 3-dimethylaminomethyl-4-hydroxy-4-(3-methoxyphenyl)-cyclohexylester;
• 3,4-Dichlor-benzoesäure 3-dimethylaminomethyl-4-hydroxy-4-(3-methoxyphenyl)-cyclohexylester;
• 4-Chlor-benzoesäure 3-dimethylaminomethyl-4-hydroxy-4-(3-methoxyphenyl)-cyclohexylester;
• 3-Chlor-benzoesäure 3-dimethylaminomethyl-4-hydroxy-4-(3-methoxyphenyl)-cyclohexylester;
• 2-Chlor-benzoesäure 3-dimethylaminomethyl-4-hydroxy-4-(3-methoxyphenyl)-cyclohexylester;
• 4-Trifluormethyl-benzoesäure 3-dimethylaminomethyl-4-hydroxy-4-(3-methoxy-phenyl)-cyclohexylester;
• 3,5-Difiuor-benzoesäure 3-dimethylaminomethyl-4-hydroxy-4-(3-methoxyphenyl)-cyclohexylester;
• 4-Fluor-benzoesäure 3-dimethylaminomethyl-4-hydroxy-4-(3-methoxy-phenyl)-cyclohexylester;
• 3-Fluor-benzoesäure 3-dimethylaminomethyl-4-hydroxy-4-(3-methoxy-phenyl)-cyclohexylester;
• 2-Fluor-benzoesäure 3-dimethylaminomethyl-4-hydroxy-4-(3-methoxy-phenyl)-cyclohexylester;
• Cyclopentancarbonsäure 3-dimethylaminomethyl-4-hydroxy-4-(3-methoxyphenyl)-cyclohexylester;
• (4-Methoxy-phenyl)-essigsäure 3-dimethylaminomethyl-4-hydroxy-4-(3-methoxy-phenyl)-cyclohexylester;
• (3-Methoxy-phenyl)-essigsäure 3-dimethylaminomethyl-4-hydroxy-4-(3-methoxy-phenyl)-cyclohexylester
• Phenyl-essigsäure 3-dimethylaminomethyl-4-hydroxy-4-(3-methoxy-phenyl)-cyclohexylester;
• 3,4,5-Trimethoxy-benzoesäure 3-dimethylaminomethyl-4-hydroxy-4-(3-methoxy-phenyl)-cyclohexylester;
• 3,5-Dimethoxy-benzoesäure 3-dimethylaminomethyl-4-hydroxy-4-(3-methoxyphenyl)-cyclohexylester;
• Naphthalin-1-carbonsäure 3-dimethylaminomethyl-4-hydroxy-4-(3-methoxyphenyl)-cyclohexylester;
• 2-Methoxy-benzoesäure 3-dimethylaminomethyl-4-hydroxy-4-(3-methoxyphenyl)-cyclohexylester;
• Naphthalin-2-carbonsäure 3-dimethylaminomethyl-4-hydroxy-4-(3-methoxyphenyl)-cyclohexylester;
• 2-Hydroxy-benzoesäure 3-dimethylaminomethyl-4-hydroxy-4-(3-methoxyphenyl)-cyclohexylester;
• 4-Methyl-benzoesäure 3-dimethylaminomethyl-4-hydroxy-4-(3-methoxyphenyl)-cyclohexylester;
• 3,4-Dichlor-benzoesäure 3-dimethylaminomethyl-4-hydroxy-4-(3-methoxyphenyl)-cyclohexylester;
• 4-Chlor-benzoesäure 3-dimethylaminomethyl-4-hydroxy-4-(3-methoxyphenyl)-cyclohexylester;
• 3-Chlor-benzoesäure 3-dimethylaminomethyl-4-hydroxy-4-(3-methoxyphenyl)-cyclohexylester;
• 2-Chlor-benzoesäure 3-dimethylaminomethyl-4-hydroxy-4-(3-methoxyphenyl)-cyclohexylester;
• 4-Trifluormethyl-benzoesäure 3-dimethylaminomethyl-4-hydroxy-4-(3-methoxy-phenyl)-cyclohexylester;
• 3,5-Difluor-benzoesäure 3-dimethylaminomethyl-4-hydroxy-4-(3-methoxyphenyl)-cyclohexylester;
• 4-Fluor-benzoesäure 3-dimethylaminomethyl-4-hydroxy-4-(3-methoxy-phenyl)-cyclohexylester;
• 3-Fluor-benzoesäure 3-dimethylaminomethyl-4-hydroxy-4-(3-methoxy-phenyl)-cyclohexylester;
• 2-Fluor-benzoesäure 3-dimethylaminomethyl-4-hydroxy-4-(3-methoxy-phenyl)-cyclohexylester;
• Cyclopentancarbonsäure 3-dimethylaminomethyl-4-hydroxy-4-(3-methoxy phenyl)-cyclohexylester;
• (4-Methoxy-phenyl)-essigsäure 3-dimethylaminomethyl-4-hydroxy-3234-(3-methoxy-phenyl)-cyclohexylester;
• (3-Methoxy-phenyl)-essigsäure 3-dimethylaminomethyl-4-hydroxy-4-(3-methoxy-phenyl)-cyclohexylester;
• Phenyl-essigsäure 3-dimethylaminomethyl-4-hydroxy-4-(3-methoxy-phenyl)-cyclohexylester;
• 3,4,5-Trimethoxy-benzoesäure 3-dimethylaminomethyl-4-hydroxy-4-(3-methoxy-phenyl)-cyclohexylester;
• 3,5-Dimethoxy-benzoesäure 3-dimethylaminomethyl-4-hydroxy-4-(3-methoxyphenyl)-cyclohexylester;
• Naphthalin-1-carbonsäure 3-dimethylaminomethyl-4-hydroxy-4-(3-methoxyphenyl)-cyclohexylester;
• 2-Methoxy-benzoesäure 3-dimethylaminomethyl-4-hydroxy-4-(3-methoxyphenyl)-cyclohexylester;
• 2-Dimethylaminomethyl-1,4-bis-(3-methoxy-phenyl)-cyclohexan-1,4-diol;
• 2-Dimethylaminomethyl-1,4-bis-(3-methoxy-phenyl)-cyclohexan-1,4-diol;
• 6-(4-Benzyloxy-2-dimethylaminomethyl-1-hydroxy-cyclohexyl)-naphthalin-2-ol;
• 4-Benzyloxy-2-dimethylaminomethyl-1-(6-methoxy-naphthalin-2-yl)-cyclohexanol;
• Buttersäure 3-dimethylaminomethyl-4-hydroxy-4-(3-methoxy-phenyl)-cyclohexylester
• Buttersäure 3-dimethylaminomethyl-4-hydroxy-4-(3-methoxy-phenyl)-cyclohexylester
• 2-Dimethylaminomethyl-1-(3-methoxy-phenyl)-4-methylamino-cyclohexanol;
• 2-Dimethylaminomethyl-1-(3-methoxy-phenyl)-4-methylamino-cyclohexanol;
• Valeriansäure 3-dimethylaminomethyl-4-hydroxy-4-(3-methoxy-phenyl)-cyclohexylester;
• [3-Dimethylaminomethyl4-hydroxy4-(3-methoxy-phenyl)-cyclohexylidene]-essigsäure ethylester;
• [3-Dimethylaminomethyl-4-hydroxy-4-(3-methoxy-phenyl)-cyclohexylidene]-essigsäure ethylester;
• 3-[2-Dimethylaminomethyl-4-(4-fluor-benzyloxy)-1-hydroxy-cyclohexyl]-phenol;
• 4-Benzyloxy-2-dimethylaminomethyl-1-(3-phenyl-propyl)-cyclohexanol;
• 2-Dimethylaminomethyl-4-(4-fluor-benzyloxy)-1-(3-phenyl-propyl)-cyclohexanol;
• 2-Dimethylaminomethyl-1-(3-fluor-benzyl)-4-(4-fluor-benzyloxy)-cyclohexanol;
• 4-Benzyloxy-2-dimethylaminomethyl-1-(3-fluor-benzyl)-cyclohexanol;
• 2-Dimethylaminomethyl-4-(4-fluor-benzyloxy)-1-p-tolyl-cyclohexanol;
• 4-Benzyloxy-2-dimethylaminomethyl-1-p-tolyl-cyclohexanol;
• 2-Dimethylaminomethyl-1-(2,5-dimethyl-benzyl)-4-(4-fluor-benzyloxy)-cyclohexanol;
• 4-Benzyloxy-2.dimethytaminomethyl-1-(2,5-dimethyl-benzyl)-cyclohexanol;
• 1-(4-Chlor-3-trifluormethyl-phenyl)-2-dimethylaminomethyl-4-(4-fluorbenzyloxy)-cyclohexanol;
• 4-Benzyloxy-1-(4-chlor-3-trifluormethyl-phenyl)-2-dimethylaminomethyl-cyclohexanol;
• [2,5-Bis-(4-fluor-benzyloxy)-2-(3-methoxy-phenyl)-cyclohexylmethyl]-dimemylamin;
• 2-Dimethylaminomethyl-1-(6-methoxynaphthalin-2-yl)cyclohexan-1,4-diol
• Buttersäure 3-dimethylaminomethyl-4-hydroxy-4-(6-methoxynaphthalin-2-yl)cyclohexylester
• Benzoesäure 4-hydroxy-3-(4-methyl-piperazin-1-ylmethyl)-4-naphthalin-2-yl-cyclohexyl ester;
• 2-Chlor-benzoesäure 4-hydroxy-3-(4-methyl-piperazin-1-ylmethyl)-4-naphthalin-2-yl-cyclohexyl ester;
• 3-Chlor-benzoesäure 4-hydroxy-3-(4-methy)-piperazin-1-ylmethyl)-4-naphthalin-2-yl-cyclohexyl ester;
• 4-Chlor-benzoesäure 4-hydroxy-3-(4-methyl-piperazin-1-ylmethyl)-4-naphthalin-2-yl-cyclohexyl ester;
• 2-Fluor-benzoesäure 4-hydroxy-3-(4-methyl-piperazin-1-ylmethyl)-4-naphthalin-2-yl-cyclohexyl ester;
• 3- Fluor -benzoesäure 4-hydroxy-3-(4-methyl-piperazin-1-ylmethyl)-4-naphthalin-2-yl-cyclohexyl ester;
• 4- Fluor -benzoesäure 4-hydroxy-3-(4-methyl-piperazin-1-ylmethyl)-4-naphthalin-2-yl-cyclohexyl ester;
• 2-Methyl-benzoesäure 4-hydroxy-3-(4-methyl-piperazin-1-ylmethyl)-4-naphthalin-2-yl-cyclohexyl ester;
• 3- Methyl -benzoesäure 4-hydroxy-3-(4-methyl-piperazin-1-ylmethyl)-4-naphthalin-2-yl-cyclohexyl ester;
• 4- Methyl -benzoesäure 4-hydroxy-3-(4-methyl-piperazin-1-ylmethyl)-4-naphthalin-2-yl-cyclohexyl ester;
• 2-Methoxy-benzoesäure 4-hydroxy-3-(4-methyl-piperazin-1-ylmethyl)-4-naphthalin-2-yl-cyclohexyl ester;
• 3- Methoxy -benzoesäure 4-hydroxy-3-(4-methyl-piperazin-1-ylmethyl)-4-naphthalin-2-yl-cyclohexyl erster;
• 4- Methoxy -benzoesäure 4-hydroxy-3-(4-methyl-piperazin-1-ylmethyl)-4-naphthalin-2-yl-cyclohexyl ester;
• 2,6-Dichlor-benzoesäure 4-hydroxy-3-(4-methyl-piperazin-1-ylmethyl)-4-naphthalin-2-yl-cyclohexyl ester;
• 2,6-Difluor-benzoesäure 4-hydroxy-3-(4-methyl-piperazin-1-ylmethyl)-4-naphthalin-2-yl-cyclohexyl ester;
• 2-Chlor-5-fluor-benzoesäure 4-hydroxy-3-(4-methyl-piperazin-1-ylmethyl)-4-naphthalin-2-yl-cyclohexyl ester;
• Biphenyl-4-carbonsäure 4-hydroxy-3-(4-methyl-piperazin-1-ylmethyl)-4-naphthalin-2-yl-cyclohexylester;
• 2-Chlor-benzoesäure 4-hydroxy-4-(6-methoxy-naphthalin-2-yl)-3-(4-methylpiperazin-1-ylmethyl)-cyclohexyl ester;
• 3-Chlor-benzoesäure 4-hydroxy-4-(6-methoxy-naphthalin-2-yl)-3-(4-methylpiperazin-1-yimethyl)-cyclohexyl ester;
• 4-Chlor-benzoesäure 4-hydroxy-4-(6-methoxy-naphthalin-2-yl)-3-(4-methylpiperazin-1-ylmethyl)-cyclohexyl ester;
• 2-Fluor-benzoesäure 4-hydroxy-4-(6-methoxy-naphthalin-2-yl)-3-(4-methylpiperazin-1-ylmethyl)-cyclohexyl ester;
• 3-Fluor-benzoesäure 4-hydroxy-4-(6-methoxy-naphthalin-2-yl)-3-(4-methylpiperazin-1-ylmethyl)-cyclohexyl ester;
• 4-Fluor-benzoesäure 4-hydroxy-4-(6-methoxy-naphthalin-2-yl)-3-(4-methylpiperazin-1-yimethyl)-cyclohexyl ester;
• 2-Methyl-benzoesäure 4-hydroxy-4-(6-methoxy-naphthalin-2-yl)-3-(4-methylpiperazin-1-ylmethyl)-cyclohexyl ester;
• 3-Methyl-benzoesäure 4-hydroxy-4-(6-methoxy-naphthalin-2-yl)-3-(4-methylpiperazin-1-ylmethyl)-cyclohexyl ester;
• 4-Methyl-benzoesäure 4-hydroxy-4-(6-methoxy-naphthalin-2-yl)-3-(4-methylpiperazin-1-ylmethyl)-cyclohexyl ester;
• 2-Methoxy-benzoesäure 4-hydroxy-4-(6-methoxy-naphthalin-2-yl)-3-(4-methylpiperazin-1-ylmethyl)-cyclohexyl ester;
• 3-Methoxy-benzoesäure 4-hydroxy-4-(6-methoxy-naphthalin-2-yl)-3-(4-methylpiperazin-1-yimethyl)-cyclohexyl ester;
• 4-Methoxy-benzoesäure 4-hydroxy-4-(6-methoxy-naphthalin-2-yl)-3-(4-methylpiperazin-1-ylmethyl)-cyclohexyl ester;
• 2,6-Dichlor-benzoesäure 4-hydroxy-4-(6-methoxy-naphthalin-2-yl)-3-(4-methylpiperazin-1-ylmethyl)-cyclohexyl ester;
• 2,6-Fluor-benzoesäure 4-hydroxy-4-(6-methoxy-naphthalin-2-yl)-3-(4-methylpiperazin-1-yimethyl)-cyclohexyl ester;
• 2-Chlor-6-fluor-benzoesäure 4-hydroxy-4-(6-methoxy-naphthalin-2-yl)-3-(4-methyl-piperazin-1-ylmethyl)-cyclohexyl ester;
• Biphenyl-4-carbonsäure 4-hydroxy-4-(6-methoxy-naphthalin-2-yl)-3-(4-methylplperazin-1-ylmethyl)-cyclohexyl ester;
• 4-(2-Chlor-benzyloxy)-2-(4-methyl-piperazin-1-ylmethyl)-1-naphthalin-2-ylcyclohexanol;
• 4-(3-Chlor-benzyloxy)-2-(4-methyl-piperazin-1-ylmethyl)-1-naphthalin-2-ylcyclohexanol;
• 4-(4-Chlor-benzyloxy)-2-(4-mathyl-piperazin-1-ylmethyl)-1-naphthalin-2-ylcyclohexanol;
• 4-(2-Fluor-benzyloxy)-2-(4-methyl-piperazin-1-ylmethyl)-1-naphthalin-2-yl-cyclohexanol:
• 4-(3-Fluor-benzyloxy)-2-(4-methyl-piperazin-1-ylmethyl)-1-naphthalin-2-yl-cyclohexanol;
• 4-(4-Fluor-benzyloxy)-2-(4-methyl-piperazin-1-ylmethyl)-1-naphthalin-2-yl-cyclohexanol;
• 4-(2-Methyl-benzyloxy)-2-(4-methyl-piperazin-1-ylmethyl)-1-naphthalin-2-yl-cyclohexanol;
• 4-(3-Methyl-benzyloxy)-2-(4-methyl-piperazin-1-ylmethyl)-1-naphthalin-2-yl-cyclohexanol;
• 4-(4-Methyl-benzyloxy)-2-(4-methyl-piperazin-1-ylmethyl)-1-naphthalin-2-yl-cyclohexanol;
• 4-(2-Methoxy-benzyloxy)-2-(4-methyl-piperazin-1-ylmethyl)-1-naphthalin-2-yl-cyclohexanol;
• 4-(3-Methoxy-benzyloxy)-2-(4-methyl-piperazin-1-ylmethyl)-1-naphthalin-2-yl-cyclohexanol;
• 4-(4-Methoxy-benzyloxy)-2-(4-methyl-piperazin-1-ylmethyl)-1-naphthalin-2-yl-cyclohexanol;
• 4-(2,6-DiChlor-benzyloxy)-2-(4-methyl-piperazin-1-ylmethyl)-1-naphthalin-2-yl-cyclohexanol,
• 4-(2,6-Difluor-benzyloxy)-2-(4-methyl-plperazin-1-ylmethyl)-1-naphthalin-2-yl-cyclohexanol,
• 4-(2-Chlor-6-fluor-benzylox)-2-(4-methyl-piperazin-1-ylmethyl)-1-naphthalin-2-yl-cyclohexanol,
• 4-(2-Chlor-benzyloxy)-1-(6-methoxy-naphthalin-2-yl)-2-(4-methyl-piperazin-1-ylmethyl)-cyclohexanol;
• 4-(3-Chlor-benzyloxy)-1-(6-methoxy-naphthalin-2-yl)-2-(4-methyl-piperazin-1-ylmethyl)-cyclohexanol;
• 4-(4-Chlor-benzyloxy)-1-(6-methoxy-naphthalin-2-yl)-2-(4-methyl-piperazin-1-ylmethyl)-cyclohexanol;
• 4-(2-Fluor-benzyloxy)-1-(6-methoxy-naphthalin-2-yl)-2-(4-methyl-piparazin-1-ylmethyl)-cyclohexanol;
• 4-(3-Fluor-benzyloxy)-1-(6-methoxy-naphthalin-2-yl)2-(4-methyl-piperazin-1-ylmethyl)-cyclohexanol;
• 4-(4-Fluor-benzyloxy)-1-(6-methoxy-naphthalin-2-yl)-2-(4-methyl-piperazin-1-ylmethyl)-cyclohexanol;
• 4-(2-Methyl-benzyloxy)-1-(6-methoxy-naphthalin-2-yl)-2-(4-methyl-piperazin-1-ylmethyl)-cyclohexanol;
• 4-(3-Methyl-benzyloxy)-1-(6-methoxy-naphthalin-2-yl)-2-(4-methyl-piperazin-1-ylmethyl)-cyclohexanol;
• 4-(4-Methyl-benzyloxy)-1-(6-methoxy-naphthalin-2-yl)-2-(4-methyl-piperazin-1-ylmethyl)-cyclohexanol;
• 4-(2-Methoxy-benzyloxy)-1-(6-methoxy-naphthalin-2-yl)-2-(4-methyl-piperazin-1-ylmethyl)-cyclohexanol;
• 4-(3-Methoxy-benzyloxy)-1-(6-methoxy-naphthalin-2-yl)-2-(4-methyl-piperazin-1-ylmethyl)-cyclohexanol;
• 4-(4-Methoxy-benzyloxy)-1-(6-methoxy-naphthalin-2-yl)-2-(4-methyl-piperazin-1-ylmethyl)-cyclohexanol;
• 4-(2,6-Dichlor-benzyloxy)-1-(6-methoxy-naphthalin-2-yl)-2-(4-methyl-piperazin-1-ylmethyl)-cyclohexanol;
• 4-(2,6-Difluor-benzyloxy)-1-(6-methoxy-naphthalin-2-yl)-2-(4-methyl-piperazin-1-ylmethyl)-cyclohexanol;
• 4-(2-Chlor-6-Fluor-benzyloxy)-1-(6-methoxy-naphthalin-2-yl)-2-(4-methylpiperazin-1-ylmethyl)-cyclohexanol;
• 2-Dimethylaminomethyl-4-(4-fluor-benzyloxy)-1-naphthalin-2-yl-cyclohexanol
• 4-Fluor-benzoesäure 3-dimethylaminomethyl-4-hydroxy-4-(6-methoxy-naphthalin-2-yl)-cyclohexylester
• 4-Fluor-benzoesäure 3-dimethylaminomethyl-4-hydroxy-4-naphthalin-2-yl-cyclohexylester
• 2-Dimethylaminomethyl-4-(4-fluor-benzyloxy)-1-(6-methoxy-naphthalin-2-yl)-cyclohexanol
• 6-[2-Dimethylaminomethyl-4-(4-fluor-benzyloxy)-1-hydroxy-cyclohexyl]-naphthalin-2-ol
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, insbesondere des Hydrochlorid- oder des Bishydrochloridsalzes.

Die erfindungsgemäßen Substanzen sind toxikologisch unbedenklich, so daß sie sich als pharmazeutischer Wirkstoff in Arzneimittel eignen. Ein weiterer Gegenstand der Erfindung sind daher Arzneimittel enthaltend wenigstens ein erfindungsgemäßes substituiertes C-Cyclohexylmethylamin-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten C-Cyclohexylmethylamin-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte C-Cyclohexylmethylamin-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten C-Cyclohexylmethylamin-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten C-Cyclohexylmethylamin-Derivats appliziert.

In einer bevorzugten Form des Arzneimittel liegt ein enthaltenes erfindungsgemäßes substituiertes C-Cyclohexylmethylamin-Derivat als reines Diastereomer und/oder Enantiomer, als Razemat oder als nicht-äquimolare oder äquimolare Mischung der Diastereomere und/oder Enantiomere vor.

Ein weiterer Gegenstand der Erfindung ist die Verwendung eines erfiondungsgemäßen substituierten C-Cyclohexylmethylamin-Derivats zur Herstellung eines Arzneimittels zur Behandlung von Schmerz, insbesondere von neuropathischem oder chronischem Schmerz. Dabei kann es bevorzugt sein, wenn ein verwendetes substituiertes C-Cyclohexylmethylamin-Derivat wie hierin beschrieben, als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Herstellung eines erfindungsgemäßen substituierten C-Cyclohexylmethylamin-Derivats wie in der folgenden Beschreibung und Beispielen ausgeführt.

Einige Verfahren zur Herstellung erfindungsgemäßer Verbindungen gemäß der allg. Formel I, die hier analog angewandt werden, sind schon in DE 19547766 beschrieben worden (I. Graudums et al., Grünenthal GmbH, 1995). Zur Herstellung erfindungsgemäßer Substanzen der allgemeinen Formel I, in denen A nicht Wasserstoff bedeutet, wurden die von Graudums et al. beschriebenen Verfahren mit literaturbekannten Verfahren zur Darstellung substituierter Mannichbasen kombiniert (Risch et al., Houben-Weyl - Methoden der Organischen Chemie, E21b (1995) 1925-1929; Angew. Chem. 106 (1994) 2531-2533; Synlett (1997) 974-976).

Zur Herstellung von Substanzen, in denen die Olefine 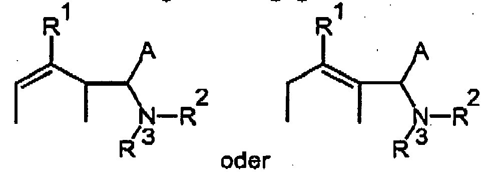 für die Gruppierung 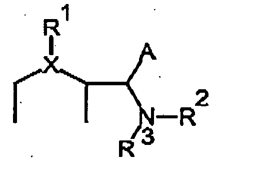 stehen, wurden tertiäre Alkohole 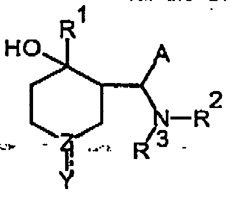 gegebenenfalls in einem Lösungsmittel oder Lösungsmittelgemisch bei Temperaturen zwischen 20 und 120 °C mit organischen oder anorganischen Säuren oder anderen wasserentziehenden Mitteln wie Säurehalogeniden behandelt. Bevorzugt wurden Ameisensäure oder Bromwasserstoffsäure eingesetzt. In Abhängigkeit von den gewählten Reaktionsbedingungen wird auf diesen Weg eines der oben aufgeführten Olefine bevorzugt oder auch ausschließlich erhalten. Aus diesen Olefinen 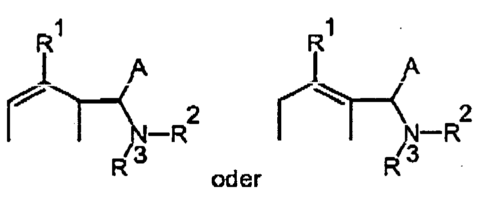 wurden durch Reduktion der CC-Doppelbindung erfindungsgemäße Verbindungen erhalten, bei denen 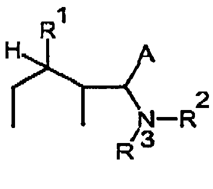 für die Gruppierung 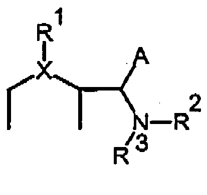 steht. Hierzu wurden die Olefine bevorzugt unter Wasserstoffatmosphäre oder auch Wasserstoffüberdruck in Gegenwart eines Übergangsmetalls, das ggf. an einen Träger gebunden war, hydriert.

Die Umsetzung der tertiären Alkohole 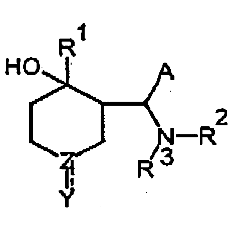 mit Salzsäure bei Temperaturen zwischen -20 und +20 °C führte zu Chlorderivaten 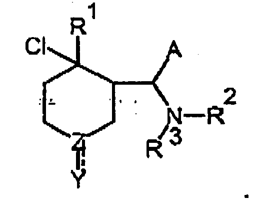 Bei dieser Umsetzung hat die Reaktionsdauer, die Konzentration der verwendeten Salzsäure und der Einsatz eines ggf. zugesetzten Lösungsmittels Einfluß auf den Verlauf der Reaktion. Unter drastischeren Bedingungen, also bei Verwendung konzentrierterer Salzsäure, langen Reaktionszeiten oder Anwendung höherer Temperaturen, wurden bei dieser Umsetzung auch Olefine 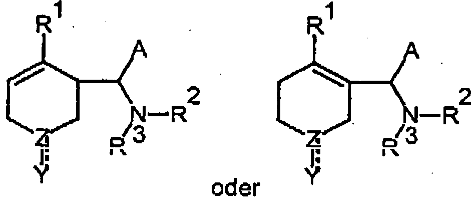 erhalten. Die Umsetzung der tertiären Alkohole 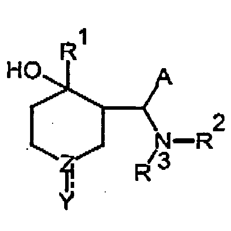 mit Fluorierungs-Reagenzien wie Dimethylaminoschwefeltrifluorid (DAST) oder Deoxofluor® führte zu Fluorderivaten

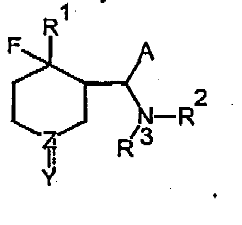

Zur Herstellung erfindungsgemäßer Substanzen, in denen 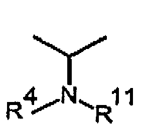 für die Gruppierung 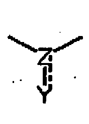 steht, wurden Ketone 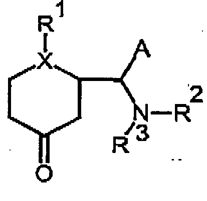 mit Ammoniak bzw. Ammoniaksalzen, primären oder sekundären Aminen unter reduktiven Bedingungen umgesetzt. Eine Vielzahl literaturbekannter Verfahren ist hierfür geeignet (R.C. Larock; Comprehensive Organic Transformations; VCH Publishers; New York, Weinheim, Cambridge 1989). Bevorzugt wurden komplexe Hydride wie Natrlumborhydrid, Natriumcyanoborhydrid oder besonders bevorzugt Natriumtriacetoxyborhydrid eingesetzt. Soweit auf diesem Weg primäre oder sekundäre Amine erhalten wurden, können diese nach literaturbekannten Methoden mit Säurehalogeniden oder Säuren zu Amiden umgesetzt werden, mit Alkyl-, Arylalkyl- oder Heteroarylalkylhalogeniden nach literaturbekannten Methoden alkyliert oder mit Aldehyden nach ebenfalls literaturbekannten Methoden reduktiv laminiert werden (R.C. Larock: Comprehensive Organic Transformations; VCH Publishers; New York, Weinheim, Cambridge 1989).

Die Herstellung erfindungsgemäße Substanzen, in denen 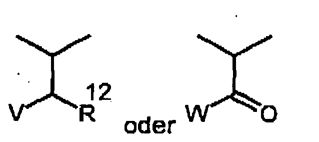 für die Gruppierung 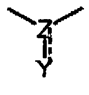 steht, kann nach einer Vielzahl von literaturbekannten Methoden (R.C. Larock; Comprehensive Organic Transformations; VCH Publishers; New York, Weinheim, Cambridge 1989) unter anderem ausgehend vom Ester 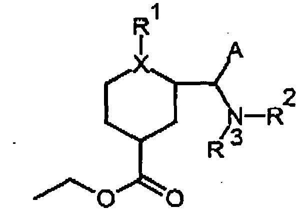 und/oder vom Aldehyd 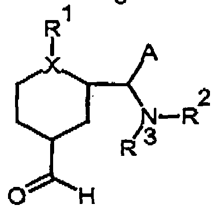 durchgeführt werden. Der Ester 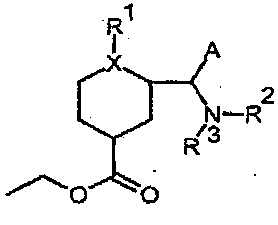 ist dabei nach den oben beschriebenen Verfahren ausgehend vom kommerziell erhältlichen Cydohexanon-4-carbonsäureethylester zügänglich. Der Aldehyd 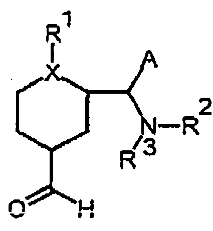 kann aus dem Keton 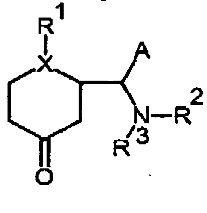 beispielsweise durch Wittig-Reaktion mit Methoxymethylphosphoniumchlorid oder-bromid und saure Spaltung des zunächst gebildeten Enolethers erhalten werden (G. Wittig et al., Chem. Ber. 95 (1962) 2514-2525; S.O. Bhanot et al., J. Chem. Soc. C (1968) 2583-2588).

Ein weiterer Gegenstand der Erfindung ist ein Verfahren zur Behandlung, insbesondere Schmerzbehandlung, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten C-Cyclohexylmethylamin-Derivats, oder eines erfindungsgemäßen Arzneimittels.

Im folgenden wird die Erfindung weiter durch Beispiele erläutert, ohne sie darauf zu beschränken.

### Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:
Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCl etc.) oder synthetisiert.
Die Analytik erfolgte über mittels NMR-Spektroskopie, ESI-Massenspektrometrie und/oder HPLC.

### Grignard-Reaktion 1

### Reaktionsgleichung:

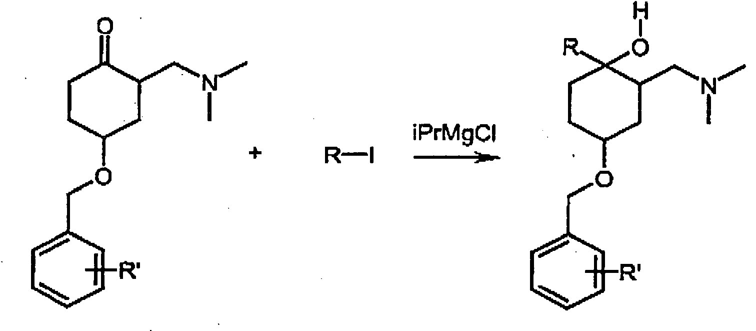

### Allgemeine Arbeitsvorschrift 1 (AAV 1):

Das Reaktionsgefäß wurde im Trockenschrank ausgeheizt. Das Aryliodid wurde vorgelegt und mit 1 ml THF versetzt. Bei -20 °C wurde Isopropylmagnesiumchlorid-Lsg. zugegeben und 60 Minuten gerührt, bevor die Mannichbase sowie weitere 0,25 ml THF zugegeben wurden. Unter Rühren ließ man den Ansatz langsam auf Raumtemperatur auftauen und über Nacht nachrühren. Anschließend wurde erneut auf -20 °C abgekühlt und mit Ammoniumchlorid-Lsg. hydrolisiert.

Das Reaktionsgemisch wurde dreimal mit jeweils 3 ml Ether extrahiert. Die vereinigten organischen Phasen wurden über Magnesiumsulfat getrocknet und bei 40 °C im Vakuum eingeengt. Die Reinigung erfolgte über eine Hydrochloridfällung: Lösen der Rohbase in ca. 10 ml 2-Butanon je Gramm Base; Zugabe eines halben Moläquivalents Wasser, gefolgt von 1,1 Moläquivalenten Chlortrimethylsilan und Rühren über Nacht. Bildete sich auch bei Kühlung auf ca. 4 °C kein Hydrochlorid, wurde der Fällungsansatz im doppelten Volumen Wasser aufgenommen, mit drei kleinen Portionen Ether gewaschen, die wäßrige Phase mit wenig ca. 30 %iger Natronlauge alkalisch gestellt und dreimal mit Ether extrahiert ("Säure-Base-Extraktion"). Diese letzten Extrakte wurden wiederum vereinigt und einer erneuten Hydrochloridfäilung zugeführt.

| | |
|---|---|
| Ansatz A: R' = H | (Beispiel 1 bis 28) |
| | |
| 2.68 mmol (700 mg) | Mannichbase (1,22 M in THF) |
| 5.36 mmol | Aryliodid (R-I) |
| 4,02 mmol (2,01 ml) | Isopropymagnesiumchlorid-Lsg. (2 M in THF) |
| 1,25 ml | THF |
| 2 ml | 20%-ige Ammoniumchlorid-Lsg. |

sowie zur Aufarbeitung Ether und Magnesiumsulfat.

| | |
|---|---|
| Ansatz B: R' = 4-F | (Beispiel 29 bis 50) |
| | |
| 2,15 mmol (600 mg) | Mannichbase (1,00 M in THF) |
| 4,30 mmol | Aryliodid (R-I) |
| 3,22 mmol (1,61 ml) | Isopropymagnesiumchlorid-Lsg. (2 M in THF) |
| 1,25 ml | THF |
| 2 ml | 20%-ige Ammoniumchlorid-Lsg. |

sowie zur Aufarbeitung Ether und Magnesiumsulfat.

| **Beispiel** | | **Aryliodid** | **Ausbeute** | **Reinigung** |
|---|---|---|---|---|
| Nr. | | | g Hydrochlorid | Säure-Base-Extraktion |
| 1 | | 1-Brom-4-lodbenzol | 0.190 | |
| 2 | | 1-Iod-4-trifluormethylbenzol | 0,310 | |
| 3 | | 1,2-Difluor-4-iodbenzol | 0.172 | x |
| 4 | | 2-Fluor-4-iodtoluol | 0.098 | x |
| 5 | | 4-Iod-1,2-dichlorbenzol | 0,340 | |
| 6 | | 1-lodnaphthalin | 0,191 | x |
| 7 | | 4-Iod-1,2-dimethylbenzol | 0,246 | x |
| 8 | | 2-Fluor-5-iodtoluol | 0,167 | x |
| 9 | | 4-Fluor-2-iodtoluol | 0,148 | x |
| 10 | | 1-Chlor-2-fluor-4-iodbenzol | 0,151 | x |
| 11 | | 4-Chlor-2-iodanisol | 0,556 | x |
| 12 | | 1-Brom-2-iodbenzol | 0,208 | x |
| 13 | | 2-lodanisol | 0,644 | |
| 14 | | 1-Iod-2-methylsulfanyl-benzol | 0,665 | |
| 15 | | 1-Chlor-4-iodbenzol | 0,194 | x |
| 16 | | 1-Brom-3-iodbenzol | 0,152 | x |
| 17 | | 1-Brom-2-fluor-4-iodbenzol | 0,287 | x |
| 18 | | 4-Iodanisol | 0,296 | x |
| 19 | | 1-Chlor-2-iodbenzol | 0.218 | x |
| 20 | | 1-Iod-3-trifluormethyl-benzol | 0,240 | x |
| 21 | | 1-Iod-4-pentylbenzol | 0,164 | |
| 22 | | 2-lod-1,4-dimethylbenzol | 0,084 | x |
| 23 | | 1-Iod-4-isopropylbenzol | 0,151 | x |
| 24 | | 1-Iod-2,3-dimethylbenzol | 0,127 | x |
| 25 | | 1-Fluor-3-iodbenzol | 0,195 | x |
| 26 | | 1-Iod-3,5-bis(trifluor-methyl)benzol | 0,220 | |
| 27 | | 1-Ethyl-4-iodbenzol | 0,096 | x |
| 28 | | 3.5-Dichlor5-iodbenzol | 0,349 | |
| 29 | | 2-lodtoluol | 0,083 | x |
| 30 | | 4-Iodtoluol | 0,105 | x |
| 31 | | 2-Iod-1,4-dimethylbenzol | 0,089 | |
| 32 | | 4-Iod-1,2-dimethylbenzol | 0,066 | x |
| 33 | | 1-Iod-3,5-dimethylbenzol | 0,098 | |
| 34 | | 1-Iod-4-isopropylbenzol | 0,129 | x |
| 35 | | 1-Fluor-3-lodbenzol | 0,065 | x |
| 36 | | 1-Fluor-4-iodbenzol | 0,100 | x |
| 37 | | 1,2-Difluor-4-iodbenzol | 0,081 | |
| 38 | | 2-Fluor-4-iodtoluol | 0,161 | |
| 39 | | 2-Fluor-5-lodtoluol | 0,105 | |
| 40 | | 1-Chlor-2-iodbenzol | 0,077 | |
| 41 | | 1-Chlor-3-iodbenzol | 0,212 | |
| 42 | | 1-Chlor-4-iodbanzol | 0,107 | |
| 43 | | 1,2-Dichlor-4-iodbenzol | 0,255 | |
| 44 | | 1-Chlor-2-fluorr-4-iodbenzol | 0,092 | |
| 45 | | 1,3-Dichlor-5-iodbenzol | 0,183 | |
| 46 | | 4-Chlor-2-iodanisol | 0,173 | x |
| 47 | | 1-Iod-4-trifluormethyl-benzol | 0,106 | |
| 48 | | 1-Iod-3,5-bis(trifluormethyl)benzol | 0,087 | |
| 49 | | 2-Iodanisol | 0,243 | x |
| 50 | | 1-Iod-2-methylsulfanylbenzol | 0,240 | x |

### Grignard-Reaktion unter Eliminierung 1

### Reaktionsgleichung:

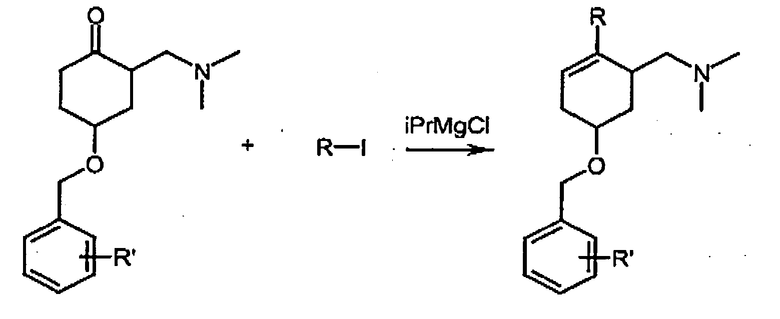

### Durchführung: Siehe AAV 1.

| | |
|---|---|
| Ansatz A: R' = H | (Beispiel 51) |
| | |
| Ansatz B: R' = 4-F | (Beispiel 52) |

### REFERENZ

| **Beispiel** | | **Aryllodid** | **Ausbeute** | **Reinigung** |
|---|---|---|---|---|
| Nr. | | | g Hydrochlorid | Säure-Base-Extraktion |
| 51 | | 2-lodthiophen | 0,308 | x |
| 52 | | 2-Iodthiophen | 0,102 | |

### Acylierung 1

### Reaktionsgleichung:

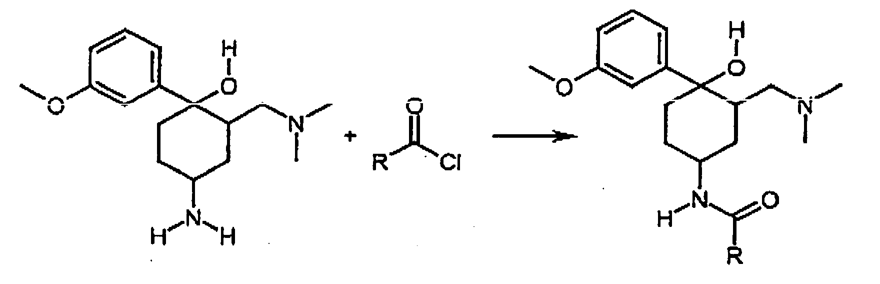

### Allgemeine Arbeitsvorschrift 2 (AAV 2):

Die Apparatur wurde im Trockenschrank ausgeheizt. Das Säurechlorid wurde vorgelegt und mit 1 ml Dichlormethan versetzt. Bei -10 °C wurde Triethylamin zugegeben und 20 Minuten gerührt, bevor das Amin, gelöst in 4 ml Dichlormethan, zugegeben wurde. Unter Rühren ließ man den Ansatz langsam auf Raumtemperatur auftauen und über Nacht nachrühren.
Bei Raumtemperatur wurde verdünnte Kaliumhydroxidlösung zugefügt und anschließend zentrifugiert. Die Phasen wurden getrennt, die organische Phase mittels Magnesiumsulfat getrocknet und bei 40 °C im Vakuum eingeengt. Die Reinigung erfolgte über Hydrochloridfällung, ggf. nach Säure-Base-Extraktion (siehe AAV 1).

| Ansatz A: | (Beispiel 53 bis 62) |
|---|---|
| | |
| 3,59 mmol (1,00 g) | 4-Amino-2-dimethylaminomethyl-1-(3-methoxyphenyl)cyclohexanol |
| 5,39 mmol | Säurechlorid (RCOCI) |
| 9,74 mmol (1,36 ml) | Triethylamin |
| 5 ml | Dichlormethan |
| 2 ml | Kaliumhydroxidlösung (2 M) |

sowie zur Aufarbeitung Magnesiumsulfat.

| Ansatz B: | (Beispiel 63 und 64) |
|---|---|
| | |
| 2,16 mmol (600 mg) | 4-Amino-2-dimethylaminomethyl-1-(3-methoxyphenyl)cyclohexanol |
| 3,23 mmol | Säurechlorid (RCOCl) |
| 4,31 mmol (0,60 ml) | Triethylamin |
| 3 ml | Dichlormethan |
| 1,5 ml | Kaliumhydroxidlösung (2 M) |

sowie zur Aufarbeitung Magnesiumsulfat.

| **Beispiel** | | **Säurechlorid** | **Ausbeute** | **Reinigung** |
|---|---|---|---|---|
| Nr. | | | g Hydrochlorid | Säure-Base-Extraktion |
| 53 | | 3,4-Dichlorbenzoylchlorid | 0,627 | |
| 54 | | 2-Naphthoylchlorid | 0,512 | |
| 55 | | 3-Phenylpropionylchlorid | 0,119 | |
| 56 | | 4-Nitrobenzoylchlorid | 0,975 | |
| 57 | | 4-Methyl-3-nitro-benzoylchlorid | 0,656 | |
| 58 | | 3,4,5-Trimethoxybenzoylchlorid | 0,236 | x |
| 59 | | 4-Chlorphenoxyacetylchlorid | 0,639 | x |
| 60 | | 3-Nitrobenzoylchlorid | 0,302 | x |
| 61 | | 2-Furoylchlorid | 0,746 | x |
| 62 | | Phenoxyacetylchlorid | 0,527 | x |
| 63 | | 4-(Trifluormethyl)benzoylchlorid | 0,212 | x |
| 64 | | 4-Methoxybenzoylchlorid | 0,510 | x |

### Grignard-Reaktion 2

### Reaktionsgleichung:

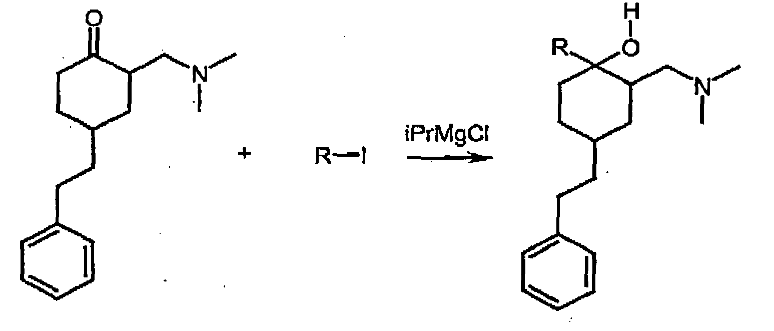

### Allgemeine Arbeitsvorschrift 3 (AAV 3):

Die Apparatur wurde ausgeheizt und mit Stickstoff belüftet. Das Aryliodid wurde vorgelegt und mit 1 ml THF versetzt. Bei -20°C wurde Isopropylmagnesiumchlorid-Lsg. zugegeben und 60 Minuten gerührt, bevor die Mannichbase sowie weitere 0,25 ml THF zugegeben wurden. Unter Rühren ließ man den Ansatz langsam auf Raumtemperatur auftauen und über Nacht nachrühren. Anschließend wurde erneut auf -20°C abgekühlt und mit Ammoniumchlorid-Lsg. hydrolisiert.

Das Reaktionsgemisch wurde dreimal mit jeweils 10 ml Ether extrahiert. Die vereinigten organischen Phasen wurden über Magnesiumsulfat getrocknet und bei 40°C im Vakuum eingeengt. Die Reinigung erfolgte über Säulenchromatographie. Anschließend wurde das gereinigte Produkt als Hydrochlorid gefällt und umkristaflisiert (siehe AAV 1).

| Ansatz: | (Beispiel 65 bis 97) |
|---|---|
| 2,00 mmol (0,52g) | Mannichbase (1,0 M in THF) |
| 4,00 mmol | Aryliodid (R-I) |
| 3,00 mmol (1,73 ml) | Isopropymagnesiumchlorid-Lsg. (2 M in THF) |
| 1,25 ml | THF |
| 2 ml | 20%-ige Ammoniumchlorid-Lsg. |

sowie zur Aufarbeitung Ether und Magnesiumsulfat

| **Beispiel** | | **Aryliodid** | **Ausbeute** | **Reinigung** | |
|---|---|---|---|---|---|
| Nr. | | | g Hydrochlorid | Säulen-chromatographie | Kristallisation |
| 65* | | 1-Fluor-4-iodbenzol | 0,134 | Ether/Hexan/Me OH (25/25/1) | 2-Butanon/ Ether |
| 66* | | 1-Fluor-4-iod-benzol | 0,072 | Ether/Hexan/Me OH (25/25/1) | 2-Butanon/ Ether |
| 67 | | 1-Brom-4-iodbenzol | 0,154 | Ether/Hexan/Me OH (25/25/1) | 2-Butanon/ Ether |
| 68* | | 1-Ethyl-4-iodbenzol | 0,114 | Hexan/Essigester/MeOH (9/3/1) | 2-Butanon/ Ether |
| 69* | | 1-Ethyl-4-iodbenzol | 0,225 | Ether/Hexan/Me OH (25/25/1) | 2-Butanon/ Ether |
| 70 | | 1-Iod-4-isopropylbenzol | 0.093 | Ether/Haxan/Me OH (25/25/1) | 2-Butanon/ Ether |
| 71* | | 4-Iodanisol | 0,175 | Ether/Hexan/Me OH (25/25/1) | 2-Butanon/Ether |
| 72* | | 4-Iodanisol | 0,107 | Ether/Hexan/Me OH (25/25/1) | 2-Butanon/Ether |
| 73 | | 1-Iod-2,4-di methylbenzol | 0,136 | Ether/Hexan/Me OH (25/25/1) | 2-Butanon/Ether |
| 74 | | 1-Iod-2-methyl-sulfanyl-benzol | 0,204 | Hexan/Essig- ester/MeOH (9/3/1) | 2-Butanon/Ether |
| 75 | | 4-Fluor-2-iod 2-Butanon/ toluol | 0,136 | Ether/Hexan/MeOH (25/25/1) | 2-Butanon/Ether |
| 76 | | 2-Iod-1,4-di-methylbenzol | 0,176 | Hexan/Essigester/MeOH (12/3/1) | 2-Butanon/Ether |
| 77 | | 4-Iodtoluol | 0,140 | Ether/Hexan/Me 2-Butanon/ OH (25/25/1) | 2-Butanon/Ether |
| 78 | | 1-Fluor-3-iod-benzol | 0,126 | Hexan/Essig-ester/MeOH (12/3/1) | 2-Butanon/Ether |
| 79 | | 1-Chlor-3-iod-benzol | 0,255 | Ether/Hexan/Me OH (25/25/1) | 2-Butanon/Ether |
| 80 | | 1-Chlor-2-fluor-4-iod-benzol | 0,110 | Ether/Hexan/Me OH (25/25/1) | 2-Butanon/Ether |
| 81 | | 1-Iod-3-trifluormethylbenzol | 0,220 | Ether/Hexan/MeOH (25/25/1) | 2-Butanon/Ether |
| 82 | | 1,2-Dichlor-5-iodbenzol | 0,324 | Ether/Hexan/Me OH (25/25/1) | 2-Butanon/Ether |
| 83 | | 4-Iod-1,2-dimethylbenzol | 0,170 | Ether/Hexan/Me OH (25/25/1) | 2-Butanon/Ether |
| 84 | | 1,2-Dichlor-4-iodbenzol | 0,390 | Ether/Hexan/Me OH (25/25/1) | 2-Butanon/Ether |
| 85 | | 1-Chlor-4-iod-benzol | 0,202 | Ether/Hexan/Me OH (25/25/1) | 2-Butanon/Ether |
| 86 | | 2-Fluor-4-iod-toluol | 0,173 | Ether/Hexan/Me OH (25/25/1) | 2-Butanon/Ether |
| 87 | | 1-Iod-3,5-di-methylbenzol | 0,083 | Ether/Hexan/Me OH (25/25/1) | 2-Butanon/Ether |
| 88 | | 1-Brom-2-fluor-4-iod-benzol | 0,096 | Ether/Hexan/Me 2-Butanon/ OH (25/25/1) | 2-Butanon/Ether |
| 69* | | 1-Iod-2,3-dimethylbenzol | 0,145 | Ether/Hexan/Me 2-Butanon/ OH (25/25/1) | 2-Butanon/Ether |
| 90* | | 1-Iod-2,3-dimethylbenzol 0,096 | 0.096 | Ether/Hexan/Me OH (25/25/1) | 2-Butanon/Ether |
| 91 | | 1-Iod-naphthalin | 0,082 | Ether/Hexan/Me OH (25/25/1) | 2-Butanon/Ether |
| 92 | | 1-Iod-3,5-bis-trifluorethylbenzol | 0,168 | Ether/Hexan/Me OH (25/25/1) | 2-Butanon/Ether |
| 93 | | 1-Brom-3-iodbenzol | 0,081 | Ether/Hexan/Me OH (25/25/1) | 2-Butanon/Ether |
| 94 | | 2-Fluor-5-iod-toluol | 0,216 | Ether/Hexan/Me OH (25/25/1) | 2-Butanon/Ether |
| 95* | | 3-Iodtoluol | 0,079 | Ether/Hexan/Me OH (25/25/1) | 2-Butanon/Ether |
| 96* | | 3-Iodtoluol | 0,088 | Ether/Hexan/Me OH (25/25/1) | 2-Butanon/Ether |

| | | | | | |
|---|---|---|---|---|---|
| Bei den mit einem * gekennzeichneten Beispielen konnten Diastereomere isoliert werden | | | | | |

### Grignard-Reaktion 3

### Reaktionsgleichung:

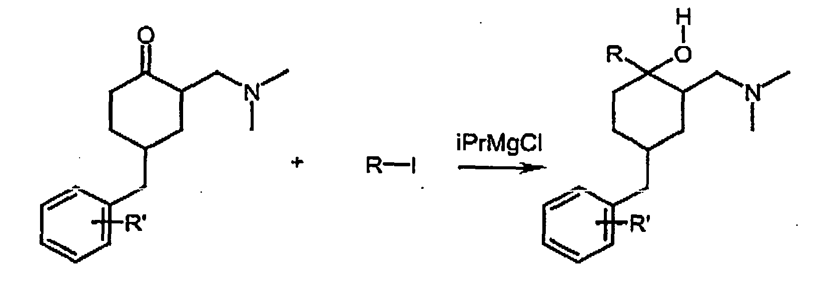

### Durchführung: Siehe AAV 3.

| | | |
|---|---|---|
| Ansatz A: | R' = H | (Beispiel 97 und 99) |
| Ansatz B: | R' = 3-OCH₃ | (Beispiel 100 bis 102) |
| Ansatz C: | R' = 4-F | (Beispiel 103 und 104) |

| **Beispiel** | | **Aryliodid** | **Ausbeute** | **Reinigung** | |
|---|---|---|---|---|---|
| Nr. | | | g Hydrochlorid | Säulenchromatographie | Kristallisation |
| 97 | | 3-Iodtoluol | 0,033 | Ether/Hexan/Me OH (25/25/1) | Ether/ Essigester |
| 98 | | 1.2-Difluor-4-iodbenzol | 0,125 | Ether/Hexan/Me (25/25/1) | Ether/ Essigester |
| 99 | | 4-Iodtoluol | 0,080 | Ether/Hexan/Me OH (25/25/1) | Ether/ Essigester |
| 100 | | 4-Iodtoluol | 0,097 | Ether/Hexan/Me OH (25/25/1) | Ether/ Essigester |
| 101 | | 1-Chlor-4-iodbenzol | 0,104 | Ether/Hexan/Me OH (25/25/1) | Ether Essigester |
| 102 | | 1-Brom-2-fluor-4-iodbenzol | 0,101 | Ether/Hexan/Me OH (25/25/1) | Ether/ Essigester |
| 103 | | 4-Iodtoluol | 0.080 | Ether/Hexan/Me OH (25/25/1) | Ether/ Essigester |
| 104 | | 1-Chlor-4-iodbenzol | 0,080 | Ether/Hexan/Me OH (25/25/1) | Ether/ Essigester |

Grignard-Reaktion unter Eliminierung 2

### Reaktionsgleichung:

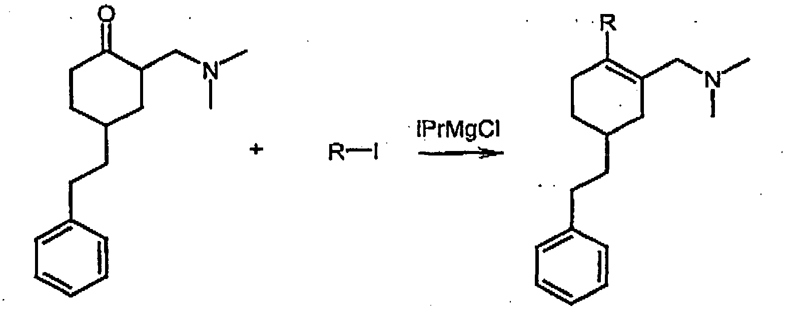

### Durchführung: Siehe AAV 3.

### REFERENZ

| **Beispiel** | | **Aryliodid** | **Ausbeute** | **Reinigung** | |
|---|---|---|---|---|---|
| Nr. | | | g Hydrochlorid | Säulenchromatographie | Kristallisation |
| 105 | | 2-lod-5-methylthiophen | 0.122 | Ether/Hexan/Me OH (25/25/1) | 2-Butanon/Ether |

### Eliminierung 1

### Reaktionsgleichung:

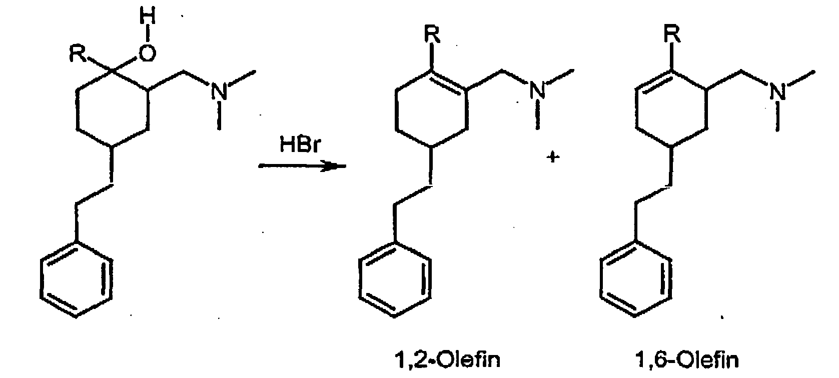

### Allgemeine Arbeitsvorschrift 4 (AAV 4):

Der tertiäre Alokohol wurde vorgelegt und mit Bromwasserstoffsäure versetzt. Bei einer Ölbadtemperatur von 80 °C wurde 4 Stunden gerührt (gegebenenfalls wurde über Nacht bei Raumtemperatur nachgerührt). Anschließend wurde der Ansatz mit 40 g Eis versetzt und unter Kühlung mit Natronlauge auf pH 10-11 eingestellt, bevor mit je ca. 20 ml Essigester dreimal extrahiert wurde. Die organischen Phasen wurden vereinigt, mit Magnesiumsulfat getrocknet und im Vakuum eingeengt. Die Trennung der isomeren Olefine erfolgte über Säulenchromatographie. Anschließend wurde das gereinigte Produkt als Hydrochlorid gefällt und umkristallisiert.

| | |
|---|---|
| Ansatz: | (Beispiel 106 bis 110) |
| 3,00 mmol | tert. Alkohol (als Hydrochlorid) |
| 30,0 ml | 48 %ige Bromwasserstoffsäure |

sowie zur Aufarbeitung 32 %ige Natronlauge und Essigester.

### 1,2-Olefine:

### REFERENZ

| **Beispiel** | **Code** | **Ausbeute** | **Reinigung** | |
|---|---|---|---|---|
| Nr. | | g Hydrochlorid | Säulenchromatographie | Kristallisation |
| 106 | | 0,050 | Ether *l* Hexan / MeOH (25/25/1) | MEK / Ether |
| 107 | | 0,214 | Ether / Hexan / MeOH (25/25/1) | MEK / Ether |
| 108 | | 0,074 | Ether / Hexan *l* MeOH (25/25/1) | MEK / Ether |
| 109 | | 0,098 | Essigester / Hexan / MeOH (3/12/1) | MEK / Ether |

### 1,6-Olefine:

### REFERENZ

| **Beispiel** | | **Ausbeute** | **Reinigung** | |
|---|---|---|---|---|
| Nr. | | g Hydrochlorid | Säulenchromatographie | Kristallisation |
| 110 | | 0,175 | Ether / Hexan/ MeOH (25/25/1) | MEK / Ether MEK / Ether |
| 111 | | 0,027 | Ether / Hexan / MeOH (25/25/1) | MEK / Ether |
| 112 | | 0.161 | Ether / Hexan / MeOH (25/25/1) | MEK / Ether |

### Fluorierung 1

### Reaktionsgleichung:

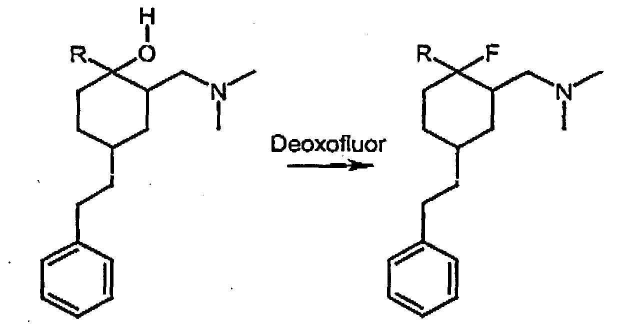

### Allgemeine Arbeitsvorschrift 5 (AAV 5):

Die Apparatur wurde ausgeheizt und mit Stickstoff belüftet. Anschließend wurden 5 ml Dichlormethan vorgelegt und mit Deoxofluor versetzt. Bei einer Temperatur von -15 °C wurde das Edukt, gelöst im restlichen Dichlormethan, langsam zugetropft und 90 Minuten nachgerührt. Unter Kühlung wurde dann etwas Wasser zugegeben und mittels Natriumcarbonat-Lsg. auf pH 11 gestellt. Anschließend wurde dreimal mit Dichlormethan extrahiert. Die vereinigten organischen Phasen wurden mittels Magnesiumsulfat getrocknet und im Vakuum eingeengt. Die Reinigung erfolgte über Säulenchromatographie. Anschließend wurde das gereinigte Produkt als Hydrochlorid gefällt und umkristallisiert.

| | |
|---|---|
| Ansatz: | (Beispiel 113) |
| 1,50 mmol | Edukt (tert. Alkohol) |
| 5,00 mmol | Deoxofluor |
| 15 ml | Dichlormethan (getrocknet) |

sowie zur Aufarbeitung 32 %ige Natronlauge und Essigester.

| **Beispiel** | | **Ausbeute** | **Reinigung** | |
|---|---|---|---|---|
| Nr. | | g Hydro-chlorid | Säulenchromatographie Kristallisation | |
| 113 | | 0,050 | ther / Hexan/ MeOH (25/25/1) | EMEK / Ether |

### Fluorierung 2

### Reaktionsgleichung:

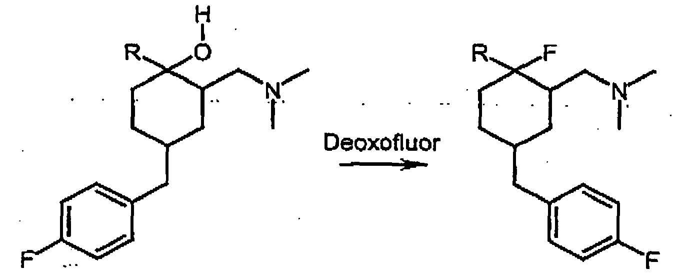

### Durchführung: Siehe AAV 5:

**Beispiel Ausbeute Reinigung** Nr. g Hydro- Säulenchromatographie Kristallisation chlorid 114 0,032 Essigester Ether

### Grignard-Reaktion 4

### Reaktionsgleichung:

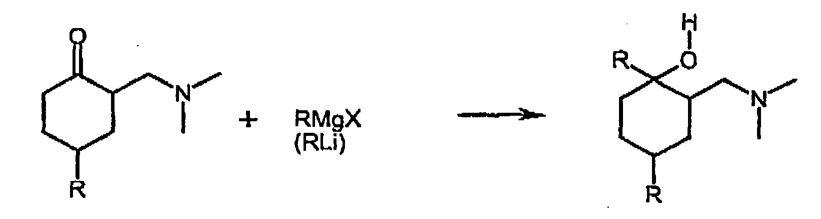

### Allgemeine Arbeitsvorschrift 6 (AAV 6)

In einem ausgeheizten und unter Inertgas auf - 10°C abgekühlten Reaktionsgefäß wurde die in THF gelöste Mannichbase (400µl, 0,5 M) vorgelegt. Unter Rühren wurden daraufhin 2 Äquivalente des vorbereiteten Grignard- oder Organolithium-Reagenzes zugegeben (0,5 M in THF oder Diethylether, 800 µl). Das Reaktionsgemisch wurde bei Raumtemperatur gerührt. Nach drei Stunden wurde erneut auf-10°C gekühlt und mit Ammoniumchlorid-Lösung hydrolisiert.
Das Reaktionsgemisch wurde zweimal mit Ethylacetat extrahiert und bei 40°C im Vakuum eingeengt.

Zur Charakterisierung wurde ein ESI-MS aufgenommen.

Die eingesetzten Grignard- oder Organolithium-Reagenzien wurden hergestellt aus:
Phenylmagnesiumbromid
4-Chlorphenylmagnesiumbromid
Benzylmagnesiumchlorid
4-Fluor-3-methylphenylmagnesiumbromid
o-Tolylmagnesiumbromid
Vinylmagnesiumbromid
4-t-Butylphenylmagnesiumbromid
Cyclopentylmagnesiumchlorid
m-Tolylmagnesiumchlorid
Cyclohexylmagnesiumchlorid
4-Fluorphenylmagnesiumbromid
Phenethylmagnesiumbromid
Lithiumphenylacetylid
2-Thienyllithium
1-Brommagnesium-2,4-dichlorbenzol
3-Bromanisolmagnesiumbromid
Phenylpropylmagnesiumbromid
2,3-Dichlorphenylmagnesiumbromid
p-Toluylmagnesiumbromid
4-Bromanisolmagnesiumbromid
Cyclohexylmethylmagnesiumbromid
2-Brommagnesium-4-fluoranisol
3-Flourphenylmagnesiumbromid
3-Chlorphenylmagnesiumbromid
3,5-Dichlorphenylmagnesiumbromid
2-Chlorbenzylmagnesiumchlorid
4-Fluorbenzylmagnesiumchlorid
3-Methoxybenzylmagnesiumchlorid
5-Brommagnesium-2-chlorbenzotrifluorid
3-Fluorbenzylmagnesiumchlorid
2-Methoxyphenylmagnesiumbromid
2-Methylbenzylmagnesiumchlorid
3-Chlor-4-fluorphenylmagnesiumbromid
3-Brommagnesiumbenzotrifluorid
3-Methylbenzylmagnesiumchlorid
4-Chlorbenzylmagnesiumchlorid
2-Chlor-6-fluorbenzylmagnesiumchlorid
2,5-Dimethylbenzylmagnesiumchlorid
3-Chlorbenzylmagnesiumchlorid
2,4-Dichlorbenzylmagnesiumchlorid
2-Brommethyl-1,4-dimethylbenzol
4-Brom-1-chlor-2-trifluormethylbenzol

| **Beispiel** | **Name** | **berechnete Masse** | **gefundene Masse** |
|---|---|---|---|
| 115 | 1-Benzyl-2-(dimethylamino-phenyl-methyl)-4-phenyl-cyclohexanol | 399.57 | 400,4 |
| 116 | 2-(Dimethylamino-phenyl-methyl)-4-phenyl-1-vinyl-cyclohexanol | 335.49 | 336,3 |
| 117 | 1-(4-*tert*-Butyl-phenyl)-2-(dimethylamino-phenyl-methyl)-4-phenyl-cyclohexanol | 441.65 | 442,4 |
| 118 | 2-(Dimethylamino-phenyl-methyl)-4-phenyl-1-m-tolyl-cyclohexanol | 399.57 | 400,3 |
| 119 | 2-(Dimethylamino-phenyl-methyl)-1-phenethyl-4-phenyl-cyclohexanol | 413.6 | 414,5 |
| 120 | 2-(Dimethylamino-phenyl-methyl)-4-phenyl-1-phenylethynyl-cyclohexanol | 409.57 | 410,3 |
| 121 | 2-(Dimethylamino-phenyl-methyl)-1-(3-methoxy-phenyl)-4-phenyl-cyclohexanol | 415.57 | 416,3 |
| 122 | 2-(Dimethylamino-phenyl-methyl)-4-phenyl-1-(3-Phenyl-propyl)-cyclohexanol | 427.63 | 428,5 |
| 123 | 2-(Dimethylamino-phenyl-methyl)-1-(4-methoxy-phenyl)-4-phenyl-cyclohexanol | 415.57 | 416,3 |
| 124 | 2-(Dimethylamino-phenyl-methyl)-1-(2-methoxy-phenyl)-4-phenyl-cyclohexanol | 415.57 | 416,3 |
| 125 | 4-Benzyloxy-2-dimethylaminomethyl-1-phenyl-cyclohexanol | 339.47 | 340,4 |
| 126 | 1-Benzyl-4-benzyloxy-2-dimethylaminomethyl-cyclohexanol | 353.5 | 354,4 |
| 127 | 4-Benzyloxy-2-dimethylaminomethyl-1-(4-fluor-3-methyl-phenyl)-cyclohexanol | 371.49 | 372,4 |
| 128 | 4-Benzyloxy-2-dimethylaminomethyl-1-o-tolyl-cyclohexanol | 353.5 | 354,4 |
| 129 | 4-Benzyloxy-2-dimethylamlnomethyl-1-vinyl-cyclohexanol | 289.41 | 290,3 |
| 130 | 4-Benzyloxy-1-cyclopentyl-2-dimethylaminomethyl-cyclohexanol | 331.49 | 332,4 |
| 131 | 4-Benzyloxy-2-dimethylaminomethyl-1-m-tolyl-cyclohexanol | 353.5 | 354,4 |
| 132 | 4-Benzyloxy-2-dimethylaminomethyl-bicyclohexyl-1-ol | 345.52 | 346,4 |
| 133 | 4-Benzyloxy-2-dimethylaminomethyl-1-(4-fluor-phenyl)-cyclohexanol | 357.46 | 358,3 |
| 134 | 4-Benzyloxy-2-dimethylaminomethyl-1-phenylethyl-cyclohexanol | 363.5 | 364,3 |
| 135 | 4-Benzyloxy-2-dimethylaminomethyl-1-thiophen-2-yl-cyclohexanol | 345.5 | 346,4 |
| 136 | 4-Benzyloxy-2-dimethylaminomethyl-1-(3-methoxy-phenyl)-cyclohexanol | 369.5 | 370,4 |
| 137 | 4-Benzyloxy-2-dimethylaminomethyl-1-(3-phenyl-propyl)-cyclohexanol | 381.55 | 382,4 |
| 138 | 4-Benzyloxy-2-dimethylaminomethyl-1-p-tolyl-cyclohexanol | 353.5 | 354,4 |
| 139 | 4-Benzyloxy-2-dimethylaminomethyl-1-(4-methoxy-phenyl)-cyclohexanol | 369.5 | 370,3 |
| 140 | 4-Benzyloxy-2-dimethylaminomethyl-1-(3-fluor-phenyl)-cyclohexanol | 357.46 | 358,4 |
| 141 | 4-Benzyloxy-1-(3-chlor-phenyl)-2-dimethylaminomethyl-cyclohexanol | 373.92 | 374,4 |
| 142 | 4-Benzyloxy-2-dimethylaminomethyl-1-(3-methoxy-benzyl)-cyclohexanol | 383.53 | 384,4 |
| 143 | 4-Benzyloxy-1-(4-chlor-3-trifluormethyl-phenyl)-2-dimethylaminomethyl-cyclohexanol | 441.92 | 442,3 |
| 144 | 4-Benzyloxy-2-dimethylaminomethyl-1-(3-fluor-benzyl)-cyclohexanol | 371.49 | 372,4 |
| 145 | 4-Benzyloxy-2-dimethylamlnomethyl-1-(2-methyl-benzyl)-cyclohexanol | 367.53 | 368,4 |
| 146 | 4-Benzyloxy-2-dimethylaminomethyl-1-(2,5-dimethyl-benzyl)-cyclohexanol | 381.55 | 382,4 |
| 147 | 4-(4-Chlor-benzyl)-2-dimethylaminomethyl-1-(3-methoxy-phenyl)-cyclohexanol | 387.95 | 388,8 |
| 148 | 4-(4-Chlor-benzyl)-2-dimethylaminomethyl-1-(3-phenyl-propyl)-cyclohexanol | 400 | 400,8 |
| 149 | 4-(4-Chlor-benzyl)-1-(2,3-dichlor-phenyl)-2-dimethylaminomathyl-cyclohexanol | 426.81 | 427,0/428, 9 |
| 150 | 4-(4-Chlor-benzyl)-1-cyclohexylmethyl-2-dimethylaminomethyl-cyclohexanol | 377.99 | 378,8 |
| 151 | 4-(4-Chlor-benzyl)-2-dimethylaminamethyl-1-(5-fluor-2-methoxy-phenyl)-cyclohexanol | 405.94 | 406,7 |
| 152 | 4-(4-Chlor-benzyl)-2-dimethylaminomethyl-1-(3-fluor-phenyl)-cyclohexanol | 375.91 | 376,7 |
| 153 | 4-(4-Chlor-benzyl)-1-(3-chlor-phenyl)-2-dimethylaminomethyl-cyclohexanol | 392.36 | 393,0 |
| 154 | 4-(4-Chlor-benzyl)-(3,5-dichlor-phenyl)-2-dimethylaminomethyl-cyclohexanol | 426.81 | 426,5/428, 3 |
| 155 | 4-(4-Chlor-benzyl)-1-(2-chlor-benzyl)-2-dimethylaminomethyl-cyclohexanol | 406.39 | 406,9 |
| 156 | 4-(4-Chlor-benzyl)-2-dimethylaminomethyl-1-(4-fluor-benzyl)-cyclohexanol | 389.94 | 390,8 |
| 157 | 4-(4-Chlor-benzyl)-2-dimethylaminomethyl-1-(3-fluor-benzyl)-cyclohexanol | 389.94 | 390,7 |
| 158 | 4-(4-Chlor-benzyl)-2-dimethylaminomethyl-1-(2-methoxy-phenyl)-cyclohexanol | 387.95 | 388,8 |
| 159 | 4-(4-Chlor-benzyl)-2-dimethylaminomethyl-1-(2-methyl-benzyl)-cyclohexanol | 385.97 | 386,8 |
| 160 | 4-(4-Chlor-benzyl)-2-dimethylaminomethyl-1-(3-methyl-benzyl)-cyclohexanol | 385.97 | 386,7 |
| 161 | 1,4-Bis-(4-chlor-benzyl)-2-dimethylaminomethyl-cyclohexanol | 406.39 | 407,1 |
| 162 | 4-(4-Chlor-benzyl)-1-(2-chlor-6-fluor-benzyl)-2-dimethylaminomethyl-cyclohexanol | 424.38 | 425,1 |
| 163 | 4-(4-Chlor-benzyl)-2-dimethylaminomethyl-1-(2,5-dimethyl-benzyl)-cyclohexanol | 400 | 400,8 |
| 164 | 4-(4-Chlor-benzyl)-(3-chlor-benzyl)-2-dimethylaminomethyl-cyclohexanol | 406.39 | 407,0 |
| 165 | 4-(4-Chlor-benzyl)-1-(2,4-dichlor-benzyl)-2-dimethylaminomethyl-cyclohexanol | 440.84 | 440,9/442, 4 |
| 166 | 4-(4-Chlor-benzyl)-2-dimethylaminomethyl-1-phenyl-cyclohexanol | 357.92 | 358,5 |
| 167 | 4-(4-Chlor-benzyl)-1-(4-chlor-phenyl)-2 dimethylaminomethyl-cyclohexanol | 392.36 | 392,7/394,5 |
| 168 | 4-(4-Chlor-benzyl)-2-dimethylaminomethyl-1-(4-fluor-3-methyl-phenyl)-cyclohexanol | 389.94 | 390,6 |
| 169 | 4-(4-Chlor-benzyl)-2-dimethylaminomethyl-1-o-tolyl-cyclohexanol | 371.95 | 372,5 |
| 170 | 4-(4-Chlor-benzyl)-2-dimethylaminomethyl-1-vinyl-cyclohexanol | 307.86 | 308,5 |
| 171 | 4-(4-Chlor-benzyl)-1-cyclopentyl-2-dimethylaminomethyl-cyclohexanol | 349.94 | 350,3 |
| 172 | 4-(4-Chlor-benzyl)-2-dimethylaminomethyl-1-m-tolyl-cyclohexanol | 371.95 | 372,5 |
| 173 | 4-(4-Chlor-benzyl)-2-dimethylaminomethyl-bicyclohexyl-1-ol | 363.97 | 364,4 |
| 174 | 4-(4-Chlor-benzyl)-2-dimethylaminomethyl-1-(4-fluor-phenyl)-cyclohexanol | 375.91 | 376,5 |
| 175 | 4-(4-Chlor-benzyl)-2-dimethylaminomethyl-1-phenethyl-cyclohexanol | 385.97 | 386,5 |
| 176 | 4-(4-Chlor-benzyl)-2-dimethylaminomethyl-1-phenylethynyl-cyclohexanol | 381.94 | 382,5 |
| 177 | 4-(4-Chlor-benzyl-2-dimethylaminomethyl-1-thiophen-2-yl-cyclohexanol | 363.95 | 364,4 |
| 178 | 4-(4-Chlor-benzyl)-2-dimethylaminomethyl-1-p-tolyl-cyclohexanol | 371.95 | 372,6 |
| 179 | 4-(4-Chlor-benzyl)-2-dimethylaminomethyl-1-(4-methoxy-phenyl)-cyclohexanol | 387.95 | 388,6 |
| 180 | 4-(4-Chlor-benzyl)-2-dimethylaminomethyl-1-trimethylsilanylethynyl-cyclohexanol | 378.03 | 378,7 |
| 181 | 4-(4-Chlor-benzyl)-1-(4-chlor-3-trifluormethyl-phenyl)-2- dimethylaminomethyl-cyclohexanol | 460.36 | 460,8/462,1 |
| 182 | 4-(4-Chlor-benzyl)-1-(3-chlor-4-fluor-phenyl)-2-dimethylaminomethyl-cyclohexanol | 410.35 | 410,6/412,4 |
| 183 | 4-(4-Chlor-benzyl)-2-dimethylaminomethyl-1-(3-trifluormethyl-phenyl)-cyclohexanol | 425.92 | 426,6 |
| 184 | 2-Dimethylaminomethyl-4-(4-fluor-benzyl)-1-phenyl-cyclohexanol | 341.46 | 342,4 |
| 185 | 1-(4-chlor-phenyl)-2-dimethylaminomethyl-4-(4-fluor-benzyl)-cyclohexanol | 375.91 | 376,5 |
| 186 | 1-Benzyl-2-dimethylaminomethyl-4-(4-fluor-benzyl)-cyclohexanol | 355.49 | 356,4 |
| 187 | 2-Dimethylaminomethyl-4-(4-fluor-benzyl)-1-(4-fluor-3-methyl-phenyl)-cyclohexanol | 373.48 | 374,4 |
| 188 | 2-Dimethylaminomethyl-4-(4-fluor-benzyl)-1-o-tolyl-cyclohexanol | 355.49 | 356,4 |
| 189 | 2-Dimethylaminomethyl-4-(4-fluor-benzyl)-1-vinyl-cyclohexanol | 291.4 | 292,3 |
| 190 | 1-(4-*tert*-Butyl-phenyl)-2-dimethylaminomethyl-4-(4-fluor-benzyl)-cyclohexanol | 397.57 | 398,4 |
| 191 | 1-Cyclopentyl-2-dimethylaminomethyl-4-(4-fluor-benzyl)-cyclohexanol | 333.49 | 334,5 |
| 192 | 2-Dimethylaminomethyl-4-(4-fluor-benzyl)-1-m-tolyl-cyclohexanol | 355.49 | 356,3 |
| 193 | 2-Dimethylaminomethyl-4-(4-fluor-benzyl)-bicyclohexyl-1-ol | 347.51 | 348,4 |
| 194 | 2-Dimethylaminomethyl-4-(4-fluor-benzyl)-1-(4-fluor-phenyl)-cyclohexanol | 359.45 | 360,4 |
| 195 | 2-Dimethylaminomethyl-4-(4-fluor-benzyl-phenethyl-cyclohexanol | 369.52 | 370,4 |
| 196 | 2-Dimethylaminomethyl-4-(4-fluor-benzyl)-1-phenylethynyl-cyclohexanol | 365.49 | 366,3 |
| 197 | 2-Dimethylaminomethyl-4-(4-fluor-benzyl)-thiophen-2-yl-cyclohexanol | 347.49 | 348,3 |
| 198 | 2-Dimethylaminomethyl-4-(4-fluor-benzyl)-1-(3-methoxy-phenyl)-cyclohexanol | 371.49 | 372,3 |
| 199 | 2-Dimethylaminomethyl-4-(4-fluor-benzyl)-1-(3-phenyl-propyl)-cyclohexanol | 383.55 | 384,4 |
| 200 | 1-(2,3-Dichlor-phenyl)-2-dimethylaminomethyl-4-(4-fluor-benzyl)-cyclohexanol | 410.35 | 410,6/412,4 |
| 201 | 2-Dimethylaminomethyl-4-(4-fluor-benzyl)-p-tolyl-cyclohexanol | 355.49 | 356,4 |
| 202 | 2-Dimethylaminomethyl-4-(4-methoxy-phenyl)-cyclohexanol | 371.49 | 372,3 |
| 203 | 1-Cyclohexylmethyl-2-dimethylaminomethyl-4-(4-fluor-benzyl)-cyclohexanol | 361.54 | 362,4 |
| 204 | 2-Dimethylaminomethyl-(4-fluor-benzyl)-1-(5-fluor-2-methoxy-phenyl)-cyclohexanol | 389.48 | 390,4 |
| 205 | 2-Dimethylaminomethyl-4-(4-fluor-benzyl)-1-(3-flour-phenyl)-cyclohexanol | 359.45 | 360,4 |
| 206 | 1-(3-chlor-phenyl)-2-dimethylaminomethyl-4-(4-fluor-benzyl)-cyclohexanol | 375.91 | 376,5/377,5 |
| 207 | 1-(3,5-Dichlor-phepyl)-2-dimethylaminomethyl-4-(4-fluor-benzyl)-cyclohexanol | 410.35 | 410,6/412,3 |
| 208 | 1-(2-chlor-benzyl)-2-dimethylaminomethyl-(4-fluor-benzyl)-cyclohexanol | 389.94 | 390,7 |
| 209 | 2-Dimethylaminomethyl-1,4-bis-(4-fluor-benzyl)-cyclohexanol | 373.48 | 374,5 |
| 210 | 2-Dimethylaminomethyl-4-(4-fluor-benzyl)-1-(3-methoxy-benzyl)-cyclohexanol | 385.52 | 386,4 |
| 211 | 1-(4-chlor-3-trifluormethyl-phenyl)2-dimethylaminomethyl-4-(4-fluor-benzyl)-cyclohexanol | 443.91 | 444,5/445,4 |
| 212 | 2-Dimethylaminomethyl-4-(4-fluor-benzyl-1-(3-fluor-benzyl)-cyclohexanol | 373.48 | 374,4 |
| 213 | 2-Dimethylaminomethyl-4-(4-fluor-benzyl)-1-(2-methoxy-phenyl)-cyclohexanol | 371.49 | 372,4 |
| 214 | 2-Dimethylaminomethyl-4-(4-fluor-benzyl)-1-(2-methyl-benzyl)-cyclohexanol | 369.52 | 370,4 |
| 215 | 1-(3-chlor-4-fluor-phenyl)-2-dimethylaminomethyl-(4-fluor-benzyl)-cyclohexanol | 393.9 | 394,5 |
| 216 | 2-Dimethylaminomethyl-4-(4-fluor-benzyl)-1-(3-trifluormethyl-phenyl)-cyclohexanol | 409.46 | 410,5 |
| 217 | 2-Dimethylaminomethyl-4-(4-fluor-benzyl)-1-(3-methyl-benzyl)-cyclohexanol | 369.52 | 370,4 |
| 218 | 1-(4-chlor-benzyl)2-dimethylaminomethyl-4-(4-fluor-benzyl)-cyclohexanol | 389.94 | 390,5 |
| 219 | 1-(2-chlor-6-fluor-benzyl)-2-dimethylaminomethyl-4-(4-fluor-benzyl)-cyclohexanol | 407.93 | 408,5 |
| 220 | 2-Dimethylaminomethyl-1-(2,5-dimethyl-benzyl)4-(4-fluor-benzyl)-cyclohexanol | 383.55 | 384,4 |
| 221 | 1-(3-chlor-benzyl)-2-dimethylaminomethyl-4-(4-fluor-benzyl)-cyclohexanol | 389.94 | 390,4/391,3 |
| 222 | 1-(2,4-Dichlor-benzyl)-2-dimethylaminomethyl-4-(4-fluor-benzyl)-cyclohexanol | 424.38 | 424,5/426,3 |
| 223 | 2-Dimethylaminomethyl-4-(3-methoxy-benzyl)-1-phenyl-cyclohexanol | 353.5 | 354,5 |
| 224 | 1-Benzyl-2-dimethylaminomethyl-4-(3-methoxy-benzyl)-cyclohexanol | 367.53 | 368,6 |
| 225 | 2-Dimethylaminomethyl-1-(4-fluor-3-methyl-phenyl)-4-(3-methoxy-benzyl)-cyclohexanol | 385.52 | 386,4 |
| 226 | 2-Dimethylaminomethyl-4-(3-methoxy-benzyl)-1-o-tolyl-cyclohexanol | 367.53 | 368,5 |
| 227 | 2-Dimethylaminomethyl-4-(3-methoxy-benzyl)-1-vinyl-cyclohexanol | 303.44 | 304,3 |
| 228 | 1-(4-*tert*-Butylphenyl)-2-dimethylaminomethyl-4-(3-methoxy-benzyl)-cyclohexanol | 409.61 | 410,7 |
| 229 | 1-Cyclopentyl-2-dimethylaminomethyl-4-(3- methoxy-benzyl)-cyclohexanol | 345.52 | 346,4 |
| 230 | 2-Dimethylaminomethyl-4-(3-methoxy-benzyl)-1-m-tolyl-cyclohexanol | 367.53 | 368,4 |
| 231 | 2-Dimethylaminomethyl-4-(3-methoxybenzyl)-bicyclohexyl-1-ol | 359.55 | 360,4 |
| 232 | 2-Dimethylaminomethyl-4-(3-methoxy-benzyl)-phenethyl-cyclohexanol | 381.55 | 382,4 |
| 233 | 2-Dimethylaminomethyl-4-(3-methoxybenzyl)-1-phenylethynyl-cyclohexanol | 377.52 | 378,4 |
| 234 | 2-Dimethylaminomethyl-4-(3-methoxybenzyl)-1-thiophen-2-yl-cyclohexanol | 359.53 | 360,3 |
| 235 | 2-Dimethylaminomethyl-4-(3-methoxy-benzyl)-1-(3-methoxy-phenyl)-cyclohexanol | 383.53 | 384,4 |
| 236 | 2-Dimethylaminomethyl-4-(3-methoxy-benzyl)-1-(3-phenyl-propyl)-cyclohexanol | 395.58 | 396,6 |
| 237 | 1-(2,3-Dichlor-phenyl)-2-dimethylaminomethyl-4-(3-methoxy-benzyl)-cyclohexanol | 422.39 | 422,8/424,5 |
| 238 | 2-Dimethylaminomethyl-4-(3-methoxy-benzyl)-1-p-tolyl-cyclohexanol | 367.53 | 368,5 |
| 239 | 1-Cyclohexylmethyl-2-dimethylaminomethyl-4-(3-methoxy-benzyl)-cyclohexanol | 373.58 | 374,8 |
| 240 | 2-Dimethylaminomethyl-1-(5-fluor-2-methoxy-phenyl)-4-(3-methoxy-benzyl)-cyclohexanol | 401.52 | 402,5 |
| 241 | 1-(3-chlor-phenyl)-2-dimethylaminomethyl-4-(3-methoxy-benzyl)-cyclohexanol | 387.95 | 388.5 |
| 242 | 1-(3,5-Dichlor-phenyl)-2-dimethylaminomethyl-4-(3-methoxy-benzyl)-cyclohexanol | 422.39 | 422,7/424,5 |
| 243 | 1-(4-chlor-3-trifluormethyl-phenyl)-2-dimethylaminomethyl-4-(3-methoxy-benzyl)-cyclohexanol | 455.94 | 456,6/457,4 |
| 244 | 2-Dimethylaminomethyl-1-(3-fluor-benzyl)-4-(3-methoxy-benzyl)-cyclohexanol | 385.52 | 386,4 |
| 245 | 2-Dimethylaminomethyl-4-(3-methoxy-benzyl)-1-(2-methoxy-phenyl)-cyclohexanol | 383.53 | 384,4 |
| 246 | 2-Dimethylaminomethyl-4-(3-methoxy-benzyl)-1-(3-trifluormethyl-phenyl)-cyclohexanol | 421.5 | 422,6 |
| 247 | 1-(2-chlor-6-fluor-benzyl)-2-dimethylaminomethyl-4-(3-methoxy-benzyl)-cyclohexanol | 419.96 | 420,9/422,6 |
| 248 | 2-Dimethylaminomethyl-1-(2,5-dimethyl-benzyl)-4-(3-methoxy-benzyl)-cyclohexanol | 395.58 | 396,7 |
| 249 | 1-(3-chlor-benzyl)-2-dimethylaminomethyl-4-(3-methoxy-benzyl)-cyclohexanol | 401.97 | 402,9 |
| 250 | 2-Dimethylaminomethyl-4-(4-fluor-benzyloxy)-1-phenyl-cyclohexanol | 357.46 | 358,4 |
| 251 | 1-Benzyl-2-dimethylaminomethyl-4-(4-fluor-benzyloxy)-cyclohexanol | 371.49 | 372,8 |
| 252 | 2-Dimethylaminomethyl-4-(4-fluor-benzyloxy)-1-(4-fluor-3-methyl-phenyl)-cyclohexanol | 389.48 | 390,4 |
| 253 | 2-Dimethylaminomethyl-4-(4-fluor-benzyloxy)-1-vinyl-cyclohexanol | 307.4 | 308,6 |
| 254 | 1-(4-*tert*-Butyl-phenyl)-2-dimethylaminomethyl-4-(4-fluor-benzyloxy)-cyclohexanol | 413.57 | 414,6 |
| 255 | 1-Cyclopentyl-2-dimethylaminomethyl-4-(4-fluor-benzyloxy)-cyclohexanol | 349.48 | 350,4 |
| 256 | 2-Dimethylaminomethyl-4-(4-fluor-benzyloxy)-1-m-tolyl-cyclohexanol | 371.49 | 372,5 |
| 257 | 2-Dimethylaminomethyl-4-(4-fluor-benzyloxy)-bicyclohexyl-1-ol | 363.51 | 364,6 |
| 258 | 2-Dimethylaminomethyl-4-(4-fluor-benzyloxy)-1-phenethyl-cyclohexanol | 385.52 | 386,5 |
| 259 | 2-Dimethylaminomethyl-4-(4-fluor-benzyloxy)-1-phenylethynyl-cyclohexanol | 381.49 | 382,6 |
| 260 | 2-Dimethylaminomethyl-4-(4-fluor benzyloxy)-1-thiophen-2-yl-cyclohexanol | 363.49 | 364,6 |
| 261 | 2-Dimethylaminomethyl-4-(4-fluorbenzyloxy)-1-(3-methoxy-phenyl)-cyclohexanol | 387.49 | 388,6 |
| 262 | 2-Dimethylaminomethyl-4-(4-fluorbenzyloxy)-1-p-tolyl-cyclohexanol | 371.49 | 372,6 |
| 263 | 4-Cyclohexylmethyl-2-dimethylaminomethyl-4-(4-fluor-benzyloxy)-cyclohexanol | 377.54 | 379,0 |
| 264 | 2-Dimethylaminomethyl-1-(3-fluor-benzyl)-4-(4-fluor-benzyloxy)-cyclohexanol | 389.48 | 390,7 |
| 265 | 2-Dimethylaminomethyl-4-(4-fluor-benzyloxy)-1-(2-methoxy-phenyl)-cyclohexanol | 387.49 | 388,8 |
| 266 | 2-Dimethylaminomethyl-4-(4-fluor-benzyloxy)-(3-methyl-benzyl)-cyclohexanol | 385.52 | 387,0 |
| 267 | 2-Dimethylaminomethyl-1-(2,5-dimethyl-benzyl)4-(4-fluor-benzyloxy)-cyclohexanol | 399.54 | 400,9 |
| 268 | 1-(3-Chlor-benzyl)-2-dimethylaminomethyl-4-(4-fluor-benzyloxy)-cyclohexanol | 405.94 | 406,8 |
| 340 | 4-Benzyloxy-2-dimethylaminomethyl-1-(3-phenyl-propyl)-cyclohexanol; Hydrochlorid | | |
| 341 | 2-Dimethylaminomethyl-4-(4-fluor-benzyloxy)-1-(3-phenyl-propyl)-cyclohexanol; Hydrochlorid | | |
| 342 | 2-Dimethylaminomethyl-1-(3-fluorbenzyl)-4-(4-fluorbenzyloxy)-cyclohexanol; Hydrochlorid | | |
| 343 | 4-Benzyloxy-2-dimethylaminomethyl-1-(3-fluor-benzyl)-cyclohexanol; Hydrochlorid | | |
| 344 | 2-Dimethylaminomethyl-4-(4-fluorbenzyloxy)-p-tolyl-cyclohexanol; Hydrochlorid | | |
| 345 | 4-Benzyloxy-2-dimethylaminomethyl-1-p-tolyl-cyclohexanol; Hydrochlorid | | |
| 346 | 2-Dimethylaminomethyl-1-(2,5-dimethyl-benzyl)-4-(4-fluor-benzyloxy)-cyclohexanol; Hydrochlorid | | |
| 347 | 4-Benzyloxy-2-dimethylaminomethyl-1-(2,5-dimethyl-benzyl)-cyclohexanol; Hydrochlorid | | |
| 348 | 1-(4-Chlor-3-trifluormethyl-phenyl)-2-dimethylaminomethyl-4-(4-fluor-benzyloxy)-cyclohexanol; Hydrochlorid | | |
| 349 | 4-Benzyloxy-1-(4-chlor-3-trifluormethyl-phenyl)-2-dimethylaminomethyl-cyclohexanol; Hydrochlorid | | |

### Alkylierung

### Reaktionsgleichung:

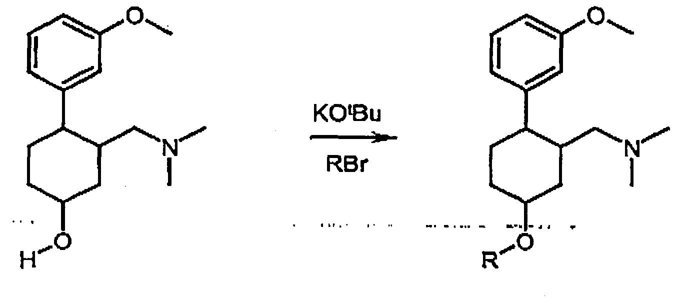

Allgemeine Arbeitsvorschrift 7 (AAV 7):
360 mg 3-Dimethylaminomethyl-4-(3-methoxyphenyl)cyclohexanol Hydrochlorid wurden in 3 ml THF p.a. vorgelegt, 340 mg Kalium-tert.-butylat (2,5 Moläquivalente) zugegeben und 15 Minuten nachgerührt. Anschließend wurde das entsprechende substituierte Benzylbromid (1,5 Moläquivalente) gelöst in 0,6 ml THF p.a. zugetropft und 16 Stunden bei Raumtemperatur nachgerührt.
Zur Aufarbeitung wurden 2,2 ml Wasser zugegeben, zweimal mit je 10 ml Ethylacetat extrahiert, die vereinigten Extrakte über Natriumsulfat getrocknet, filtriert und eingeengt Die Rohprodukte wurden mit Ethylacetat/Methanol/Hexan (V/V/V = 1:1:1) an Kieselgel chromatographiert. Die erhaltenen Produkte wurden in Analogie zur AAV 1 mit Chlortrimethylsilan in wäßrigem 2-Butanon in die korrespondierenden Hydrochloride überführt.
Bei der Herstellung von [5-(4-Methoxybenzyloxy)-2-(3-methoxyphenyl)cyclohexylmethyl]dimethylamin wurde das Cyclohexenderivat [5-(4-Methoxy-benzyloxy)-2-(3-methoxyphenyl)cyclohex-2-enylmethyl]dimethylamin als Nebenprodukt isoliert, da das eingesetzte 3-Dimethylaminomethyl-4-(3-methoxyphenyl)cyclohexanol Hydrochlorid mit der Vorstufe 5-Dimethylaminomethyl-4-(3-methoxy-phenyl)-cyclohex-3-enol Hydrochlorid verunreinigt war, die zur Herstellung von 3-Dimethylaminomethyl-4-(3-methoxyphenyl)cyclohexanol katalytisch über Palladium hydriert wurde.

**Beispiele gemäß AAV 7:**

| | |
|---|---|
| 269 | [5-Benzyloxy-2-(3-methoxy-phenyl)-cyclohexylmethyl]-dimethyl-amin; Hydrochlorid |
| 270 | [5-(3-Chlor-benzyloxy)-2-(3-methoxy-phenyl)-cyclohexylmethyl]-dimethyl-amin; Hydrochlorid |
| 271 | [2-(3-Methoxy-phenyl)-5-(naphthalin-2-ylmethoxy)-cyclohexylmethyl]-dimethyl-amin; Hydrochlorid |
| 272 | [5-(3-Methoxy-benzyloxy)-2-(3-methoxy-phenyl)-cyclohexylmethyl]-dimethyl-amin; Hydrochlorid |
| 273 | [5-(4-Chlor-benzyloxy)-2-(3-methoxy-phenyl)-cyclohexylmethyl]-dimethyl-amin; Hydrochlorid |
| 274 | [5-(4-Methoxy-benzyloxy)-2-(3-methoxy-phenyl)-cyclohexylmethyl]-dimethyl-amin; Hydrochlorid |
| 275 | 2-Dimethylaminomethyl-1-(3-methoxy-phenyl)-4-(naphthalin-2-ylmethoxy)-cyclohexanol; Hydrochlorid |
| 276* | [5-(4-Methoxy-benzyloxy)-2-(3-methoxy-phenyl)-cyclohex-2-enylmethyl]-dimethyl-min; Hydrochlorid |

| | |
|---|---|
| * REFERENZBEISPIEL | |

### Acylierung 2

### Reaktionsgleichung:

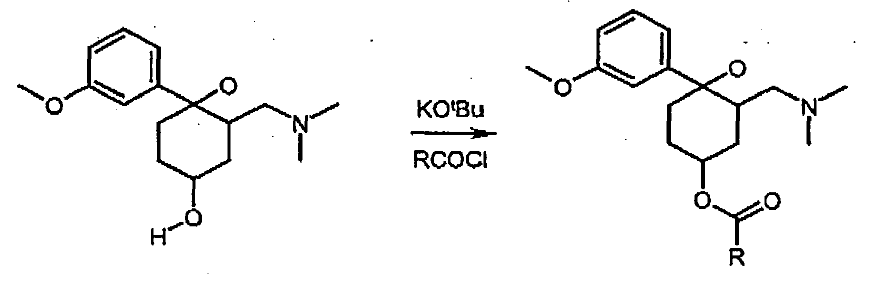

### Allgemeine Arbeitsvorschrift 9 (AAV 8):

Im getrockneten Reaktionsgefäß wurden 5 ml einer 0,5 M Lösung von Kalium-tert.-butylat in THF p.a. vorgelegt, bei -10 °C 2 ml einer 1 M Lösung des jeweiligen Alkohols in THF p.a. zugegeben, 30 Minuten unter Erwärmung auf Raumtemperatur gerührt, erneut auf -10°C gekühlt, 2 ml einer 1,25 M Lösung des korrespondierenden Säurechlorids in THF p.a. zugegeben und eine Stunde bei 30 °C gerührt.

Zur Aufarbeitung wurden bei 0 °C 2 ml 1 M Natriumhydrogencarbonatlösung zugegeben, die überstehende THF-Phase abgetrennt, mit Diisopropylether/Methanol (V/V = 1/1) an Kieselgel chromatographiert und das erhaltene Produkt in Analogie zur AAV 1 durch Lösen in 2-Butanon und Zugabe von Wasser und Chlortrimethylsilan in das Hydrochlorid überführt.

Ansätze gemäß AAV 8 mit 2-Dimethylaminomethyl-1-(3-methoxyphenyl)cyclohexan-1,4-diol Hydrochlorid (äquatorialer Alkohol) :

| | |
|---|---|
| 282 | 2,2-Dimethyl-propionsäure 3-dimethylaminomethyl-4-hydroxy-4-(3-methoxy-phenyl)-cyclchexylester; Hydrochlorid |
| 281 | 3-Methoxy-benzoesäure 3-dimethylaminomethyl-4-hydroxy-4-(3-methoxy-phenyl)-cyclohexylester; Hydrochlorid |
| 280 | 4-Methoxy-benzoesäure 3-dimethylaminomethyl-4-hydroxy4-(3-methoxy-phenyl)-cyclohexylester; Hydrochlorid |
| 307 | 2-Methoxy-benzoesäure 3-dimethylaminomethyl-4-hydroxy-4-(3-methoxy-phenyl)-cyclohexylester; Hydrochlorid |
| 306 | Naphthalin-1-carbonsäure 3-dimethylaminomethyl-4-hydroxy-4-(3-methoxy-phenyl)-cyclohexylester; Hydrochlorid |
| 305 | 3,5-Dimethoxy-benzoesäure 3-dimethylaminomethyl-4-hydroxy-4-(3-methoxy-phenyl)-cyclohexylester; Hydrochlorid |
| 304 | 3,4,5-Trimethoxy-benzoesäure 3-dimethylaminomethyl-4-hydroxy-4-(3-methoxy-phenyl)-cyclohexylester; Hydrochlorid |
| 303 | Phenyl-essigsäure 3-dimethylaminomethyl-4-hydroxy-4-(3-methoxy-phenyl)-cyclohexylester; Hydrochlorid |
| 302 | (3-Methoxy-phenyl)-essigsäure 3-dimethylaminomethyl-4-hydroxy-4-(3-methoxy-phenyl)-cyclohexylester; Hydrochlorid |
| 301 | (4-Methoxy-phenyl)-essigsäure 3-dimethylaminomethyl-4-hydroxy-4-(3-methoxy-phenyl)-cyclohexylester; Hydrochlorid |
| 300 | Cyclopentancarbonsäure 3-dimethylaminomethyl-4-hydroxy-4-(3-methoxy-phenyl)-cyclohexylester; Hydrochlorid |
| 299 | 2-Fluor-benzoesäure 3-dimethylaminomethyl-4-hydroxy-4-(3-methoxy-phenyl)-cyclohexylester; Hydrochlorid |
| 298 | 3-Fluor-benzoesäure 3-dimethylaminomethyl-4-hydroxy-4-(3-methoxy-phenyl)-cyclohexylester; Hydrochlorid |
| 297 | 4-Fluor-benzoesäure 3-dimethylaminomethyl-4-hydroxy-4-(3-methoxy-phenyl)-cyclohexylester; Hydrochlorid |
| 296 | 3,5-Difluor-benzoesäure 3-dimethylaminomethyl-4-hydroxy-4-(3-methoxy-phenyl)-cyclohexylester; Hydrochlorid |
| 295 | 4-Trifluormethyl-benzoesäure 3-dimethylaminomethyl-4-hydroxy-4-(3-methoxy-phenyl)-cyclohexylester; Hydrochlorid |
| 294 | 2-Chlor-benzoesäure 3-dimethylaminomethyl-4-hydroxy-4-(3-methoxy-phenyl)-cyclohexylester; Hydrochlorid |
| 293 | 3-Chlor-benzoesäure 3-dimethylaminomethyl-4-hydroxy-4-(3-methoxy-phenyl)-cyclohexylester; Hydrochlorid |
| 292 | 4-Chlor-benzoesäure 3-dimethylaminomethyl-4-hydroxy-4-(3-methoxy-phenyl)-cyclohexylester; Hydrochlorid |
| 291 | 3,4-Dichlor-benzoesäure 3-dimethylaminomethyl-4-hydroxy-4-(3-methoxy-phenyl)-cyclohexylester; Hydrochlorid |
| 290 | 4-Methyl-benzoesäure 3-dimethylaminomethyl-4-hydroxy-4-(3-methoxy-phenyl)-cyclohexylester; Hydrochlorid |
| 309 | 2-Hydroxy-benzoesäure 3-dimethylaminomethyl-4-hydroxy-4-(3-methoxy-phenyyl)-cyclohexylester; Hydrochlorid |
| 289 | Naphthalin-2-carbonsäure 3-dimethylaminomethyl-4-hydroxy-4-(3-methoxy-phenyl)-cyclohexylester; Hydrochlorid |
| 287 | 3,4-Dimethoxy-benzoesäure 3-dimethylaminomethyl-4-hydroxy-4-(3-methoxy-phenyl)-cyclohexylester; Hydrochlorid |
| 336 | Valeriansäure 3-dimethylaminomethyl-4-hydroxy-4-(3-methoxy-phenyl)-cyclohexylester; Hydrochlorid |

Ansätze gemäß AAV 8 mit 2-Dimethylaminomethyl-1-(3-methoxylphenyl)cyclohexan-1,4-diol Hydrochlorid (axialer Alkohol):

| | |
|---|---|
| 277 | Buttersäure 3-dimethylaminomethyl-4-hydroxy-4-(3-methoxy-phenyl)-cyclohexylester; Hydrochlorid |
| 286 | 2,2-Dimethyl-propionsäure 3-dimethylaminomethyl-4-hydroxy-4-(3-methoxy-phenyl)-cyclohexylester; Hydrochlorid |
| 285 | 3-Methoxy-benzoesäure 3-dimethylaminomethyl-4-hydroxy-4-(3-methoxy-phenyl)-cyclohexyloster; Hydrochlorid |
| 284 | 4-Methoxy-benzoesäure 3-dimethylaminomethyl-4-hydroxy-4-(3-methoxy-phenyl)-cyclohexylester; Hydrochlorid |
| 327 | 2-Methoxy-benzoesäure 3-dimethylaminomethyl-4-hydroxy-4-(3-methoxy-phenyl)-cyclohexylester; Hydrochlorid |
| 326 | Naphthalin-1-carbonsäure 3-dimethylaminomethyl-4-hydroxy-4-(3-methoxy-phenyl)-cyclohexylester; Hydrochlorid |
| 325 | 3,5-Dimethoxy-benzoesäure 3-dimethylaminomethyl-4-hydroxy-4-(3-methoxy-phenyl)-cyclohexylester; Hydrochlorid |
| 324 | 3,4,5-Trimethoxy-benzoesäure 3-dimethylaminomethyl-4-hydroxy-4-(3-methoxy-phenyl)-cyclohexylester; Hydrochlorid |
| 323 | Phenyl-essigsäure 3-dimethylaminomethyl-4-hydroxy-4-(3-methoxy-phenyl)-cyclohexylester; Hydrochlorid |
| 322 | (3-Methoxy-phenyl)-essigsäure 3-dimethylaminomethyl-4-hydroxy-4-(3-methoxy-phenyl)-cyclohexylester; Hydrochlorid |
| 321 | (4-Methoxy-phenyl)-essigsäure 3-dimethylaminomethyl-4-hydroxy-4-(3-methoxy-phenyl)-cyclohexylester; Hydrochlorid |
| 320 | Cyclopentancarbonsäure 3-dimethylaminomethyl-4-hydroxy-4-(3-methoxy-phenyl)-cyclohexylester; Hydrochlorid |
| 319 | 2-Fluor-benzoesäure 3-dimethylaminomethyl-4-hydroxy-4-(3-methoxy-phenyl)-cyclohexylester; Hydrochlorid |
| 318 | 3-Fluor-benzoesäure 3-dimethylaminomethyl-4-hydroxy-4-(3-methoxy-phenyl)-cyclohexylester; Hydrochlorid |
| 317 | 4-Fluor-benzoesäure 3-dimethylaminomethyl-4-hydroxy-4-(3-methoxy-phenyl)-cyclohexylester; Hydrochlorid |
| 316 | 3,5-Difluor-benzoesäure 3-dimethylaminomethyl-4-hydroxy-4-(3-methoxy-phenyl)-cyclohexylester; Hydrochlorid |
| 315 | 4-Trifluormethyl-benzoesäure 3-dimethylaminomethyl-4-hydroxy-4-(3-methoxy-phenyl)-cyclohexylester; Hydrochlorid |
| 314 | 2-Chlor-benzoesäure 3-dimethylaminomethyl-4-hydroxy-4-(3-methoxy-phenyl)-cyclohexylester; Hydrochlorid |
| 313 | 3-Chlor-benzoesäure 3-dimethylaminomethyl-4-hydroxy-4-(3-methoxy-phenyl)-cyclohexylester; Hydrochlorid |
| 312 | 4-Chlor-benzoesäure 3-dimethylaminomethyl-4-hydroxy-4-(3-methoxy-phenyl)-cyclohexylester; Hydrochlorid |
| 311 | 3,4-Dichlor-benzoesäure 3-dimethylaminomethyl-4-hydroxy-4-(3-methoxy-phenyl)-cyclohexylester; Hydrochlorid |
| 310 | 4-Methyl-benzoesäure 3-dimethylaminomethyl-4-hydroxy-4-(3-methoxy-phenyl)-cyclohexylester; Hydrochlorid |
| 308 | Naphthalin-2-carbonsäure 3-dimethylaminomethyl-4-hydroxy-4-(3-methoxy-phenyl)-cyclohexylester; Hydrochlorid |
| 288 | 3,4-Dimethoxy-benzoesäure 3-dimethylaminomethyl-4-hydroxy-4-(3-methoxy-phenyl)-cyclohexylester; Hydrochlorid |

### Beispiel 278:

### 4-Amino-2-dimethylaminomethyl-1-(3-methoxyphenyl)cyclohexanol Dihydrochlorid:

In eine Lösung von 2,64 g Natriumcyanoborhydrid in 45 ml getrocknetem Methanol wurden unter Rühren portionsweise 2,43 g trockenes Zinkchlorid eingetragen und 30 Minuten nachgerührt. Diese Lösung wurde langsam zu einer Suspension von 16,7 g trockenem Ammoniumacetat und 9,0 g 3-Dimethylaminomethyl-4-hydroxy-4-(3-methoxyphenyl)cyclohexanon in 45 ml trockenem Methanol getropft und 72 Stunden bei Raumtemperatur nachgerührt.

Zur Aufarbeitung wurden im Eisbad 45 ml halbkonzentrierte Salzsäure zugetropft, nach beendeter Zugabe eine Stunde nachgerührt und das Methanol im Vakuum entfernt Zum Rückstand wurden unter Eiskühlung 30 g Kaliumhydroxid gegeben und dreimal mit je 25 ml Dichlormethan extrahiert, die vereinigten Extrakte über Kaliumcarbonat getrocknet, filtriert und eingeengt. Das isolierte Rohprodukt (8,8 g) wurde an Kieselgel chromatographiert. Die erhaltenen 6,25 g 4-Amino-2-dimethylaminomethyl-1-(3-methoxyphenyl)cyclohexanol wurden in Analogie zur AAV 1 in das korrespondierende Dihydrochlorid überführt.

### Beispiel 334 und 335:

### 2-Dimethylaminomethyl-1-(3-methoxy-phenyl)-4-methylamino-cyclohexanol Dihydrochlorid (äquatoriales und axiales Amin)

18,8 g 3-Dimethylaminomethyl-4-hydroxy-4-(3-methoxyphenyl)cyclohexanon wurden unter Stickstoffatmosphäre in 210 ml THF p.a. gelöst, im Eis-/Methanol-Bad 24,3 ml 5,6 M Methylaminlösung in THF zugegeben, gefolgt von 5,72 ml Eisessig und portionsweise insgesamt 20,0 g Natriumtriacetoxyborhydrid. Nach beendeter Zugabe wurde das Kühlbad entfernt und 16 Stunden unter Erwärmung auf Raumtemperatur nachgerührt.

Zur Aufarbeitung wurden 120 ml Natronlauge zugegeben, dreimal mit je 100 ml Diethylether extrahiert, die Extrakte vereinigt, zweimal mit je 50 ml Wasser gewaschen, über Natriumsulfat getrocknet, filtriert und eingeengt. 2,5 g des erhaltenen Rohprodukts (17,4 g) wurden mit Methanol an Kieselgel chromatographiert. Es wurden 580 bzw. 600 mg zweier diastereomerer 2-Dimethylaminomethyl-1-(3-methoxy-phenyl)-methylaminocyclohexanole erhalten, die dem axialen und dem äquatorialen Produkt entsprechen, und diese in Analogie zur AAV 1 in die korrespondierenden Dihydrochloride überführt.

### Beispiel 279:

### 2-Dimethylaminomethyl-1-(3-methoxy-phenyl)-(naphthalin-2-ylmethoxy)cyclohexanol Hydrochlorid:

2-Dimethylaminomethyl-1-(3-methoxy-phenyl)-4-(naphthalin-2-ylmethoxy)cyclohexanol und das entsprechende Hydrochlorid wurden analog zur AAV 7 aus 2-Dimethylaminomethyl-1-(3-methoxy-phenyl)-cyclohexan-1,4-diol Hydrochlorid und 2-Brommethylnaphthalin hergestellt.

### Beispiel 350:

### [2,5-Bis-(4-fluor-benzyloxy)-2-(3-methoxy-phenyl)-cyclohexylmethyl]-dimethyl-amin; Hydrochlorid:

Bei der Umsetzung von 2-Dimethylaminomethyl-1-(3-methoxy-phenyl)-cyclohexan-1,4-diol Hydrochlorid mit 4-Fluorbenzylbromid gemäß AAV 7 wurde als Nebenprodukt nach chromatographischer Reinigung an Kieselgel auch [2,5-Bis-(4-fluorbenzyloxy)-2-(3-methoxyphenyl)cyclohexylmethyl]dimethylamin erhalten, das in Analogie zur AAV 1 in das korrespondierende Hydrochlorid überführt wurde.

### Beispiel 283:

### Buttersäure 3-dimethylaminomethyl-4-hydroxy-4-(3-methoxy-phenyl)-cyclohexylester Hydrochlorid:

28,0 g 3-Dimethylaminomethyl-4-hydroxy-4-(3-methoxyphenyl)cyclohexanon wurden unter Stickstoffatmosphäre in 140 ml Isopropanol gelöst, portionsweise 1,72 g Natriumboranat zugegeben und eine Stunde nachgerührt. Zur Aufarbeitung wurden zunächst 91 ml 2 M Salzsäure, dann 20 ml 10 M Natronlauge zugegeben, zweimal mit je 100 ml Dichlormethan extrahiert, die vereinigten Extrakte über Natriumsulfat getrocknet, filtriert und eingeengt. Das erhaltene Rohprodukt (29,8 g) wurde in 500 ml Aceton gelöst und durch Zugabe von 1,82 ml Wasser gefolgt von 12,7 ml Chlortrimethylsilan und Rühren über Nacht in 25,7 g 2-Dimethylaminomethyl-1-(3-methoxyphenyl)cyclohexan-1,4-diol Hydrochlorid (äquatorialer Alkohol) überführt. Aus der Mutterlauge konnten nach Zugabe von 2 M Natriumcarbonatlösung und zweimaliger Extraktion mit Ethylacetat 5,6 g des angereicherten diastereoisomeren Reduktionsproduktes isoliert werden, das durch Chromatographie an Kieselgel mit Ethylacetat/Methanol (V/V = 1:3) gereinigt wurde. Nach Hydrochloridfällung wurden 4,1 g 2-Dimethylaminomethyl-1-(3-methoxyphenyl)cyclohexan-1,4-diol Hydrochlorid (axialer Alkohol) erhalten.

205 g 2-Dimethylaminomethyl-1-(3-methoxyphenyl)cyclohexan-1,4-diol Hydrochlorid (äquatorialer Alkohol) wurden unter Stickstoffatmosphäre in 2050 ml THF p.a. suspendiert, langsam 149 g Kalium-tert.-butylat zugegeben, eine Stunde nachgerührt, 71 ml Butyrylchlorid zugetropft und eine weitere Stunde nachgerührt, bevor nochmals 36 g Kalium-tert.-butylat und 34 ml Butyrylchlorid zugegeben wurden. Zur Aufarbeitung wurden 980 ml Wasser zugegeben, zweimal mit je 1000 ml Ethylacetat extrahiert, die vereinigten Extrakte mit wenig 1 M Natriumhydrogencarbonatlösung gewaschen, über Magnesiumsulfat getrocknet, filtriert und eingeengt. Das erhaltene Rohprodukt (236 g) wurde mit einer weiteren, analog hergestellten Charge dieses Rohprodukts (232 g) vereinigt, in 2400 ml Aceton und 470 ml trockenem Ethanol gelöst und durch Zugabe von einem halben Moläquivalent Wasser und einem Moläquivalent Chlortrimethylsilan 442 g des Hydrochlorids von Buttersäure 3-dimethylaminomethyl-4-hydroxy-4-(3-methoxyphenyl)cyclohexylester erhalten.

### Beispiel 332 und 333:

### (+)- und (-)-Buttersäure 3-dimethylaminomethyl-4-hydroxy-4-(3-methoxyphenyl)cyclohexylester

Die enantiomeren (+)- und (-)-Buttersäure 3-dimethylaminomethyl-4-hydroxy-4-(3-methoxyphenyl)cyclohexylester wurden durch enzymatische Racematspaltung von Buttersäure 3-dimethylaminomethyl-4-hydroxy-4-(3-methoxyphenyl)cyclohexylester und anschließende Isolierung des verbliebenen Butyrats bzw. erneute Veresterung des erhaltenen 2-Dimethylaminomethyl-1-(3-methoxyphenyl)cyclohexan-1,4-diols wie für das Racemat beschrieben hergestellt (Gais, Hans-Joachim; Griebel, Carsten; Buschmann, Helmut; Tetrahedron: Asymmetry 11 (2000) 917-928).

### Beispiel 351: 5-Dimethylaminomethyl-4-(3-methoxy-phenyl)-cyclohex-3-enol Hydrochlorid:

20,0 g 2-Dimethylaminomethyl-1-(3-methoxyphenyl)cyclohexan-1,4-diol (äquatorialer Alkohol) wurden in 300 ml konz. Ameisensäure gelöst, 6,75 ml Acetylchlorid zugegeben und zwei Stunden zum Rückfluß erhitzt. Nach Abkühlung wurde eingeengt, der Rückstand mit 2 M Natronlauge aufgenommen und mit Ethylacetat extrahiert. Die vereinigten Extrakte wurden über Natriumsulfat getrocknet, filtriert, eingeengt, das erhaltene Rohprodukt (16,7 g) in 170 ml 2-Butanon gelöst und durch Zugabe von 1,15 ml Wasser und 8,1 ml Chlortrimethylsilan 5-Dimethylaminomethyl-4-(3-methoxy-phenyl)-cyclohex-3-enol Hydrochlorid (17,7 g) ausgefällt.

### Beispiel 328 und 329:

### 2-Dimethylaminomethyl-1,4-bis-(3-methoxy-phenyl)-cyclohexan-1,4-diol Hydrochlorid (cis- und trans-Diol):

Aus 7,47 g Magnesium und 38,9 ml 3-Bromanisol wurde in 60 ml THF p.a. das korrespondierende Grignardreagenz hergestellt, bei ca. 10 °C eine Lösung von 40 g 1,4-Dioxa-spiro[4.5]decan-8-on in 75 ml THF p.a. zugetropft und eine Stunde nachgerührt. Zur Aufarbeitung wurde im Eisbad 20 massenprozentige Ammoniumchloridlösung zugegeben, mit Ethylacetat extrahiert, die vereinigten Extrakte über Natriumsulfat getrocknet, filtriert und eingeengt. Zum erhaltenen Rohprodukt (61,6 g) wurden im Eisbad 128 ml Wasser, 62 g Eis und eine Lösung von 31,5 ml konz. Salzsäure in 385 ml Wasser gegeben, die Mischung fünf Stunden bei 5 - 10 °C gerührt, mit Ethylacetat extrahiert, die vereinigten Extrakte über Natriumsulfat getrocknet, filtriert und eingeengt. Das Rohprodukt wurde mit Diisopropylether verrührt und der verbliebene Feststoff abfiltriert. Es wurden 14,0 g 4-Hydroxy-4-(3-methoxyphenyl)cyclohexanon erhalten.

Zu 10,0 g 4-Hydroxy-4-(3-methoxyphenyl)cyclohexanon wurden in 100 ml Acetonitril 4,23 g Dimethylmethylenammoniumchlorid und ein Tropfen Acetylchlorid gegeben und die Mischung 16 Stunden gerührt. Der ausgefallene Feststoff wurde abfiltriert, mit wenig Acetonitril gewaschen und im Vakuum getrocknet. Es wurden 12,2 g 2-Dimethylaminomethyl-4-hydroxy-4-(3-methoxyphenyl)cyclohexanon erhalten.

Aus 0,75 g Magnesium und 3,9 ml 3-Bromanisol wurde in 20 ml THF p.a. das korrespondierende Grignardreagenz hergestellt, bei ca. 10 °C eine Lösung von 3,87 g 2-Dimethylaminomethyl-4-hydroxy4-(3-methoxyphenyl)cyclohexanon in 25 ml THF p.a. zugetropft und eine Stunde nachgerührt. Zur Aufarbeitung wurde im Eisbad 20 massenprozentige Ammoniumchloridlösung zugegeben, mit Ethylacetat extrahiert, die vereinigten Extrakte über Natriumsulfat getrocknet, filtriert und eingeengt. Das erhaltene Rohprodukt (5,44 g) wurde mit Ethylacetat/Methanol (V/V = 1:1) an Kieselgel chromatographiert. Die erhaltenen diastereomeren 2-Dimethylaminomethyl-1,4-bis-(3-methoxy-phenyl)-cyclohexan-1,4-diole (cis- und trans-Diol) wurden in Analogie zur AAV 1 mit Chlortrimethylsilan in wäßrigem 2-Butanon in die korrespondierenden Hydrochloride (240 bzw. 777 mg) überführt.

### Beispiel 337und 338:

### (E)- und (Z)-[3-Dimethylaminomethyl4-hydroxy-4-(3-methoxyphenyl)cyclohexyliden]essigsäureethylester Hydrochlorid

100 g 1,4-Dioxa-spiro[4.5]decan-8-on wurden in 800 ml Toluol gelöst, 534 ml 32 massenprozentige Natronlauge zugegeben, unter Eiskühlung und intensivem Rühren eine Lösung von 127 ml Triethylphosphonoacetat in 250 ml Toluol zugetropft und eine Stunde nachgerührt. Die organische Phase wurde abgetrennt, die wäßrige Phase mit Toluol extrahiert, die vereinigten organischen Phasen dreimal mit Wasser gewaschen, über Natriumsulfat getrocknet, filtriert und eingeengt. Das erhaltene Rohprodukt wurde mit Diisopropylether/Hexan (V/V = 1:1) an Kieselgel chromatographiert. Es wurden 92,9 g (1,4-Dioxa-spiro[4.5]dec-8-yliden)essigsäureethylester erhalten.
92,8 g (1,4-Dioxa-spiro[4.5]dec-8-yliden)essigsäureethylester wurden in 465 ml Diisopropylether gelöst, 186 ml Wasser und 137 ml konz. Salzsäure zugegeben, eine Stunde gerührt, die Phasen getrennt, die wäßrige Phase zweimal mit Diisopropylether extrahiert, die vereinigten organischen Phasen über Natriumsulfat getrocknet, filtriert und eingeengt. Das erhaltene Rohprodukt (95,9 g) wurde mit Diisopropylether/Hexan (V/V = 2:1) an Kieselgel chromatographiert. Neben 17,2 g nicht umgesetztem (1,4-Dioxa-spiro[4.5]dec-8-yliden)essigsäureethylester wurden 76,3 g (4-Oxocyclohexyliden)essigsäureethylester erhalten.

Zu 87,6 g (4-Oxocyclohexyliden)essigsäureethylester in 260 ml Acetonitril wurden 7,5 g Dimethylmethylenammoniumchlorid gegeben, eine Stunde auf 60 °C erwärmt, 250 ml Diisopropylether zugegeben und über Nacht bei Raumtemperatur gerührt.

Der ausgefallene Feststoff wurde abfiltriert, mit wenig Acetonitril gewaschen und im Vakuum getrocknet. Es wurden 30,4 g (*E*)-(3-Dimethylaminomethyl-4-oxocyclo-hexyliden)essigsäureethylester erhalten. Die Mutterlauge wurde zur Trockne eingeengt und der Rückstand (95 g) aus 475 ml 2-Butanon umkristallisiert. Es wurden 62,3 g (*Z*)-(3-Dimethylaminomethyl-4-oxocyclohexyliden)essigsäureethylester erhalten.

Aus 0,37 g Magnesium und 1,9 ml 3-Bromanisol wurde in 10 ml THF p.a. das korrespondierende Grignardreagenz hergestellt, bei Eiskühlung eine Lösung von 2,40 g (Z)-(3-Dimethylaminomethyl-4-oxocyclohexyliden)essigsäureethylester in 24 ml THF p.a. zugetropft und eine Stunde nachgerührt. Zur Aufarbeitung wurde im Eisbad 5 ml 4 M Ammoniumchloridlösung zugegeben, die organische Phase abgetrennt, die wäßrige Phase zweimal mit Ethylacetat extrahiert, die vereinigten organischen Phasen über Natriumsulfat getrocknet, filtriert und eingeengt. Das erhaltene Rohprodukt (3,20 g) wurde mit Dilsopropylether/Methanol (V/V = 1:1) an Kieselgel chromatographiert. Es wurden 1,61 g (*Z*)-[3-Dimethylaminomethyl-4-hydroxy-4-(3-methoxyphenyl)cyclohexyliden]essigsäureethylester erhalten und diese in Analogie zur AAV 1 in das korrespondierende Hydrochlorid überführt. Analog wurden aus 2,40 g (*E*)-(3-Dimethlaminomethyl-4-oxocyclohexyliden)essigsäureethylester 1,48 g des Hydrochlorids von (*E*)-[3-Dimethylaminomethyl-4-hydroxy-4-(3-methoxyphenyl)cyclohexyliden]essigsäureethylester hergestellt.

### Beispiel 352:

### 2-Dimethylaminomethyl-1 -(6-methoxynaphthalin-2-yl)cyclohexan-1,4-diol Hydrochlorid

Aus 18,2 g Magnesium und 177 g 2-Brom-6-methoxynaphthalin wurde in 720 ml THF p.a. das korrespondierende Grignardreagenz hergestellt, bei Eiskühlung eine Lösung von 105 g 7-Dimethylaminomethyl-1,4-dioxa-spiro[4.5]decan-8-on in 315 ml THF p.a. zugetropft und eine Stunde nachgerührt. Zur Aufarbeitung wurde im Eisbad 375 ml 4 M Ammoniumchloridlösung zugegeben, die organische Phase abgetrennt, die wäßrige Phase zweimal mit Ethylacetat extrahiert, die vereinigten organischen Phasen über Natriumsulfat getrocknet, filtriert und eingeengt. Es wurden 229 g 7-Dimethylaminomethyl-8-(6-methoxynaphthalin-2-yl)-1,4-dioxa-spiro[4.5]decan-8-ol erhalten.

229 g 7-Dimethylaminomethyl-8-(6-methoxynaphthalin-2-yl)-1,4-dioxaspiro[4.5]decan-8-ol wurden in 1150 ml THF gelöst, 164 ml Wasser und 41ml konz. Salzsäure zugegeben, fünf Stunden gerührt, mit 32 massenprozentiger Natronlauge neutralisiert, zweimal mit Ethylacetat/THF (V/V = 1:1) extrahiert, die vereinigten organischen Phasen zweimal mit gesättigter Kochsalzlösung gewaschen, über Magnesiumsulfat getrocknet, filtriert und eingeengt. Das erhaltene Rohprodukt (196 g) wurde mit 400 ml Methanol und 600 ml Diisopropylether versetzt, filtriert, das Filtrat eingeengt, in 1600 ml 2-Butanon gelöst und durch Zugabe von Wasser und Chlortrimethylsilan in Analogie zur AAV 1 das Hydrochlorid gefällt. Es wurden 106 g des Hydrochlorid von 3-Dimethylarninomethyl-4-hydroxy-4-(6-methoxynaphthalin-2-yl)cyclohexanon erhalten.

4,3 g Natriumboranat wurden in 82 ml Ethanol p.a. vorgelegt, 82,2 g 3-Dimethylaminomethyl-4-hydroxy-4-(6-methoxynaphthalin-2-yl)cyclohexanon, gelöst in 330 ml Ethanol p.a., unter Eiskühlung zugetropft und eine Stunde nachgerührt. Zur Aufarbeitung wurden unter Eiskühlung zunächst 44 ml konz. Salzsäure, dann 40 ml 32 massenprozentige Natronlauge zugegeben, zweimal mit Dichlormethan extrahiert, die vereinigten organischen Phasen über Magnesiumsulfat getrocknet, filtriert und eingeengt. Aus dem erhaltenen Rohprodukt (88.2 g) wurden in Analogie zur AAV 1 77,4 g des Hydrochlorids von 2-Dimethylaminomethyl-1-(6-methoxy-naphthalin-2-yl)cyclohexan-1,4-diol erhalten.

### Beispiel 353:

### Buttersäure 3-dimethylaminomethyl-4-hydroxy-4-(6-methoxynaphthalin-2-yl)cyclohexylester Hydrochlorid:

70,0 g 2-Dimethylaminomethyl-1-(6-methoxynaphthalin-2-yl)cyclohexan-1,4-diol wurden in 560 ml THF p.a. suspendiert, portionsweise 44,0 g Kalium-tert-butylat zugegeben, zwanzig Minuten gerührt, 21,0 ml Butyrylchlorid zugetropft, weitere zwanzig Minuten gerührt und noch zweimal weitere je 21,0 g Kalium-tert.-butylat und 20,0 ml Butyrylchlorid nach obigem Schema zugegeben. Zur Aufarbeitung wurden unter Eisbadkühlung 290 ml Wasser zugegeben, die Phasen getrennt, einmal mit Ethylacetat extrahiert, die vereinigten organischen Phasen mit 1 M Natriumhydrogencarbonatlösung gewaschen, über Magnesiumsulfat getrocknet, filtriert und eingeengt. Aus dem erhaltenen Rohprodukt (90 g) wurden in Analogie zur AAV 1 47,1 g des Hydrochlorids von Buttersäure 3-dimethylaminomethyl-4-hydroxy-4-(6-methoxynaphthalin-2-yl)cyclohexylester erhalten.

### Beispiel 331:

### 4-Benzyloxy-2-dimethylaminomethyl-1-(6-methoxynaphthalin-2-yl)cyclohexanol Hydrochlorid:

Aus 0,28 g Magnesium und 2,72 g 2-Brom-6-methoxynaphthalin wurde in 11 ml THF p.a. das korrespondierende Grignardreagenz hergestellt, bei Eiskühlung eine Lösung von 2,00 g 4-Benzyloxy-2-dimethylaminomethylcyclohexanon in 8 ml THF p.a. zugetropft und eine Stunde nachgerührt. Zur Aufarbeitung wurden im Eisbad 4 ml 4 M Ammoniumchloridlösung zugegeben, die organische Phase abgetrennt, die wäßrige Phase zweimal mit Diethylether extrahiert, die vereinigten organischen Phasen über Natriumsulfat getrocknet, filtriert und eingeengt. Das erhaltene Rohprodukt (3,20 g) wurde mit Ethylacetat/Methanol/Hexan (V/V/V = 1:1:1) an Kieselgel chromatographiert. Es wurden 1,52 g 4-Benzyloxy-2-dimethylaminomethyl-1-(6-methoxynaphthalin-2-yl)cyclohexanol erhalten und diese in Analogie zur AAV 1 in das korrespondierende Hydrochlorid (1,32 g) überführt.

### Beispiel 330:

### 6-(4-Benzyloxy-2-dimethylaminomethyl-1-hydroxy-cyclohexyl)-naphthalin-2-ol Hydrochlorid:

200 g 6-Bromnaphthalin-2-ol, 143 g imidazol und 151 g tert.-Butylchlordimethylsilan wurden in 1000 ml Dimethylformamid gelöst, zwei Stunden bei Raumtemperatur gerührt, die Lösung weitgehend eingeengt (506 g), der Rückstand in Ethylacetat und Wasser gelöst, 1500 ml gesättigte Natriumchloridlösung zugegeben, die Phasen getrennt, die wäßrige Phase zweimal mit Ethylacetat extrahiert und die organischen Phasen vereinigt und eingeengt. Das erhaltene Rohprodukt wurde in siedendem Hexan gelöst und anschließend über Nacht bei 4 °C aufbewahrt. Nach Filtration wurden 158 g (6-Bromnaphthalin-2-yloxy)-tert.-butyldimethylsilan isoliert. Erneutes einengen der Mutterlauge und Umkristallisation aus Methanol erbrachte weitere 54 g (6-Bromnaphthalin-2-yloxy)-tert.-butyldimethylsilan.
3,87 g (6-Bromnaphthalin-2-yloxy)-tert.-butyldimethylsilan wurden in 19 ml THF p.a. gelöst, bei Trockeneiskühlung 6,0 ml 1.6 M Butyllithiumlösung in Hexan zugetropft, kurz nachgerührt, dann eine Lösung von 2,00 g 4-Benzyloxy-2-dimethylamino-methylcyclohexanon in 20 ml THF p.a. zugetropft und eine Stunde unter langsamer Erwärmung nachgerührt. Zur Aufarbeitung wurden im Eisbad 4 ml Wasser zugegeben, die organische Phase abgetrennt, die wäßrige Phase zweimal mit Diethylether extrahiert, die vereinigten organischen Phasen über Natriumsulfat getrocknet, filtriert und eingeengt. Das erhaltene Rohprodukt (5,00 g) wurde mit Ethylacetat/Methanol (V/V = 1:1) an Kieselgel chromatographiert. Es wurden 2,20 g 6-(4-Benzyloxy-2-dimethylaminomethyl-1-hydroxy-cyclohexyl)-naphthalin-2-ol erhalten und diese in Analogie zur AAV 1 in das korrespondierende Hydrochlorid (1,33 g) überführt.

### Algemeine Ergänzung der Beispiele:

Ergänzend wurden folgende weitere Beispiele nach mindestens einer der vorstehenden (Grund-)Verfahren oder AAV's hergestellt:

| **Beispiel Nr.:** | **Verbindungsname:** |
|---|---|
| 354 | Benzoesäure 4-hydroxy-3-(4-methyl-piperazin-1-ylmethyl)-4-naphthalin-2-yl-cyclohexyl ester; Bishydrochlorid |
| 355 | 2-Chlor-benzoesäure 4-hydroxy-3-(4-methyl-piperazin-1-ylmethyl)-4-naphthalin-2-yl-cyclohexyl ester; Bishydrochlorid |
| 356 | 3-Chlor-benzoesäure 4-hydroxy-3-(4-methyl-piperazin-1-ylmethyl)-4-naphthalin-2-yl-cyclohexyl ester; Bishydrochlorid |
| 357 | 4-Chlor-benzoesäure 4-hydroxy-3-(4-methyl-piperazin-1-ylmethyl)-4-naphthalin-2-yl-cyclohexyl ester; Bishydrochlorid |
| 358 | 2-Fluor-benzoesäure 4-hydroxy-3-(4-methyl-piperazin-1-ylmethyl)-4-naphthalin-2-yl-cyclohexyl ester; Bishydrochlorid |
| 359 | 3- Fluor-benzoesäure 4-hydroxy-3-(4-methyl-piperazin-1-ylmethyl)-4-naphthalin-2-yl-cyclohexyl ester; Bishydrochlorid |
| 360 | 4- Fluor -benzoesäure 4-hydroxy-3-(4-methyl-piperazin-1-ylmethyl)-4-naphthalin-2-yl-cyclohexyl ester; Bishydrochlorid |
| 361 | 2-Methyl-benzoesäure 4-hydroxy-3-(4-methyl-piperazin-1-ylmethyl)-4-naphthalin-2-yl-cyclohexyl ester; Bishydrochlorid |
| 362 | 3- Methyl -benzoesäure 4-hydroxy-3-(4-methyl-piperazin-1-ylmethyl)-4-naphthalin-2-yl-cyclahexyl ester; Bishydrochlorid |
| 363 | 4- Methyl -benzoesäure 4-hydroxy-3-(4-methyl-piperazin-1-ylmethyl)-4-naphthalin-2-yl-cyclohexyl ester; Bishydrochlorid |
| 364 | 2-Methoxy-benzoesäure 4-hydroxy-3-(4-methyl-piperazin-1-ylmethyl)-4-naphthalin-2-yl-cyclohexyl ester; Bishydrochlorid |
| 365 | 3- Methoxy -benzoesäure 4-hydroxy-3-(4-methyl-piperazin-1-ylmethyl)-4-naphthalin-2-yl-cyclohee ester; Bishydrochlorid |
| 366 | 4- Methoxy -benzoesäure 4-hydroxy-3-(4-methyl-piperazin-1-ylmethyl)-4-naphthalin-2-yl-cyclohexyl ester; Bishydrochlorid |
| 367 | 2,6-Dichlor-benzoesäure 4-hydroxy-3-(4-methyl-piperazin-1-ylmethyl)-4-naphthalin-2-yl-cyclohexyl ester; Bishydrochlorid |
| 368 | 2,6-Difluor-benzoesäure 4-hydroxy-3-(4-methyl-piperazin-1-ylmethyl)-4-naphthalin-2-yl-cyclohexyl ester; Bishydrochlorid |
| 369 | 2-Chlor-5-fluor-benzoesäure 4-hydroxy-3-(4-methyl-piperazin-1-ylmethyl)-4-naphthalin-2-yl-cyclohexyl ester; Bishydrochlorid |
| 370 | Biphenyl-4-carbonsäure 4-hydroxy-3-(4-methyl-piperazin-1-ylmethyl)-4-naphthalin-2-yl-cyclohexylester; Bishydrochlorid |
| 371 | 2-Chlor-benzoesäure 4-hydroxy-4-(6-methoxy-naphthalin-2-yl)-3-(4-methyl-piperazin-1-ylmethyl)-cyclohexyl ester; Bishydrochlorid |
| 372 | 3-Chlor-benzoesäure 4-hydroxy-4-(6-methoxy-naphthalin-2-yl)-3-(4-methyl-piperazin-1-ylmethyl)-cyclohexyl ester; Bishydrochlorid |
| 373 | 4-Chlor-benzoesäure 4-hydroxy-4-(6-methoxy-naphthalin-2-yl)-3-(4-methyl-piperazin-1-ylmethyl)-cyclohexyl ester; Bishydrochlorid |
| 374 | 2-Fluor-benzoesäure 4-hydroxy-4-(6-methoxy-naphthalin-2-yl-3-(4-methyl-piperazin-1-ylmethyl)-cyclohexyl ester; Bishydrochlorid |
| 375 | 3-Fluor-benzoesäure 4-hydroxy-4-(6-methoxy-naphthalin-2-yl)-3-(4-methyl-piperazin-1-ylmethyl)-cyclohexyl ester; Bishydrochlorid |
| 376 | 4-Fluor-benzoesäure 4-hydroxy-4-(6-methoxy-naphthalin-2-yl)-3-(4-methyl-piperazin-1-ylmethyl)-cyclohexyl ester; Bishydrochlorid |
| 377 | 2-Methyl-benzoesäure 4-hydroxy-4-(6-methoxy-naphthalin-2-yl)-3-(4-methyl-piperazin-1-ylmethyl)-cyclohexy! ester; Bishydrochlorid |
| 378 | 3-Methyl-benzoesäure 4-hydroxy-4-(6-methoxy-naphthalin-2-yl)-3-(4-methyl-piperazin-1-ylmethyl)-cyclohexyl ester; Bishydrochlorid |
| 379 | 4-Methyl-benzoesäure 4-hydroxy-4-(6-methoxy-naphthalln-2-yl)-3-(4-methyl-piperazin-1-ylmethyl)-cyclohexyl ester; Bishydrochlorid |
| 380 | 2-Methoxy-benzoesäure 4-hydroxy-4-(6-methoxy-naphthalin-2-yl)-3-(4-methyl-piperazin-1-ylmethyl)-cyclohexyl ester; Bishydrochlorid |
| 381 | 3-Methoxy-benzoesäure 4-hydroxy-4-(6-methoxy-naphthalin-2-yl)-3-(4-methyl-piperazin-1-ylmethyl)-cyclohexyl ester; Bishydrochlorid |
| 382 | 4-Methoxy-benzoesäure 4-hydroxy-4-(6-methoxy-naphthalin-2-yl)-3-(4-methyl-piperazin-1-ylmethyl)-cyclohexyl ester; Bishydrochlorid |
| 383 | 2,6-Dichlor-benzoesäure 4-hydroxy-4-(6-methoxy-naphthalin-2-yl)-3-(4-methyl-piperazin-1-ylmethyl)-cyclohexyl ester; Bishydrochlorid |
| 384 | 2,6-Fluor-benzoesäure 4-hydroxy-4-l6-methoxy-naphthalin-2-yl)-3-(4-methyl-piperazin-1-ylmethyl)-cyclohexyl ester; Bishydrochlorid |
| 385 | 2-Chlor-6-fluor-benzoesäure 4-hydroxy-4-(6-methoxy-naphthalin-2-yl)-3-(4-methyl-piperazin-1-ylmethyl)-cyclohexyl ester; Bishydrochlorid |
| 386 | Biphenyl-4-carbonsäure 4-hydroxy-4-(C-methoxy-naphthalin-2-yl)-3-(4-methyl-piperazin-1-ylmethyl)-cyclohexyl ester; Bishydrochlorid |
| 387 | 4-(2-Chlor-benzyloxy)-2-(4-methyl-piperazin-1-ylmethyl)-1-naphthalin-2-yl-cyclohexanol; Bishydrochlorid |
| 388 | 4-(3-Chlor-benzyloxy)-2-(4-methyl-piperazin-1-ylmethyl)-1-naphthalin-2-yl-cyclohexanol: Bishydrochlorid |
| 389 | 4-(4-Chlor-benzyloxy)-2-(4-methyl-piperazin-1-ylmethyl)-1-naphthalin-2-yl-cyclohexanol; Bishydrochlorid |
| 390 | 4-(2-Fluor-benzyloxy)-2-(4-methyl-piperazin-1-ylmethyl)-1-naphthalin-2-yl-cyclohexanol; Bishydrochlorid |
| 391 | 4-(3-Fluor-benzyloxy)-2-(4-methyl-piperazin-1-ylmethyl)-1-naphthalin-2-yl-cyclohexanol; Bishydrochlorid |
| 392 | 4-(4-Fluor-benzyloxy)-2-(4-methyl-piperazin-1-ylmethyl)-1-naphthalin-2-yl-cyclohexanol; Bishydrochlorid |
| 393 | 4-(2-Methyl-benzyloxy)-2-(4-methyl-piperazin-1-ylmethyl)-1-naphthalin-2-yl-cyclohexanol; Bishydrochlorid |
| 394 | 4-(3-Methyl-benzyloxy)-2-(4-methyl-piperazin-1-ylmethyl)-1-naphthalin-2-yl-cyclohexanol; Bishydrochlorid |
| 395 | 4-(4-Methyl-benzyloxy)-2-(4-methyl-piperazin-1-ylmethyl)-1-naphthalin-2-yl-cyclohexanol; Bishydrochlorid |
| 396 | 4-(2-Methoxy-benzyloxy)-2-(4-methyl-piperazin-1-ylmethyl)-1-naphthalin-2-yl-cyclohexanol; Bishydrochlorid |
| 397 | 4-(3-Methoxy-benzyloxy)-2-(4-methyl-piperazin-1-ylmethyl)-1-naphthalin-2-yl-cyclohexanol; Bishydrochlorid |
| 398 | 4-(4-Methoxy-benzyloxy)-2-(4-methyl-piperazin-1-ylmethyl)-1-naphthalin-2-yl-cyclohexanol; Bishydrochlorid |
| 399 | 4-(2,6-Dichlor-benzyloxy)-2-(4-methyl-piperazin-1-ylmethyl)-1-naphthalin-2-yl-cyclohexanol, Bishydrochlorid |
| 400 | 4-(2,6-Difluor-benzyloxy)-2-(4-methyl-piperazin-1-ylmethyl)-1-naphthalin-2-yl-cyclohexanol, Bishydrochlorid |
| 401 | 4-(2-Chlor-6-fluor-benzyloxy)-2-(4-methyl-piperazin-1-ylmethyl)-1-naphthalin-2-yl-cyclohexanol, Bishydrochlorid |
| 402 | 4-(2-Chlor-benzyloxy)-1-(6-methoxy-naphthalin-2-yl)-2-(4-methyl-piperazin-1-ylmethyl)-cyclohexanol; Bishydrochlorid |
| 403 | 4-(3-Chlor-benzyloxy)-1-(6-methoxy-naphthalin-2-yl)-2-(4-methyl-piperazin-1-ylmethyl)-cyclohexanol; Bishydrochlorid |
| 404 | 4-(4-Chlor-benzyloxy)-1-(6-methoxy-naphthalin-2-yl)-2-(4-methyl-piperazin-1-ylmethyl)-cyclohexanol; Bishydrochlorid |
| 405 | 4-(2-Fluor-benzyloxy)-1-(6-methoxy-naphthalin-2-yl)-2-(4-methyl-piperazin-1-ylmethyl)-cyclohexanol; Bishydrochlorid |
| 406 | 4-(3-Fluor-benzyloxy)-1-(6-methoxy-naphthalin-2-yl)-2-(4-methyl-piperazin-1-ylmethyl)-cyclohexanol; Bishydrochlorid |
| 407 | 4-(4-Fluor-benzyloxy)-1-(6-methoxy-naphthalin-2-yl)-2-(4-methyl-piperazin-1-ylmethyl)-cyclohexanol: Bishydrochlorid |
| 408 | 4-(2-Methyl-benzyloxy)-1-(6-methoxy-naphthalin-2-yl)-2-(4-methyl-piperazin-1-ylmethyl)-cyclohexanol; Bishydrochlorid |
| 409 | 4-(3-Methyl-benzyloxy)-1-(6-methoxy-naphthalin-2-yl)-2-(4-methyl-piperazin-1-ylmethyl)-cyclohexanol; Bishydrochlorid |
| 410 | 4-(4-Methyl-benzyloxy)-1-(6-methoxy-naphthalin-2-yl)-2-(4-methyl-piperazin-1-ylmethyly-cyclohexanol; Bishydrochlorid |
| 411 | 4-(2-Methoxy-benzyloxy)-1-(6-methoxy-naphthalin-2-yl)-2-(4-methyl-piperazin-1-ylmethyl)-cyclohexanol; Bishydrochlorid |
| 412 | 4-(3-Methoxy-benzyloxy)-1-(6-methoxy-naphthalin-2-yl)-2-(4-methyl-piperazin-1-ylmethyl)-cyclohexanol; Bishydrochlorid |
| 413 | 4-(4-Methoxy-benzyloxy)-1-(6-methoxy-naphthalin-2-yl)-2-(4-methyl-piperazin-1-ylmethyl)-cyclohexanol; Bishydrochlorid |
| 414 | 4-(2,6-Dichlor-benzyloxy)-1-(6-methoxy-naphthalin-2-yl)-2-(4-methyl-piperazin-1-ylmethyl)-cyclohexanol; Bishydrochlorid |
| 415 | 4-(2,6-Difluor-benzyloxy)-1-(6-methoxy-naphthalin-2-yl)-2-(4-methyl-plperazin-1-ylmethyl)-cyclohexanol; Bishydrochlorid |
| 416 | 4-(2-Chlor-6-fluor-benzyloxy)-1-(6-methoxy-naphthalin-2-yl)-2-(4-methyl-piperazin-1-ylmethyl)-cyclohexanol; Bishydrochlorid |
| 417 | 2-Dimethylaminomethyl-4-(4-fluor-benzyloxy)-1-naphthalin-2-yl-cyclohexanol |
| 418 | 4-Fluor-benzoesäure 3-dimethylaminomethyl-4-hydroxy-4-(6-methoxy-naphthalin-2-yl)-cyclohexylester |
| 419 | 4-Fluor-benzoesäure 3-dimethylaminomethyl-4-hydroxy-4-naphthalin-2-yl-cyclohexylester |
| 420 | 2-Dimethylaminomethyl-4-(4-fluor-benzyloxy)-1-(6-methoxy-naphthalin-2-yl)-cyclohexanol |
| 421 | 6-[2-Dimethylaminomethyl-4-(4-fluor-benzyloxy)-1-hydroxy-cyclohexyl]-naphthalin-2-ol |

## Patentansprüche

1. Substituierte C-Cyclohexylmethylamin-Derivate der allgemeinen Formel I, 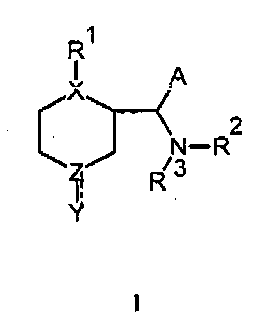 , worin
A ausgewählt ist aus H oder Phenyl;
R¹ ausgewählt ist aus C₁₋₁₀-Alkyl oder C₃₋₁₀-Cycloalkyl, jeweils gesättigt oder ungesättigt, verzweigt oder unverzweigt, unsubstituiert oder einfach oder mehrfach substituiert, Naphthyl, Phenyl mit der Massgabe, daß R¹ kein einfach in 3-Position O- oder S-substituierter Phenylring ist, Furyl, Thiophenyl, über C₁₋₃-Alkylen oder Ethinyl gebundenem Naphthyl, Phenyl, über C₁₋₃-Alkylen oder Ethinyl gebundenem C₃₋₁₀-Cycloalkyl oder über C₁₋₃-Alkylen oder Ethinyl gebundenem Furyl, Thiophenyl, jeweils unsubstituiert oder einfach oder mehrfach substituiert,
wobei die Substituenten unabhängig voneinander ausgewählt sind aus F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl oder C₃₋₁₀-Cycloalkyl; jeweils unsubstituiert oder einfach oder mehrfach, gleich oder verschieden, substituiert mit F, Cl, Br, I, NH₂, SH oder OH, oder Silyl, mit R¹⁸ ausgewählt aus H; C₁₋₁₀-Alkyl oder C₃₋₁₀-Cycloalkyl, jeweils gesättigt oder ungesättigt, verzweigt oder unverzweigt,
R¹⁹ und R²⁰ unabhängig voneinander ausgewählt aus H; C₁₋₁₀-Alkyl oder C₃₋₁₀-Cycloalkyl, jeweils gesättigt oder ungesättigt, verzweigt oder unverzweigt,
oder R¹⁹ und R²⁰ zusammen CH₂CH₂OCH₂CH₂, CH₂CH₂NR²¹CH₂CH₂ oder (CH₂)₃₋₆ bilden,
mit R²¹ ausgewählt aus H; Phenyl; C₁₋₁₀-Alkyl gesättigt oder ungesättigt, verzweigt oder unverzweigt;
R² und R³, unabhängig voneinander ausgewählt sind aus H; C₁₋₁₀-Alkyl oder C₃₋₁₀-Cycloalkyl, jeweils gesättigt oder ungesättigt, verzweigt oder unverzweigt,
oder die Reste R² und R³ zusammen CH₂CH₂OCH₂CH₂, CH₂CH₂NR⁶CH₂CH₂ oder (CH₂)₃₋₆ bedeuten,
mit R⁶ ausgewählt aus H; C₁₋₁₀-Alkyl oder C₃₋₁₀-Cycloalkyl, jeweils gesättigt oder ungesättigt, verzweigt oder unverzweigt;
X in der Formel I gemäß Teilformel la der Formel I 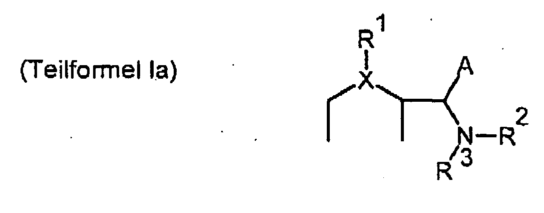 für 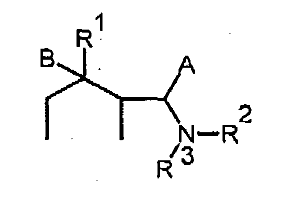 steht,
mit B ausgewählt aus OH, OR⁷, H, F, Cl oder NR⁸R⁹,
mit R⁷ ausgewählt aus C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH;
R⁸ und R⁹, unabhängig voneinander ausgewählt sind aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH; oder die Reste R⁸ und R⁹ zusammen CH₂CH₂OCH₂CH₂, CH₂CH₂NR¹⁰CH₂CH₂ oder (CH₂)₃₋₆ bilden
mit R¹⁰ ausgewählt aus H; C₁₋₁₀-Alkyl oder C₃₋₁₀-Cycloalkyl, jeweils gesättigt oder ungesättigt, verzweigt oder unverzweigt, unsubstituiert oder einfach oder mehrfach, gleich oder verschieden, substituiert mit F, Cl, Br, I, NH₂, SH oder OH; 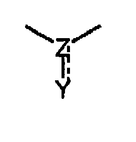 ausgewählt ist aus 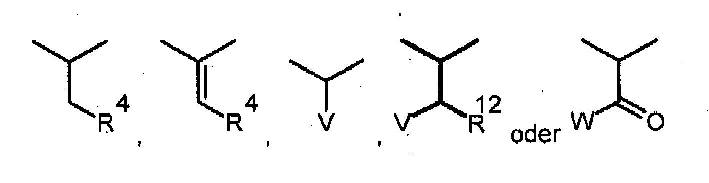 mit R⁴ ausgewählt aus COR⁵, SO₂R⁵; C₁₋₁₀-Alkyl oder C₃₋₁₀-Cycloalkyl, jeweils gesättigt oder ungesättigt, verzweigt oder unverzweigt, unsubstituiert oder einfach oder mehrfach substituiert; Naphthyl, Phenyl, Furyl oder Thiophenyl, jeweils unsubstituiert oder einfach oder mehrfach substituiert; oder über C₁₋₃-Alkylen gebundenem Napthyl, Phenyl, C₃₋₁₀-Cycloalkyl, Furyl oder Thiophenyl, jeweils unsubstituiert oder einfach oder mehrfach substituiert,
mit R⁵ ausgewählt aus C₁₋₁₀-Alkyl oder C₃₋₁₀-Cycloalkyl, jeweils gesättigt oder ungesättigt, verzweigt oder unverzweigt, unsubstituiert oder einfach oder mehrfach substituiert; Naphthyl, Phenyl, Furyl, Thiophenyl, jeweils unsubstituiert oder einfach oder mehrfach substituiert; oder über C₁₋₃-Alkylen gebundenem Naphthyl, Phenyl, C₃₋₁₀-Cycloalkyl, Furyl oder Thiophenyl, jeweils unsubstituiert oder einfach oder mehrfach substituiert,
mit V ausgewählt aus OR⁴ oder NR⁴R¹¹,
mit W ausgewählt aus R¹¹, OR¹² oder NR¹¹R¹²,
mit R¹¹ und R¹² unabhängig voneinander ausgewählt aus H, C₇₋₁₀-Alkyl oder C₃₋₁₀-Cycloalkyl, jeweils gesättigt oder ungesättigt, verzweigt oder unverzweigt, unsubstituiert oder einfach oder mehrfach substituiert; Naphthyl, Phenyl, Furyl oder Thiophenyl, jeweils unsubstituiert oder einfach oder mehrfach substituiert; oder über C₁₋₃-Alkylen gebundenem Naphthyl, Phenyl, C₃₋₁₀-Cycloalkyl, Furyl oder Thiophenyl, jeweils unsubstituiert oder einfach oder mehrfach substituiert,
wobei man im Zusammenhang mit Alkyl und Cycloalkyl von R⁴, R⁵, R¹¹ und R¹² unter substituiert die Substitution mit F, Cl, Br, I, NH₂, SH oder OH versteht, und
im Zusammenhang mit Naphthyl, Phenyl, Furyl und Thiophenyl von R⁴, R⁵, R¹¹ und R¹² unter substituiert die Substitution des Naphthyl, Phenyl, Furyl oder Thiophenyl mit R²³, OR²³, einem Halogen, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen versteht, wobei
der Rest R²³ für H, einen C₁₋₁₀-Alkyl-, einen Naphthyl-, Phenyl-, Furyl- oder Thiophenyl-Rest oder für einen über eine C₁₋₃-Alkylken-Gruppe gebundenen Naphthyl-, Phenyl-, Furyl- oder Thiophenyl-Rest steht, wobei diese Naphthyl-, Phenyl-, Furyl- und Thiophenyl-Reste nicht selbst mit Aryl- oder Heteroaryl-Resten substituiert sein dürfen,
die Reste R²⁴ und R²⁵, gleich oder verschieden, für H, einen C₁₋₁₀-Alkyl-, einen Naphthyl-, Phenyl-, Furyl- oder Thiophenyl-Rest oder einen über eine C₁₋₃-Alkylen-Gruppe gebundenen Naphthyl-, Phenyl-, Furyl- oder Thiophenyl-Rest stehen, wobei diese Naphthyl-, Phenyl-, Furyl- und Thiophenyl-Reste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, Naphthyl-, Phenyl-, Furyl- oder Thiophenyl-Rest oder für einen über eine C₁₋₃-Alkylen-Gruppe gebundenen Naphthyl-, Phenyl-, Furyl- oder Thiophenyl-Rest steht, wobei diese Naphthyl-, Phenyl-, Furyl- oder Thiophenyl-Reste nicht selbst mit Aryl- oder Heteroaryl-Resten substituiert sein dürfen,
gegebenenfalls in Form ihrer Racemate, ihrer reinen Stereoisomeren, oder in Form von Mischungen der Stereoisomeren in einem beliebigen Mischungsverhältnis, in dargestellter Form oder in Form ihrer Säuren oder ihrer Basen oder in Form ihrer Salze, oder in Form ihrer Solvate, mit Ausnahme von Verbindungen, bei denen
R², R³ unabhängig voneinander ausgewählt sind aus
H, C₁-C₁₀-Alkyl, verzweigt oder unverzweigt, einfach oder mehrfach substituiert oder unsubstituiert, gesättigt oder ungesättigt; C₃-C₇-Cycloalkyl, gesättigt oder ungesättigt, einfach oder mehrfach substituiert oder unsubstituiert, oder einem entsprechenden Heterocyclus, bei dem ein C-Atom im Ring durch N, S oder O ersetzt ist, jeweils einfach oder mehrfach substituiert oder unsubstituiert;
oder
R² und R³ zusammen einen Ring bilden und CH₂CH₂OCH₂CH₂, CH₂CH₂NR⁶CH₂CH₂ oder (CH₂)₃₋₆ bedeuten;
X in der Formel I gemäß Teilformel la der Formel I 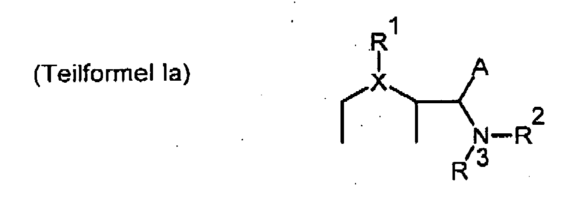 die folgende Bedeutung hat 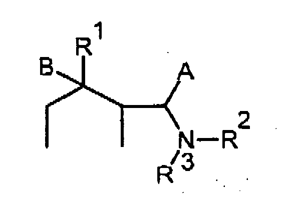 also X 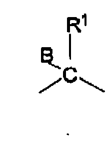 bedeutet,
A Wasserstoff ist,
R¹ ein unsubstituierter oder ein- oder zweifach substituierter Phenylring ist,
B ausgewählt ist aus
H, F, Cl, OH oder OR⁷, wobei R⁷ ausgewählt ist aus C₁-C₁₀-Alkyl, verzweigt oder unverzweigt, einfach oder mehrfach substituiert oder unsubstituiert; gesättigt oder ungesättigt; C₃-C₇-Cycloalkyl, gesättigt oder ungesättigt, einfach oder mehrfach substituiert oder unsubstituiert, oder einem entsprechenden Heterocyclus, bei dem ein C-Atom im Ring durch N, S oder O ersetzt ist; jeweils einfach oder mehrfach substituiert oder unsubstituiert; und gleichzeitig die Gruppierung 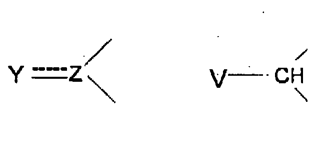 bedeutet mit V ausgewählt aus OR⁴ mit R⁴ ausgewählt aus COR⁵ und mit R⁵ ausgewählt aus C₁₋₆-Alkyl, unsubstituiert oder einfach oder mehrfach substituiert.

2. Substituierte C-Cyclohexylmethylamin-Derivate gemäß Anspruch 1, **dadurch gekennzeichnet, daß**
R¹ einer Verbindung gemäß der allgemeinen Formel II entspricht 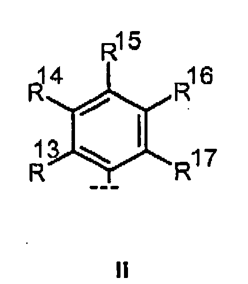 , worin
R¹³, R¹⁵ und R¹⁷ unabhängig voneinander ausgewählt sind aus H, F, Cl, Br, I, OR¹⁸, SR¹⁸, SO₂R¹⁸, SO₂OR¹⁸, CN, COOR¹⁸, NR¹⁹R²⁰; C₁₋₁₀-Alkyl, C₃₋₁₀-Cycloalkyl, jeweils unsubstituiert oder einfach oder mehrfach, gleich oder verschieden, substituiert mit F, Cl, Br, I, NH₂, SH oder OH, oder Silyl, gesättigt oder ungesättigt, verzweigt oder unverzweigt,
R¹⁴ und R¹⁶ unabhängig voneinander ausgewählt sind aus H, F, Cl, Br, I, SO₂R¹⁸, SO₂OR¹⁸, CN, COOR¹⁸, NR¹⁹R²⁰; C₁₋₁₀-Alkyl, C₃₋₁₀-Cycloalkyl, jeweils unsubstituiert oder einfach oder mehrfach, gleich oder verschieden, substituiert mit F, Cl, Br, I, NH₂, SH oder OH, oder Silyl, gesättigt oder ungesättigt, verzweigt oder unverzweigt,
oder die Reste R¹³ und R¹⁴ oder R¹⁴ und R¹⁵ zusammen jeweils die Gruppe OCH₂O, OCH₂CH₂O, CH=CHO, CH=C(CH₃)O oder CH=CHNH bilden und R¹⁵-R¹⁷ oder R¹³, R¹⁶ und R¹⁷ die oben genannte Bedeutung haben,
mit R¹⁸ ausgewählt aus H; C₁₋₁₀-Alkyl oder C₃₋₁₀-Cycloalkyl, jeweils gesättigt oder ungesättigt, verzweigt oder unverzweigt,;
R¹⁹ und R²⁰ unabhängig voneinander ausgewählt aus H; C₁₋₁₀-Alkyl oder C₃₋₁₀-Cycloalkyl, jeweils gesättigt oder ungesättigt, verzweigt oder unverzweigt,
oder R¹⁹ und R²⁰ zusammen CH₂CH₂OCH₂CH₂, CH₂CH₂NR²¹ CH₂CH₂ oder (CH₂)₃₋₆ bilden,
mit R²¹ ausgewählt aus H, C₁₋₁₀-Alkyl, gesättigt oder ungesättigt, verzweigt oder unverzweigt, jeweils unsubstituiert oder einfach oder mehrfach, gleich oder verschieden, substituiert mit F, Cl, Br, I, NH₂, SH oder OH,
und 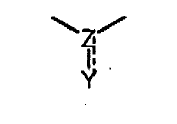 ausgewählt ist aus 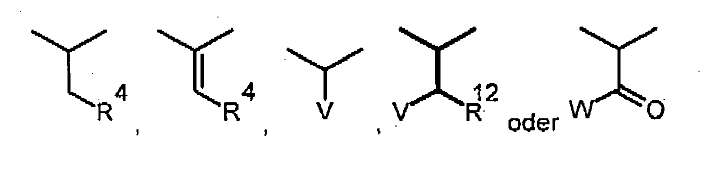 mit R⁴ ausgewählt aus COR^{5a}, SO₂R⁵; C₁₋₁₀-Alkyl oder C₃₋₁₀-Cycloalkyl, jeweils gesättigt oder ungesättigt, verzweigt oder unverzweigt, unsubstituiert oder einfach oder mehrfach substituiert; Naphthyl, Phenyl, Furyl oder Thiophenyl, jeweils unsubstituiert oder einfach oder mehrfach substituiert; oder über G₁₋₃-Alkylen gebundenem Naphthyl, Phenyl, C₃₋₁₀-Cycloalkyl, Furyl oder Thiophenyl, jeweils unsubstituiert oder einfach oder mehrfach substituiert,
mit R⁵ ausgewählt aus C₁₋₁₀-Alkyl oder C₃₋₁₀-Cycloalkyl, jeweils gesättigt oder ungesättigt, verzweigt oder unverzweigt, unsubstituiert oder einfach oder mehrfach substituiert; Naphthyl, Phenyl, Furyl oder Thiophenyl, jeweils unsubstituiert oder einfach oder mehrfach substituiert; oder über C₁₋₃-Alkylen gebundenem Phenyl, Naphthyl, C₃₋₁₀-Cycloalkyl, Furyl oder Thiophenyl, jeweils unsubstituiert oder einfach oder mehrfach substituiert,
mit R^{5a} ausgewählt aus C₃₋₁₀-Cycloalkyl, gesättigt oder ungesättigt, verzweigt oder unverzweigt, unsubstituiert oder einfach oder mehrfach substituiert; Naphthyl, Phenyl, Furyl oder Thiophenyl, jeweils unsubstituiert oder einfach oder mehrfach substituiert; oder über C₁₋₃-Alkylen gebundenem Naphthyl, Phenyl, C₃₋₁₀-Cycloalkyl, Furyl oder Thiophenyl, jeweils unsubstituiert oder einfach oder mehrfach substituiert,
mit V ausgewählt aus OR⁴ oder NR⁴R¹¹,
mit W ausgewählt aus R¹¹, OR¹² oder NR¹¹R¹²,
mit R¹¹ und R¹² unabhängig voneinander ausgewählt aus H, C₇₋₁₀-Alkyl oder C₃₋₁₀-Cycloalkyl, jeweils gesättigt oder ungesättigt, verzweigt oder unverzweigt, unsubstituiert oder einfach oder mehrfach substituiert; Naphthyl, Phenyl, Furyl, oder Thiophenyl, jeweils unsubstituiert oder einfach oder mehrfach substituiert; oder über C₁₋₃-Alkylen gebundenem Naphthyl, Phenyl, C₃₋₁₀-Cycloalkyl, Furyl oder Thiophenyl, jeweils unsubstituiert oder einfach oder mehrfach substituiert,
wobei man im Zusammenhang mit Alkyl und Cycloalkyl von R⁴, R⁵, R^{5a}, R¹¹ und R¹² unter substituiert die Substitution mit F, Cl, Br, I, NH₂, SH oder OH versteht, und im Zusammenhang mit Naphthyl, Phenyl, Furyl und Thiophenyl von R⁴, R⁵, R^{5a}, R¹¹ und R¹² unter substituiert die Substitution des Naphthyl, Phenyl, Furyl oder Thiophenyl mit R²³, OR²³, einem Halogen, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen versteht, wobei
der Rest R²³ für H, einen C₁₋₁₀-Alkyl-, einen Naphthyl-, Phenyl-, Furyl- oder Thiophenyl-Rest oder für einen über eine C₁₋₃-Alkylen-Gruppe gebundenen Naphthyl-, Phenyl-, Furyl- oder Thiophenyl-Rest steht, wobei diese Naphthyl-, Phenyl-, Furyl- oder Thiophenyl-Reste nicht selbst mit Aryl- oder Heteroaryl-Resten substituiert sein dürfen,
die Reste R²⁴ und R²⁵, gleich oder verschieden, für H, einen C₁₋₁₀-Alkyl-, einen Naphthyl-, Phenyl-, Furyl- oder Thiophenyl-Rest oder einen über eine C₁₋₃-Alkylen-Gruppe gebundenen Naphthyl-, Phenyl-, Furyl- oder Thiophenyl-Rest stehen, wobei diese Naphthyl-, Phenyl-, Furyl- und Thiophenyl-Reste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, Naphthyl-, Phenyl-, Furyl- oder Thiophenyl-Rest oder für einen über eine C₁₋₃-Alkylen-Gruppe gebundenen Naphthyl-, Phenyl-, Furyl- oder Thiophenyl-Rest steht, wobei diese Naphthyl-, Phenyl-, Furyl- oder Thiophenyl-Reste nicht selbst mit Aryl- oder Heteroaryl-Resten substituiert sein dürfen.

3. Substituierte C-Cyclohexylmethylamin-Derivate gemäß Anspruch 1 oder 2, **dadurch gekennzeichnet, daß** A Wasserstoff ist.

4. Substituierte C-Cyclohexylmethylamin-Derivate gemäß einem der Ansprüche 1 bis 3, **dadurch gekennzeichnet, daß**
R² und R³ ausgewählt sind aus C₁₋₆-Alkyl, gesättigt oder ungesättigt, verzweigt oder unverzweigt,
oder die Reste R² und R³ zusammen CH₂CH₂NR⁶CH₂CH₂ oder (CH₂)₃₋₆ bedeuten, mit R⁶ ausgewählt aus H oder C₁₋₆-Alkyl, gesättigt, verzweigt oder unverzweigt und unsubstituiert.

5. Substituierte C-Cyclohexylmethylamin-Derivate gemäß einem der Ansprüche 1 bis 4, **dadurch gekennzeichnet, daß**
R¹ ausgewählt ist aus; C₁₋₆-Alkyl, gesättigt oder ungesättigt, verzweigt oder unverzweigt; C₃₋₆-Cycloalkyl, Naphthyl, Phenyl, Furyl, Thiophenyl, über G₁₋₃-Alkylen oder Ethinyl gebundenem Naphthyl, Phenyl, C₃₋₆-Cycloalkyl oder über C₁₋₃-Alkylen oder Ethinyl gebundenem Thiophenyl oder Furyl, die gemäß Anspruch 1 unsubstituiert oder ein- oder mehrfach substituiert sein können.

6. Substituierte C-Cyclohexylmethylamin-Derivate gemäß einem der Ansprüche 1 bis 5, **dadurch gekennzeichnet, daß** 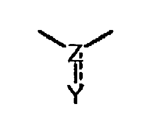 ausgewählt ist aus

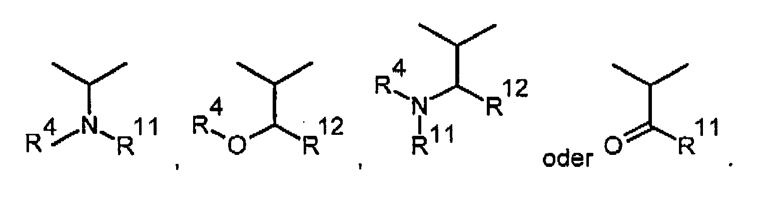

7. Substituierte C-Cyclohexylmethylamin-Derivate gemäß Anspruch 1, **dadurch gekennzeichnet, daß**
A ausgewählt ist aus H oder Phenyl,
**und/oder**
R¹ ausgewählt ist aus Naphthyl, unsubstituiert oder einfach oder mehrfach substituiert mit Resten unabhängig voneinander ausgewählt aus
F, Cl, Br, I, OR¹⁸; C₁₋₄-Alkyl, verzweigt oder unverzweigt, unsubstituiert oder einfach oder mehrfach, gleich oder verschieden, substituiert mit F, Cl, Br, I, NH₂, SH oder OH;
mit R¹⁸ ausgewählt aus H; C₁₋₄-Alkyl, verzweigt oder unverzweigt,
**und/oder**
R² und R³ ausgewählt sind aus C₁₋₆-Alkyl, gesättigt oder ungesättigt, verzweigt oder unverzweigt,
oder die Reste R² und R³ zusammen CM₂CH₂NR⁶CM₂CM₂ oder (CH₂)₄-₅ bedeuten, mit R⁶ ausgewählt aus H oder C₁₋₆-Alkyl, gesättigt, verzweigt oder unverzweigt und unsubstituiert;
**und/oder**
X in der Formel I gemäß Teilformel la der Formel I 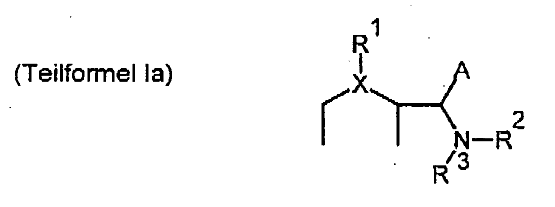 ausgewählt ist aus den folgenden Bedeutungen 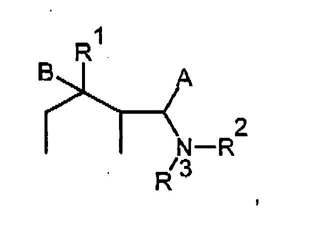 , also X 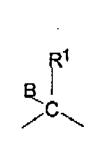 bedeutet, mit B ausgewählt aus OH, OR⁷, H, F, Cl, mit R⁷ ausgewählt aus C₁₋₄-Alkyl, verzweigt oder unverzweigt, unsubstituiert oder einfach oder mehrfach, gleich oder verschieden, substituiert mit F, Cl, Br, I, NH₂, SH oder OH;
**und/oder** 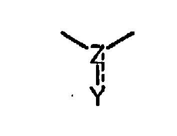 ausgewählt ist aus 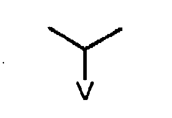 mit V ausgewählt aus OR⁴,
mit R⁴ ausgewählt aus COR⁵; Phenyl oder Benzyl, die gemäß Anspruch 1 unsubstituiert oder ein- oder mehrfach substituiert sein können;
mit R⁵ ausgewählt aus C₁₋₁₀-Alkyl, gesättigt oder ungesättigt, verzweigt oder unverzweigt, unsubstituiert oder einfach oder mehrfach, gleich oder verschieden, substituiert mit F, Cl, Br, I, NH₂, SH oder OH; oder Phenyl, das gemäß Anspruch 1 unsubstituiert oder ein- oder mehrfach substituiert sein kann.

8. Substituierte C-Cyclohexylmethylamin-Derivate gemäß einem der Ansprüche 1 bis 7, **dadurch gekennzeichnet, daß** sie ausgewählt sind aus der folgenden Gruppe:
• 4-Benzyloxy-1-(4-brom-phenyl)-2-dimethylaminomethyl-cyclohexanol;
• 4-Benzyloxy-2-dimethylaminomethyl-1-(4-trifluormethyl-phenyl)-cyclohexanol;
• 4-Benzyloxy-1-(3,4-difluor-phenyl)-2-dimethylaminomethyl-cyclohexanol;
• 4-Benzyloxy-2-dimethylaminomethyl-1-(3-fluor-4-methyl-phenyl)-cyclohexanol;
• 4-Benzyloxy-1-(3,4-dichlor-phenyl)-2-dimethylaminomethyl-cyclohexanol;
• 4-Benzyloxy-2-dimethylaminomethyl-1-naphthalen-2-yl-cyclohexanol;
• 4-Benzyloxy-2-dimethylaminomethyl-1-(3,4-dimethyl-phenyl)-cyclohexanol;
• 4-Benzyloxy-2-dimethylaminomethyl-1-(4-fluor-3-methyl-phenyl)-cyclohexanol;
• 4-Benzyloxy-2-dimethylaminomethyl-1-(5-fluor-2-methyl-phenyl)-cyclohexanol;
• 4-Benzyloxy-1-(3-chlor-4-fluor-phenyl)-2-dimethylaminomethyl-cyclohexanol;
• 4-Benzyloxy-1-(5-chlor-2-methoxy-phenyl)-2-dimethylaminomethyl-cyclohexanol;
• 4-Benzyloxy-1-(2-brom-phenyl)-2-dimethylaminomethyl-cyclohexanol;
• 4-Benzyloxy-2-dimethylaminomethyl-1-(2-methoxy-phenyl)-cyclohexanol;
• 4-Benzyloxy-2-dimethylaminomethyl-1-(2-methylsulfanyl-phenyl)-cyclohexanol;
• 4-Benryloxy-1-(4-chlor-phenyl)-2-dimethylaminomethyl-cyclohexanol;
• 4-Benzyloxy-1-(3-brom-phenyl)-2-dimethylaminomethyl-cyclohexanol:
• 4-Benzyloxy-1-(4-brom-3-fluor-phenyl)-2-dimethylaminomethyl-cyclohexanol;
• 4-Benzyloxy-2-dimethylaminomethyl-1-(4-methoxy-phenyl)-cyclohexanol:
• 4-Benzyloxy-1-(2-chlor-phenyl)-2-dimethylaminomethyl-cyclohexanol;
• 4-Benzyloxy-2-dimethylaminomethyl-1-(3-trifluormethyl-phenyl)-cyclohexanol;
• 4-Benzyloxy-2-dimethylaminomethyl-1-(4-pentyl-phenyl)-cyclohexanol;
• 4-Benzyloxy-2-dimethylaminomethyl-1-(2,5-dimethyl-phenyl)-cyclohexanol;
• 4-Benzyloxy-2-dimethylaminomethyl-1-(4-isopropyl-phenyl)-cyclohexanol;
• 4-Benzyloxy-2-dimethylaminomethyl-1-(2,3-dimethyl-phenyl)-cyclohexanol;
• 4-Benzyloxy-2-dimethylaminomethyl-1-(3-fluor-phenyl)-cyclohexanol;
• 4-Benzyloxy-1-(3,5-bis-trifluormethyl-phenyl)-2-dimethylaminomethyl-cyclohexanol;
• 4-Benzyloxy-2-dimethylaminomethyl-1-(4-ethyl-phenyl)-cyclohexanol;
• 4-Benzyloxy-1-(3,5-dichlor-phenyl)-2-dimethylaminomethyl-cyclohexanol;
• 2-Dimethylaminomethyl-4-(4-fluor-benzyloxy)-1-o-tolyl-cyclohexanol;
• 2-Dimethylaminomethyl-4-(4-fluor-benzyloxy)-1-p-tolyl-cyclohexanol;
• 2-Dimethylaminomethyl-1-(2,5-dimethyl-phenyl)-4-(4-fluor-benzyloxy)-cyclohexanol;
• 2-Dimethylaminomethyl-1-(3,4-dimethyl-phenyl)-4-(4-fluor-benzyloxy)-cyclohexanol;
• 2-Dimethylaminomethyl-1-(3,5-dimethyl-phenyl)-4-(4-fluor-benzyloxy)-cyclohexanol;
• 2-Dimethylaminomethyl-4-(4-fluor-benzyloxy)-1-(4-isopropyl-phenyl)-cyclohexanol;
• 2-Dimethylaminomethyl-4-(4-fluor-benzyloxy)-1-(3-fluor-phenyl)-cyclohexanol;
• 2-Dimethylaminomethyl-4-(4-fluor-benzyloxy)-1-(4-fluor-phenyl)-cyclohexanol;
• 1-(3,4-Difluor-phenyl)-2-dimethylaminomethyl-4-(4-fluor-benzyloxy)-cyclohexanol;
• 2-Dimethylaminomethyl-4-(4-fluor-benzyloxy)-1-(3-fluor-4-methyl-phenyl)-cyclohexanol;
• 2-Dimethylaminomethyl-4-(4-fluor-benzyloxy)-1-(4-fluor-3-methyl-phenyl)-cyclohexanol;
• 1-(2-Chlor-phenyl)-2-dimethylaminomethyl-4-(4-fluor-benzyloxy)-cyclohexanol;
• 1-(3-Chlor-phenyl)-2-dimethylaminomethyl-4-(4-fluor-benzyloxy)-cyclohexanol;
• 1-(4-Chior-phenyl)-2-dimethylaminomethyl-4-(4-fluor-benzyloxy)-cyclohexanol;
• 1-(3,4-Dichlor-phenyl)-2-dimethylaminomethyl-4-(4-fluor-benzyloxy)-cyclohexanol;
• 1-(4-Chlor-3-fluor-phenyl)-2-dimethylaminomethyl-4-(4-fluor-benzyloxy)-cyclohexanol;
• 1-(3,5-Dichlor-phenyl)-2-dimethylaminomethyl-4-(4-fluor-benzyloxy)-cyclohexanol;
• 1-(4-Chlor-2-methoxy-phenyl)-2-dimethylaminomethyl-4-(4-fluor-benzyloxy)-cyclohexanol;
• 2-Dimethylaminomethyl-4-(4-fluor-benzyloxy)-1-(4-trifluormethyl-phenyl)-cyclohexanol;
• 1-(3,5-Bis-trifluormethyl-phenyl)-2-dimethylaminomethyl-4-(4-fluor-benzyloxy)-cyclohexanol;
• 2-Dimethylaminomethyl-4-(4-fluor-benzyloxy)-1-(2-methoxy-phenyl)-cyclohexanol;
• 2-Dimethylaminomethyl-4-(4-fluor-benzyloxy)-1-(2-methylsulfanyl-phenyl)-cyclohexanol;
• 3,4-Dichlor-N-[3-dimethylaminomethyl-4-hydroxy-4-(3-methoxy-phenyl)-cyclohexyl]-benzamid;
• Naphthalene-2-carbonsäure [3-dimethylaminomethyl-4-hydroxy-4-(3-methoxyphenyl)-cyclohexyl]-amid;
• N-[3-Dimethylaminomethyl-4-hydroxy-4-(3-methoxy-phenyl)-cyclohexyl]-3-phenyl-propionamid;
• N-[3-Dimethylaminomethyl-4-hydroxy-4-(3-methoxy-phenyl)-cyclohexyl]-4-nitro-benzamid;
• N-[3-Dimethylaminomethyl-4-hydroxy-4-(3-methoxy-phenyl)-cyclohexyl]-4-methyl-3-nitro-benzamid;
• N-[3-Dimethylaminomethyl-4-hydroxy-4-(3-methoxy-phenyl)-cyclohexyl]-3,4,5-trimethoxy-benzamid;
• 2-(4-Chlor-phenoxy)-N-[3-dimethylaminomethyl-4-hydroxy-4-(3-methoxyphenyl)-cyclohexyl]-acetamid;
• N-[3-Dimethylaminomethyl-4-hydroxy-4-(3-methoxy-phenyl)-cyclohexyl]-3-nitro-benzamid;
• Furan-2-carbonsäure [3-dimethylaminomethyl-4-hydroxy-4-(3-methoxyphenyl)-cyclohexyl]-amid;
• N-[3-Dimethylaminomethyl-4-hydroxy-4-(3-methoxy-phenyl)-cyclohexyl]-2-phenoxy-acetamid;
• N-[3-Dimethylaminomethyl-4-hydroxy-4-(3-methoxy-phenyl)-cyclohexyl]-4-trifluormethyl-benzamid;
• N-[3-Dimethylaminomethyl-4-hydroxy-4-(3-methoxy-phenyl)-cyclohexyl]-4-methoxy-benzamid;
• 2-Dimethylaminomethyl-1-(4-fluor-phenyl)-4-phenethyl-cyclohexanol;
• 1-(4-Brom-phenyl)-2-dimethylaminomethyl-4-phenethyl-cyclohexanol;
• 2-Dimethylaminomethyl-1-(4-ethyl-phenyl)-4-phenethyl-cyclohexanol;
• 2-Dimethylaminomethyl-1-(4-isopropyl-phenyl)-4-phenethyl-cyclohexanol;
• 2-Dimethylaminomethyl-1-(4-methoxy-phenyl)-4-phenethyl-cyclohexanol;
• 2-Dimethylaminomethyl-1-(2,4-dimethyl-phenyl)-4-phenethyl-cyclohexanol;
• 2-Dimethylaminomethyl-1-(2-methylsulfanyl-phenyl)-4-phenethyl-cyclohexanol;
• 2-Dimethylaminomethyl-1-(5-fluor-2-methyl-phonyl)-4-phenethyl-cyclohexanol;
• 2-Dimethylaminomethyl-1-(2,5-dimethyl-phenyl)-4-phenethyl-cyclohexanol;
• 2-Dimethylaminomethyl-4-phenethyl-1-p-tolyl-cyclohexanol;
• 2-Dimethylaminomethyl-1-(3-fluor-phenyl)-4-phenethyl-cyclohexanol;
• 1-(3-Chlor-phenyl)-2-dimethylaminomethyl-4-phenethyl-cyclohexanol;
• 1-(4-Chlor-3-fluor-phenyl)-2-dimethylaminomethyl-4-phenethyl-cyclohexanol;
• 2-Dimethylaminomethyl-4-phenethyl-1-(3-trifluormethyl-phenyl)-cyclohexanol;
• 1-(3,5-Dichlor-phenyl)-2-dimethylaminomethyl-4-phenethyl-cyclohexanol;
• 2-Dimethylaminomethyl-1-(3,4-dimethyl-phenyl)-4-phenethyl-cyciohexanol;
• 1-(3,4-Dichlor-phenyl)-2-dimethylaminomethyl-4-phenethyl-cyclohexanol;
• 1-(4-Chlor-phenyl)-2-dimethylaminomethyl-4-phenethyl-cyclohexanol;
• 2-Dimethylaminomethyl-1-(3-fluor-4-methyl-phenyl)-4-phenethyl-cyclohexanol;
• 2-Dimethylaminomethyl-1-(3,5-dimethyl-phenyl)-4-phenethyl-cyclohexanol;
• 1-(4-Brom-3-fluor-phenyl)-2-dimethylaminomethyl-4-phenethyl-cyclohexanol;
• 1-(3,4-Difluor-phenyl)-2-dimethylaminomethyl-4-phenethyl-cyclohexanol;
• 2-Dimethylaminomethyl-1-(2,3-dimethyl-phenyl)-4-phenethyl-cyclohexanol;
• 2-Dimethylaminomethyl-1-naphthalin-1-yl-4-phenethyl-cyclohexanol;
• 1-(3,5-Bis-trifluormethyl-phenyl)-2-dimethylaminomethyl-4-phenethyl-cyclohexanol;
• 1-(3-Brom-phenyl)-2-dimethylaminomethyl-4-phenethyl-cyclohexanol;
• 2-Dimethylaminomethyl-1-(4-fluor-3-methyl-phenyl)-4-phenethyl-cyclohexanol;
• 2-Dimethylaminomethyl-4-phenethyl-1-m-tolyl-cyclohexanol;
• 4-Benzyl-2-dimethylaminomethyl-1-m-tolyl-cyclohexanol;
• 4-Benzyl-1-(3,4-difluor-phenyl)-2-dimethylaminomethyl-cyclohexanol;
• 4-Benzyl-2-dimethylaminomethyl-1-p-tolyl-cyclohexanol;
• 2-Dimethylaminomethyl-4-(3-methoxy-benzyl)-1-p-tolyl-cyclohexanol;
• 1-(4-Chlor-phenyl)-2-dimethylaminomethyl-4-(3-methoxy-benzyl)-cyclohexanol;
• 1-(4-Brom-3-fluor-phenyl)-2-dimethylaminomethyl-4-(3-methoxy-benzyl)-cyclohexanol;
• 2-Dimethylaminomethyl-4-(4-fluor-benzyl)-1-p-tolyl-cyclohexanol;
• 1-(4-Chlor-phenyl)-2-dimethylaminomethyl-4-(4-fluor-benzyl)-cyclohexanol;
• [2-(3-Chlor-4-fluor-phenyl)-2-fluor-5-phenethyl-cyclohexylmethyl]-dimethylamin;
• [2-Fluor-5-(4-fluor-benzyl)-2-p-tolyl-cyclohexylmethyl]-dimethyl-amin;
• Benzyl-2-(dimethylamino-phenyl-methyl)-4-phenyl-cyclohexanol
• 2-(Dimethylamino-phenyl-methyl)-4-phenyl-1-vinyl-cyclohexanol
• 1-(4-tert-Butyl-phenyl)-2-(dimethylamino-phenyl-methyl)-4-phenyl-cyclohexanol
• 2-(Dimethylamino-phenyl-methyl)-4-phenyl-1-m-tolyl-cyclohexanol
• 2-(Dimethylamino-phenyl-methyl)-1-phenethyl-4-phenyl-cyclohexanol
• 2-(Dimethylamino-phenyl-methyl)-4-phenyl-1-phenylethynyl-cyclohexanol
• 2-(Dimethylamino-phenyl-methyl)-1-(3-methoxy-phenyl)-4-phenyl-cyclohexanol
• 2-(Dimethylamino-phenyl-methyl)-4-phenyl-1-(3-phenyl-propyl)-cyclohexanol
• 2-(Dimethylamino-phenyl-methyl)-1-(4-methoxy-phenyl)-4-phenyl-cyclohexanol
• 2-(Dimethylamino-phenyl-methyl)-1-(2-methoxy-phenyl)-4-phenyl-cyclohexanol
• 4-Benzyloxy-2-dimethylaminomethyl-1-phenyl-cyclohexanol
• 1-Benzyl-4-benzyloxy-2-dimethylaminomethyl-cyclohexanol
• 4-Benzyloxy-2-dimethylaminomethyl-1-(4-fluor-3-methyl-phenyl)-cyclohexanol
• 4-Benzyloxy-2-dimethylaminomethyl-1-o-tolyl-cyclohexanol
• 4-Benzyloxy-2-dimethylaminomethyl-1-vinyl-cyclohexanol
• 4-Benzyloxy-1-cyclopentyl-2-dimethylaminomethyl-cyclohexanol
• 4-Benzyloxy-2-dimethylaminomethyl-1-m-tolyl-cyclohexanol
• 4-Benzyloxy-2-dimethylaminomethyl-bicyclohexyl-1-ol
• 4-Benzyloxy-2-dimethylaminomethyl-1-(4-fluor-phenyl)-cyclohexanol
• 4-Benzyloxy-2-dimethylaminomethyl-1-phenylethynyl-cyclohexanol
• 4-Benzyloxy-2-dimethylaminomethyl-1-thiophen-2-yl-cyclohexanol
• 4-Benzyloxy-2-dimethylaminomethyl-1-(3-methoxy-phenyl)-cyclohexanol
• 4-Benzyloxy-2-dimethylaminomethyl-1-(3-phenyl-propyl)-cyclohexanol
• 4-Benzyloxy-2-dimethylaminomethyl-1-p-tolyl-cyclohexanol
• 4-Benzyloxy-2-dimethylaminomethyl-1-(4-methoxy-phenyl)-cyclohexanol
• 4-Benzyloxy-2-dimethylaminomethyl-1-(3-fluor-phenyl)-cyclohexanol
• 4-Benzyloxy-1-(3-chlor-phenyl)-2-dimethylaminomethyl-cyclohexanol
• 4-Benzyloxy-2-dimethylaminomethyl-1-(3-methoxy-benzyl)-cyclohexanol
• 4-Benzyloxy-1-(4-chlor-3-trifluormethyl-phenyl)-2-dimethylaminomethyl-cyclohexanol
• 4-Benzyloxy-2-dimethylaminomethyl-1-(3-fluor-benzyl)-cyclohexanol
• 4-Benzyloxy-2-dimethylaminomethyl-1-(2-methyl-benzyl)-cyclohexanol
• 4-Benzyloxy-2-dimethylaminomethyl-1-(2,5-dimethyl-benzyl)-cyclohexanol
• 4-(4-Chlor-benzyl)-2-dimethylaminomethyl-1-(3-methoxy-phenyl)-cyclohexanol
• 4-(4-Chlor-benzyl)-2-dimethylaminomethyl-1-(3-phenyl-propyl)-cyclohexanol
• 4-(4-Chlor-benzyl)-1-(2,3-dichlor-phenyl)-2-dimethylaminomethyl-cyclohexanol
• 4-(4-Chlor-benzyl)-1-cyclohexylmethyl-2-dimethylaminomethyl-cyclohexanol
• 4-(4-Chlor-benzyl)-2-dimethylaminomethyl-1-(5-fluor-2-methoxy-phenyl)-cyclohexanol
• 4-(4-Chlor-benzyl)-2-dimethylaminomethyl-1-(3-fluor-phenyl)-cyclohexanol
• 4-(4-Chlor-benzyl)-1-(3-chlor-phenyl)-2-dimethylaminomethyl-cyclohexanol
• 4-(4-Chlor-benzyl)-1-(3,5-dichlor-phenyl)-2-dimethylaminomethyl-cyclohexanol
• 4-(4-Chlor-benzyl)-1-(2-chlor-benzy!)-2-dimethylaminomethyl-cyclohexanol
• 4-(4-Chlor-benzyl)-2-dimethylaminomethyl-1-(4-fluor-benzyl)-cyclohexanol
• 4-(4-Chlor-benzyl)-2-dimethylaminomethyl-1-(3-fluor-benzyl)-cyclohexanol
• 4-(4-Chlor-benzyl)-2-dimethylaminomethyl-1-(2-methoxy-phenyl)-cyclohexanol
• 4-(4-Chlor-benzyl)-2-dimethylaminomethyl-1-(2-methyl-benzyl)-cyclohexanoi
• 4-(4-Chlor-benzyl)-2-dimethylaminomethyl-1-(3-methyl-benzyl)-cyclohexanol
• 1,4-Bis-(4-chlor-benzyl)-2-dimethylaminomethyl-cyclohexanol
• 4-(4-Chlor-benzyl)-1-(2-chlor-6-fluor-benzyl)-2-dimethylaminomethyl-cyclohexanol
• 4-(4-Chlor-benzyl)-2-dimethylaminomethyl-1-(2,5-dimethyl-benzyl)-cyclohexanol
• 4-(4-Chlor-benzyl)-1-(3-chlor-benzyl)-2-dimethylaminomethyl-cyclohexanol
• 4-(4-Chlor-benzyl)-1-(2,4-dichlor-benzyl)-2-dimethylaminomethyl-cyclohexanol
• 4-(4-Chlor-benzyl)-2-dimethylaminomethyl-1-phenyl-cyclohexanol
• 4-(4-Chlor-benzyl)-1-(4-chlor-phenyl)-2-dimethylaminomethyl-cyclohexanol
• 4-(4-Chlor-benzyl)-2-dimethylaminomethyl-1-(4-fluor-3-methyl-phenyl)-cyclohexanol
• 4-(4-Chlor-benzyl)-2-dimethylaminomethyl-1-o-tolyl-cyclohexanol
• 4-(4-Chlor-benzyl)-2-dimethylaminomethyl-1-vinyl-cyclohexanol
• 4-(4-Chlor-benzyl)-1-cyclopentyl-2-dimethylaminomethyl-cyclohexanol
• 4-(4-Chlor-benzyl)-2-dimethylaminomethyl-1-m-tolyl-cyclohexanol
• 4-(4-Chlor-benzyl)-2-dimethylaminomethyl-bicyclohexyl-1-ol
• 4-(4-Chlor-benzyl)-2-dimethylaminomethyl-1-(4-fluor-phenyl)-cyclohexanol
• 4-(4-Chlor-benzyl)-2-dimethylaminomethyl-1-phenethyl-cyclohexanol
• 4-(4-Chlor-benzyl)-2-dimethylaminomethyl-1-phenylethynyl-cyclohexanol
• 4-(4-Chlor-benzyl)-2-dimethylaminomethyl-1-thiophen-2-yl-cyclohexanol
• 4-(4-Chlor-benzyl)-2-dimethylaminomethyl-1-p-tolyl-cyclohexanol
• 4-(4-Chlor-benzyl)-2-dimethylaminomethyl-1-(4-methoxy-phenyl)-cyclohexanol
• 4-(4-Chlor-benzyl)-2-dimethylaminomethyl-1-trimethylsilanylethynyl-cyclohexanol
• 4-(4-Chlor-benzyl)-1-(4-chlor-3-trifluormethyl-phenyl)-2-dimethylaminomethyl-cyclohexanol
• 4-(4-Chlor-benzyl)-1-(3-chlor-4-fluor-phenyl)-2-dimethylaminomethyl-cyclohexanol
• 4-(4-Chlor-benzyl)-2-dimethylaminomethyl-1-(3-trifluormethyl-phenyl)-cyclohexanol
• 2-Dimethylaminomethyl-4-(4-fluor-benzyl)-1-phenyl-cyclohexanol
• 1-(4-chlor-phenyl)-2-dimethylaminomethyl-4-(4-fluor-benzyl)-cyclohexanol
• 1-Benzyl-2-dimethylaminomethyl-4-(4-fluor-benzyl)-cyclohexanol
• 2-Dimethylaminomethyl-4-(4-fluor-benzyl)-1-(4-fluor-3-methyl-phenyl)-cyclohexanol
• 2-Dimethylaminomethyl-4-(4-fluor-benzyl)-1-o-tolyl-cyclohexanol
• 2-Dimethylaminomethyl-4-(4-fluor-benzyl)-1-vinyl-cyclohexanol
• 1-(4-tert-Butyl-phenyl)-2-dimethylaminomethyl-4-(4-fluor-benzyl)-cyclohexanol
• 1-Cyclopentyl-2-dimethylaminomethyl-4-(4-fluor-benzyl)-cyclohexanol
• 2-Dimethylaminomethyl-4-(4-fluor-benzyl)-1-m-tolyl-cyclohexanol
• 2-Dimethylaminomethyl-4-(4-fluor-benzyl)-bicyclohexyl-1-ol
• 2-Dimethylaminomethyl-4-(4-fluor-benzyl)-1-(4-fluor-phenyl)-cyclohexanol
• 2-Dimethylaminomethyl-4-(4-fluor-benzyl)-1-phenethyl-cyclohexanol
• 2-Dimethylaminomethyl-4-(4-fluor-benzyl)-1-phenylethynyl-cyclohexanol
• 2-Dimethylaminomethyl-4-(4-fluor-benzyl)-1-thiophen-2-yl-cyclohexanol
• 2-Dimethylaminomethyl-4-(4-fluor-benzyl)-1-(3-methoxy-phenyl)-cyclohexanol
• 2-Dimethylaminomethyl-4-(4-fluor-benzyl)-1-(3-phenyl-propyl)-cyclohexanol
• 1-(2,3-Dichlor-phenyl)-2-dimethylaminomethyl-4-(4-fluor-benzyl)-cyclohexanol
• 2-Dimethylaminomethyl-4-(4-fluor-benzyl)-1-p-tolyl-cyclohexanol
• 2-Dimethylaminomethyl-4-(4-fluor-benzyl)-1-(4-methoxy-phenyl)-cyclohexanol
• 1-Cyclohexylmethyl-2-dimethylaminomethyl-4-(4-fluor-benzyl)-cyclohexanol
• 2-Dimethylaminomethyl-4-(4-fluor-benzyl)-1-(5-fluor-2-methoxy-phenyl)-cyclohexanol
• 2-Dimethylaminomethyl-4-(4-fluor-benzyl)-1-(3-fluor-phenyl)-cyclohexanol
• 1-(3-chlor-phenyl)-2-dimethylaminomethyl-4-(4-fluor-benzyl)-cyclohexanol
• 1-(3,5-Dichlor-phenyl)-2-dimethylaminomethyl-4-(4-fluor-benzyl)-cyclohexanol
• 1-(2-chlor-benzyl)-2-dimethylaminomethyl-4-(4-fluor-benzyl)-cyclohexanol
• 2-Dimethylaminomethyl-1,4-bis-(4-fluor-benzyl)-cyclohexanol
• 2-Dimethylaminomethyl-4-(4-fluor-benzyl)-1-(3-methoxy-benzyl)-cyclohexanol
• 1-(4-chlor-3-trifluormethyl-phenyl)-2-dimethylaminomethyl-4-(4-fluor-benzyl)-cyclohexanol
• 2-Dimethylaminomethyl-4-(4-fluor-benzyl)-1-(3-fluor-benzyl)-cyclohexanol
• 2-Dimethylaminomethyl-4-(4-fluor-benzyl)-1-(2-methoxy-phenyl)-cyclohexanol
• 2-Dimethylaminomethyl-4-(4-fluor-benzyl)-1-(2-methyl-benzyl)-cyclohexanol
• 1-(3-chlor-4-fluor-phenyl)-2-dimethylaminomethyl-4-(4-fluor-benzyl)-cyclohexanol
• 2-Dimethylaminomethyl-4-(4-fluor-benzyl)-1-(3-trifluormethyl-phonyl)-cyclohexanol
• 2-Dimethylaminomethyl-4-(4-fluor-benzyl)-1-(3-methyl-benzyl)-cyclohexanol
• 1-(4-chlor-benzyl)-2-dimethylaminomethyl-4-(4-fluor-benzyl)-cyclohexanol
• 1-(2-chlor-6-fluor-benzyl)-2-dimethylaminomethyl-4-(4-fluor-benzyl)-cyclohexanol
• 2-Dimethylaminomethyl-1-(2,5-dimethyl-benzyl)-4-(4-fluor-benzyl)-cyclohexanol
• 1-(3-chlor-benzyl)-2-dimethylaminomethyl-4-(4-fluor-benzyl)-cyclohexanol
• 1-(2,4-Dichlor-benzyl)-2-dimethylaminomethyl-4-(4-fluor-benzyl)-cyclohexanol
• 2-Dimethylaminomethyl-4-(3-methoxy-benzyl)-1-phenyl-cyclohexanol
• 1-Benzyl-2-dimethylaminomethyl-4-(3-methoxy-benzyl)-cyclohexanol
• 2-Dimethylaminomethyl-1-(4-fluor-3-methyl-phenyl)-4-(3-methoxy-benzyl)-cyclohexanol
• 2-Dimethylaminomethyl-4-(3-methoxy-benzyl)-1-o-tolyl-cyclohexanol
• 2-Dimethylaminomethyl-4--(3-methoxy-benzyl)-1-vinyl-cyclohexanol
• 1-(4-tert-Butyl-phenyl)-2-dimethylaminomethyl-4-(3-methoxy-benzyl)-cyclohexanol
• 1-Cyclopentyl-2-dimethylaminomethyl-4-(3-methoxy-benzyl)-cyclohexanol
• 2-Dimethylaminomethyl-4-(3-methoxy-benzyl)-1-m-tolyl-cyclohexanol
• 2-Dimethylaminomethyl-4-(3-methoxy-benzyl)-bicyclohexyl-1-ol
• 2-Dimethylaminomethyl-4-(3-methoxy-benzyl)-1-phenethyl-cyclohexanol
• 2-Dimethylaminomethyl-4-(3-methoxy-benzyl)-1-phenylethynyl-cyclohexanol
• 2-Dimethylaminomethyl-4-(3-methoxy-benzyl)-1-thiophen-2-yl-cyclohexanol
• 2-Dimethylaminomethyl-4-(3-methoxy-benzyl)-1-(3-methoxy-phenyl)-cyclohexanol
• 2-Dimethylaminomethyl-4-(3-methoxy-benzyl)-1-(3-phenyl-propyl)-cyclohexanol
• 1-(2,3-Dichlor-phenyl)-2-dimethylaminomethyl-4-(3-methoxy-benzyl)-cyclohexanol
• 2-Dimethylaminomethyl-4-(3-methoxy-benzyl)-1-p-tolyl-cyclohexanol
• 1-Cyclohexylmethyl-2-dimethylaminomethyl-4-(3-methoxy-benzyl)-cyclohexanol
• 2-Dimethylaminomethyl-1-(5-fluor-2-methoxy-phenyl)-4-(3-methoxy-benzyl)-cyclohexanol
• 1-(3-chlor-phenyl)-2-dimethylaminomethyl-4-(3-methoxy-benzyl)-cyclohexanol
• 1-(3,5-Dichlor-phenyl)-2-dimethylaminomethyl-4-(3-methoxy-benzyl)-cyclohexanol
• 1-(4-chlor-3-trifluormethyl-phenyl)-2-dimethylaminomethyl-4-(3-methoxybenzyl)-cyclohexanol
• 2-Dimethylaminomethyl-1-(3-fluor-benzyl)-4-(3-methoxy-benzyl)-cyclohexanol
• 2-Dimethylaminomethyl-4-(3-methoxy-benzyl)-1-(2-methoxy-phenyl)-cyclohexanol
• 2-Dimethylaminomethyl-4-(3-methoxy-benzyl)-1-(3-trifluormethyl-phenyl)-cyclohexanol
• 1-(2-chlor-6-fluor-benzyl)-2-dimethylaminomethyl-4-(3-methoxy-benzyl)-cyclohexanol
• 2-Dimethylaminomethyl-1-(2,5-dimethyl-benzyl)-4-(3-methoxy-benzyl)-cyclohexanol
• 1-(3-chlor-benzyl)-2-dimethylaminomethyl-4-(3-methoxy-benzyl)-cyclohexanol
• 2-Dimethylaminomethyl-4-(4-fluor-benzyloxy)-1-phenyl-cyclohexanol
• 1-Benzyl-2-dimethylaminomethyl-4-(4-fluor-benzyloxy)-cyclohexanol
• 2-Dimethylaminomethyl-4-(4-fluor-benzyloxy)-1-(4-fluor-3-methyl-phenyl)-cyclohexanol
• 2-Dimethylaminomethyl-4-(4-fluor-benzyloxy)-1-vinyl-cyclohexanol
• 1-(4-tert-Butyl-phenyl)-2-dimethylaminomethyl-4-(4-fluor-benzyloxy)-cyclohexanol
• 1-Cyclopentyl-2-dimethylaminomethyl-4-(4-fluor-benzyloxy)-cyclohexanol
• 2-Dimethylaminomethyl-4-(4-fluor-benzyloxy)-1-m-tolyl-cyclohexanol
• 2-Dimethylaminomethyl-4-(4-fluor-benzyloxy)-bicyclohexyl-1-ol
• 2-Dimethylaminomethyl-4-(4-fluor-benzyloxy)-1-phenethyl-cyclohexanol
• 2-Dimethylaminomethyl-4-(4-fluor-benzyloxy)-1-phenylethynyl-cyclohexanol
• 2-Dimethylaminomethyl-4-(4-fluor-benzyloxy)-1-thiophen-2-yl-cyclohexanol
• 2-Dimethylaminomethyl-4-(4-fluor-benzyloxy)-1-(3-methoxy-phenyl)-cyclohexanol
• 2-Dimethylaminomethyl-4-(4-fluor-benzyloxy)-1-p-tolyl-cyclohexanol
• 1-Cyclohexylmethyl-2-dimethylaminomethyl-4-(4-fluor-benzyloxy)-cyclohexanol
• 2-Dimethylaminomethyl-1-(3-fluor-benzyl)-4-(4-fluor-benzyloxy)-cyclohexanol
• 2-Dimethylaminomethyl-4-(4-fluor-benzyloxy)-1-(2-methoxy-phenyl)-cyclohexanol
• 2-Dimethylaminomethyl-4-(4-fluor-benzyloxy)-1-(3-methyl-benzyl)-cyclohexanol
• 2-Dimethylaminomethyl-1-(2,5-dimethyl-benzyl)-4-(4-fluor-benzyloxy)-cyclohexanol
• 1-(3-Chlor-benzyl)-2-dimethylaminomethyl-4-(4-fluor-benzyloxy)-cyclohexanol
• [5-Benzyloxy-2-(3-methoxy-phenyl)-cyclohexylmethyl]-dimethyl-amin;
• [5-(3-Chlor-benzyloxy)-2-(3-methoxy-phenyl)-cyclohexylmethyl]-dimethyl-amin;
• [2-(3-Methoxy-phenyl)-5-(naphthalin-2-ylmethoxy)-cyclohexylmethyl]-dimethylamin;
• [5-(3-Methoxy-benzyloxy)-2-(3-methoxy-phenyl)-cyclohexylmethyl]-dimethylamin;
• [5-(4-Chlor-benzyloxy)-2-(3-methoxy-phenyl)-cyclohexylmethyl]-dimethyl-amin;
• [5-(4-Methoxy-benzyloxy)-2-(3-methoxy-phenyl)-cyclohexylmethyl]-dimethylamin;
• 2-Dimethylaminomethyl-1-(3-methoxy-phenyl)-4-(naphthalin-2-ylmethoxy)-cyclohexanol;
• Buttersäure 3-dimethylaminomethyl-4-hydroxy-4-(3-methoxy-phenyl)-cyclohexylester;
• 2-Dimethylaminomethyl-1-(3-methoxy-phenyl)-4-(naphthalin-2-ylmethoxy)-cyclohexanol
• 4-Methoxy-benzoesäure 3-dimethylaminomethyl-4-hydroxy-4-(3-methoxy-phenyl)-cyclohexylester;
• 3-Methoxy-benzoesäure 3-dimethylaminomethyl-4-hydroxy-4-(3-methoxyphenyl)-cyclohexylester;
• 2,2-Dimethyl-propionsäure 3-dimethylaminomethyl-4-hydroxy-4-(3-methoxyphenyl)-cyclohexylester;
• Buttersäure 3-dimethylaminomethyl-4-hydroxy-4-(3-methoxy-phenyl)-cyclohexylester;
• 4-Methoxy-benzoesäure 3-dimethylaminomethyl-4-hydroxy-4-(3-methoxyphenyl)-cyclohexylester;
• 3-Methoxy-benzoesäure 3-dimethylaminomethyl-4-hydroxy-4-(3-methoxyphenyl)-cyclohexylester;
• 2,2-Dimethyl-propionsäure 3-dimethylaminomethyl-4-hydroxy-4-(3-methoxyphenyl)-cyclohexylester;
• 3,4-Dimethoxy-benzoesäure 3-dimethylaminomethyl-4-hydroxy-4-(3-methoxyphenyl)-cyclohexylester;
• 3,4-Dimethoxy-benzoesäure 3-dimethylaminomethyl-4-hydroxy-4-(3-methoxyphenyl)-cyclohexylester;
• Naphthalin-2-carbonsäure 3-dimethylaminomethyl-4-hydroxy-4-(3-methoxy-phenyl)-cyclohexylester;
• 4-Methyl-benzoesäure 3-dimethylaminomethyl-4-hydroxy-4-(3-methoxyphenyl)-cyclohexylester;
• 3,4-Dichlor-benzoesäure 3-dimethylaminomethyl-4-hydroxy-4-(3-methoxyphenyl)-cyclohexylester;
• 4-Chlor-benzoesäure 3-dimethylaminomethyl-4-hydroxy-4-(3-methoxyphenyl)-cyclohexylester;
• 3-Chlor-benzoesäure 3-dimethylaminomethyl-4-hydroxy-4-(3-methoxyphenyl)-cyclohexylester;
• 2-Chlor-benzoesäure 3-dimethylaminomethyl-4-hydroxy-4-(3-methoxyphenyl)-cyclohexylester;
• 4-Trifluormethyl-benzoesäure 3-dinethylaminomethyl-4-hydroxy-4-(3-methoxy-phenyl)-cyclohexylester;
• 3,5-Difluor-benzoesäure 3-dimethylaminomethyl-4-hydroxy-4-(3-methoxyphenyl)-cyclohexylester;
• 4-Fluor-benzoesäure 3-dimethylaminomethyl-4-hydroxy-4-(3-methoxy-phenyl)-cyclohexylester;
• 3-Fluor-benzoesäure 3-dimethylaminomethyl-4-hydroxy-4-(3-methoxy-phenyl)-cyclohexylester;
• 2-Fluor-benzoesäure 3-dimethylaminomethyl-4-hydroxy-4-(3-methoxy-phenyl)-cyclohexylester;
• Cyclopentancarbonsäure 3-dimethylaminomethyl-4-hydroxy-4-(3-methoxyphenyl)-cyclohexylester;
• (4-Methoxy-phenyl)-essigsäure 3-dimethylaminomethyl-4-hydroxy-4-(3-methoxy-phenyl)-cyclohexylester;
• (3-Methoxy-phenyl)-essigsäure 3-dimethylaminomethyl-4-hydroxy-4-(3-methoxy-phenyl)-cyclohexylester
• Phenyl-essigsäure 3-dimethylaminomethyl-4-hydroxy-4-(3-methoxy-phenyl)-cyclohexylester;
• 3,4,5-Trimethoxy-benzoesäure 3-dimethylaminomethyl-4-hydroxy-4-(3-methoxy-phenyl)-cyclohexylester;
• 3,5-Dimethoxy-benzoesäure 3-dimethylaminomethyl-4-hydroxy-4-(3-methoxyphenyl)-cyclohexylester;
• Naphthalin-1-carbonsäure 3-dimethylaminomethyl-4-hydroxy-4-(3-methoxyphenyl)-cyclohexylester;
• 2-Methoxy-benzoesäure 3-dimethylaminomethyl-4-hydroxy-4-(3-methoxyphenyl)-cyclohexylester;
• Naphthalin-2-carbonsäure 3-dimethylaminomethyl-4-hydroxy-4-(3-methoxyphenyl)-cyclohexylester;
• 2-Hydroxy-benzoesäure 3-dimethylaminomethyl-4-hydroxy-4-(3-methoxyphenyl)-cyclohexylester;
• 4-Methyl-benzoesäure 3-dimethylaminomethyl-4-hydroxy-4-(3-methoxyphenyl)-cyclohexylester;
• 3,4-Dichlor-benzoesäure 3-dimethylaminomethyl-4-hydroxy-4-(3-methoxyphenyl)-cyclohexylester;
• 4-Chlor-benzoesäure 3-dimethylaminomethyl-4-hydroxy-4-(3-methoxyphenyl)-cyclohexylester;
• 3-Chlor-benzoesäure 3-dimethylaminomethyl-4-hydroxy-4-(3-methoxyphenyl)-cyclohexylester;
• 2-Chlor-benzoesäure 3-dimethylaminomethyl-4-hydroxy-4-(3-methoxyphenyl)-cyclohexylester;
• 4-Trifluormethyl-benzoesäure 3-dimethylaminomethyl-4-hydroxy-4-(3-methoxy-phenyl)-cyclohexylester;
• 3,5-Difluor-benzoesäure 3-dimethylaminomethyl-4-hydroxy-4-(3-methoxyphenyl)-cyclohexylester;
• 4-Fluor-benzoesäure 3-dimethylaminomethyl-4-hydroxy-4-(3-methoxy-phenyl)-cyclohexylester;
• 3-Fluor-benzoesäure 3-dimethylaminomethyl-4-hydroxy-4-(3-methoxy-phenyl)-cyclohexylester;
• 2-Fluor-benzoesäure 3-dimethylaminomethyl-4-hydroxy-4-(3-methoxy-phenyl)-cyclohexylester;
• Cyclopentancarbonsäure 3-dimethylaminomethyl-4-hydroxy-4-(3-methoxyphenyl)-cyclohexylester;
• (4-Methoxy-phenyl)-essigsäure 3-dimethylaminomethyl-4-hydroxy-3234-(3-methoxy-phenyl)-cyclohexylester;
• (3-Methoxy-phenyl)-essigsäure 3-dimethylaminomethyl-4-hydroxy-4-(3-methoxy-phenyl)-cyclohexylester;
• Phenyl-essigsäure 3-dimethylaminomethyl-4-hydroxy-4-(3-methoxy-phenyl)-cyclohexylester;
• 3,4,5-Trimethoxy-benzoesäure 3-dimethylaminomethyl-4-hydroxy-4-(3-methoxy-phenyl)-cyclohexylester;
• 3,5-Dimethoxy-benzoesäure 3-dimethylaminomethyl-4-hydroxy-4-(3-methoxyphenyl)-cyclohexylester;
• Naphthalene-1-carbonsäure 3-dimethylaminomethyl-4-hydroxy-4-(3-methoxyphenyl)-cyclohexylester;
• 2-Methoxy-benzoesäure 3-dimethylaminomethyl-4-hydroxy-4-(3-methoxyphenyl)-cyclohexylester;
• 2-Dimethylaminomethyl-1,4-bis-(3-methoxy-phenyl)-cyclohexan-1,4-diol;
• 2-Dimethylaminomethyl-1,4-bis-(3-methoxy-phenyl)-cyclohexan-1,4-diol;
• 6-(4-Benzyloxy-2-dimethylaminomethyl-1-hydroxy-cyclohexyl)-naphthalin-2-ol;
• 4-Benzyloxy-2-dimethylaminomethyl-1-(6-methoxy-naphthalin-2-yl)-cyclohexanol;
• Buttersäure 3-dimethylaminomethyl-4-hydroxy-4-(3-methoxy-phenyl)-cyclohexylester
• Buttersäure 3-dimethylaminomethyl-4-hydroxy-4-(3-methoxy-phenyl)-cyclohexylester
• 2-Dimethylaminomethyl-1-(3-methoxy-phenyl)-4-methylamino-cyclohexanol;
• 2-Dimethylaminomethyl-1-(3-methoxy-phenyl)-4-methylamino-cyclohexanol;
• Valeriansäure 3-dimethylaminomethyl-4-hydroxy-4-(3-methoxy-phenyl)-cyclohexylester;
• [3-Dimethylaminomethyl-4-hydroxy-4-(3-methoxy-phenyl)-cyclohexylidene]-essigsäure ethylester;
• [3-Dimethylaminomethyl-4-hydroxy-4-(3-methoxy-phenyl)-cyclohexylidene]-essigsäure ethylester;
• 3-[2-Dimethylaminomethyl-4-(4-fluor-benzyloxy)-1-hydroxy-cyclohexyl]-phenol;
• 4-Benzyloxy-2-dimethylaminomethyl-1-(3-phenyl-propyl)-cyclohexanol;
• 2-Dimethylaminomethyl-4-(4-fluor-benzyloxy)-1-(3-phenyl-propyl)-cyclohexanol;
• 2-Dimethylaminomethyl-1-(3-fluor-benzyl)-4-(4-fluor-benzyloxy)-cyclohexanol;
• 4-Benzyloxy-2-dimethylaminomethyl-1-(3-fluor-benzyl)-cyclohexanol;
• 2-Dimethylaminomethyl-4-(4-fluor-benzyloxy)-1-p-tolyl-cyclohexanol;
• 4-Benzyloxy-2-dimethylaminomethyl-1-p-tolyl-cyclohexanol;
• 2-Dimethylaminomethyl-1-(2,5-dimethyl-benzyl)-4-(4-fluor-benzyloxy)-cyclohexanol;
• 4-Benzyloxy-2-dimethylaminomethyl-1-(2,5-dimethyl-benzyl)-cyclohexanol;
• 1-(4-Chlor-3-trifluormethyl-phenyl)-2-dimethylaminomethyl-4-(4-fluor-benzyloxy)-cyclohexanol;
• 4-Benzyloxy-1-(4-chlor-3-trifluormethyl-phenyl)-2-dimethylaminomethyl-cyclohexanol;
• [2,5-Bis-(4-fluor-benzyloxy)-2-(3-methoxy-phenyl)-cyclohexylmethyl]-dimethylamin;
• 5-Dimethylaminomethyl-4-(3-methoxy-phenyl)-cyclohex-3-enol
• Buttersäure 3-dimethylaminomethyl-4-hydroxy-4-(6-methoxynaphthalin-2-yl)cyclohexylester
• Benzoesäure 4-hydroxy-3-(4-methyl-piperazin-1-ylmethyl)-4-naphthalen-2-yl-cyclohexyl ester;
• 2-Chloro-benzoesäure 4-hydroxy-3-(4-methyl-piperazin-1-ylmethyl)-4-naphthalen-2-yl-cyclohexyl ester;
• 3-Chloro-benzoesäure 4-hydroxy-3-(4-methyl-piperazin-1-ylmethyl)-4-naphthalen-2-yl-cyclohexyl ester;
• 4-Chloro-benzoesäure 4-hydroxy-3-(4-methyl-piperazin-1-ylmethyl)-4-naphthalen-2-yl-cyclohexyl ester;
• 2-Fluoro-benzoesäure 4-hydroxy-3-(4-methyl-piperazin-1-ylmethyl)-4-naphthalen-2-yl-cyclohexyl ester;
• 3- Fluoro -benzoesäure 4-hydroxy-3-(4-methyl-piperazin-1-ylmethyl)-4-naphthalen-2-yl-cyclohexyl ester;
• 4- Fluoro -benzoesäure 4-hydroxy-3-(4-methyl-piperazin-1-ylmethyl)-4-naphthalen-2-yl-cyclohexyl ester;
• 2-Methyl-benzoesäure 4-hydroxy-3-(4-methyl-piperazin-1-ylmethyl)-4-naphthalen-2-yl-cyclohexyl ester;
• 3- Methyl -benzoesäure 4-hydroxy-3-(4-methyl-piperazin-1-ylmethyl)-4-naphthalen-2-yl-cyclohexyl ester;
• 4- Methyl -benzoesäure 4-hydroxy-3-(4-methyl-piperazin-1-ylmethyl)-4-naphthalen-2-yl-cyclohexyl ester;
• 2-Methoxy-benzoesäure 4-hydroxy-3-(4-methyl-piperazin-1-ylmethyl)-4-naphthalen-2-yl-cyclohexyl ester;
• 3- Methoxy -benzoesäure 4-hydroxy-3-(4-methyl-piperazin-1-ylmethyl)-4-naphthalen-2-yl-cyclohexyl ester;
• 4- Methoxy -benzoesäure 4-hydroxy-3-(4-methyl-piperazin-1-ylmethyl)-4-naphthalen-2-yl-cyclohexyl ester;
• 2,6-Dichloro-benzoesäure 4-hydroxy-3-(4-methyl-piperazin-1-ylmethyl)-4-naphthalen-2-yl-cyclohexyl ester;
• 2,6-Difiuoro-benzoesäure 4-hydroxy-3-(4-methyl-piperazin-1-ylmethyl)-4-naphthalen-2-yl-cyclohexyl ester;
• 2-Chloro-5-fluoro-benzoesäure 4-hydroxy-3-(4-methyl-piperazin-1-ylmethyl)-4-naphthalen-2-yl-cyclohexyl ester;
• Biphenyl-4-carbonsäure 4-hydroxy-3-(4-methyl-piperazin-1-ylmethyl)-4-naphthalen-2-yl-cyclohexylester;
• 2-Chloro-benzoesäure 4-hydroxy-4-(6-methoxy-naphthalen-2-yl)-3-(4-methylpiperazin-1-ylmethyl)-cyclohexyl ester;
• 3-Chloro-benzoesäure 4-hydroxy-4-(6-methoxy-naphthalen-2-yl)-3-(4-methylpiperazin-1-ylmethyl)-cyclohexyl ester;
• 4-Chloro-benzoesäure 4-hydroxy-4-(6-methoxy-naphthalen-2-yl)-3-(4-methylpiperazin-1-ylmethyl)-cyclohexyl ester;
• 2-Fluoro-benzoesäure 4-hydroxy-4-(6-methoxy-naphthalen-2-yl)-3-(4-methylpiperazin-1-ylmethyl)-cyclohexyl ester;
• 3-Fluoro-benzoesäure 4-hydroxy-4-(6-methoxy-naphthalen-2-yl)-3-(4-methylpiperazin-1-ylmethyl)-cyclohexyl ester;
• 4-Fluoro-benzoesäure 4-hydroxy-4-(6-methoxy-naphthalen-2-yl)-3-(4-methylpiperazin-1-ylmethyl)-cyclohexyl ester;
• 2-Methyl-benzoesäure 4-hydroxy-4-(6-methoxy-naphthalen-2-yl)-3-(4-methylpiperazin-1-ylmethyl)-cyclohexyl ester;
• 3-Methyl-benzoesäure 4-hydroxy-4-(6-methoxy-naphthalen-2-yl)-3-(4-methylpiperazin-1-y(methyl)-cyclohexyl ester;
• 4-Methyl-benzoesäure 4-hydroxy-4-(6-methoxy-naphthalen-2-yl)-3-(4-methylpiperazin-1-ylmethyl)-cyclohexy) ester;
• 2-Methoxy-benzoesäure 4-hydroxy-4-(6-methoxy-naphthalen-2-yl)-3-(4-methyl-piperazin-1-ylmethyl)-cyclohexyl ester;
• 3-Methoxy-benzoesäure 4-hydroxy-4-(6-methoxy-naphthalen-2-yl)-3-(4-methyl-piperazin-1-ylmethyl)-cyclohexyl ester;
• 4-Methoxy-benzoesäure 4-hydroxy-4-(6-methoxy-naphthalen-2-yl)-3-(4-methyl-piperazin-1-ylmethyl)-cyclohexyl ester;
• 2,6-Dichloro-benzoesäure 4-hydroxy-4-(6-methoxy-naphthalen-2-yl)-3-(4-methyl-piperazin-1-ylmethyl)-cyclohexyl ester;
• 2,6-Fluoro-benzoesäure 4-hydroxy-4-(6-methoxy-naphthalen-2-yl)-3-(4-methyl-piperazin-1-ylmethyl)-cyclohexyl ester;
• 2-Chloro-6-fluoro-benzoesäure 4-hydroxy-4-(6-methoxy-naphthalen-2-yl)-3-(4-methyl-piperazin-1-ylmethyl)-cyclohexyl ester;
• Biphenyl-4-carbonsäure 4-hydroxy-4-(6-methoxy-naphthalen-2-yl)-3-(4-methylpiperazin-1-ylmethyl)-cyclohexyl ester;
• 4-(2-Chloro-benzyloxy)-2-(4-methyl-piperazin-1-ylmethyl)-1-naphthalen-2-yl-cyclohexanol;
• 4-(3-Chloro-benzyloxy)-2-(4-methyl-piperazin-1-ylmethyl)-1-naphthalen-2-yl-cyclohexanol;
• 4-(4-Chloro-benzyloxy)-2-(4-methyl-piperazin-1-ylmethyl)-1-naphthalen-2-yl-cyclohexanol;
• 4-(2-Fluoro-benzyloxy)-2-(4-methyl-piperazin-1-ylmethyl)-1-naphthalen-2-yl-cyclohexanol;
• 4-(3-Fluoro-benzyloxy)-2-(4-methyl-piperazin-1-ylmethyl)-1-naphthalen-2-yl-cyclohexanol;
• 4-(4-Fluoro-benzyloxy)-2-(4-methyl-piperazin-1-ylmethyl)-1-naphthalen-2-yl-cyclohexanol;
• 4-(2-Methyl-benzyloxy)-2-(4-methyl-piperazin-1-ylmethyl)-1-naphthalen-2-yl-cyclohexanol;
• 4-(3-Methyl-benzyloxy)-2-(4-methyl-piperazin-1-ylmethyl)-1-naphthalen-2-yl-cyclohexanol;
• 4-(4-Methyl-benzyloxy)-2-(4-methyl-piperazin-1-ylmethyl)-1-naphthalen-2-yl-cyclohexanol;
• 4-(2-Methoxy-benzyloxy)-2-(4-methyl-piperazin-1-ylmethyl)-1-naphthalen-2-yl-cyclohexanol;
• 4-(3-Methoxy-benzyloxy)-2-(4-methyl-piperazin-1-ylmethyl)-1-naphthalen-2-yl-cyclohexanol;
• 4-(4-Methoxy-benzyloxy)-2-(4-methyl-piperazin-1-ylmethyl)-1-naphthalen-2-yl-cyclohexanol;
• 4-(2,6-Dichloro-benzyloxy)-2-(4-methyl-piperazin-1-ylmethyl)-1-naphthalen-2-yl-cyclohexanol,
• 4-(2,6-Difluoro-benzyloxy)-2-(4-methyl-piperazin-1-ylmethyl)-1-naphthalen-2-yl-cyclohexanol,
• 4-(2-Chloro-6-fluoro-benzyloxy)-2-(4-methyl-piperazin-1-ylmethyl)-1-naphthalen-2-yl-cyclohexanol,
• 4-(2-Chloro-benzyloxy)-1-(6-methoxy-naphthalen-2-yl)-2-(4-methyl-piperazin-1-ylmethyl)-cyclohexanol;
• 4-(3-Chloro-benzyloxy)-1-(6-methoxy-naphthalen-2-yl)-2-(4-methyl-piperazin-1-ylmethyl)-cyclohexanol;
• 4-(4-Chloro-benzyloxy)-1-(6-methoxy-naphthalen-2-yl)-2-(4-methyl-piperazin-1-ylmethyl)-cyclohexanol;
• 4-(2-Fluoro-benzyloxy)-1-(6-methoxy-naphthalen-2-yl)-2-(4-methyl-piperazin-1-ylmethyl)-cyclohexanol;
• 4-(3-Fluoro-benzyloxy)-1-(6-methoxy-naphthalen-2-yl)-2-(4-methyl-piperazin-1-ylmethyl)-cyclohexanol;
• 4-(4-Fluoro-benzyloxy)-1-(6-methoxy-naphthalen-2-yl)-2-(4-methyl-piperazin-1-ylmethyl)-cyclohexanol;
• 4-(2-Methyl-benzyloxy)-1-(6-methoxy-naphthalen-2-yl)-2-(4-methyl-piperazin-1-ylmethyl)-cyclohexanol;
• 4-(3-Methyl-benzyloxy)-1-(6-methoxy-naphthalen-2-yl)-2-(4-methyl-piperazin-1-ylmethyl)-cyclohexanol;
• 4-(4-Methyl-benzyloxy)-1-(6-methoxy-naphthalen-2-yl)-2-(4-methyl-piperazin-1-ylmethyl)-cyclohexanol;
• 4-(2-Methoxy-benzyloxy)-1-(6-methoxy-naphthalen-2-yl)-2-(4-methylpiperazin-1-ylmethyl)-cyclohexanol;
• 4-(3-Methoxy-benzyloxy)-1-(6-methoxy-naphthalen-2-yl)-2-(4-methylpiperazin-1-ylmethyl)-cyclohexanol;
• 4-(4-Methoxy-benzyloxy)-1-(6-methoxy-naphthalen-2-yl)-2-(4-methylpiperazin-1-ylmethyl)-cyclohexanol;
• 4-(2,6-Dichloro-benzyloxy)-1-(6-methoxy-naphthalen-2-yl)-2-(4-methylpiperazin-1-ylmethyl)-cyclohexanol;
• 4-(2,6-Difluoro-benzyloxy)-1-(6-methoxy-naphthalen-2-yl)-2-(4-methylpiperazin-1-ylmethyl)-cyclohexanol;
• 4-(2-Chloro-6-fluoro-benzyloxy)-1-(6-methoxy-naphthalen-2-yl)-2-(4-methylpiperazin-1-ylmethyl)-cyclohexanol;
• 2-Dimethylaminomethyl-4-(4-fluor-benzyloxy)-1-naphthalin-2-yl-cyclohexanol
• 4-Fluor-benzoesäure 3-dimethylaminomethyl-4-hydroxy-4-(6-methoxy-naphthalin-2-yl)-cyclohexylester
• 4-Fluor-benzesäure 3-dimethylaminomethyl-4-hydroxy-4-naphthalin-2-yl-cyclohexylester
gegebenenfalls in Form ihrer Racemate, ihrer reinen Stereoisomeren, oder in Form von Mischungen der Stereoisomeren in einem beliebigen Mischungsverhältnis, in dargestellter Form oder in Form ihrer Säuren oder ihrer Basen oder in Form ihrer Salze, oder in Form ihrer Solvate,

9. Arzneimittel enthaltend wenigstens ein substituiertes C-Cyclohexylmethylamin-Derivat gemäß einem der Ansprüche 1 bis 8, sowie gegebenenfalls geeignete Zusatz- und/oder Hilfsstoffe und/oder gegebenenfalls weitere Wirkstoffe.

10. Arzneimittel gemäß Anspruch 9, **dadurch gekennzeichnet, daß** ein enthaltenes substituiertes C-Cyclohexylmethylamin-Derivat gemäß einem der Ansprüche 1 bis 8, als reines Diastereomer und/oder Enantiomer, als Razemat oder als nicht-äquimolare oder äquimolare Mischung der Diastereomere und/oder Enantiomere vorliegt.

11. Verwendung eines substituierten C-Cyclohexylmethylamin-Derivats gemäß einem der Ansprüche 1 bis 8 zur Herstellung eines Arzneimittels zur Behandlung von Schmerz, insbesondere von neuropathischem oder chronischem Schmerz.

12. Verwendung gemäß Anspruch 11, **dadurch gekennzeichnet, daß** ein verwendetes substituiertes C-Cyclohexylmethylamin-Derivat gemäß einem der Ansprüche 1 bis 8, als reines Diastereomere und/oder Enantiomer, als Razemat oder als nicht-äquimolare oder äquimolare Mischung der Diastereomere und/oder Enantiomere vorliegt.

## Claims

1. Substituted C-cyclohexylmethylamine derivatives of general formula I, 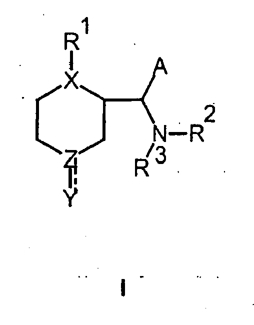 wherein
A is selected from H or phenyl;
R¹ is selected from respectively saturated or unsaturated, branched or unbranched, singly or multiply substituted or unsubstituted C₁₋₁₀ alkyl or C₃₋₁₀ cycloalkyl; naphthyl, phenyl with the condition that R¹ is not a phenyl ring singly O- or S-substituted in the 3 position, furyl, thiophenyl, or naphthyl or phenyl bound by C₁₋₃ alkylene or ethinyl, C₃₋₁₀ cycloalkyl bound by C₁₋₃ alkylene or ethinyl, or furyl or thiophenyl bound by C₁₋₃ alkylene or ethinyl, respectively unsubstituted or singly or multiply substituted,
the substituents being selected independently of one another from
F, Cl, Br, I, OR¹⁸, SR¹⁸, SO₂R¹⁸, SO₂OR¹⁸, CN, COOR¹⁸, NR¹⁹R²⁰; C₁₋₁₀ alkyl or C₃₋₁₀ cycloalkyl, respectively unsubstituted or singly or multiply substituted identically or differently by F, Cl, Br, I, NH₂, SH or OH, or silyl,
wherein R¹⁸ is selected from H; respectively saturated or unsaturated, branched or unbranched, C₁₋₁₀ alkyl or C₃₋₁₀ cycloalkyl,
R¹⁹ and R²⁰ independently of one another are selected from H; respectively saturated or unsaturated, branched or unbranched, C₁₋₁₀ alkyl or C₃₋₁₀ cycloalkyl;
or R¹⁹ and R²⁰ together form CH₂CH₂OCH₂CH₂, CH₂CH₂NR²¹CH₂CH₂ or (CH₂)₃₋₆,
wherein R²¹ is selected from H phenyl; saturated or unsaturated, branched or unbranched C₁₋₁₀ alkyl;
R² and R³ independently of one another are selected from H; respectively saturated or unsaturated, branched or unbranched, C₁₋₁₀ alkyl or C₃₋₁₀ cycloalkyl;
or the radicals R² and R³ together represent CH₂CH₂OCH₂CH₂, CH₂CH₂NR⁶CH₂CH₂ or (CH₂)₃₋₆,
wherein R⁶ is selected from H; respectively saturated or unsaturated, branched or unbranched, C₁₋₁₀ alkyl or C₃₋₁₀ cycloalkyl;
X in formula I according to part formula Ia of formula I 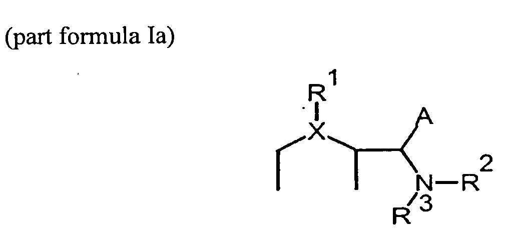 represents 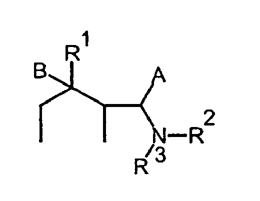 wherein B is selected from OH, OR⁷, H, F, Cl or NR⁸R⁹,
wherein R⁷ is selected from C₁₋₁₀ alkyl or C₃₋₁₀ cycloalkyl, respectively saturated or unsaturated, branched or unbranched, unsubstituted or singly or multiply substituted identically or differently by F, Cl, Br, I, NH₂, SH or OH;
R⁸ and R⁹ independently of one another are selected from H; C₁₋₁₀ alkyl or C₃₋₁₀ cycloalkyl, respectively saturated or unsaturated, branched or unbranched, unsubstituted or singly or multiply substituted identically or differently by F, Cl, Br, I, NH₂, SH or OH;
or the radicals R⁸ and R⁹ together form CH₂CH₂OCH₂CH₂, CH₂CH₂NR¹⁰CH₂CH₂ or (CH₂)₃₋₆
wherein R¹⁰ is selected from H; C₁₋₁₀ alkyl or C₃₋₁₀ cycloalkyl, respectively saturated or unsaturated, branched or unbranched, unsubstituted or singly or multiply substituted identically or differently by F, Cl, Br, I, NH₂, SH or OH; 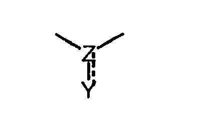 is selected from 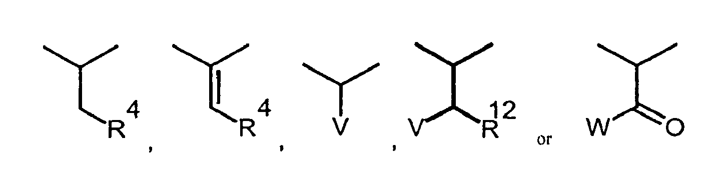 wherein R⁴ is selected from COR⁵, SO₂R⁵; respectively saturated or unsaturated, branched or unbranched, singly or multiply substituted or unsubstituted C₁₋₁₀ alkyl or C₃₋₁₀ cycloalkyl; respectively singly or multiply substituted or unsubstituted naphthyl, phenyl, furyl or thiophenyl; or respectively singly or multiply substituted or unsubstituted phenyl, naphthyl, C₃₋₁₀ cycloalkyl, furyl or thiophenyl bound by C₁₋₃ alkylene,
wherein R⁵ is selected from respectively saturated or unsaturated, branched or unbranched, singly or multiply substituted or unsubstituted C₁₋₁₀ alkyl or C₃₋₁₀ cycloalkyl; respectively singly or multiply substituted or unsubstituted phenyl, naphthyl, furyl or thiophenyl; or respectively singly or multiply substituted or unsubstituted phenyl, naphthyl, C₃₋₁₀ cycloalkyl, furyl or thiophenyl bound by C₁₋₃ alkylene,
wherein V is selected from OR⁴ or NR⁴R¹¹,
wherein W is selected from R¹¹, OR¹² or NR¹¹R¹²,
wherein R¹¹ and R¹² independently of one another are selected from H; respectively saturated or unsaturated, branched or unbranched, singly or multiply substituted or unsubstituted C₇₋₁₀ alkyl or C₃₋₁₀ cycloalkyl; respectively singly or multiply substituted or unsubstituted naphthyl, phenyl, furyl or thiophenyl; or respectively singly or multiply substituted or unsubstituted phenyl, naphthyl, C₃₋₁₀ cycloalkyl, furyl or thiophenyl bound by C₁₋₃ alkylene,
wherein the term "substitution", in relation to alkyl and cycloalkyl of R⁴, R⁵, R¹¹ and R¹², is understood as meaning substitution with F, Cl, Br, I, NH₂, SH or OH, and
the term "substitution", in relation to naphthyl, phenyl, furyl and thiophenyl of R⁴, R⁵, R¹¹ and R¹², is understood as meaning substitution of the naphthyl, phenyl, furyl or thiophenyl by R²³, OR²³, a halogen, a CF₃, a CN, an NO₂, an NR²⁴R²⁵, a C¹⁻⁶ alkyl (saturated), a C₁₋₆ alkoxy, a C₃₋₈ cycloalkoxy, a C₃₋₈ cycloalkyl or a C₂₋₆ alkylene, wherein
the radical R²³ represents H, a C₁₋₁₀ alkyl, a naphthyl, phenyl, furyl or thiophenyl radical, or a naphthyl, phenyl, furyl or thiophenyl radical bound by a C₁₋₃ alkylene group, wherein said naphthyl, phenyl, furyl and thiophenyl radicals cannot themselves be substituted by aryl or heteroaryl radicals,
the radicals R²⁴ and R²⁵, the same or different, represent H, a C₁₋₁₀ alkyl, a naphthyl, phenyl, furyl or thiophenyl radical, or a naphthyl, phenyl, furyl or thiophenyl radical bound by a C₁₋₃ alkylene group, wherein said naphthyl, phenyl, furyl and thiophenyl radicals cannot themselves be substituted by aryl or heteroaryl radicals,
or the radicals R²⁴ and R²⁵ together form CH₂CH₂OCH₂CH₂, CH₂CH₂NR²⁶CH₂CH₂ or (CH₂)₃₋₆,
the radical R²⁶ represents H, a C₁₋₁₀ alkyl, naphthyl, phenyl, furyl or thiophenyl radical, or a naphthyl, phenyl, furyl or thiophenyl radical bound by a C₁₋₃ alkylene group, wherein said naphthyl, phenyl, furyl and thiophenyl radicals cannot themselves be substituted by aryl or heteroaryl radicals,
optionally in the form of their racemates, their pure stereoisomers, or in the form of blends of stereoisomers in any mixing ratio; in the illustrated form or in the form of their acids or their bases or in the form of their salts, or in the form of their solvates,
to the exclusion of compounds in which
R² and R³ are selected independently of one another from
H, branched or unbranched, singly or multiply substituted or unsubstituted, saturated or unsaturated C₁-C₁₀ alkyl; saturated or unsaturated, singly or multiply substituted or unsubstituted C₃-C₇ cycloalkyl, or a corresponding heterocycle, wherein a C atom in the ring is replaced by N, S or O; in each case singly or multiply substituted or unsubstituted;
or
R² and R³ together form a ring and represent CH₂CH₂OCH₂CH₂, CH₂CH₂NR⁶CH₂CH₂ or (CH₂)₃₋₆;
X in formula I according to part formula Ia of formula I 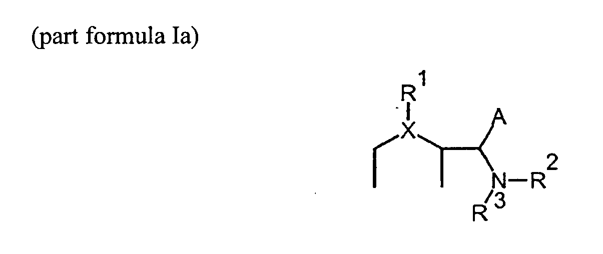 has the following meaning 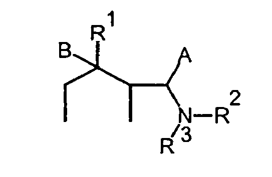 , in other words X represents 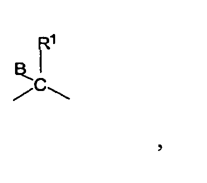 A is hydrogen,
R¹ is an unsubstituted or singly or doubly substituted phenyl ring,
B is selected from
H, F, Cl, OH or OR⁷, wherein R⁷ is selected from branched or unbranched, singly or multiply substituted or unsubstituted C₁-C₁₀ alkyl; saturated or unsaturated, singly or multiply substituted or unsubstituted C₃-C₇ cycloalkyl, or a corresponding heterocycle, wherein a C atom in the ring is replaced by N, S or O; in each case singly or multiply substituted or unsubstituted;
and at the same time the grouping 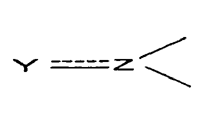 represents [scan 2nd Fig. on p. 8; = Fig. from top p. 21 desc.]
wherein V is selected from OR⁴ with R⁴ selected from COR⁵ and with R⁵ selected from unsubstituted or singly or multiply substituted C₁₋₆ alkyl.

2. Substituted C-cyclohexylmethylamine derivatives according to claim 1,
**characterised in that**
R¹ corresponds to a compound according to general formula II 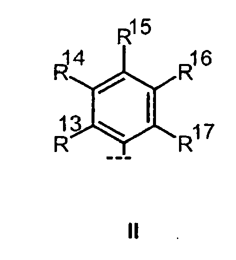 wherein
R¹³, R¹⁵ and R¹⁷ independently of one another are selected from H, F, Cl, Br, I, OR¹⁸, SR¹⁸, SO₂R¹⁸, SO₂OR¹⁸, CN, COOR¹⁸, NR¹⁹R²⁰; C₁₋₁₀ alkyl or C₃₋₁₀ cycloalkyl, respectively unsubstituted or singly or multiply substituted identically or differently by F, Cl, Br, I, NH₂, SH or OH, or silyl, saturated or unsaturated, branched or unbranched;
R¹⁴ and R¹⁶ independently of one another are selected from H, F, Cl, Br, I, SO₂R¹⁸, SO₂OR¹⁸, CN, COOR¹⁸, NR¹⁹R²⁰; C₁₋₁₀ alkyl or C₃₋₁₀ cycloalkyl, respectively unsubstituted or singly or multiply identically or differently substituted by F, Cl, Br, I, NH₂, SH or OH, or silyl saturated or unsaturated, branched or unbranched;
or the radicals R¹³ and R¹⁴ or R¹⁴ and R¹⁵ together respectively form the group OCH₂O, OCH₂CH₂O, CH=CHO, CH=C(CH₃)O or CH=CHNH and R¹⁵-R¹⁷ or R¹³, R¹⁶ and R¹⁷ have the meaning given above,
wherein R¹⁸ is selected from H, respectively saturated or unsaturated, branched or unbranched, C₁₋₁₀ alkyl or C₃₋₁₀ cycloalkyl,
R¹⁹ and R²⁰ independently of one another are selected from H, respectively saturated or unsaturated, branched or unbranched, C₁₋₁₀ alkyl or C₃₋₁₀ cycloalkyl,
or R¹⁹ and R²⁰ together form CH₂CH₂OCH₂CH₂, CH₂CH₂NR²¹CH₂CH₂ or (CH₂)₃₋₆,
wherein R²¹ is selected from H, C₁₋₁₀ alkyl, preferably saturated or unsaturated, branched or unbranched C₁₋₆ alkyl, respectively unsubstituted or singly or multiply substituted identically or differently by F, Cl, Br, NH₂, SH or OH,
and 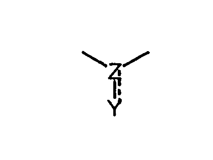 is selected from 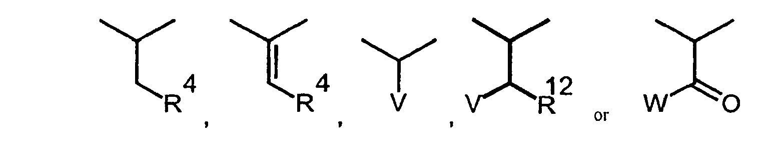 wherein R⁴ is selected from H, COR^{5a}, SO₂R⁵; respectively saturated or unsaturated, branched or unbranched, singly or multiply substituted or unsubstituted C₁₋₁₀ alkyl or C₃₋₁₀ cycloalkyl; respectively singly or multiply substituted or unsubstituted phenyl, naphthyl, furyl or thiophenyl; or respectively singly or multiply substituted or unsubstituted naphthyl, phenyl, C₃₋₁₀ cycloalkyl, furyl or thiophenyl bound by C₁₋₃ alkylene,
wherein R⁵ is selected from respectively saturated or unsaturated, branched or unbranched, singly or multiply substituted or unsubstituted C₁₋₁₀ alkyl or C₃₋₁₀ cycloalkyl; respectively singly or multiply substituted or unsubstituted phenyl, naphthyl, furyl or thiophenyl; or respectively singly or multiply substituted or unsubstituted phenyl, naphthyl, C₃₋₁₀ cycloalkyl, furyl or thiophenyl bound by C₁₋₃ alkylene,
wherein R^{5a} is selected from saturated or unsaturated, branched or unbranched, singly or multiply substituted or unsubstituted C₃₋₁₀ cycloalkyl; respectively singly or multiply substituted or unsubstituted naphthyl, phenyl, furyl or thiophenyl; or respectively singly or multiply substituted or unsubstituted phenyl, naphthyl, C₃₋₁₀ cycloalkyl, furyl or thiophenyl bound by C₁₋₃ alkylene,
wherein V is selected from OR⁴ or NR⁴R¹¹,
wherein W is selected from R¹¹, OR¹² or NR¹¹R¹²,
wherein R¹¹ and R¹² independently of one another are selected from H; respectively saturated or unsaturated, branched or unbranched, singly or multiply substituted or unsubstituted C₇₋₁₀ alkyl or C₃₋₁₀ cycloalkyl; respectively singly or multiply substituted or unsubstituted phenyl, naphthyl, furyl or thiophenyl; or respectively singly or multiply substituted or unsubstituted phenyl, naphthyl, C₃₋₁₀ cycloalkyl, furyl or thiophenyl bound by C₁₋₃ alkylene,
wherein the term "substitution", in relation to alkyl and cycloalkyl of R⁴, R⁵, R^{5a}, R¹¹ and R¹², is understood as meaning substitution with F, Cl, Br, I, NH₂, SH or OH, and
the term "substitution", in relation to naphthyl, phenyl, furyl and thiophenyl of R⁴, R⁵, R^{5a}, R¹¹ and R¹², is understood as meaning substitution of the naphthyl, phenyl, furyl or thiophenyl by R²³, OR²³, a halogen, a CF₃, a CN, an NO₂, an NR²⁴R²⁵, a C¹⁻⁶ alkyl (saturated), a C₁₋₆ alkoxy, a C₃₋₈ cycloalkoxy, a C₃₋₈ cycloalkyl or a C₂₋₆ alkylene, wherein
the radical R²³ represents H, a C₁₋₁₀ alkyl, a naphthyl, phenyl, furyl or thiophenyl radical, or a naphthyl, phenyl, furyl or thiophenyl radical bound by a C₁₋₃ alkylene group, wherein said naphthyl, phenyl, furyl and thiophenyl radicals cannot themselves be substituted by aryl or heteroaryl radicals,
the radicals R²⁴ and R²⁵, the same or different, represent H, a C₁₋₁₀ alkyl, a naphthyl, phenyl, furyl or thiophenyl radical, or a naphthyl, phenyl, furyl or thiophenyl radical bound by a C₁₋₃ alkylene group, wherein said naphthyl, phenyl, furyl and thiophenyl radicals cannot themselves be substituted by aryl or heteroaryl radicals,
or the radicals R²⁴ and R²⁵ together form CH₂CH₂OCH₂CH₂, CH₂CH₂NR²⁶CH₂CH₂ or (CH₂)₃₋₆,
the radical R²⁶ represents H, a C₁₋₁₀ alkyl, naphthyl, phenyl, furyl or thiophenyl radical, or a naphthyl, phenyl, furyl or thiophenyl radical bound by a C₁₋₃ alkylene group, wherein said naphthyl, phenyl, furyl and thiophenyl radicals cannot themselves be substituted by aryl or heteroaryl radicals.

3. Substituted C-cyclohexylmethylamine derivatives according to either claim 1 or claim 2, **characterised in that** A is hydrogen.

4. Substituted C-cyclohexylmethylamine derivatives according to any one of claims 1 to 3, **characterised in that**
R² and R³ are selected from C₁₋₆ alkyl, saturated or unsaturated, branched or unbranched,
or the radicals R² and R³ together represent CH₂CH₂NR⁶CH₂CH₂ or (CH₂)₃₋₆, wherein R⁶ is selected from H or C₁₋₆ alkyl, saturated, branched or unbranched and unsubstituted.

5. Substituted C-cyclohexylmethylamine derivatives according to any one of claims 1 to 4, **characterised in that**
R¹ is selected from saturated or unsaturated, branched or unbranched C₁₋₆ alkyl; C₃₋₆ cycloalkyl, naphthyl, phenyl, furyl, thiophenyl, naphthyl bound by C₁₋₃ alkylene or ethinyl, phenyl, C₃₋₆ cycloalkyl or thiophenyl bound by C₁₋₃ alkylene or ethinyl or furyl, which according to claim 1 can be unsubstituted or singly or multiply unsubstituted.

6. Substituted C-cyclohexylmethylamine derivatives according to any one of claims 1 to 5, **characterised in that** 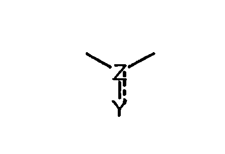 is selected from

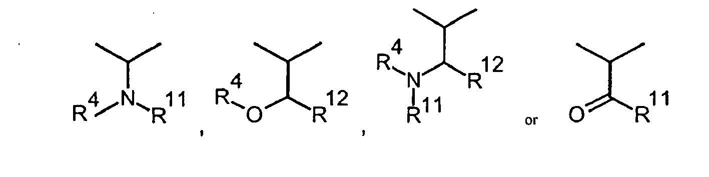

7. Substituted C-cyclohexylmethylamine derivatives according to claim 1,
**characterised in that**
A is selected from H or phenyl
**and/or**
R¹ is selected from naphthyl, unsubstituted or singly or multiply substituted by radicals, selected independently of one another from
F, Cl, Br, I, OR¹⁸; the same or different, branched or unbranched, unsubstituted C₁₋₄ alkyl or singly or multiply substituted by F, Cl, Br, I, NH₂, SH or OH;
wherein R¹⁸ is selected from H; branched or unbranched C₁₋₄ alkyl,
**and/or**
R² and R³ are selected from saturated or unsaturated, branched or unbranched C₁₋₆ alkyl,
or the radicals R² and R³ together represent CH₂CH₂NR⁶CH₂CH₂ or (CH₂)₄₋₅, wherein R⁶ is selected from H or saturated, branched or unbranched, and unsubstituted C₁₋₆ alkyl,
**and/or**
X in formula I according to part formula Ia of formula I 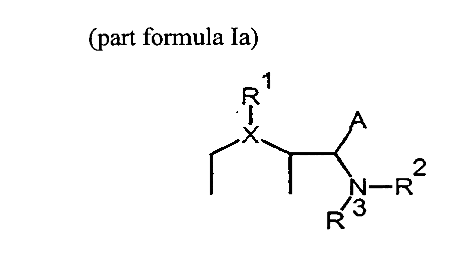 has the following meaning 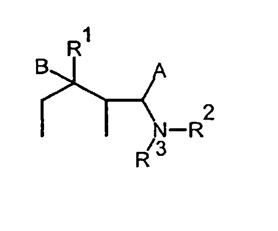 , in other words X represents 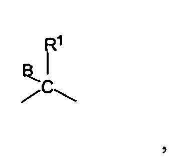 wherein B is selected from OH, OR⁷, H, F, Cl,
wherein R⁷ is selected from C₁₋₄ alkyl, branched or unbranched, unsubstituted or singly or multiply substituted identically or differently with F, Cl, Br, I, NH₂, SH or OH;
and/or 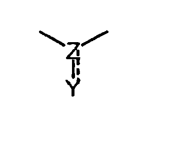 is selected from 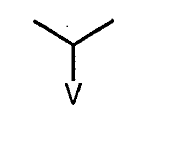 wherein V is selected from OR⁴,
wherein R⁴ is selected from COR⁵; singly or phenyl or benzyl which may be unsubstituted or singly or multiply substituted in accordance with claim 1;
wherein R⁵ is selected from saturated or unsaturated, branched or unbranched C₁₋₁₀ alkyl, unsubstituted or singly or multiply substituted, identically or differently, with F, Cl, Br, I, NH₂, SH or OH; or unsubstituted or singly or multiply substituted phenyl in accordance with claim 1.

8. Substituted C-cyclohexylmethylamine derivatives according to any one of claims 1 to 7, **characterised in that** they are selected from the following group:
• 4-benzyloxy-1-(4-bromo-phenyl)-2-dimethylaminomethyl-cyclohexanol;
• 4-benzyloxy-2-dimethylaminomethyl-1-(4-trifluoromethyl-phenyl)-cyclohexanol;
• 4-benzyloxy-1-(3,4-difluoro-phenyl)-2-dimethylaminomethyl-cyclohexanol
• 4-benzyloxy-2-dimethylaminomethyl-1-(3-fluoro-4-methyl-phenyl)-cyclohexanol;
• 4-benzyloxy-1-(3,4-dichloro-phenyl)-2-dimethylaminomethyl-cyclohexanol;
• 4-benzyloxy-2-dimethylaminomethyl-1-naphthalin-2-yl-cyclohexanol;
• 4-benzyloxy-2-dimethylaminomethyl-1-(3,4-dimethyl-phenyl)-cyclohexanol;
• 4-benzyloxy-2-dimethylaminomethyl-1-(4-fluoro-3-methyl-phenyl)-cyclohexanol;
• 4-benzyloxy-2-dimethylaminomethyl-1-(5-fluoro-2-methyl-phenyl)-cyclohexanol;
• 4-benzyloxy-1-(3-chloro-4-fluoro-phenyl)-2-dimethylaminomethyl-cyclohexanol;
• 4-benzyloxy-1-(5-chloro-2-methoxy-phenyl)-2-dimethylaminomethyl-cyclohexanol;
• 4-benzyloxy-1-(2-bromo-phenyl)-2-dimethylaminomethyl-cyclohexanol;
• 4-benzyloxy-2-dimethylaminomethyl-1-(2-methoxy-phenyl)-cyclohexanol;
• 4-benzyloxy-2-dimethylaminomethyl-1-(2-methylsulphanyl-phenyl)-cyclohexanol;
• 4-benzyloxy-1-(4-chloro-phenyl)-2-dimethylaminomethyl-cyclohexanol;
• 4-benzyloxy-1-(3-bromo-phenyl)-2-dimethylaminomethyl-cyclohexanol;
• 4-benzyloxy-1-(4-bromo-3-fluoro-phenyl)-2-dimethylaminomethyl-cyclohexanol;
• 4-benzyloxy-2-dimethylaminomethyl-1-(4-methoxy-phenyl)-cyclohexanol;
• 4-benzyloxy-1-(2-chloro-phenyl)-2-dimethylaminomethyl-cyclohexanol;
• 4-benzyloxy-2-dimethylaminomethyl-1-(3-trifluoromethyl-phenyl)-cyclohexanol;
• 4-benzyloxy-2-dimethylaminomethyl-1-(4-pentyl-phenyl)-cyclohexanol;
• 4-benzyloxy-2-dimethylaminomethyl-1-(2,5-dimethyl-phenyl)-cyclohexanol;
• 4-benzyloxy-2-dimethylaminomethyl-1-(4-isopropyl-phenyl)-cyclohexanol;
• 4-benzyloxy-2-dimethylaminomethyl-1-(2,3-dimethyl-phenyl)-cyclohexanol;
• 4-benzyloxy-2-dimethylaminomethyl-1-(3-fluoro-phenyl)-cyclohexanol;
• 4-benzyloxy-1-(3,5-bis-trifluoromethyl-phenyl)-2-dimethylaminomethyl-cyclohexanol;
• 4-benzyloxy-2-dimethylaminomethyl-1-(4-ethyl-phenyl)-cyclohexanol;
• 4-benzyloxy-1-(3,5-dichloro-phenyl)-2-dimethylaminomethyl-cyclohexanol;
• 2-dimethylaminomethyl-4-(4-fluoro-benzyloxy)-1-o-tolyl-cyclohexanol;
• 2-dimethylaminomethyl-4-(4-fluoro-benzyloxy)-1-p-tolyl-cyclohexanol;
• 2-dimethylaminomethyl-1-(2,5-dimethyl-phenyl)-4-(4-fluoro-benzyloxy)-cyclohexanol;
• 2-dimethylaminomethyl-1-(3,4-dimethyl-phenyl)-4-(4-fluoro-benzyloxy)-cyclohexanol;
• 2-dimethylaminomethyl-1-(3,5-dimethyl-phenyl)-4-(4-fluoro-benzyloxy)-cyclohexanol;
• 2-dimethylaminomethyl-4-(4-fluoro-benzyloxy)-1-(4-isopropyl-phenyl)-cyclohexanol;
• 2-dimethylaminomethyl-4-(4-fluoro-benzyloxy)-1-(3-fluoro-phenyl)-cyclohexanol;
• 2-dimethylaminomethyl-4-(4-fluoro-benzyloxy)-1-(4-fluoro-phenyl)-cyclohexanol;
• 1-(3,4-difluoro-phenyl)-2-dimethylaminomethyl-4-(4-fluoro-benzyloxy)-cyclohexanol;
• 2-dimethylaminomethyl-4-(4-fluoro-benzyloxy)-1-(3-fluoro-4-methylphenyl)-cyclohexanol;
• 2-dimethylaminomethyl-4-(4-fluoro-benzyloxy)-1-(4-fluoro-3-methylphenyl)-cyclohexanol;
• 1-(2-chloro-phenyl)-2-dimethylaminomethyl-4-(4-fluoro-benzyloxy)-cyclohexanol;
• 1-(3-chloro-phenyl)-2-dimethylaminomethyl-4-(4-fluoro-benzyloxy)-cyclohexanol;
• 1-(4-chloro-phenyl)-2-dimethylaminomethyl-4-(4-fluoro-benzyloxy)-cyclohexanol;
• 1-(3,4-dichloro-phenyl)-2-dimethylaminomethyl-4-(4-fluoro-benzyloxy)-cyclohexanol;
• 1-(4-chloro-3-fluoro-phenyl)-2-dimethylaminomethyl-4-(4-fluoro-benzyloxy)-cyclohexanol;
• 1-(3,5-dichloro-phenyl)-2-dimethylaminomethyl-4-(4-fluoro-benzyloxy)-cyclohexanol;
• 1-(4-chloro-2-methoxy-phenyl)-2-dimethylaminomethyl-4-(4-fluoro-benzyloxy)-cyclohexanol;
• 2-dimethylaminomethyl-4-(4-fluoro-benzyloxy)-1-(4-trifluoromethylphenyl)-cyclohexanol;
• 1-(3,5-bis-trifluoromethyl-phenyl)-2-dimethylaminomethyl-4-(4-fluoro-benzyloxy)-cyclohexanol;
• 2-dimethylaminomethyl-4-(4-fluoro-benzyloxy)-1-(2-methoxy-phenyl)-cyclohexanol;
• 2-dimethylaminomethyl-4-(4-fluoro-benzyloxy)-1-(2-methylsulphanyl-phenyl)-cyclohexanol;
• 3,4-dichloro-N-[3-dimethylaminomethyl-4-hydroxy-4-(3-methoxy-phenyl)-cyclohexyl]-benzamide;
• Naphthalene-2-carboxylic acid [3-dimethylaminomethyl-4-hydroxy-4-(3-methoxy-phenyl)-cyclohexyl]-amide;
• N-[3-dimethylaminomethyl-4-hydroxy-4-(3-methoxy-phenyl)-cyclohexyl]-3-phenyl-propionamide;
• N-[3-dimethylaminomethyl-4-hydroxy-4-(3-methoxy-phenyl)-cyclohexyl]-4-nitro-benzamide;
• N-[3-dimethylaminomethyl-4-hydroxy-4-(3-methoxy-phenyl)-cyclohexyl]-4-methyl-3-nitro-benzamide;
• N-[3-dimethylaminomethyl-4-hydroxy-4-(3-methoxy-phenyl)-cyclohexyl]-3,4,5-trimethoxy-benzamide;
• 2-(4-chloro-phenoxy)-N-[3-dimethylaminomethyl-4-hydroxy-4-(3-methoxy-phenyl)-cyclohexyl]-acetamide;
• N-[3-dimethylaminomethyl-4-hydroxy-4-(3-methoxy-phenyl)-cyclohexyl]-3-nitro-benzamide
• furan-2-carboxylic acid [3-dimethylaminomethyl-4-hydroxy-4-(3-methoxyphenyl)-cyclohexyl]-amide
• N-[3-dimethylaminomethyl-4-hydroxy-4-(3-methoxy-phenyl)-cyclohexyl]-2-phenoxy-acetamide
• N-[3-dimethylaminomethyl-4-hydroxy-4-(3-methoxy-phenyl)-cyclohexyl]-4-trifluoromethyl-benzamide;
• N-[3-dimethylaminomethyl-4-hydroxy-4-(3-methoxy-phenyl)-cyclohexyl]-4-methoxy-benzamide;
• 2-dimethylaminomethyl-1-(4-fluoro-phenyl)-4-phenethyl-cyclohexanol;
• 1-(4-bromo-phenyl)-2-dimethylaminomethyl-4-phenethyl-cyclohexanol;
• 2-dimethylaminomethyl-1-(4-ethyl-phenyl)-4-phenethyl-cyclohexanol;
• 2-dimethylaminomethyl-1-(4-isopropyl-phenyl)-4-phenethyl-cyclohexanol;
• 2-dimethylaminomethyl-1-(4-methoxy-phenyl)-4-phenethyl-cyclohexanol;
• 2-dimethylaminomethyl-1-(2,4-dimethyl-phenyl)-4-phenethyl-cyclohexanol;
• 2-dimethylaminomethyl-1-(2-methylsulphanyl-phenyl)-4-phenethyl-cyclohexanol;
• 2-dimethylaminomethyl-1-(5-fluoro-2-methyl-phenyl)-4-phenethyl-cyclohexanol;
• 2-dimethylaminomethyl-1-(2,5-dimethyl-phenyl)-4-phenethyl-cyclohexanol;
• 2-dimethylaminomethyl-4-phenethyl-1-p-tolyl-cyclohexanol;
• 2-dimethylaminomethyl-1-(3-fluoro-phenyl)-4-phenethyl-cyclohexanol;
• 1-(3-chloro-phenyl)-2-dimethylaminomethyl-4-phenethyl-cyclohexanol;
• 1-(4-chloro-3-fluoro-phenyl)-2-dimethylaminomethyl-4-phenethyl-cyclohexanol;
• 2-dimethylaminomethyl-4-phenethyl-1-(3-trifluoromethyl-phenyl)-cyclohexanol
• 1-(3,5-dichloro-phenyl)-2-dimethylaminomethyl-4-phenethyl-cyclohexanol;
• 2-dimethylaminomethyl-1-(3,4-dimethyl-phenyl)-4-phenethyl-cyclohexanol;
• 1-(3,4-dichloro-phenyl)-2-dimethylaminomethyl-4-phenethyl-cyclohexanol;
• 1-(4-chloro-phenyl)-2-dimethylaminomethyl-4-phenethyl-cyclohexanol;
• 2-dimethylaminomethyl-1-(3-fluoro-4-methyl-phenyl)-4-phenethyl-cyclohexanol;
• 2-dimethylaminomethyl-1-(3,5-dimethyl-phenyl)-4-phenethyl-cyclohexanol;
• 1-(4-bromo-3-fluoro-phenyl)-2-dimethylaminomethyl-4-phenethyl-cyclohexanol;
• 1-(3,4-difluoro-phenyl)-2-dimethylaminomethyl-4-phenethyl-cyclohexanol;
• 2-dimethylaminomethyl-1-(2,3-dimethyl-phenyl)-4-phenethyl-cyclohexanol;
• 2-dimethylaminomethyl-1-naphthalin-1-yl-4-phenethyl-cyclohexanol;
• 1-(3,5-bis-trifluoromethyl-phenyl)-2-dimethylaminomethyl-4-phenethyl-cyclohexanol;
• 1-(3-bromo-phenyl)-2-dimethylaminomethyl-4-phenethyl-cyclohexanol;
• 2-dimethylaminomethyl-1-(4-fluoro-3-methyl-phenyl)-4-phenethyl-cyclohexanol;
• 2-dimethylaminomethyl-4-phenethyl-1-m-tolyl-cyclohexanol;
• 4-benzyl-2-dimethylaminomethyl-1-m-tolyl-cyclohexanol;
• 4-benzyl-1-(3,4-difluoro-phenyl)-2-dimethylaminomethyl-cyclohexanol;
• 4-benzyl-2-dimethylaminomethyl-1-p-tolyl-cyclohexanol;
• 2-dimethylaminomethyl-4-(3-methoxy-benzyl)-1-p-tolyl-cyclohexanol;
• 1-(4-chloro-phenyl)-2-dimethylaminomethyl-4-(3-methoxy-benzyl)-cyclohexanol;
• 1-(4-bromo-3-fluoro-phenyl)-2-dimethylaminomethyl-4-(3-methoxybenzyl)-cyclohexanol;
• 2-dimethylaminomethyl-4-(4-fluoro-benzyl)-1-p-tolyl-cyclohexanol;
• 1-(4-chloro-phenyl)-2-dimethylaminomethyl-4-(4-fluoro-benzyl)-cyclohexanol;
• [2-(3-chloro-4-fluoro-phenyl)-2-fluoro-5-phenethyl-cyclohexylmethyl]-dimethyl-amine;
• [2-fluoro-5-(4-fluoro-benzyl)-2-p-tolyl-cyclohexylmethyl]-dimethyl-amine;
• benzyl-2-(dimethylamino-phenyl-methyl)-4-phenyl-cyclohexanol
• 2-(dimethylamino-phenyl-methyl)-4-phenyl-1-vinyl-cyclohexanol
• 1-(4-tert-Butyl-phenyl)-2-(dimethylamino-phenyl-methyl)-4-phenyl-cyclohexanol
• 2-(dimethylamino-phenyl-methyl)-4-phenyl-1-m-tolyl-cyclohexanol
• 2-(dimethylamino-phenyl-methyl)-1-phenethyl-4-phenyl-cyclohexanol
• 2-(dimethylamino-phenyl-methyl)-4-phenyl-1-phenylethynyl-cyclohexanol
• 2-(dimethylamino-phenyl-methyl)-1-(3-methoxy-phenyl)-4-phenyl-cyclohexanol
• 2-(dimethylamino-phenyl-methyl)-4-phenyl-1-(3-phenyl-propyl)-cyclohexanol
• 2-(dimethylamino-phenyl-methyl)-1-(4-methoxy-phenyl)-4-phenyl-cyclohexanol
• 2-(dimethylamino-phenyl-methyl)-1-(2-methoxy-phenyl)-4-phenyl-cyclohexanol
• 4-benzyloxy-2-dimethylaminomethyl-1-phenyl-cyclohexanol
• 1-benzyl-4-benzyloxy-2-dimethylaminomethyl-cyclohexanol
• 4-benzyloxy-2-dimethylaminomethyl-1-(4-fluoro-2methyl-phenyl)-cyclohexanol
• 4-benzyloxy-2-dimethylaminomethyl-1-o-tolyl-cyclohexanol
• 4-benzyloxy-2-dimethylaminomethyl-1-vinyl-cyclohexanol
• 4-benzyloxy-1-cyclopentyl-2-dimethylaminomethyl-cyclohexanol
• 4-benzyloxy-2-dimethylaminomethyl-1-m-tolyl-cyclohexanol
• 4-benzyloxy-2-dimethylaminomethyl-bicyclohexyl-1-ol
• 4-benzyloxy-2-dimethylaminomethyl-1-(4-fluoro-phenyl)-cyclohexanol
• 4-benzyloxy-2-dimethylaminomethyl-1-phenylethynyl-cyclohexanol
• 4-benzyloxy-2-dimethylaminomethyl-1-thiophen-2-yl-cyclohexanol
• 4-benzyloxy-2-dimethylaminomethyl-1-(3-methoxy-phenyl)-cyclohexanol
• 4-benzyloxy-2-dimethylaminomethyl-1-(3-phenyl-propyl)-cyclohexanol
• 4-benzyloxy-2-dimethylaminomethyl-1-p-tolyl-cyclohexanol
• 4-benzyloxy-2-dimethylaminomethyl-1-(4-methoxy-phenyl)-cyclohexanol
• 4-benzyloxy-2-dimethylaminomethyl-1-(3-fluoro-phenyl)-cyclohexanol
• 4-benzyloxy-1-(3-chloro-phenyl)-2-dimethylaminomethyl-cyclohexanol
• 4-benzyloxy-2-dimethylaminomethyl-1-(3-methoxy-benzyl)-cyclohexanol
• 4-benzyloxy-1-(4-chloro-3-trifluoromethyl-phenyl)-2-dimethylaminomethyl-cyclohexanol
• 4-benzyloxy-2-dimethylaminomethyl-1-(3-fluoro-benzyl)-cyclohexanol
• 4-benzyloxy-2-dimethylaminomethyl-1-(2-methyl-benzyl)-cyclohexanol
• 4-benzyloxy-2-dimethylaminomethyl-1-(2,5-dimethyl-benzyl)-cyclohexanol
• 4-(4-chloro-benzyl)-2-dimethylaminomethyl-1-(3-methoxy-phenyl)-cyclohexanol
• 4-(4-chloro-benzyl)-2-dimethylaminomethyl-1-(3-phenyl-propyl)-cyclohexanol
• 4-(4-chloro-benzyl)-1-(2,3-dichloro-phenyl)-2-dimethylaminomethyl-cyclohexanol
• 4-(4-chloro-benzyl)-1-cyclohexylmethyl-2-dimethylaminomethyl-cyclohexanol
• 4-(4-chloro-benzyl)-2-dimethylaminomethyl-1-(5-fluoro-2-methoxyphenyl)-cyclohexanol
• 4-(4-chloro-benzyl)-2-dimethylaminomethyl-1-(3-fluoro-phenyl)-cyclohexanol
• 4-(4-chloro-benzyl)-1-(3-chloro-phenyl)-2-dimethylaminomethyl-cyclohexanol
• 4-(4-chloro-benzyl)-1-(3,5-dichloro-phenyl)-2-dimethylaminomethyl-cyclohexanol
• 4-(4-chloro-benzyl)-1-(2-chloro-benzyl)-2-dimethylaminomethyl-cyclohexanol
• 4-(4-chloro-benzyl)-2-dimethylaminomethyl-1-(4-fluoro-benzyl)-cyclohexanol
• 4-(4-chloro-benzyl)-2-dimethylaminomethyl-1-(3-fluoro-benzyl)-cyclohexanol
• 4-(4-chloro-benzyl)-2-dimethylaminomethyl-1-(2-methoxy-phenyl)-cyclohexanol
• 4-(4-chloro-benzyl)-2-dimethylaminomethyl-1-(2-methyl-benzyl)-cyclohexanol
• 4-(4-chloro-benzyl)-2-dimethylaminomethyl-1-(3-methyl-benzyl)-cyclohexanol
• 1,4-bis-(4-chloro-benzyl)-2-dimethylaminomethyl-cyclohexanol
• 4-(4-chloro-benzyl)-1-(2-chloro-6-fluoro-benzyl)-2-dimethylaminomethyl-cyclohexanol
• 4-(4-chloro-benzyl)-2-dimethylaminomethyl-1-(2,5-dimethyl-benzyl)-cyclohexanol
• 4-(4-chloro-benzyl)-1-(3-chloro-benzyl)-2-dimethylaminomethyl-cyclohexanol
• 4-(4-chloro-benzyl)-1-(2,4-dichloro-benzyl)-2-dimethylaminomethyl-cyclohexanol
• 4-(4-chloro-benzyl)-2-dimethylaminomethyl-1-phenyl-cyclohexanol
• 4-(4-chloro-benzyl)-1-(4-chloro-phenyl)-2-dimethylaminomethyl-cyclohexanol
• 4-(4-chloro-benzyl)-2-dimethylaminomethyl-1-(4-fluoro-3-methyl-phenyl)-cyclohexanol
• 4-(4-chloro-benzyl)-2-dimethylaminomethyl-1-o-tolyl-cyclohexanol
• 4-(4-chloro-benzyl)-2-dimethyl aminomethyl-1-vinyl-cyclohexanol
• 4-(4-chloro-benzyl)-1-cyclopentyl-2-dimethylaminomethyl-cyclohexanol
• 4-(4-chloro-benzyl)-2-dimethylaminomethyl-1-m-tolyl-cyclohexanol
• 4-(4-chloro-benzyl)-2-dimethylaminomethyl-bicyclohexyl-1-ol
• 4-(4-chloro-benzyl)-2-dimethylaminomethyl-1-(4-fluoro-phenyl)-cyclohexanol
• 4-(4-chloro-benzyl)-2-dimethylaminomethyl-1-phenethyl-cyclohexanol
• 4-(4-chloro-benzyl)-2-dimethylaminomethyl-1-phenylethynyl-cyclohexanol
• 4-(4-chloro-benzyl)-2-dimethylaminomethyl-1-thiophen-2-yl-cyclohexanol
• 4-(4-chloro-benzyl)-2-dimethylaminomethyl-1-p-tolyl-cyclohexanol;
• 4-(4-chloro-benzyl)-2-dimethylaminomethyl-1-(4-methoxy-phenyl)-cyclohexanol
• 4-(4-chloro-benzyl)-2-dimethylaminomethyl-1-trimethylsilanylethynyl-cyclohexanol
• 4-(4-chloro-benzyl)-1-(4-chloro-3-trifluoromethyl-phenyl)-2-dimethylaminomethyl-cyclohexanol
• 4-(4-chloro-benzyl)-1-(3-chloro-4-fluoro-phenyl)-2-dimethylaminomethyl-cyclohexanol
• 4-(4-chloro-benzyl)-2-dimethylaminomethyl-1-(trifluoromethyl-phenyl)-cyclohexanol
• 2-dimethylaminomethyl-4-(4-fluoro-benzyl)-1-phenyl-cyclohexanol
• 1-(4-chloro-phenyl)-2-dimethylaminomethyl-4-(4-fluoro-benzyl)-cyclohexanol
• 1-benzyl-2-dimethylaminomethyl-4-(4-fluoro-benzyl)-cyclohexanol
• 2-dimethylaminomethyl-4-(4-fluoro-benzyl)-1-(4-fluoro-3-methyl-phenyl)-cyclohexanol
• 2-dimethylaminomethyl-4-(4-fluoro-benzyl)-1-o-tolyl-cyclohexanol
• 2-dimethylaminomethyl-4-(4-fluoro-benzyl)-1-vinyl-cyclohexanol
• 1-(4-tert-butyl-phenyl)-2-dimethylaminomethyl-4-(4-fluoro-benzyl)-cyclohexanol
• 1-cyclopentyl-2-dimethylaminomethyl-4-(4-fluoro-benzyl)-cyclohexanol
• 2-dimethylaminomethyl-4-(4-fluoro-benzyl)-1-m-tolyl-cyclohexanol
• 2-dimethylaminomethyl-4-(4-fluoro-benzyl)-bicyclohexyl-1-ol
• 2-dimethylaminomethyl-4-(4-fluoro-benzyl)-1-(4-fluoro-phenyl)-cyclohexanol
• 2-dimethylaminomethyl-4-(4-fluoro-benzyl)-1-phenethyl-cyclohexanol
• 2-dimethylaminomethyl-4-(4-fluoro-benzyl)-1-phenylethynyl-cyclohexanol
• 2-dimethylaminomethyl-4-(4-fluoro-benzyl)-1-thiophen-2-yl-cyclohexanol
• 2-dimethylaminomethyl-4-(4-fluoro-benzyl)-1-(3-methoxy-phenyl)-cyclohexanol
• 2-dimethylaminomethyl-4-(4-fluoro-benzyl)-1-(3-phenyl-propyl)-cyclohexanol
• 1-(2,3-dichloro-phenyl)-2-dimethylaminomethyl-4-(4-fluoro-benzyl)-cyclohexanol
• 2-dimethylaminomethyl-4-(4-fluoro-benzyl)-1-p-tolyl-cyclohexanol
• 2-dimethylaminomethyl-4-(4-fluoro-benzyl)-1-(4-methoxy-phenyl)-cyclohexanol
• 1-cyclohexylmethyl-2-dimethylaminomethyl-4-(4-fluoro-benzyl)-cyclohexanol
• 2-dimethylaminomethyl-4-(4-fluoro-benzyl)-1-(5-fluoro-2-methoxyphenyl)-cyclohexanol
• 2-dimethylaminomethyl-4-(4-fluoro-benzyl)-1-(3-fluoro-phenyl)-cyclohexanol
• 1-(3-chloro-phenyl)-2-dimethylaminomethyl-4-(4-fluoro-benzyl)-cyclohexanol
• 1-(3,5-dichloro-phenyl)-2-dimethylaminomethyl-4-(4-fluoro-benzyl)-cyclohexanol
• 1-(2-chloro-benzyl)-2-dimethylaminomethyl-4-(4-fluoro-benzyl)-cyclohexanol
• 2-dimethylaminomethyl-1,4-bis-(4-fluoro-benzyl)-cyclohexanol
• 2-dimethylaminomethyl-4-(4-fluoro-benzyl)-1-(3-methoxy-benzyl)-cyclohexanol
• 1-(4-chloro-3-trifluoromethyl-phenyl)-2-dimethylaminomethyl-4-(4-fluorobenzyl)-cyclohexanol
• 2-dimethylaminomethyl-4-(4-fluoro-benzyl)-1-(3-fluoro-benzyl)-cyclohexanol
• 2-dimethylaminomethyl-4-(4-fluoro-benzyl)-1-(2-methoxy-benzyl)-cyclohexanol
• 2-dimethylaminomethyl-4-(4-fluoro-benzyl)-1-(2-methyl-benzyl)-cyclohexanol
• 1-(3-chloro-4-fluoro-phenyl)-2-dimethylaminomethyl-4-(4-fluoro-benzyl)-cyclohexanol
• 2-dimethylaminomethyl-4-(4-fluoro-benzyl)-1-(3-trifluoromethyl-phenyl)-cyclohexanol
• 2-dimethylaminomethyl-4-(4-fluoro-benzyl)-1-(3-methyl-benzyl)-cyclohexanol
• 1-(4-chloro-benzyl)-2-dimethylaminomethyl-4-(4-fluoro-benzyl)-cyclohexanol
• 1-(2-chloro-6-fluoro-benzyl)-2-dimethylaminomethyl-4-(4-fluoro-benzyl)-cyclohexanol
• 2-dimethylaminomethyl-1-(2,5-dimethyl-benzyl)-4-(4-fluoro-benzyl)-cyclohexanol
• 1-(3-chloro-benzyl)-2-dimethylaminomethyl-4-(4-fluoro-benzyl)-cyclohexanol
• 1-(2,4-dichloro-benzyl)-2-dimethylaminomethyl-4-(4-fluoro-benzyl)-cyclohexanol
• 2-dimethylaminomethyl-4-(3-methoxy-benzyl)-1-phenyl-cyclohexanol
• 1-benzyl-2-dimethylaminomethyl-4-(3-methoxy-benzyl)-cyclohexanol
• 2-dimethylaminomethyl-1-(4-fluoro-3-methyl-phenyl)-4-(3-methoxybenzyl)-cyclohexanol
• 2-dimethylaminomethyl-4-(3-methoxy-benzyl)-1-o-tolyl-cyclohexanol
• 2-dimethylaminomethyl-4-(3-methoxy-benzyl)-1-vinyl-cyclohexanol
• 1-(4-tert-butyl-phnyl)-2-dimethylaminomethyl-4-(3-methoxy-benzyl)-cyclohexanol
• 1-cyclopentyl-2-dimethylaminomethyl-4-(3-methoxy-benzyl)-cyclohexanol
• 2-dimethylaminomethyl-4-(3-methoxy-benzyl)-1-m-tolyl-cyclohexanol
• 2-dimethylaminomethyl-4-(3-methoxy-benzyl)-1-bicyclohexyl-1-ol
• 2-dimethylaminomethyl-4-(3-methoxy-benzyl)-1-phenethyl-cyclohexanol
• 2-dimethylaminomethyl-4-(3-methoxy-benzyl)-1-phenylethynyl-cyclohexanol
• 2-dimethylaminomethyl-4-(3-methoxy-benzyl)-1-thiophen-2-yl-cyclohexanol
• 2-dimethylaminomethyl-4-(3-methoxy-benzyl)-1-(3-methoxy-phenyl)-cyclohexanol
• 2-dimethylaminomethyl-4-(3-methoxy-benzyl)-1-(3-phenyl-propyl)-cyclohexanol
• 1-(2,3-dichloro-phenyl)-2-dimethylaminomethyl-4-(3-methoxy-benzyl)-cyclohexanol
• 2-dimethylaminomethyl-4-(3-methoxy-benzyl)-1-p-tolyl-cyclohexanol
• 1-cyclohexylmethyl-2-dimethylaminomethyl-4-(3-methoxy-benzyl)-cyclohexanol
• 2-dimethylaminomethyl-1-(5-fluoro-2-methoxy-phenyl)-4-(3-methoxybenzyl)-cyclohexanol
• 1-(3-chloro-phenyl)-2-dimethylaminomethyl-4-(3-methoxy-benzyl)-cyclohexanol
• 1-(3,5-dichloro-phenyl)-2-dimethylaminomethyl-4-(3-methoxy-benzyl)-cyclohexanol
• 1-(4-chloro-3-trifluoromethyl-phenyl)-2-dimethylaminomethyl-4-(3-methoxy-benzyl)-cyclohexanol
• 2-dimethylaminomethyl-1-(3-fluoro-benzyl)-4-(3-methoxy-benzyl)-cyclohexanol
• 2-dimethylaminomethyl-4-(3-methoxy-benzyl)-1-(2-methoxy-phenyl)-cyclohexanol
• 2-dimethylaminomethyl-4-(3-methoxy-benzyl)-1-(3-trifluoromethylphenyl)-cyclohexanol
• 1-(2-chloro-6-fluoro-benzyl)-2-dimethylaminomethyl-4-(3-methoxybenzyl)-cyclohexanol
• 2-dimethylaminomethyl-1-(2,5-dimethyl-benzyl)-4-(3-methoxy-benzyl)-cyclohexanol
• 1-(3-chloro-benzyl)-2-dimethylaminomethyl-4-(3-methoxy-benzyl)-cyclohexanol
• 2-dimethylaminomethyl-4-(4-fluoro-benzyloxy)-1-phenyl-cyclohexanol
• 1-benzyl-2-dimethylaminomethyl-4-(4-fluoro-benzyloxy)-cyclohexanol
• 2-dimethylaminomethyl-4-(4-fluoro-benzyloxy)-1-(4-fluoro-3-methylphenyl)-cyclohexanol
• 2-dimethylaminomethyl-4-(4-fluoro-benzyloxy)-1-vinyl-cyclohexanol
• 1-(4-tert-butyl-phenyl)-2-dimethylaminomethyl-4-(4-fluoro-benzyloxy)-cyclohexanol
• 1-cyclopentyl-2-dimethylaminomethyl-4-(4-fluoro-benzyloxy)-cyclohexanol
• 2-dimethylaminomethyl-4-(4-fluoro-benzyloxy)-1-m-tolyl-cyclohexanol
• 2-dimethylaminomethyl-4-(4-fluoro-benzyloxy)-1-bicyclohexyl-1-ol
• 2-dimethylaminomethyl-4-(4-fluoro-benzyloxy)-1-phenethyl-cyclohexanol
• 2-dimethylaminomethyl-4-(4-fluoro-benzyloxy)-1-phenylethynyl-cyclohexanol
• 2-dimethylaminomethyl-4-(4-fluoro-benzyloxy)-1-thiophen-2-yl-cyclohexanol
• 2-dimethylaminomethyl-4-(4-fluoro-benzyloxy)-1-(3-methoxy-phenyl)-cyclohexanol
• 2-dimethylaminomethyl-4-(4-fluoro-benzyloxy)-1-p-tolyl-cyclohexanol
• 1-cyclohexylmethyl-2-dimethylaminomethyl-4-(4-fluoro-benzyloxy)-cyclohexanol
• 2-dimethylaminomethyl-1-(3-fluoro-benzyl)-4-(4-fluoro-benzyloxy)-cyclohexanol
• 2-dimethylaminomethyl-4-(4-fluoro-benzyloxy)-1-(2-methoxy-phenyl)-cyclohexanol
• 2-dimethylaminomethyl-4-(4-fluoro-benzyloxy)-1-(3-methyl-benzyl)-cyclohexanol
• 2-dimethylaminomethyl-1-(2,5-dimethyl-benzyl)-4-(4-fluoro-benzyloxy)-cyclohexanol
• 1-(3-chloro-benzyl)-2-dimethylaminomethyl-4-(4-fluoro-benzyloxy)-cyclohexanol
• [5-benzyloxy-2-(3-methoxy-phenyl)-cyclohexylmethyl]-dimethyl-amine;
• [5-(3-chloro-benzyloxy)-2-(3-methoxy-phenyl)-cyclohexylmethyl]-dimethyl-amine;
• [2-(3-methoxy-phenyl)-5-(naphthalen-2-ylmethoxy)-cyclohexylmethyl]-dimethyl-amine;
• [5-(3-methoxy-benzyloxy)-2-(3-methoxy-phenyl)-cyclohexylmethyl]-dimethyl-amine;
• [5-(4-chloro-benzyloxy)-2-(3-methoxy-phenyl)-cyclohexylmethyl]-dimethyl-amine;
• [5-(4-methoxy-benzyloxy)-2-(3-methoxy-phenyl)-cyclohexylmethyl]-dimethyl-amine;
• 2-dimethylaminomethyl-1-(3-methoxy-phenyl)-4-(naphthalene-2-ylmethoxy)-cyclohexanol;
• butyric acid 3-dimethylaminomethyl-4-hydroxy-4-(3-methoxy-phenyl)-cyclohexanol;
• 4-methoxy-benzoic acid 3-dimethylaminomethyl-4-hydroxy-4-(3-methoxyphenyl)-cyclohexylester;
• 3-methoxy-benzoic acid 3-dimethylaminomethyl-4-hydroxy-4-(3-methoxyphenyl)-cyclohexylester;
• 2,2-dimethyl-propionic acid 3-dimethylaminomethyl-4-hydroxy-4-(3-methoxy-phenyl)-cyclohexylester;
• butyric acid 3-dimethylaminomethyl-4-hydroxy-4-(3-methoxy-phenyl)-cyclohexanol;
• 4-methoxy-benzoic acid 3-dimethylaminomethyl-4-hydroxy-4-(3-methoxyphenyl)-cyclohexylester;
• 3-methoxy-benzoic acid 3-dimethylaminomethyl-4-hydroxy-4-(3-methoxyphenyl)-cyclohexylester;
• 2,2-dimethyl-propionic acid 3-dimethylaminomethyl-4-hydroxy-4-(3-methoxy-phenyl)-cyclohexylester;
• 3,4-dimethoxy-benzoic acid 3-dimethylaminomethyl-4-hydroxy-4-(3-methoxy-phenyl)-cyclohexylester;
• 3,4-dimethoxy-benzoic acid 3-dimethylaminomethyl-4-hydroxy-4-(3-methoxy-phenyl)-cyclohexylester;
• Naphthalene-2-carboxylic acid 3-dimethylaminomethyl-4-hydroxy-4-(3-methoxy-phenyl)-cyclohexylester;
• 4-methyl-benzoic acid 3-dimethylaminomethyl-4-hydroxy-4-(3-methoxyphenyl)-cyclohexylester;
• 3,4-dichloro-benzoic acid 3-dimethylaminomethyl-4-hydroxy-4-(3-methoxy-phenyl)-cyclohexylester;
• 4-chloro-benzoic acid 3-dimethylaminomethyl-4-hydroxy-4-(3-methoxyphenyl)-cyclohexylester;
• 3-chloro-benzoic acid 3-dimethylaminomethyl-4-hydroxy-4-(3-methoxyphenyl)-cyclohexylester;
• 2-chloro-benzoic acid 3-dimethylaminomethyl-4-hydroxy-4-(3-methoxyphenyl)-cyclohexylester;
• 4-trifluoromethyl-benzoic acid 3-dimethylaminomethyl-4-hydroxy-4-(3-methoxy-phenyl)-cyclohexylester;
• 3,5-difluoro-benzoic acid 3-dimethylaminomethyl-4-hydroxy-4-(3-methoxy-phenyl)-cyclohexylester;
• 4-fluoro-benzoic acid 3-dimethylaminomethyl-4-hydroxy-4-(3-methoxyphenyl)-cyclohexylester;
• 3-fluoro-benzoic acid 3-dimethylaminomethyl-4-hydroxy-4-(3-methoxyphenyl)-cyclohexylester;
• 2-fluoro-benzoic acid 3-dimethylaminomethyl-4-hydroxy-4-(3-methoxyphenyl)-cyclohexylester;
• Cyclopentane carboxylic acid 3-dimethylaminomethyl-4-hydroxy-4-(3-methoxy-phenyl)-cyclohexylester;
• (4-methoxy-phenyl)-acetic acid 3-dimethylaminomethyl-4-hydroxy-4-(3-methoxy-phenyl)-cyclohexylester;
• (3-methoxy-phenyl)-acetic acid 3-dimethylaminomethyl-4-hydroxy-4-(3-methoxy-phenyl)-cyclohexylester;
• phenyl-acetic acid 3-dimethylaminomethyl-4-hydroxy-4-(3-methoxyphenyl)-cyclohexylester;
• 3,4,5-trimethoxy-benzoic acid 3-dimethylaminomethyl-4-hydroxy-4-(3-methoxy-phenyl)-cyclohexylester;
• 3,5-dimethoxy-benzoic acid 3-dimethylaminomethyl-4-hydroxy-4-(3-methoxy-phenyl)-cyclohexylester;
• Naphthalene-1-carboxylic acid 3-dimethylaminomethyl-4-hydroxy-4-(3-methoxy-phenyl)-cyclohexylester;
• 2-methoxy-benzoic acid 3-dimethylaminomethyl-4-hydroxy-4-(3-methoxyphenyl)-cyclohexylester;
• Naphthalene-2-carboxylic acid 3-dimethylaminomethyl-4-hydroxy-4-(3-methoxy-phenyl)-cyclohexylester;
• 2-hydroxy-benzoic acid 3-dimethylaminomethyl-4-hydroxy-4-(3-methoxyphenyl)-cyclohexylester;
• 4-methyl-benzoic acid 3-dimethylaminomethyl-4-hydroxy-4-(3-methoxyphenyl)-cyclohexylester;
• 3,4-dichloro-benzoic acid 3-dimethylaminomethyl-4-hydroxy-4-(3-methoxy-phenyl)-cyclohexylester;
• 4-chloro-benzoic acid 3-dimethylaminomethyl-4-hydroxy-4-(3-methoxyphenyl)-cyclohexylester;
• 3-chloro-benzoic acid 3-dimethylaminomethyl-4-hydroxy-4-(3-methoxyphenyl)-cyclohexylester;
• 2-chloro-benzoic acid 3-dimethylaminomethyl-4-hydroxy-4-(3-methoxyphenyl)-cyclohexylester;
• 4-trifluoromethyl-benzoic acid 3-dimethylaminomethyl-4-hydroxy-4-(3-methoxy-phenyl)-cyclohexylester;
• 3,5-difluoro-benzoic acid 3-dimethylaminomethyl-4-hydroxy-4-(3-methoxy-phenyl)-cyclohexylester;
• 4-fluoro-benzoic acid 3-dimethylaminomethyl-4-hydroxy-4-(3-methoxyphenyl)-cyclohexylester;
• 3-fluoro-benzoic acid 3-dimethylaminomethyl-4-hydroxy-4-(3-methoxyphenyl)-cyclohexylester;
• 2-fluoro-benzoic acid 3-dimethylaminomethyl-4-hydroxy-4-(3-methoxyphenyl)-cyclohexylester;
• Cyclopentane carboxylic acid 3-dimethylaminomethyl-4-hydroxy-4-(3-methoxy-phenyl)-cyclohexylester;
• (4-methoxy-phenyl)-acetic acid 3-dimethylaminomethyl-4-hydroxy-3234-(3-methoxy-phenyl)-cyclohexylester;
• (3-methoxy-phenyl)-acetic acid 3-dimethylaminomethyl-4-hydroxy-4-(3-methoxy-phenyl)-cyclohexylester;
• phenyl-acetic acid 3-dimethylaminomethyl-4-hydroxy-4-(3-methoxyphenyl)-cyclohexylester;
• 3,4,5-trimethoxy-benzoic acid 3-dimethylaminomethyl-4-hydroxy-4-(3-methoxy-phenyl)-cyclohexylester;
• 3,5-dimethoxy-benzoic acid 3-dimethylaminomethyl-4-hydroxy-4-(3-methoxy-phenyl)-cyclohexylester;
• Naphthalene-1-carboxylic acid 3-dimethylaminomethyl-4-hydroxy-4-(3-methoxy-phenyl)-cyclohexylester;
• 2-methoxy-benzoic acid 3-dimethylaminomethyl-4-hydroxy-4-(3-methoxyphenyl)-cyclohexylester;
• 2-dimethylaminomethyl-1,4-bis-(3-methoxy-phenyl)-cyclohexane-1,4-diol;
• 2-dimethylaminomethyl-1,4-bis-(3-methoxy-phenyl)-cyclohexane-1,4-diol;
• 6-(4-benzyloxy-2-dimethylaminomethyl-1-hydroxy-cyclohexyl)-naphthalen-2-ol;
• 4-benzyloxy-2-dimethylaminomethyl-1-(6-methoxy-naphthalen-2-yl)-cyclohexanol;
• Butyric acid 3-dimethylaminomethyl-4-hydroxy-4-(3-methoxy-phenyl)-cyclohexylester;
• Butyric acid 3-dimethylaminomethyl-4-hydroxy-4-(3-methoxy-phenyl)-cyclohexylester;
• 2-dimethylaminomethyl-1-(3-methoxy-phenyl)-4-methylamino-cyclohexanol;
• 2-dimethylaminomethyl-1-(3-methoxy-phenyl)-4-methylamino-cyclohexanol;
• Valeric acid 3-dimethylaminomethyl-4-hydroxy-4-(3-methoxy-phenyl)-cyclohexylester;
• [3-dimethylaminomethyl-4-hydroxy-4-(3-methoxy-phenyl)-cyclohexylidene]-acetic acid ethylester;
• [3-dimethylaminomethyl-4-hydroxy-4-(3-methoxy-phenyl)-cyclohexylidene]-acetic acid ethylester;
• 3-[2-dimethylaminomethyl-4-(4-fluoro-benzyloxy)-1-hydroxy-cyclohexyl]-phenol;
• 4-benzyloxy-2-dimethylaminomethyl-1-(3-phenyl-propyl)-cyclohexanol;
• 2-dimethylaminomethyl-4-(4-fluoro-benzyloxy)-1-(3-phenyl-propyl)-cyclohexanol;
• 2-dimethylaminomethyl-1-(3-fluoro-benzyl)-4-(4-fluoro-benzyloxy)-cyclohexanol;
• 4-benzyloxy-2-dimethylaminomethyl-1-(3-fluoro-benzyl)-cyclohexanol;
• 2-dimethylaminomethyl-4-(4-fluoro-benzyloxy)-1-p-tolyl-cyclohexanol;
• 4-benzyloxy-2-dimethylaminomethyl-1-p-tolyl-cyclohexanol;
• 2-dimethylaminomethyl-1-(2,5-dimethyl-benzyl)-4-(4-fluoro-benzyloxy)-cyclohexanol;
• 4-benzyloxy-2-dimethylaminomethyl-1-(2,5-dimethyl-benzyl)-cyclohexanol;
• 1-(4-chloro-3-trifluoromethyl-phenyl)-2-dimethylaminomethyl-4-(4-fluoro-benzyloxy)-cyclohexanol;
• 4-benzyloxy-1-(4-chloro-3-trifluoromethyl-phenyl)-2-dimethylaminomethyl-cyclohexanol;
• [2,5-bis-(4-fluoro-benzyloxy)-2-(3-methoxy-phenyl)-cyclohexylmethyl]-dimethyl-amine;
• 2-dimethylaminomethyl-1-(6-methoxynaphthalen-2-yl)cyclohexan-1,4-diol;
• Butyric acid 3-dimethylaminomethyl-4-hydroxy-4-(6-methoxynaphthalen-2-yl)cyclohexylester;
• Benzoic acid 4-hydroxy-3-(4-methyl-piperazin-1-ylmethyl)-4-naphthalen-2-yl-cyclohexyl ester;
• 2-chloro-benzoic acid 4-hydroxy-3-(4-methyl-piperazin-1-ylmethyl)-4-naphthalen-2-yl-cyclohexyl ester;
• 3-chloro-benzoic acid 4-hydroxy-3-(4-methyl-piperazin-1-ylmethyl)-4-naphthalen-2-yl-cyclohexyl ester;
• 4-chloro-benzoic acid 4-hydroxy-3-(4-methyl-piperazin-1-ylmethyl)-4-naphthalen-2-yl-cyclohexyl ester;
• 2-fluoro-benzoic acid 4-hydroxy-3-(4-methyl-piperazin-1-ylmethyl)-4-naphthalen-2-yl-cyclohexyl ester;
• 3-fluoro-benzoic acid 4-hydroxy-3-(4-methyl-piperazin-1-ylmethyl)-4-naphthalen-2-yl-cyclohexyl ester;
• 4-fluoro-benzoic acid 4-hydroxy-3-(4-methyl-piperazin-1-ylmethyl)-4-naphthalen-2-yl-cyclohexyl ester;
• 2-methyl-benzoic acid 4-hydroxy-3-(4-methyl-piperazin-1-ylmethyl)-4-naphthalen-2-yl-cyclohexyl ester;
• 3-methyl-benzoic acid 4-hydroxy-3-(4-methyl-piperazin-1-ylmethyl)-4-naphthalen-2-yl-cyclohexyl ester;
• 4-methyl-benzoic acid 4-hydroxy-3-(4-methyl-piperazin-1-ylmethyl)-4-naphthalen-2-yl-cyclohexyl ester;
• 2-methoxy-benzoic acid 4-hydroxy-3-(4-methyl-piperazin-1-ylmethyl)-4-naphthalen-2-yl-cyclohexyl ester;
• 3-methoxy-benzoic acid 4-hydroxy-3-(4-methyl-piperazin-1-ylmethyl)-4-naphthalen-2-yl-cyclohexyl ester;
• 4-methoxy-benzoic acid 4-hydroxy-3-(4-methyl-piperazin-1-ylmethyl)-4-naphthalen-2-yl-cyclohexyl ester;
• 2,6-dichloro-benzoic acid 4-hydroxy-3-(4-methyl-piperazin-1-ylmethyl)-4-naphthalen-2-yl-cyclohexyl ester;
• 2,6-difluoro-benzoic acid 4-hydroxy-3-(4-methyl-piperazin-1-ylmethyl)-4-naphthalen-2-yl-cyclohexyl ester;
• 2-chloro-5-fluoro-benzoic acid 4-hydroxy-3-(4-methyl-piperazin-1-ylmethyl)-4-naphthalen-2-yl-cyclohexyl ester;
• Biphenyl-4-carboxylic acid 4-hydroxy-3-(4-methyl-piperazin-1-ylmethyl)-4-naphthalen-2-yl-cyclohexyl ester;
• 2-chloro-benzoic acid 4-hydroxy-4-(6-methoxy-naphthalen-2-yl)-3-(4-methyl-piperazin-1-ylmethyl)-cyclohexyl ester;
• 3-chloro-benzoic acid 4-hydroxy-4-(6-methoxy-naphthalen-2-yl)-3-(4-methyl-piperazin-1-ylmethyl)-cyclohexyl ester;
• 4-chloro-benzoic acid 4-hydroxy-4-(6-methoxy-naphthalen-2-yl)-3-(4-methyl-piperazin-1-ylmethyl)-cyclohexyl ester;
• 2-fluoro-benzoic acid 4-hydroxy-4-(6-methoxy-naphthalen-2-yl)-3-(4-methyl-piperazin-1-ylmethyl)-cyclohexyl ester;
• 3-fluoro-benzoic acid 4-hydroxy-4-(6-methoxy-naphthalen-2-yl)-3-(4-methyl-piperazin-1-ylmethyl)-cyclohexyl ester;
• 4-fluoro-benzoic acid 4-hydroxy-4-(6-methoxy-naphthalen-2-yl)-3-(4-methyl-piperazin-1-ylmethyl)-cyclohexyl ester;
• 2-methyl-benzoic acid 4-hydroxy-4-(6-methoxy-naphthalen-2-yl)-3-(4-methyl-piperazin-1-ylmethyl)-cyclohexyl ester;
• 3-methyl-benzoic acid 4-hydroxy-4-(6-methoxy-naphthalen-2-yl)-3-(4-methyl-piperazin-1-ylmethyl)-cyclohexyl ester;
• 4-methyl-benzoic acid 4-hydroxy-4-(6-methoxy-naphthalen-2-yl)-3-(4-methyl-piperazin-1-ylmethyl)-cyclohexyl ester;
• 2-methoxy-benzoic acid 4-hydroxy-4-(6-methoxy-naphthalen-2-yl)-3-(4-methyl-piperazin-1-ylmethyl)-cyclohexyl ester;
• 3-methoxy-benzoic acid 4-hydroxy-4-(6-methoxy-naphthalen-2-yl)-3-(4-methyl-piperazin-1-ylmethyl)-cyclohexyl ester;
• 4-methoxy-benzoic acid 4-hydroxy-4-(6-methoxy-naphthalen-2-yl)-3-(4-methyl-piperazin-1-ylmethyl)-cyclohexyl ester;
• 2,6-dichloro-benzoic acid 4-hydroxy-4-(6-methoxy-naphthalen-2-yl)-3-(4-methyl-piperazin-1-ylmethyl)-cyclohexyl ester;
• 2,6-fluoro-benzoic acid 4-hydroxy-4-(6-methoxy-naphthalen-2-yl)-3-(4-methyl-piperazin-1-ylmethyl)-cyclohexyl ester;
• 2-chloro-6-fluoro-benzoic acid 4-hydroxy-4-(6-methoxy-naphthalen-2-yl)-3-(4-methyl-piperazin-1-ylmethyl)-cyclohexyl ester;
• Biphenyl-4-carboxylic acid 4-hydroxy-4-(6-methoxy-naphthalen-2-yl)-3-(4-methyl-piperazin-1-ylmethyl)-cyclohexyl ester;
• 4-(2-chloro-benzyloxy)-2-(4-methyl-piperazin-1-ylmethyl)-1-naphthalen-2-yl-cyclohexanol;
• 4-(3-chloro-benzyloxy)-2-(4-methyl-piperazin-1-ylmethyl)-1-naphthalen-2-yl-cyclohexanol;
• 4-(4-chloro-benzyloxy)-2-(4-methyl-piperazin-1-ylmethyl)-1-naphthalen-2-yl-cyclohexanol;
• 4-(2-fluoro-benzyloxy)-2-(4-methyl-piperazin-1-ylmethyl)-1-naphthalen-2-yl-cyclohexanol;
• 4-(3-fluoro-benzyloxy)-2-(4-methyl-piperazin-1-ylmethyl)-1-naphthalen-2-yl-cyclohexanol;
• 4-(4-fluoro-benzyloxy)-2-(4-methyl-piperazin-1-ylmethyl)-1-naphthalen-2-yl-cyclohexanol;
• 4-(2-methyl-benzyloxy)-2-(4-methyl-piperazin-1-ylmethyl)-1-naphthalen-2-yl-cyclohexanol;
• 4-(3-methyl-benzyloxy)-2-(4-methyl-piperazin-1-ylmethyl)-1-naphthalen-2-yl-cyclohexanol;
• 4-(4-methyl-benzyloxy)-2-(4-methyl-piperazin-1-ylmethyl)-1-naphthalen-2-yl-cyclohexanol;
• 4-(2-methoxy-benzyloxy)-2-(4-methyl-piperazin-1-ylmethyl)-1-naphthalen-2-yl-cyclohexanol;
• 4-(3-methoxy-benzyloxy)-2-(4-methyl-piperazin-1-ylmethyl)-1-naphthalen-2-yl-cyclohexanol;
• 4-(4-methoxy-benzyloxy)-2-(4-methyl-piperazin-1-ylmethyl)-1-naphthalen-2-yl-cyclohexanol;
• 4-(2,6-dichloro-benzyloxy)-2-(4-methyl-piperazin-1-ylmethyl)-1-naphthalen-2-yl-cyclohexanol;
• 4-(2,6-difluoro-benzyloxy)-2-(4-methyl-piperazin-1-ylmethyl)-1-naphthalen-2-yl-cyclohexanol;
• 4-(2-chloro-6-fluoro-benzyloxy)-2-(4-methyl-piperazin-1-ylmethyl)-1-naphthalen-2-yl-cyclohexanol;
• 4-(2-chloro-benzyloxy)-1-(6-methoxy-naphthalen-2-yl)-2-(4-methyl-piperazin-1-ylmethyl)-cyclohexanol;
• 4-(3-chloro-benzyloxy)-1-(6-methoxy-naphthalen-2-yl)-2-(4-methyl-piperazin-1-ylmethyl)-cyclohexanol;
• 4-(4-chloro-benzyloxy)-1-(6-methoxy-naphthalen-2-yl)-2-(4-methyl-piperazin-1-ylmethyl)-cyclohexanol;
• 4-(2-fluoro-benzyloxy)-1-(6-methoxy-naphthalen-2-yl)-2-(4-methyl-piperazin-1-ylmethyl)-cyclohexanol;
• 4-(3-fluoro-benzyloxy)-1-(6-methoxy-naphthalen-2-yl)-2-(4-methyl-piperazin-1-ylmethyl)-cyclohexanol;
• 4-(4-fluoro-benzyloxy)-1-(6-methoxy-naphthalen-2-yl)-2-(4-methyl-piperazin-1-ylmethyl)-cyclohexanol;
• 4-(2-methyl-benzyloxy)-1-(6-methoxy-naphthalen-2-yl)-2-(4-methyl-piperazin-1-ylmethyl)-cyclohexanol;
• 4-(3-methyl-benzyloxy)-1-(6-methoxy-naphthalen-2-yl)-2-(4-methyl-piperazin-1-ylmethyl)-cyclohexanol;
• 4-(4-methyl-benzyloxy)-1-(6-methoxy-naphthalen-2-yl)-2-(4-methyl-piperazin-1-ylmethyl)-cyclohexanol;
• 4-(2-methoxy-benzyloxy)-1-(6-methoxy-naphthalen-2-yl)-2-(4-methyl-piperazin-1-ylmethyl)-cyclohexanol;
• 4-(3-methoxy-benzyloxy)-1-(6-methoxy-naphthalen-2-yl)-2-(4-methyl-piperazin-1-ylmethyl)-cyclohexanol;
• 4-(4-methoxy-benzyloxy)-1-(6-methoxy-naphthalen-2-yl)-2-(4-methyl-piperazin-1-ylmethyl)-cyclohexanol;
• 4-(2,6-dichloro-benzyloxy)-1-(6-methoxy-naphthalen-2-yl)-2-(4-methyl-piperazin-1-ylmethyl)-cyclohexanol;
• 4-(2,6-difluoro-benzyloxy)-1-(6-methoxy-naphthalen-2-yl)-2-(4-methyl-piperazin-1-ylmethyl)-cyclohexanol;
• 4-(2-chloro-6-fluoro-benzyloxy)-1-(6-methoxy-naphthalen-2-yl)-2-(4-methyl-piperazin-1-ylmethyl)-cyclohexanol;
• 2-dimethylaminomethyl-4-(4-fluoro-benzyloxy)-1-naphthalen-2-yl-cyclohexanol;
• 4-fluoro-benzoic acid 3-dimethylaminomethyl-4-hydroxy-4-(6-methoxy-naphthalen-2-yl)-cyclohexylester;
• 4-fluoro-benzoic acid 3-dimethylaminomethyl-4-hydroxy-4-naphthalen-2-yl-cyclohexylester;
optionally in the form of their racemates, their pure stereoisomers, or in the form of blends of stereoisomers in any mixing ratio; in the illustrated form or in the form of their acids or their bases or in the form of their salts, or in the form of their solvates.

9. Pharmaceutical composition containing at least one substituted C-cyclohexylmethylamine derivative according to any one of claims 1 to 8, and optionally suitable additives and/or auxiliaries and/or optionally further active ingredients.

10. Pharmaceutical composition according to claim 9, **characterised in that** a contained substituted C-cyclohexylmethylamine derivative according to any one of claims 1 to 8 is present as a pure diastereomer and/or enantiomer, as a racemate or as a non-equimolar or equimolar blend of the diastereomers and/or enantiomers.

11. Use of a substituted C-cyclohexylmethylamine derivative according to any one of claims 1 to 8 for producing a pharmaceutical composition for treating pain, in particular neuropathic or chronic pain.

12. Use according to claim 11, **characterised in that** a used substituted C-cyclohexylmethylamine derivative according to any one of claims 1 to 8 is present as a pure diastereomer and/or enantiomer, as a racemate or as a non-equimolar or equimolar blend of the diastereomers and/or enantiomers.

## Revendications

1. Dérivés de cyclohexylméthylamine C-substitués de formule générale I, 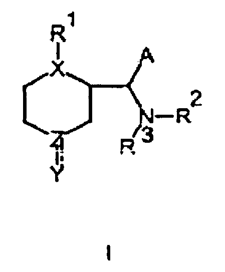 dans laquelle
A est choisi entre H ou un reste phényle ;
R¹ est choisi entre un reste alkyle en C₁ à C₁₀ ou cycloalkyle en C₃ à C₁₀, chacun saturé ou non saturé, ramifié ou non ramifié, substitué une ou plusieurs fois ou non substitué, un reste naphtyle, phényle, sous réserve que R¹ ne soit pas un reste phényle une fois O- ou S-substitué en position 3, un reste furyle, thiophényle, un reste naphtyle, phényle lié par l'intermédiaire d'un groupe alkylène en C₁ à C₃ ou éthynyle, un reste cycloalkyle en C₃ à C₁₀ lié par l'intermédiaire d'un groupe alkylène en C₁ à C₃ ou éthynyle, ou un reste furyle, thiophényle, lié par l'intermédiaire d'un groupe alkylène en C₁ à C₃ ou éthynyle, chacun non substitué ou substitué une ou plusieurs fois,
les substituants étant choisis indépendamment les uns des autres entre
F, Cl, Br, I, OR¹⁸, SR¹⁸, SO₂R¹⁸, SO₂OR¹⁸_{,} CN, COOR¹⁸, NR¹⁹R²⁰ ; alkyle en C₁ à C₁₀ ou cycloalkyle en C₃ à C₁₀, chacun non substitué ou substitué une ou plusieurs fois, identiques ou différentes, avec F, Cl, Br, I, NH₂, SH ou OH, ou un radical silyle,
R¹⁸ étant choisi entre H ; un reste alkyle en C₁ à C₁₀ ou cycloalkyle en C₃ à C₁₀, chacun saturé ou non saturé, ramifié ou non ramifié,
R¹⁹ et R²⁰ sont choisis indépendamment l'un de l'autre entre H ; un reste alkyle en C₁ à C₁₀ ou cycloalkyle en C₃ à C₁₀, chacun saturé ou non saturé, ramifié ou non ramifié,
ou bien R¹⁹ et R²⁰ forment ensemble un groupe CH₂CH₂OCH₂CH₂, CH₂CH₂NR²¹CH₂CH₂ ou (CH₂)₃₋₆,
R²¹ étant choisi entre H; un reste phényle ; alkyle en C₁ à C₁₀ saturé ou non saturé, ramifié ou non ramifié ;
R² et R³ sont choisis, indépendamment l'un de l'autre, entre H ; un reste alkyle en C₁ à C₁₀ ou cycloalkyle en C₃ à C₁₀, chacun saturé ou non saturé, ramifié ou non ramifié,
ou bien les restes R² et R³ forment ensemble un groupe CH₂CH₂OCH₂CH₂, CH₂CH₂NR⁶CH₂CH₂ ou (CH₂)₃₋₆,
R⁶ étant choisi entre H ; un radical alkyle en C₁ à C₁₀ ou cycloalkyle en C₃ à C₁₀, chacun saturé ou non saturé, ramifié ou non ramifié ;
X dans la formule I, conformément à la formule partielle Ia de la formule I 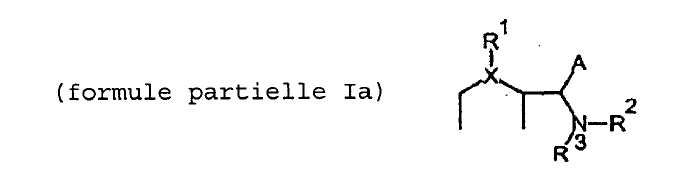 représente 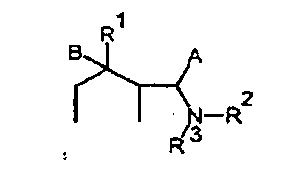 B étant choisi entre OH, OR⁷, H, F, Cl ou NR⁸R⁹,
R⁷ étant choisi entre un reste alkyle en C₁ à C₁₀ ou un reste cycloalkyle en C₃ à C₁₀, chacun saturé ou non saturé, ramifié ou non ramifié, non substitué ou substitué une ou plusieurs fois, identiques ou différentes, avec F, Cl, Br, I, NH₂, SH ou OH ;
R⁸ et R⁹ sont choisis, indépendamment l'un de l'autre, entre H ; un reste alkyle en C₁ à C₁₀ ou cycloalkyle en C₃ à C₁₀, chacun saturé ou non saturé, ramifié ou non ramifié, non substitué ou substitué une ou plusieurs fois, identiques ou différentes, avec F, Cl, Br, I, NH₂, SH ou OH ;
ou bien les restes R⁸ et R⁹ forment ensemble un groupe CH₂CH₂OCH₂CH₂, CH₂CH₂NR¹⁰CH₂CH₂ ou (CH₂)₃₋₆,
R¹⁰ étant choisi entre H ; un reste alkyle en C₁ à C₁₀ ou cycloalkyle en C₃ à C₁₀, chacun saturé ou non saturé, ramifié ou non ramifié, non substitué ou substitué une ou plusieurs fois, identiques ou différentes, avec F, Cl, Br, I, NH₂, SH ou OH ; 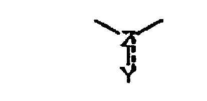 est choisi entre 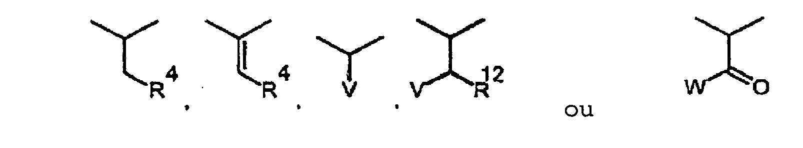 R⁴ étant choisi entre COR⁵, SO₂R⁵; un reste alkyle en C₁ à C₁₀ ou cycloalkyle en C₃ à C₁₀, chacun saturé ou non saturé, ramifié ou non ramifié, substitué une ou plusieurs fois ou non substitué ; un reste naphtyle, phényle, furyle ou thiophényle, chacun substitué une ou plusieurs fois ou non substitué ; ou un reste naphtyle, phényle, cycloalkyle en C₃ à C₁₀, furyle ou thiophényle lié par l'intermédiaire d'un groupe alkylène en C₁ à C₃, chacun substitué une ou plusieurs fois ou non substitué,
R⁵ étant choisi entre un reste alkyle en C₁ à C₁₀ ou cycloalkyle en C₃ à C₁₀, chacun saturé ou non saturé, ramifié ou non ramifié, substitué une ou plusieurs fois ou non substitué ; un reste naphtyle, phényle, furyle, thiophényle, chacun substitué une ou plusieurs fois ou non substitué ; ou un reste naphtyle, phényle, cycloalkyle en C₃ à C₁₀, furyle ou thiophényle lié par l'intermédiaire d'un groupe alkylène en C₁ à C₃, chacun substitué une ou plusieurs fois ou non substitué,
V est choisi entre OR⁴ et NR⁴R¹¹,
W est choisi entre R¹¹, OR¹² et NR¹¹R¹²,
R¹¹ et R¹² étant choisis indépendamment l'un de l'autre entre H, un reste alkyle en C₇ à C₁₀ ou cycloalkyle en C₃ à C₁₀, chacun saturé ou non saturé, ramifié ou non ramifié, substitué une ou plusieurs fois ou non substitué ; un reste naphtyle, phényle, furyle, ou thiophényle, chacun substitué une ou plusieurs fois ou non substitué ; ou un reste naphtyle, phényle, cycloalkyle en C₃ à C₁₀, furyle ou thiophényle lié par l'intermédiaire d'un groupe alkylène en C₁ à C₃, chacun substitué une ou plusieurs fois ou non substitué,
le terme substitué en rapport avec alkyle et cycloalkyle de R⁴, R⁵, R¹¹ et R¹² signifiant la substitution avec F, Cl, Br, I, NH₂, SH ou OH, et le terme substitué en rapport avec naphtyle, phényle, furyle et thiophényle de R⁴, R⁵, R¹¹ et R¹² signifiant la substitution de naphtyle, phényle, furyle ou thiophényle avec R²³ , OR²³, un halogène, un radical CF₃, un radical CN, un radical NO₂, un radical NR²⁴R²⁵, un radical alkyle en C₁ à C₆ (saturé), un radical alkoxy en C₁ à C₆, un radical cycloalkoxy en C₃ à C₈, un radical cycloalkyle en C₃ à C₈ ou un radical alkylène en C₂ à C₆,
le reste R²³ représentant H, un reste alkyle en C₁ à C₁₀, un reste naphtyle, phényle, furyle ou thiophényle ou un reste naphtyle, phényle, furyle ou thiophényle lié par l'intermédiaire d'un groupe alkylène en C₁ à C₃, ces restes naphtyle, phényle, furyle et thiophényle ne pouvant pas être substitués eux-mêmes avec des restes aryle ou hétéroaryle,
les restes R²⁴ et R²⁵, identiques ou différents, représentant H, un reste alkyle en C₁ à C₁₀, un reste naphtyle, phényle, furyle ou thiophényle ou un reste naphtyle, phényle, furyle ou thiophényle lié par l'intermédiaire d'un groupe alkylène en C₁ à C₃, ces restes naphtyle, phényle, furyle et thiophényle ne pouvant pas eux-mêmes être substitués avec des restes aryle ou hétéroaryle,
ou bien les restes R²⁴ et R²⁵ forment ensemble un groupe CH₂CH₂OCH₂CH₂, CH₂CH₂NR²⁶CH₂CH₂ ou (CH₂)₃₋₆, et
le reste R²⁶ représentant H, un reste alkyle en C₁ à C₁₀, un reste naphtyle, phényle, furyle ou thiophényle ou un reste naphtyle, phényle, furyle ou thiophényle lié par l'intermédiaire d'un groupe alkylène en C₁ à C₃, ces restes naphtyle, phényle, furyle ou thiophényle ne pouvant pas être substitués eux-mêmes avec des restes aryle ou hétéroaryle,
le cas échéant sous forme de leurs racémates, de leurs stéréo-isomères, ou sous forme de mélanges des stéréo-isomères, dans un rapport de mélange quelconque, sous la forme représentée ou sous la forme de leurs acides ou de leurs bases ou sous forme de leurs sels, ou sous forme de leurs produits de solvatation,
à l'exception de composés dans lesquels
R², R³ sont choisis indépendamment l'un de l'autre entre H, alkyle en C₁ à C₁₀, ramifié ou non ramifié, substitué une ou plusieurs fois ou non substitué, saturé ou non saturé; cycloalkyle en C₃ à C₇, saturé ou non saturé, substitué une ou plusieurs fois ou non substitué, ou un hétérocycle correspondant dans lequel un atome C du noyau est remplacé par N, S ou O, dans chaque cas substitué une ou plusieurs fois ou non substitué ;
ou bien
R² et R³ forment ensemble un noyau et représentent un groupe CH₂CH₂OCH₂CH₂, CH₂CH₂NR⁶CH₂CH₂ ou (CH₂)₃₋₆ ;
X dans la formule I, conformément à la formule partielle Ia de la formule I 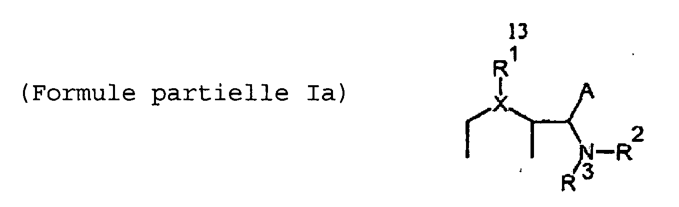 a la définition suivante 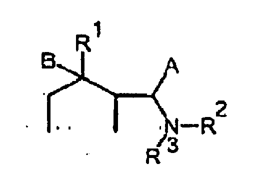 c'est-à-dire que X représente 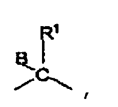 A représente l'hydrogène,
R¹ est un noyau phényle non substitué ou substitué une ou deux fois,
B est choisi entre
H, F, Cl, OH ou OR⁷, R⁷ étant choisi entre un radical alkyle en C₁ à C₁₀, ramifié ou non ramifié, substitué une ou plusieurs fois ou non substitué, saturé ou non saturé; un radical cycloalkyle en C₃ à C₇, saturé ou non saturé, substitué une ou plusieurs fois ou non substitué, ou un hétérocycle correspondant dans lequel un atome de carbone du noyau est remplacé par N, S ou O, chacun substitué une ou plusieurs fois ou non substitué,
et en même temps le groupement 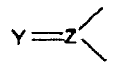 représente 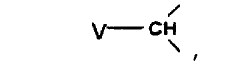 V étant choisi entre des groupes OR⁴ dans lesquels R⁴ est choisi entre COR⁵, R⁵ étant choisi entre des radicaux alkyle en C₁ à C₆, non substitué ou substitué une ou plusieurs fois.

2. Dérivés de cyclohexylméthylamine C-substitués suivant la revendication 1, **caractérisés en ce que**
R¹ correspond à un composé répondant à la formule générale II 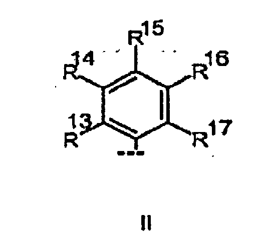 dans laquelle
R¹³, R¹⁵ et R¹⁷ sont choisis, indépendamment les uns des autres, entre H, F, Cl, Br, I, OR¹⁸, SR¹⁸, SO₂R¹⁸, SO₂OR¹⁸, CN, COOR¹⁸, NR¹⁹R²⁰ ; alkyle en C₁ à C₁₀, cycloalkyle en C₃ à C₁₀, chacun non substitué ou substitué une ou plusieurs fois, identiques ou différentes, avec F, Cl, Br, I, NH₂, SH ou OH, ou un radical silyle, saturé ou non saturé, ramifié
ou non ramifié,
R¹⁴ et R¹⁶ sont choisis indépendamment l'un de l'autre entre H, F, Cl, Br, I, SO₂R¹⁸, SO₂OR¹⁸, CN, COOR¹⁸, NR¹⁹R²⁰ ; alkyle en C₁ à C₁₀, cycloalkyle en C₃ à C₁₀, chacun non substitué ou substitué une ou plusieurs fois, identiques ou différentes, avec F, Cl, Br, NH₂, I, SH ou OH, ou un radical silyle, saturé ou non saturé, ramifié ou non ramifié,
ou bien les restes R¹³ et R¹⁴ ou R¹⁴ et R¹⁵ forment ensemble dans chaque cas le groupe OCH₂O, OCH₂CH₂O, CH=CHO, CH=C(CH₃)O ou CH=CHNH et R¹⁵-R¹⁷ ou R¹³, R¹⁶ et R¹⁷ ont la définition indiquée ci-dessus,
R¹⁸ étant choisi entre H ; un reste alkyle en C₁ à C₁₀ ou cycloalkyle en C₃ à C₁₀, chacun saturé ou non saturé, ramifié ou non ramifié ;
R¹⁹ et R¹⁰ étant choisis indépendamment l'un de l'autre entre H ; un radical alkyle en C₁ à C₁₀ ou cycloalkyle en C₃ à C₁₀, chacun saturé ou non saturé, ramifié ou non ramifié,
ou bien R¹⁹ et R²⁰ forment ensemble un groupe CH₂CH₂OCH₂CH₂, CH₂CH₂NR²¹CH₂CH₂ ou (CH₂)₃₋₆,
R²¹ étant choisi entre H, un reste alkyle en C₁ à C₁₀, saturé ou non saturé, ramifié ou non ramifié, dans chaque cas non substitué ou substitué une ou plusieurs fois, identiques ou différentes, avec F, Cl, Br, I, NH₂, SH ou OH,
et 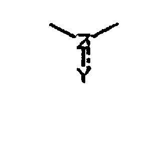 est choisi entre 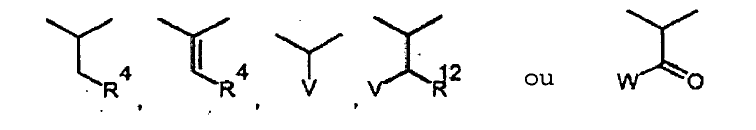 R⁴ étant choisi entre COR^{5a}, SO₂R⁵; un reste alkyle en C₁ à C₁₀ ou cycloalkyle en C₃ à C₁₀, chacun saturé ou non saturé, ramifié ou non ramifié, substitué une ou plusieurs fois ou non substitué ; un reste naphtyle, phényle, furyle ou thiophényle, chacun substitué une ou plusieurs fois ou non substitué ; ou un reste naphtyle, phényle, cycloalkyle en C₃ à C₁₀, furyle ou thiophényle, lié par l'intermédiaire d'un groupe alkylène en C₁ à C₃, chacun substitué une ou plusieurs fois ou non substitué,
R⁵ étant choisi entre un reste alkyle en C₁ à C₁₀ ou un reste cycloalkyle en C₃ à C₁₀, chacun saturé ou non saturé, ramifié ou non ramifié, substitué une ou plusieurs fois ou non substitué ; un reste naphtyle, phényle, furyle ou thiophényle, chacun substitué une ou plusieurs fois ou non substitué ; ou un reste phényle, naphtyle, cycloalkyle en C₃ à C₁₀, furyle ou thiophényle, lié par l'intermédiaire d'un groupe alkylène en C₁ à C₃, chacun substitué une ou plusieurs fois ou non substitué,
R^{5a} étant choisi entre un reste cycloalkyle en C₃ à C₁₀, saturé ou non saturé, ramifié ou non ramifié, substitué une ou plusieurs fois ou non substitué ; un reste naphtyle, phényle, furyle ou thiophényle, chacun substitué une ou plusieurs fois ou non substitué ; ou un reste naphtyle, phényle, cycloalkyle en C₃ à C₁₀, furyle ou thiophényle lié par l'intermédiaire d'un groupe alkylène en C₁ à C₃, chacun substitué une ou plusieurs fois ou non substitué,
V est choisi entre OR⁴ ou NR⁴R¹¹,
W est choisi entre R¹¹, OR¹² ou NR¹¹R¹²,
R¹¹ et R¹² étant choisis indépendamment l'un de l'autre entre H, un reste alkyle en C₇ à C₁₀ ou cycloalkyle en C₃ à C₁₀, chacun saturé ou non saturé, ramifié ou non ramifié, substitué une ou plusieurs fois ou non substitué; un reste naphtyle, phényle, furyle ou thiophényle, chacun substitué une ou plusieurs fois ou non substitué; ou un reste naphtyle, phényle, cycloalkyle en C₃ à C₁₀, furyle ou thiophényle lié par un groupe alkylène en C₁ à C₃, chacun substitué une ou plusieurs fois ou non substitué,
le terme substitué à propos d'alkyle et de cycloalkyle de R⁴, R⁵, R^{5a}, R¹¹ et R¹² signifiant la substitution avec F, Cl, Br, I, NH₂, SH ou OH ; et
le terme substitué à propose de naphtyle, phényle, furyle et thiophényle de R⁴, R⁵, R^{5a}, R¹¹ et R¹² signifiant la substitution du reste naphtyle, phényle, furyle ou thiophényle avec R²³ , OR²³, un halogène, un groupe CF₃, un groupe CN, un groupe NO₂, un groupe NR²⁴R²⁵, un groupe alkyle en C₁ à C₆ (saturé), un groupe alkoxy en C₁ à C₆, un groupe cycloalkoxy en C₃ à C₈, un groupe cycloalkyle en C₃ à C₈ ou un groupe alkylène en C₂ à C₆,
le reste R²³ représentant H, un reste alkyle en C₁ à C₁₀, un reste naphtyle, phényle, furyle ou thiophényle, ou un reste naphtyle, phényle, furyle ou thiophényle lié par l'intermédiaire d'un groupe alkylène en C₁ à C₃, ces restes naphtyle, phényle, furyle ou thiophényle ne pouvant pas eux-mêmes être substitués avec des restes aryle ou hétéroaryle,
les restes R²⁴ et R²⁵, identiques ou différents, représentent H, un reste alkyle en C₁ à C₁₀, un reste naphtyle, phényle, furyle ou thiophényle, ou un reste naphtyle, phényle, furyle ou thiophényle lié par l'intermédiaire d'un groupe alkylène en C₁ à C₃, ces restes naphtyle, phényle, furyle et thiophényle ne pouvant pas eux-mêmes être substitués avec des restes aryle
ou hétéroaryle,
ou bien les restes R²⁴ et R²⁵ forment ensemble un groupe CH₂CH₂OCH₂CH₂, CH₂CH₂NR²⁶CH₂CH₂ ou (CH₂)₃₋₆ et
le reste R²⁶ représente H, un reste alkyle en C₁ à C₁₀, naphtyle, phényle, furyle ou thiophényle ou un reste naphtyle, phényle, furyle ou thiophényle lié par l'intermédiaire d'un groupe alkylène en C₁ à C₃, ces restes naphtyle, phényle, furyle, ou thiophényle ne pouvant pas eux-mêmes être substitués avec des restes aryle ou hétéroaryle.

3. Dérivés de cyclohexylméthylamine C-substitués suivant la revendication 1 ou 2, **caractérisés en ce que** A représente l'hydrogène.

4. Dérivés de cyclohexylméthylamine C-substitués suivant l'une des revendications 1 à 3, **caractérisés en ce que**
R² et R³ sont choisis entre des restes alkyle en C₁ à C₆, saturés ou non saturés, ramifiés ou non ramifiés, ou bien les restes R² et R³ forment ensemble un groupe CH₂CH₂NR⁶CH₂CH₂ ou (CH₂)₃₋₆, R⁶ étant choisi entre H ou un radical alkyle en C₁ à C₆, saturé, ramifié ou non ramifié et non substitué.

5. Dérivés de cyclohexylméthylamine C-substitués suivant l'une des revendications 1 à 4, **caractérisés en ce que**
R¹ est choisi entre : un reste alkyle en C₁ à C₆, saturé ou non saturé, ramifié ou non ramifié; un reste cycloalkyle en C₃ à C₆, naphtyle, phényle, furyle, thiophényle, un reste naphtyle, phényle, cycloalkyle en C₃ à C₆, lié par l'intermédiaire d'un groupe alkylène en C₁ à C₃ ou éthynyle, ou un reste thiophényle ou furyle lié par l'intermédiaire d'un groupe alkylène en C₁ à C₃ ou éthynyle, qui peuvent être non substitués ou substitués une ou plusieurs fois conformément à la revendication 1.

6. Dérivés de cyclohexylméthylamine C-substitués suivant l'une des revendications 1 à 5, **caractérisés en ce que** le groupe 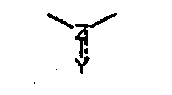 est choisi entre

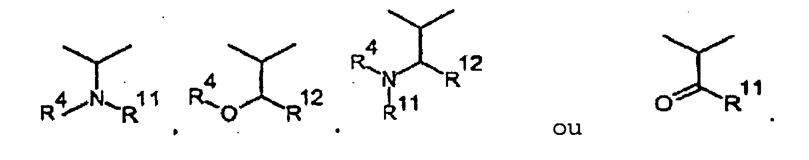

7. Dérivés de cyclohexylméthylamine C-substitués suivant la revendication 1, **caractérisés en ce que**
A est choisi entre H ou un reste phényle,
et/ou
R¹ est choisi entre un radical naphtyle non substitué ou substitué une ou plusieurs fois avec des restes choisis indépendamment les uns des autres entre
F, Cl, Br, I, OR¹⁸ ; alkyle en C₁ à C₄, ramifié ou non ramifié, non substitué ou substitué une ou plusieurs fois identiques ou différentes avec F, Cl, Br, I, NH₂, SH ou OH ;
R¹⁸ étant choisi entre H ; des restes alkyle en C₁ à C₄, ramifiés ou non ramifiés,
et/ou
R² et R³ sont choisis entre des restes alkyle en C₁ à C₆, saturés ou non saturés, ramifiés ou non ramifiés,
ou bien les restes R² et R³ forment ensemble un groupe CH₂CH₂NR⁶CH₂CH₂ ou (CH₂)₄₋₅, R⁶ étant choisi entre H ou un reste alkyle en C₁ à C₆, saturé, ramifié ou non ramifié et non substitué ;
et/ou
X dans la formule I, conformément à la formule partielle Ia de la formule I 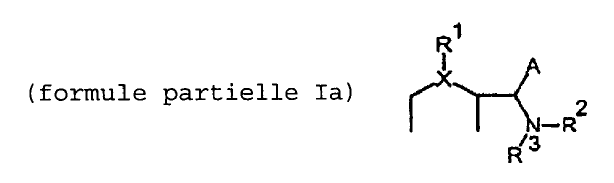 est choisi parmi les définitions suivantes 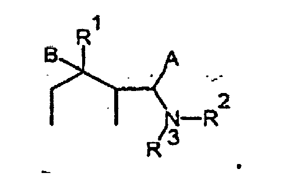 c'est-à-dire que X représente 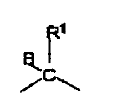 B étant choisi entre OH, OR⁷, H, F, Cl, R⁷ étant choisi entre des restes alkyle en C₁ à C₄ ramifiés ou non ramifiés, non substitués ou substitués une ou plusieurs fois, identiques ou différentes, avec F, Cl, Br, I, NH₂, SH ou OH ;
et/ou 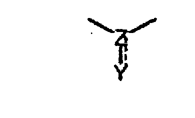 est choisi entre des groupes 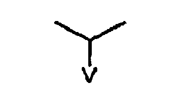 où V est choisi entre des radicaux OR⁴,
dans lesquels R⁴ est choisi entre COR⁵; un reste phényle ou un reste benzyle, substitués une ou plusieurs fois ou non substitués selon la revendication 1 ;
R⁵ étant choisi entre des restes alkyle en C₁ à C₁₀, saturés ou non saturés, ramifiés ou non ramifiés, substitués une ou plusieurs fois ou non substitués, identiques ou différentes, avec F, Cl, Br, I, NH₂, SH ou OH ; ou un reste phényle, qui peut être substitué une ou plusieurs fois ou non substitué selon la revendication 1.

8. Dérivés de cyclohexylméthylamine C-substitués suivant l'une des revendications 1 à 7, **caractérisés en ce qu'**ils sont choisis dans le groupe suivant :
• 4-benzyloxy-1-(4-bromophényl)-2-diméthylaminométhyl-cyclohexanol ;
• 4-benzyloxy-2-diméthylaminométhyl-1-(4-trifluorométhyl-phényl)-cyclohexanol ;
• 4-benzyloxy-1-(3,4-difluorophényl)-2-diméthylaminométhyl-cyclohexanol ;
• 4-benzyloxy-2-diméthylaminométhyl-1-(3-fluoro-4-méthyl-phényl)-cyclohexanol ;
• 4-benzyloxy-1-(3,4-dichlorophényl)-2-diméthylaminométhyl-cyclohexanol ;
• 4-benzyloxy-2-diméthylaminométhyl-1-naphtalène-2-yl-cyclohexanol ;
• 4-benzyloxy-2-diméthylaminométhyl-1-(3,4-diméthylphényl)-cyclohexanol ;
• 4-benzyloxy-2-diméthylaminométhyl-1-(4-fluoro-3-méthyl-phényl)-cyclohexanol ;
• 4-benzyloxy-2-diméthylaminométhyl-1-(5-fluoro-2-méthyl-phényl)-cyclohexanol ;
• 4-benzyloxy-1-(3-chloro-4-fluorophényl)-2-diméthylamino-méthyl-cyclohexanol ;
• 4-benzyloxy-1-(5-chloro-2-méthoxyphényl)-2-diméthylamino-méthyl-cyclohexanol ;
• 4-benzyloxy-1-(2-bromophényl)-2-diméthylaminométhyl-cyclohexanol ;
• 4-benzyloxy-2-diméthylaminométhyl-1-(2-méthoxyphényl)-cyclohexanol ;
• 4-benzyloxy-2-diméthylaminométhyl-1-(2-méthylsulfanyl-phényl)-cyclohexanol ;
• 4-benzyloxy-1-(4-chlorophényl)-2-diméthylaminométhyl-cyclohexanol ;
• 4-benzyloxy-1-(3-bromophényl)-2-diméthylaminométhyl-cyclohexanol ;
• 4-benzyloxy-1-(4-bromo-3-fluorophényl)-2-diméthylamino-méthyl-cyclohexanol ;
• 4-benzyloxy-2-diméthylaminométhyl-1-(4-méthoxyphényl)-cyclohexanol ;
• 4-benzyloxy-1-(2-chlorophényl)-2-diméthylaminométhyl-cyclohexanol ;
• 4-benzyloxy-2-diméthylaminométhyl-1-(4-trifluorométhyl-phényl)-cyclohexanol ;
• 4-benzyloxy-2-diméthylaminométhyl-1-(4-pentyl-phényl)-cyclohexanol ;
• 4-benzyloxy-2-diméthylaminométhyl-1-(2,5-diméthyl-phényl)-cyclohexanol ;
• 4-benzyloxy-2-diméthylaminométhyl-1-(4-isopropyl-phényl)-cyclohexanol ;
• 4-benzyloxy-2-diméthylaminométhyl-1-(2,3-diméthyl-phényl)-cyclohexanol ;
• 4-benzyloxy-2-diméthylaminométhyl-1-(3-fluorophényl)-cyclohexanol ;
• 4-benzyloxy-1-(3,5-bis-trifluorométhyl-phényl)-2-diméthylaminométhyl-cyclohexanol ;
• 4-benzyloxy-2-diméthylaminométhyl-1-(4-éthyl-phényl)-cyclohexanol ;
• 4-benzyloxy-1-(3,5-dichlorophényl)-2-diméthylaminométhyl-cyclohexanol ;
• 2-diméthylaminométhyl-4-(4-fluorobenzyloxy)-1-o-tolyl-cyclohexanol ;
• 2-diméthylaminométhyl-4-(4-fluorobenzyloxy)-1-p-tolyl-cyclohexanol ;
• 2-diméthylaminométhyl-1-(2,5-diméthyl-phényl)-4-(4-fluorobenzyloxy)-cyclohexanol ;
• 2-diméthylaminométhyl-1-(3,4-diméthyl-phényl)-4-(4-fluorobenzyloxy)-cyclohexanol ;
• 2-diméthylaminométhyl-1-(3,5-diméthyl-phényl)-4-(4-fluorobenzyloxy)-cyclohexanol ;
• 2-diméthylaminométhyl-4-(4-fluorobenzyloxy)-1-(4-isoproyl-phényl)-cyclohexanol ;
• 2-diméthylaminométhyl-4-(4-fluorobenzyloxy)-1-(3-fluoro-phényl)-cyclohexanol ;
• 2-diméthylaminométhyl-4-(4-fluorobenzyloxy)-1-(4-fluoro-phényl)-cyclohexanol ;
• 1-(3,4-difluorophényl)-2-diméthylaminométhyl-4-(4-fluoro-benzyloxy)-cyclohexanol ;
• 2-diméthylaminométhyl-4-(4-fluorobenzyloxy)-1-(3-fluoro-4-méthyl-phényl)-cyclohexanol ;
• 2-diméthylaminométhyl-4-(4-fluorobenzyloxy)-1-(4-fluoro-3-méthyl-phényl)-cyclohexanol ;
• 1-(2-chlorophényl)-2-diméthylaminométhyl-4-(4-fluoro-benzyloxy)-cyclohexanol ;
• 1-(3-chlorophényl)-2-diméthylaminométhyl-4-(4-fluoro-benzyloxy)-cyclohexanol ;
• 1-(4-chlorophényl)-2-diméthylaminométhyl-4-(4-fluoro-benzyloxy)-cyclohexanol ;
• 1-(3,4-dichlorophényl)-2-diméthylaminométhyl-4-(4-fluoro-benzyloxy)-cyclohexanol ;
• 1-(4-chloro-3-fluorophényl)-2-diméthylaminométhyl-4-(4-fluorobenzyloxy)-cyclohexanol ;
• 1-(3,5-dichlorophényl)-2-diméthylaminométhyl-4-(4-fluoro-benzyloxy)-cyclohexanol ;
• 1-(4-chloro-2-méthoxyphényl)-2-diméthylaminométhyl-4-(4-fluorobenzyloxy)-cyclohexanol ;
• 2-diméthylaminométhyl-4-(4-fluorobenzyloxy)-1-(4-trifluorométhyl-phényl)-cyclohexanol ;
• 1-(3,5-bis-trifluorophényl)-2-diméthylaminométhyl-4-(4-fluorobenzyloxy)-cyclohexanol ;
• 2-diméthylaminométhyl-4-(4-fluorobenzyloxy)-1-(2-méthoxy-phényl)-cyclohexanol ;
• 2-diméthylaminométhyl-4-(4-fluorobenzyloxy)-1-(2-méthyl-sulfanyl-phényl)-cyclohexanol ;
• 3,4-dichloro-N-[3-diméthylaminométhyl-4-hydroxy-4-(3-méthoxyphényl)-cyclohexyl]-benzamide ;
• [3-diméthylaminométhyl-4-hydroxy-4-(3-méthoxyphényl)-cyclohexyl]-amide d'acide naphtalène-2-carboxylique ;
• N-[3-diméthylaminométhyl-4-hydroxy-4-(3-méthoxyphényl)-cyclohexyl]-3-phényl-propionamide ;
• N-[3-diméthylaminométhyl-4-hydroxy-4-(3-méthoxyphényl)-cyclohexyl]-4-nitrobenzamide ;
• N-[3-diméthylaminométhyl-4-hydroxy-4-(3-méthoxyphényl)-cyclohexyl]-4-méthyl-3-nitrobenzamide ;
• N-[3-diméthylaminométhyl-4-hydroxy-4-(3-méthoxyphényl)-cyclohexyl]-3,4,5-triméthoxybenzamide ;
• 2-(4-chlorophénoxy)-N-[3-diméthylaminométhyl-4-hydroxy-4-(3-méthoxyphényl)-cyclohexyl]-acétamide ;
• N-[3-diméthylaminométhyl-4-hydroxy-4-(3-méthoxyphényl)-cyclohexyl]-3-nitrobenzamide ;
• [3-diméthylaminométhyl-4-hydroxy-4-(3-méthoxyphényl)-cyclohexyl]-amide d'acide furanne-2-carboxylique ;
• N-[3-diméthylaminométhyl-4-hydroxy-4-(3-méthoxyphényl)-cyclohexyl]-2-phénoxyacétamide ;
• N-[3-diméthylaminométhyl-4-hydroxy-4-(3-méthoxyphényl)-cyclohexyl]-4-trifluorométhyl-benzamide ;
• N-[3-diméthylaminométhyl-4-hydroxy-4-(3-méthoxyphényl)-cyclohexyl]-4-méthoxybenzamide ;
• 2-diméthylaminométhyl-1-(4-fluorophényl)-4-phénéthyl-cyclohexanol ;
• 1-(4-bromophényl)-2-diméthylaminométhyl-4-phénéthyl-cyclohexanol ;
• 2-diméthylaminométhyl-1-(4-éthyl-phényl)-4-phénéthyl-cyclohexanol ;
• 2-diméthylaminométhyl-1-(4-isopropyl-phényl)-4-phénéthyl-cyclohexanol ;
• 2-diméthylaminométhyl-1-(4-méthoxyphényl)-4-phénéthyl-cyclohexanol ;
• 2-diméthylaminométhyl-1-(2,4-diméthyl-phényl)-4-phénéthyl-cyclohexanol ;
• 2-diméthylaminométhyl-1-(2-méthylsulfanyl-phényl)-4-phénéthyl-cyclohexanol ;
• 2-diméthylaminométhyl-1-(5-fluoro-2-méthyl-phényl)-4-phénéthyl-cyclohexanol ;
• 2-diméthylaminométhyl-1-(2,5-diméthyl-phényl)-4-phénéthyl-cyclohexanol ;
• 2-diméthylaminométhyl-4-phénéthyl-1-p-tolyl-cyclohexanol ;
• 2-diméthylaminométhyl-1-(3-fluorophényl)-4-phénéthyl-cyclohexanol ;
• 1-(3-chlorophényl)-2-diméthylaminométhyl-4-phénéthyl-cyclohexanol ;
• 1-(4-chloro-3-fluorophényl)-2-diméthylaminométhyl-4-phénéthyl-cyclohexanol ;
• 2-diméthylaminométhyl-4-phénéthyl-1-(3-trifluorométhyl-phényl)-cyclohexanol ;
• 1-(3,5-dichlorophényl)-2-diméthylaminométhyl-4-phénéthyl-cyclohexanol ;
• 2-diméthylaminométhyl-1-(3,4-diméthyl-phényl)-4-phénéthyl-cyclohexanol ;
• 1-(3,4-dichlorophényl)-2-diméthylaminométhyl-4-phénéthyl-cyclohexanol ;
• 1-(4-chlorophényl)-2-diméthylaminométhyl-4-phénéthyl-cyclohexanol ;
• 2-diméthylaminométhyl-1-(3-fluoro-4-méthyl-phényl)-4-phénéthyl-cyclohexanol ;
• 2-diméthylaminométhyl-1-(3,5-diméthyl-phényl)-4-phénéthyl-cyclohexanol ;
• 1-(4-bromo-3-fluorophényl)-2-diméthylaminométhyl-4-phénéthyl-cyclohexanol ;
• 1-(3,4-difluorophényl)-2-diméthylaminométhyl-4-phénéthyl-cyclohexanol ;
• 2-diméthylaminométhyl-1-(2,3-diméthyl-phényl)-4-phénéthyl-cyclohexanol ;
• 2-diméthylaminométhyl-1-naphtalène-1-yl-4-phénéthyl-cyclohexanol ;
• 1-(3,5-bis-trifluorométhyl-phényl)-2-diméthylaminométhyl-4-phénéthyl-cyclohexanol ;
• 1-(3-bromophényl)-2-diméthylaminométhyl-4-phénéthyl-cyclohexanol ;
• 2-diméthylaminométhyl-1-(4-fluoro-3-méthyl-phényl)-4-phénéthyl-cyclohexanol ;
• 2-diméthylamino-4-phénéthyl-1-m-tolyl-cyclohexanol ;
• 4-benzyl-2-diméthylaminométhyl-1-m-tolyl-cyclohexanol ;
• 4-benzyl-1-(3,4-difluorophényl)-2-diméthylaminométhyl-cyclohexanol ;
• 4-benzyl-2-diméthylaminométhyl-1-p-tolyl-cyclohexanol ;
• 2-diméthylaminométhyl-4-(3-méthoxybenzyl)-1-p-tolyl-cyclohexanol ;
• 1-(4-chlorophényl)-2-diméthylaminométhyl-4-(3-méthoxy-benzyl)-cyclohexanol ;
• 1-(4-bromo-3-fluorophényl)-2-diméthylaminométhyl-4-(3-méthoxy-benzyl)-cyclohexanol ;
• 2-diméthylaminométhyl-4-(4-fluorobenzyl)-1-p-tolyl-cyclohexanol ;
• 1-(4-chlorophényl)-2-diméthylaminométhyl-4-(4-fluoro-benzyl)-cyclohexanol ;
• [2-(3-chloro-4-fluorophényl)-2-fluoro-5-phénéthyl-cyclohexylméthyl]-diméthylamine ;
• [2-fluoro-5-(4-fluorobenzyl)-2-p-tolyl-cyclohexylméthyl]-diméthylamine ;
• benzyl-2-(diméthylaminophényl-méthyl)-4-phényl-cyclohexanol ;
• 2-(diméthylaminophényl-méthyl)-4-phényl-1-vinyl-cyclohexanol ;
• 1-(4-tertiobutyl-phényl)-2-(diméthylaminophényl-méthyl)-4-phényl-cyclohexanol ;
• 2-(diméthylaminophényl-méthyl)-4-phényl-1-m-tolyl-cyclohexanol ;
• 2-(diméthylaminophényl-méthyl)-1-phénéthyl-4-phényl-cyclohexanol ;
• 2-(diméthylaminophényl-méthyl)-4-phényl-1-phényléthynyl-cyclohexanol ;
• 2-(diméthylaminophényl-méthyl)-1-(3-méthoxyphényl)-4-phényl-cyclohexanol ;
• 2-(diméthylaminophényl-méthyl)-4-phényl-1-(3-phényl-propyl)-cyclohexanol ;
• 2-(diméthylaminophényl-méthyl)-1-(4-méthoxyphényl)-4-phényl-cyclohexanol ;
• 2-(diméthylaminophényl-méthyl)-1-(2-méthoxyphényl)-4-phényl-cyclohexanol ;
• 4-benzyloxy-2-diméthylaminométhyl-1-phényl-cyclohexanol ;
• 1-benzyl-4-benzyloxy-2-diméthylaminométhyl-cyclohexanol ;
• 4-benzyloxy-2-diméthylaminométhyl-1-(4-fluoro-3-méthyl-phényl)-cyclohexanol ;
• 4-benzyloxy-2-diméthylaminométhyl-1-o-tolyl-cyclohexanol ;
• 4-benzyloxy-2-diméthylaminométhyl-1-vinyl-cyclohexanol ;
• 4-benzyloxy-1-cyclopentyl-2-diméthylaminométhyl-cyclohexanol ;
• 4-benzyloxy-2-diméthylaminométhyl-1-m-tolyl-cyclohexanol ;
• 4-benzyloxy-2-diméthylaminométhyl-bicyclohexyl-1-ol ;
• 4-benzyloxy-2-diméthylaminométhyl-1-(4-fluorophényl)-cyclohexanol ;
• 4-benzyloxy-2-diméthylaminométhyl-1-phényléthynyl-cyclohexanol ;
• 4-benzyloxy-2-diméthylaminométhyl-1-thiophène-2-yl-cyclohexanol ;
• 4-benzyloxy-2-diméthylaminométhyl-1-(3-méthoxyphényl)-cyclohexanol ;
• 4-benzyloxy-2-diméthylaminométhyl-1-(3-phényl-propyl)-cyclohexanol ;
• 4-benzyloxy-2-diméthylaminométhyl-1-p-tolyl-cyclohexanol ;
• 4-benzyloxy-2-diméthylaminométhyl-1-(4-méthoxyphényl)-cyclohexanol ;
• 4-benzyloxy-2-diméthylaminométhyl-1-(3-fluorophényl)-cyclohexanol ;
• 4-benzyloxy-1-(3-chlorophényl)-2-diméthylaminométhyl-cyclohexanol ;
• 4-benzyloxy-2-diméthylaminométhyl-1-(3-méthoxybenzyl)-cyclohexanol ;
• 4-benzyloxy-1-(4-chloro-3-méthyl-phényl)-2-diméthylamino-méthyl-cyclohexanol ;
• 4-benzyloxy-2-diméthylaminométhyl-1-(3-fluorobenzyl)-cyclohexanol ;
• 4-benzyloxy-2-diméthylaminométhyl-1-(2-méthylbenzyl)-cyclohexanol ;
• 4-benzyloxy-2-diméthylaminométhyl-1-(2,5-diméthyl-benzyl)-cyclohexanol ;
• 4-(4-chlorobenzyl)-2-diméthylaminométhyl-1-(3-méthoxy-phényl)-cyclohexanol ;
• 4-(4-chlorobenzyl)-2-diméthylaminométhyl-1-(3-phényl-propyl)-cyclohexanol ;
• 4-(4-chlorobenzyl)-1-(2,3-dichlorophényl)-2-diméthyl-aminométhyl-cyclohexanol ;
• 4-(4-chlorobenzyl)-1-cyclohexylméthyl-2-diméthylamino-méthyl-cyclohexanol ;
• 4-(4-chlorobenzyl)-2-diméthylaminométhyl-1-(5-fluoro-2-méthoxyphényl)-cyclohexanol ;
• 4-(4-chlorobenzyl)-2-diméthylaminométhyl-1-(3-fluoro-phényl)-cyclohexanol ;
• 4-(4-chlorobenzyl)-1-(3-chlorophényl)-2-diméthylamino-méthyl-cyclohexanol ;
• 4-(4-chlorobenzyl)-1-(3,5-dichlorophényl)-2-diméthyl-aminométhyl-cyclohexanol ;
• 4-(4-chlorobenzyl)-1-(2-chlorobenzyl)-2-diméthylamino-méthyl-cyclohexanol ;
• 4-(4-chlorobenzyl)-2-diméthylaminométhyl-1-(4-fluoro-benzyl)-cyclohexanol ;
• 4-(4-chlorobenzyl)-2-diméthylaminométhyl-1-(3-fluoro-benzyl)-cyclohexanol ;
• 4-(4-chlorobenzyl)-2-diméthylaminométhyl-1-(2-méthoxy-phényl)-cyclohexanol ;
• 4-(4-chlorobenzyl)-2-diméthylaminométhyl-1-(2-méthyl-benzyl)-cyclohexanol ;
• 4-(4-chlorobenzyl)-2-diméthylaminométhyl-1-(3-méthyl-benzyl)-cyclohexanol ;
• 1,4bis-(4-chlorobenzyl)-2-diméthylaminométhyl-cyclohexanol ;
• 4-(4-chlorobenzyl)-1-(2-chloro-6-fluorobenzyl)-2-diméthylaminométhyl-cyclohexanol ;
• 4-(4-chlorobenzyl)-2-diméthylaminométhyl-1-(2,5-diméthyl-benzyl)-cyclohexanol ;
• 4-(4-chlorobenzyl)-1-(3-chlorobenzyl)-2-diméthylamino-méthyl-cyclohexanol ;
• 4-(4-chlorobenzyl)-1-(2,4-dichlorobenzyl)-2-diméthyl-aminométhyl-cyclohexanol ;
• 4-(4-chlorobenzyl)-2-diméthylaminométhyl-1-phényl-cyclohexanol ;
• 4-(4-chlorobenzyl)-1-(4-chlorophényl)-2-diméthylamino-méthyl-cyclohexanol ;
• 4-(4-chlorobenzyl)-2-diméthylaminométhyl-1(4-fluoro-3-méthyl-phényl) cyclohexanol ;
• 4-(4-chlorobenzyl)-2-diméthylaminométhyl-1-o-tolyl-cyclohexanol ;
• 4-(4-chlorobenzyl)-2-diméthylaminométhyl-1-vinyl-cyclohexanol ;
• 4-(4-chlorobenzyl)-1-cyclopentyl-2-diméthylaminométhyl-cyclohexanol ;
• 4-(4-chlorobenzyl)-2-diméthylaminométhyl-1-m-tolyl-cyclohexanol ;
• 4-(4-chlorobenzyl)-2-diméthylaminométhyl-bicyclohexyl-1-ol ;
• 4-(4-chlorobenzyl)-2-diméthylaminométhyl-1-(4-fluoro-phényl)-cyclohexanol ;
• 4-(4-chlorobenzyl)-2-diméthylaminométhyl-1-phénéthyl-cyclohexanol ;
• 4-(4-chlorobenzyl)-2-diméthylaminométhyl-1-phényléthynyl-cyclohexanol ;
• 4-(4-chlorobenzyl)-2-diméthylaminométhyl-1-thiophène-2-yl-cyclohexanol ;
• 4-(4-chlorobenzyl)-2-diméthylaminométhyl-1-p-tolyl-cyclohexanol ;
• 4-(4-chlorobenzyl)-2-diméthylaminométhyl-1-(4-méthoxy-phényl)-cyclohexanol ;
• 4-(4-chlorobenzyl)-2-diméthylaminométhyl-1-triméthyl-silanyléthynyl-cyclohexanol ;
• 4-(4-chlorobenzyl)-1-(4-chloro-3-trifluorométhyl-phényl)-2-diméthylaminométhyl-cyclohexanol ;
• 4-(4-chlorobenzyl)-1-(3-chloro-4-fluorophényl)-2-diméthylaminométhyl-cyclohexanol ;
• 4-(4-chlorobenzyl)-2-diméthylaminométhyl-1-(3-trifluorométhyl-phényl)-cyclohexanol ;
• 2-diméthylaminométhyl-4-(4-fluorobenzyl)-1-phényl-cyclohexanol ;
• 1-(4-chlorophényl)-2-diméthylaminométhyl-4-(4-fluoro-benzyl)-cyclohexanol ;
• 1-benzyl-2-diméthylaminométhyl-4-(4-fluorobenzyl)-cyclohexanol ;
• 2-diméthylaminométhyl-4-(4-fluorobenzyl)-1-(4-fluoro-3-méthyl-phényl)-cyclohexanol ;
• 2-diméthylaminométhyl-4-(4-fluorobenzyl)-1-o-tolyl-cyclohexanol ;
• 2-diméthylaminométhyl-4-(4-fluorobenzyl)-1-vinyl-cyclohexanol ;
• 1-(4-tertiobutyl-phényl)-2-diméthylaminométhyl-4-(4-fluorobenzyl)-cyclohexanol ;
• 1-cyclopentyl-2-diméthylaminométhyl-4-(4-fluorobenzyl) cyclohexanol ;
• 2-diméthylaminométhyl-4-(4-fluorobenzyl)-1-m-tolyl-cyclohexanol ;
• 2-diméthylaminométhyl-4-(4-fluorobenzyl)-bicyclohexyl-1-ol ;
• 2-diméthylaminométhyl-4-(4-fluorobenzyl)-1-(4-fluoro-phényl)-cyclohexanol ;
• 2-diméthylaminométhyl-4-(4-fluorobenzyl)-1-phénéthyl-cyclohexanol ;
• 2-diméthylaminométhyl-4-(4-fluorobenzyl)-1-phényléthynyl-cyclohexanol ;
• 2-diméthylaminométhyl-4-(4-fluorobenzyl)-1-thiophène-2-yl-cyclohexanol ;
• 2-diméthylaminométhyl-4-(4-fluorobenzyl)-1-(3-méthoxy-phényl)-cyclohexanol ;
• 2-diméthylaminométhyl-4-(4-fluorobenzyl)-1-(3-phényl-propyl)-cyclohexanol ;
• 1-(2,3-dichlorophényl)-2-diméthylaminométhyl-4-(4-fluoro-benzyl)-cyclohexanol ;
• 2-diméthylaminométhyl-4-(4-fluorobenzyl)-1-p-tolyl-cyclohexanol ;
• 2-diméthylaminométhyl-4-(4-fluorobenzyl)-1-(4-méthoxy-phényl)-cyclohexanol ;
• 1-cyclohexylméthyl-2-diméthylaminométhyl-4-(4-fluoro-benzyl)-cyclohexanol ;
• 2-diméthylaminométhyl-4-(4-fluorobenzyl)-1-(5-fluoro-2-méthoxyphényl)-cyclohexanol ;
• 2-diméthylaminométhyl-4-(4-fluorobenzyl)-1-(3-fluoro-phényl)-cyclohexanol ;
• 1-(3-chlorophényl)-2-diméthylaminométhyl-4-(4-fluoro-benzyl)-cyclohexanol ;
• 1-(3,5-dichlorophényl)-2-diméthylaminométhyl-4-(4-fluoro-benzyl)-cyclohexanol ;
• 1-(2-chlorobenzyl)-2-diméthylaminométhyl-4-(4-fluoro-benzyl)-cyclohexanol ;
• 2-diméthylaminométhyl-1,4-bis-(4-fluorobenzyl)-cyclohexanol ;
• 2-diméthylaminométhyl-4-(4-fluorobenzyl)-1-(3-méthoxy-benzyl)-cyclohexanol ;
• 1-(4-chloro-3-trifluorométhyl-phényl)-2-diméthylamino-méthyl-4-(4-fluorobenzyl)-cyclohexanol ;
• 2-diméthylaminométhyl-4-(4-fluorobenzyl)-1-(3-fluoro-benzyl)-cyclohexanol ;
• 2-diméthylaminométhyl-4-(4-fluorobenzyl)-1-(2-méthoxy-benzyl)-cyclohexanol ;
• 2-diméthylaminométhyl-4-(4-fluorobenzyl)-1-(2-méthyl-benzyl)-cyclohexanol ;
• 1-(3-chloro-4-fluorophényl)-2-diméthylaminométhyl-4-(4-fluorobenzyl)-cyclohexanol ;
• 2-diméthylaminométhyl-4-(4-fluorobenzyl)-1-(3-trifluorométhyl-phényl)-cyclohexanol ;
• 2-diméthylaminométhyl-4-(4-fluorobenzyl)-1-(3-méthyl-benzyl)-cyclohexanol ;
• 1-(4-chlorobenzyl)-2-diméthylaminométhyl-4-(4-fluoro-benzyl)-cyclohexanol ;
• 1-(2-chloro-6-fluorobenzyl)-2-diméthylminométhyl-4-(4-fluorobenzyl)-cyclohexanol ;
• 2-diméthylaminométhyl-4-(2,5-diméthyl-benzyl)-4-(4-fluorobenzyl)-cyclohexanol ;
• 1-(3-chlorobenzyl)-2-diméthylaminométhyl-4-(4-fluoro-benzyl)-cyclohexanol ;
• 1-(2,4-dichlorobenzyl)-2-diméthylaminométhyl-4-(4-fluoro-benzyl)-cyclohexanol ;
• 2-diméthylaminométhyl-4-(3-méthoxybenzyl)-1-phényl-cyclohexanol ;
• 1-benzyl-2-diméthylaminométhyl-4-(3-méthoxybenzyl)-cyclohexanol ;
• 2-diméthylaminométhyl-4-(4-fluoro-3-méthyl-phényl)-4-(3-méthoxybenzyl)-cyclohexanol ;
• 2-diméthylaminométhyl-4-(3-méthoxybenzyl)-1-o-tolyl-cyclohexanol ;
• 2-diméthylaminométhyl-4-(3-méthoxybenzyl)-1-vinyl-cyclohexanol ;
• 1-(4-tertiobutyl-phényl)-2-diméthylaminométhyl-4-(3-méthoxybenzyl)-cyclohexanol ;
• 1-cyclopentyl-2-diméthylaminométhyl-4-(3-méthoxybenzyl)-cyclohexanol ;
• 2-diméthylaminométhyl-4-(3-méthoxybenzyl)-1-m-tolyl-cyclohexanol ;
• 2-diméthylaminométhyl-4-(3-méthoxybenzyl)-bicyclohexyl-1-ol ;
• 2-diméthylaminométhyl-4-(3-méthoxybenzyl)-1-phénéthyl-cyclohexanol ;
• 2-diméthylaminométhyl-4-(3-méthoxybenzyl)-1-phényl-éthynyl-cyclohexanol ;
• 2-diméthylaminométhyl-4-(3-méthoxybenzyl)-1-thiophène-2-yl-cyclohexanol ;
• 2-diméthylaminométhyl-4-(3-méthoxybenzyl)-1-(3-méthoxy-phényl)-cyclohexanol ;
• 2-diméthylaminométhyl-4-(3-méthoxybenzyl)-1-(3-phényl-propyl)-cyclohexanol ;
• 1-(2,3-dichlorophényl)-2-diméthylaminométhyl-4-(3-méthoxybenzyl)-cyclohexanol ;
• 2-diméthylaminométhyl-4-(3-méthoxybenzyl)-1-p-tolyl-cyclohexanol ;
• 1-cyclohexylméthyl-2-diméthylaminométhyl-4-(3-méthoxy-benzyl)-cyclohexanol ;
• 1-diméthylaminométhyl-1-(5-fluoro-2-méthoxyphényl)-4-(3-méthoxybenzyl)-cyclohexanol ;
• 1-(3-chlorophényl)-2-diméthylaminométhyl-4-(3-méthoxy-benzyl)-cyclohexanol ;
• 1-(3,5-dichlorophényl)-2-diméthylaminométhyl-4-(3-méthoxy-benzyl)-cyclohexanol ;
• 1-(4-chloro-3-trifluorométhyl-phényl)-2-diméthylamino-méthyl-4-(3-méthoxybenzyl)-cyclohexanol ;
• 2-diméthylamino-1-(3-fluorobenzyl)-4-(3-méthoxybenzyl)-cyclohexanol ;
• 2-diméthylaminométhyl-4-(3-méthoxybenzyl)-1-(2-méthoxy-phényl)-cyclohexanol ;
• 2-diméthylaminométhyl-4-(3-méthoxybenzyl)-1-(3-trifluorométhyl-phényl)-cyclohexanol ;
• 1-(2-chloro-6-fluorobenzyl)-2-diméthylaminométhyl-4-(3-méthoxybenzyl)-cyclohexanol ;
• 2-diméthylaminométhyl-1-(2,5-diméthyl-benzyl)-4-(3-méthoxybenzyl)-cyclohexanol ;
• 1-(3-chlorobenzyl)-2-diméthylaminométhyl-4-(3-méthoxy-benzyl)-cyclohexanol ;
• 2-diméthylaminométhyl-4-(4-fluorobenzyloxy)-1-phényl-cyclohexanol ;
• 1-benzyl-2-diméthylaminométhyl-4-(4-fluorobenzyloxy)-cyclohexanol ;
• 2-diméthylaminométhyl-4-(4-fluorobenzyloxy)-1-(4-fluoro-3-méthyl-phényl)-cyclohexanol ;
• 2-diméthylaminométhyl-4-(4-fluorobenzyloxy)-1-vinyl-cyclohexanol ;
• 1-(4-tertiobutyl-phényl)-2-diméthylaminométhyl-4-(4-fluorobenzyloxy)-cyclohexanol ;
• 1-cyclopentyl-2-diméthylaminométhyl-4-(4-fluorobenzyl-oxy)-cyclohexanol ;
• 2-diméthylaminométhyl-4-(4-fluorobenzyloxy)-1-m-tolyl-cyclohexanol ;
• 2-diméthylaminométhyl-4-(4-fluorobenzyloxy)-bicyclohexyl-1-ol ;
• 2-diméthylaminométhyl-4-(4-fluorobenzyloxy)-1-phénéthyl-cyclohexanol ;
• 2-diméthylaminométhyl-4-(4-fluorobenzyloxy)-1-phényl-éthynyl-cyclohexanol ;
• 2-diméthylaminométhyl-4-(4-fluorobenzyloxy)-1-thiophène-2-yl-cyclohexanol ;
• 2-diméthylaminométhyl-4-(4-fluorobenzyloxy)-1-(3-méthoxy-phényl)-cyclohexanol ;
• 2-diméthylaminométhyl-4-(4-fluorobenzyloxy)-1-p-tolyl-cyclohexanol ;
• 1-cyclohexylméthyl-2-diméthylaminométhyl-4-(4-fluoro-benzyloxy)-cyclohexanol ;
• 2-diméthylaminométhyl-1-(3-fluorobenzyl)-4-(4-fluoro-benzyloxy)-cyclohexanol ;
• 2-diméthylaminométhyl-4-(4-fluorobenzyloxy)-1-(2-méthoxyphényl)-cyclohexanol ;
• 2-diméthylaminométhyl-4-(4-fluorobenzyloxy)-1-(3-méthyl-benzyl)-cyclohexanol ;
• 2-diméthylaminométhyl-1-(2,5-diméthyl-benzyl)-4-(4-fluorobenzyloxy)-cyclohexanol ;
• 1-(3-chlorobenzyl)-2-diméthylaminométhyl-4-(4-fluoro-benzyloxy)-cyclohexanol ;
• [5-benzyloxy-2-(3-méthoxyphényl)-cyclohexylméthyl]-diméthylamine ;
• [5-(3-chlorobenzyloxy)-2-(3-méthoxyphényl)-cyclohexyl-méthyl]-diméthylamine ;
• [2-(3-méthoxyphényl)-5-(naphtalène-2-ylméthoxy)-cyclohexyl-méthyl]-diméthylamine ;
• [5-(3-méthoxybenzyloxy)-2-(3-méthoxyphényl)-cyclohexyl-méthyl]-diméthylamine ;
• [5-(4-chlorobenzyloxy)-2-(3-méthoxyphényl)-cyclohexyl-méthyl]-diméthylamine ;
• [5-(4-méthoxybenzyloxy)-2-(3-méthoxyphényl)-cyclohexyl-méthyl]-diméthylamine ;
• 2-diméthylaminométhyl-1-(3-méthoxyphényl)-4-(naphtalène-2-yl-méthoxy)-cyclohexanol ;
• ester de 3-diméthylaminométhyl-4-hydroxy-4-(3-méthoxy-phényl)-cyclohexyle d'acide butyrique ;
• 2-diméthylaminométhyl-1-(3-méthoxyphényl)-4-(naphtalène-2-yl-méthoxy)-cyclohexanol ;
• ester de 3-diméthylaminométhyl-4-hydroxy-4-(3-méthoxy-phényl)-cyclohexyle d'acide 4-méthoxybenzoïque ;
• ester de 3-diméthylaminométhyl-4-hydroxy-4-(3-méthoxy-phényl)-cyclohexyle d'acide 4-méthoxybenzoïque ;
• ester de 3-diméthylaminométhyl-4-hydroxy-4-(3-méthoxy-phényl)-cyclohexyle d'acide 2,2-diméthyl-propionique ;
• ester de 3-diméthylaminométhyl-4-hydroxy-4-(3-méthoxy-phényl)-cyclohexyle d'acide butyrique ;
• ester de 3-diméthylaminométhyl-4-hydroxy-4-(3-méthoxy-phényl)-cyclohexyle d'acide 4-méthoxybenzoïque ;
• ester de 3-diméthylaminométhyl-4-hydroxy-4-(3-méthoxy-phényl)-cyclohexyle d'acide 3-méthoxybenzoïque ;
• ester de 3-diméthylaminométhyl-4-hydroxy-4-(3-méthoxy-phényl)-cyclohexyle d'acide 2,2-diméthyl-propionique ;
• ester de 3-diméthylaminométhyl-4-hydroxy-4-(3-méthoxy-phényl)-cyclohexyle d'acide 3,4-diméthoxybenzoïque ;
• ester de 3-diméthylaminométhyl-4-hydroxy-4-(3-méthoxy-phényl)-cyclohexyle d'acide 3,4-diméthoxybenzoïque ;
• ester de 3-diméthylaminométhyl-4-hydroxy-4-(3-méthoxy-phényl)-cyclohexyle d'acide naphtalène-2-carboxylique ;
• ester de 3-diméthylaminométhyl-4-hydroxy-4-(3-méthoxy-phényl)-cyclohexyle d'acide 4-méthyl-benzoïque ;
• ester de 3-diméthylaminométhyl-4-hydroxy-4-(3-méthoxy-phényl)-cyclohexyle d'acide 3,4-dichlorobenzoïque ;
• ester de 3-diméthylaminométhyl-4-hydroxy-4-(3-méthoxy-phényl)-cyclohexyle d'acide 4-chlorobenzoïque ;
• ester de 3-diméthylaminométhyl-4-hydroxy-4-(3-méthoxy-phényl)-cyclohexyle d'acide 3-chlorobenzoïque ;
• ester de 3-diméthylaminométhyl-4-hydroxy-4-(3-méthoxy-phényl)-cyclohexyle d'acide 2-chlorobenzoïque ;
• ester de 3-diméthylaminométhyl-4-hydroxy-4-(3-méthoxy-phényl)-cyclohexyle d'acide 4-trifluorométhyl-benzoïque ;
• ester de 3-diméthylaminométhyl-4-hydroxy-4-(3-méthoxy-phényl)-cyclohexyle d'acide 3,5-difluorobenzoïque ;
• ester de 3-diméthylaminométhyl-4-hydroxy-4-(3-méthoxy-phényl)-cyclohexyle d'acide 4-fluorobenzoïque ;
• ester de 3-diméthylaminométhyl-4-hydroxy-4-(3-méthoxy-phényl)-cyclohexyle d'acide 3-fluorobenzoïque ;
• ester de 3-diméthylaminométhyl-4-hydroxy-4-(3-méthoxy-phényl)-cyclohexyle d'acide 2-fluorobenzoïque ;
• ester de 3-diméthylaminométhyl-4-hydroxy-4-(3-méthoxy-phényl)-cyclohexyle d'acide cyclopentanecarboxylique ;
• ester de 3-diméthylaminométhyl-4-hydroxy-4-(3-méthoxy-phényl)-cyclohexyle d'acide (4-méthoxyphényl)-acétique ;
• ester de 3-diméthylaminométhyl-4-hydroxy-4-(3-méthoxy-phényl)-cyclohexyle d'acide (3-méthoxyphényl)-acétique ;
• ester de 3-diméthylaminométhyl-4-hydroxy-4-(3-méthoxy-phényl)-cyclohexyle d'acide phénylacétique ;
• ester de 3-diméthylaminométhyl-4-hydroxy-4-(3-méthoxy-phényl)-cyclohexyle d'acide 3,4,5-triméthoxybenzoïque ;
• ester de 3-diméthylaminométhyl-4-hydroxy-4-(3-méthoxy-phényl)-cyclohexyle d'acide 3,5-diméthoxybenzoïque ;
• ester de 3-diméthylaminométhyl-4-hydroxy-4-(3-méthoxy-phényl)-cyclohexyle d'acide naphtalène-1-carboxylique ;
• ester de 3-diméthylaminométhyl-4-hydroxy-4-(3-méthoxy-phényl)-cyclohexyle d'acide 2-méthoxybenzoïque ;
• ester de 3-diméthylaminométhyl-4-hydroxy-4-(3-méthoxy-phényl)-cyclohexyle d'acide naphtalène-2-carboxylique ;
• ester de 3-diméthylaminométhyl-4-hydroxy-4-(3-méthoxy-phényl)-cyclohexyle d'acide 2-hydroxybenzoïque ;
• ester de 3-diméthylaminométhyl-4-hydroxy-4-(3-méthoxy-phényl)-cyclohexyle d'acide 4-méthyl-benzoïque ;
• ester de 3-diméthylaminométhyl-4-hydroxy-4-(3-méthoxy-phényl)-cyclohexyle d'acide 3,4-dichlorobenzoïque ;
• ester de 3-diméthylaminométhyl-4-hydroxy-4-(3-méthoxy-phényl)-cyclohexyle d'acide 4-chlorobenzoïque ;
• ester de 3-diméthylaminométhyl-4-hydroxy-4-(3-méthoxy-phényl)-cyclohexyle d'acide 3-chlorobenzoïque ;
• ester de 3-diméthylaminométhyl-4-hydroxy-4-(3-méthoxy-phényl)-cyclohexyle d'acide 2-chlorobenzoïque ;
• ester de 3-diméthylaminométhyl-4-hydroxy-4-(3-méthoxy-phényl)-cyclohexyle d'acide 4-trifluorométhyl-benzoïque ;
• ester de 3-diméthylaminométhyl-4-hydroxy-4-(3-méthoxy-phényl)-cyclohexyle d'acide 3,5-difluorobenzoïque ;
• ester de 3-diméthylaminométhyl-4-hydroxy-4-(3-méthoxy-phényl)-cyclohexyle d'acide 4-fluorobenzoïque ;
• ester de 3-diméthylaminométhyl-4-hydroxy-4-(3-méthoxy-phényl)-cyclohexyle d'acide 3-fluorobenzoïque ;
• ester de 3-diméthylaminométhyl-4-hydroxy-4-(3-méthoxy-phényl)-cyclohexyle d'acide 2-fluorobenzoïque ;
• ester de 3-diméthylaminométhyl-4-hydroxy-4-(3-méthoxy-phényl)-cyclohexyle d'acide cyclopentane-carboxylique ;
• ester de 3-diméthylaminométhyl-4-hydroxy-4-(3-méthoxy-phényl)-cyclohexyle d'acide (4-méthoxyphényl)-acétique ;
• ester de 3-diméthylaminométhyl-4-hydroxy-4-(3-méthoxy-phényl)-cyclohexyle d'acide (3-méthoxyphényl)-acétique ;
• ester de 3-diméthylaminométhyl-4-hydroxy-4-(3-méthoxy-phényl)-cyclohexyle d'acide phénylacétique ;
• ester de 3-diméthylaminométhyl-4-hydroxy-4-(3-méthoxy-phényl)-cyclohexyle d'acide 3,4,5-triméthoxybenzoïque ;
• ester de 3-diméthylaminométhyl-4-hydroxy-4-(3-méthoxy-phényl)-cyclohexyle d'acide 3,5-diméthoxybenzoïque ;
• ester de 3-diméthylaminométhyl-4-hydroxy-4-(3-méthoxy-phényl)-cyclohexyle d'acide naphtalène-1-carboxylique ;
• ester de 3-diméthylaminométhyl-4-hydroxy-4-(3-méthoxy-phényl)-cyclohexyle d'acide 2-méthoxybenzoïque ;
• 2-diméthylaminométhyl-1,4-bis-(3-méthoxyphényl)-cyclohexane-1,4-diol ;
• 2-diméthylaminométhyl-1,4-bis-(3-méthoxyphényl)-cyclohexane-1,4-diol ;
• 6-(4-benzyloxy-2-diméthylaminométhyl-1-hydroxycyclo-hexyl)-naphtalène-2-ol ;
• 4-benzyloxy-2-diméthylaminométhyl-1-(6-méthoxynaphtalène-2-yl)-cyclohexanol ;
• ester de 3-diméthylaminométhyl-4-hydroxy-4-(3-méthoxy-phényl)-cyclohexyle d'acide butyrique ;
• ester de 3-diméthylaminométhyl-4-hydroxy-4-(3-méthoxy-phényl)-cyclohexyle d'acide butyrique ;
• 2-diméthylaminométhyl-1-(3-méthoxyphényl)-4-méthylamino-cyclohexanol ;
• 2-diméthylaminométhyl-1-(3-méthoxyphényl)-4-méthylamino-cyclohexanol ;
• ester de 3-diméthylaminométhyl-4-hydroxy-4-(3-méthoxy-phényl)-cyclohexyle d'acide valérique ;
• ester éthylique d'acide [3-diméthylaminométhyl-4-hydroxy-4-(3-méthoxyphényl)-cyclohexylidène]-acétique ;
• ester éthylique d'acide [3-diméthylaminométhyl-4-hydroxy-4-(3-méthoxyphényl)-cyclohexylidène]-acétique ;
• 3-[2-diméthylaminométhyl-4-(4-fluorobenzyloxy)-1-hydroxy-cyclohexyl]-phénol ;
• 4-benzyloxy-2-diméthylaminométhyl-1-(3-phényl-propyl)-cyclohexanol ;
• 2-diméthylaminométhyl-4-(4-fluorobenzyloxy)-1-(3-phényl-propyl)-cyclohexanol ;
• 2-diméthylaminométhyl-1-(3-fluorobenzyl)-4-(4-fluoro-benzyloxy)-cyclohexanol ;
• 4-benzyloxy-2-diméthylaminométhyl-1-(3-fluorobenzyl)-cyclohexanol ;
• 2-diméthylamino-4-(4-fluorobenzyloxy)-1-p-tolyl-cyclohexanol ;
• 4-benzyloxy-2-diméthylaminométhyl-1-p-tolyl-cyclohexanol ;
• 2-diméthylaminométhyl-1-(2,5-diméthyl-benzyl)-4-(4-fluorobenzyloxy)-cyclohexanol ;
• 4-benzyloxy-2-diméthylaminométhyl-1-(2,5-diméthylbenzyl)-cyclohexanol ;
• 1-(4-chloro-3-trifluorométhyl-phényl)-2-diméthylamino-méthyl-4-(4-fluorobenzyloxy)-cyclohexanol ;
• 4-benzyloxy-1-(4-chloro-3-trifluorométhyl-phényl)-2-diméthylaminométhyl-cyclohexanol ;
•[2,5-bis-(4-fluorobenzyloxy)-2-(3-méthoxyphényl)-cyclohexyl-méthyl]-diméthylamine ;
• 5-diméthylaminométhyl-4-(3-méthoxyphényl)-cyclohex-3-énol ;
• ester de 3-diméthylaminométhyl-4-hydroxy-4-(6-méthoxy-naphtalène-2-yl)-cyclohexyle d'acide butyrique ;
• ester de 4-hydroxy-3-(4-méthyl-pipérazine-1-yl-méthyl)-4-naphtalène-2-yl-cyclohexyle d'acide benzoïque ;
• ester de 4-hydroxy-3-(4-méthyl-pipérazine-1-yl-méthyl)-4-naphtalène-2-yl-cyclohexyle d'acide 2-chlorobenzoïque ;
• ester de 4-hydroxy-3-(4-méthyl-pipérazine-1-yl-méthyl)-4-naphtalène-2-yl-cyclohexyle d'acide 3-chlorobenzoïque ;
• ester de 4-hydroxy-3-(4-méthyl-pipérazine-1-yl-méthyl)-4-naphtalène-2-yl-cyclohexyle d'acide 4-chlorobenzoïque ;
• ester de 4-hydroxy-3-(4-méthyl-pipérazine-1-yl-méthyl)-4-naphtalène-2-yl-cyclohexyle d'acide 2-fluorobenzoïque ;
• ester de 4-hydroxy-3-(4-méthyl-pipérazine-1-yl-méthyl)-4-naphtalène-2-yl-cyclohexyle d'acide 3-fluorobenzoïque ;
• ester de 4-hydroxy-3-(4-méthyl-pipérazine-1-yl-méthyl)-4-naphtalène-2-yl-cyclohexyle d'acide 4-fluorobenzoïque ;
• ester de 4-hydroxy-3-(4-méthyl-pipérazine-1-yl-méthyl)-4-naphtalène-2-yl-cyclohexyle d'acide 2-méthyl-benzoïque ;
• ester de 4-hydroxy-3-(4-méthyl-pipérazine-1-yl-méthyl)-4-naphtalène-2-yl-cyclohexyle d'acide 3-méthyl-benzoïque ;
• ester de 4-hydroxy-3-(4-méthyl-pipérazine-1-yl-méthyl)-4-naphtalène-2-yl-cyclohexyle d'acide 4-méthyl-benzoïque ;
• ester de 4-hydroxy-3-(4-méthyl-pipérazine-1-yl-méthyl)-4-naphtalène-2-yl-cyclohexyle d'acide 2-méthoxybenzoïque ;
• ester de 4-hydroxy-3-(4-méthyl-pipérazine-1-yl-méthyl)-4-naphtalène-2-yl-cyclohexyle d'acide 3-méthoxybenzoïque ;
• ester de 4-hydroxy-3-(4-méthyl-pipérazine-1-yl-méthyl)-4-naphtalène-2-yl-cyclohexyle d'acide 4-méthoxybenzoïque ;
• ester de 4-hydroxy-3-(4-méthyl-pipérazine-1-yl-méthyl)-4-naphtalène-2-yl-cyclohexyle d'acide 2,6-dichlorobenzoïque ;
• ester de 4-hydroxy-3-(4-méthyl-pipérazine-1-yl-méthyl)-4-naphtalène-2-yl-cyclohexyle d'acide 2,6-difluorobenzoïque ;
• ester de 4-hydroxy-3-(4-méthyl-pipérazine-1-yl-méthyl)-4-naphtalène-2-yl-cyclohexyle d'acide 2-chloro-5-fluorobenzoïque ;
• ester de 4-hydroxy-3-(4-méthyl-pipérazine-1-yl-méthyl)-4-naphtalène-2-yl-cyclohexyle d'acide biphényl-4-carboxylique ;
• ester de 4-hydroxy-4-(6-méthoxynaphtalène-2-yl)-3-(4-méthyl-pipérazine-1-yl-méthyl)-cyclohexyle d'acide 2-chlorobenzoïque ;
• ester de 4-hydroxy-4-(6-méthoxynaphtalène-2-yl)-3-(4-méthyl-pipérazine-1-yl-méthyl)-cyclohexyle d'acide 3-chlorobenzoïque ;
• ester de 4-hydroxy-4-(6-méthoxynaphtalène-2-yl)-3-(4-méthyl-pipérazine-1-yl-méthyl)-cyclohexyle d'acide 4-chlorobenzoïque ;
• ester de 4-hydroxy-4-(6-méthoxynaphtalène-2-yl)-3-(4-méthyl-pipérazine-1-yl-méthyl)-cyclohexyle d'acide 2-fluorobenzoïque ;
• ester de 4-hydroxy-4-(6-méthoxynaphtalène-2-yl)-3-(4-méthyl-pipérazine-1-yl-méthyl)-cyclohexyle d'acide 3-fluorobenzoïque ;
• ester de 4-hydroxy-4-(6-méthoxynaphtalène-2-yl)-3-(4-méthyl-pipérazine-1-yl-méthyl)-cyclohexyle d'acide 4-fluorobenzoïque ;
• ester de 4-hydroxy-4-(6-méthoxynaphtalène-2-yl)-3-(4-méthyl-pipérazine-1-yl-méthyl)-cyclohexyle d'acide 2-méthyl-benzoïque ;
• ester de 4-hydroxy-4-(6-méthoxynaphtalène-2-yl)-3-(4-méthyl-pipérazine-1-yl-méthyl)-cyclohexyle d'acide 3-méthyl-benzoïque ;
• ester de 4-hydroxy-4-(6-méthoxynaphtalène-2-yl)-3-(4-méthyl-pipérazine-1-yl-méthyl)-cyclohexyle d'acide 4-méthyl-benzoïque ;
• ester de 4-hydroxy-4-(6-méthoxynaphtalène-2-yl)-3-(4-méthyl-pipérazine-1-yl-méthyl)-cyclohexyle d'acide 2-méthoxybenzoïque ;
• ester de 4-hydroxy-4-(6-méthoxynaphtalène-2-yl)-3-(4-méthyl-pipérazine-1-yl-méthyl)-cyclohexyle d'acide 3-méthoxybenzoïque ;
• ester de 4-hydroxy-4-(6-méthoxynaphtalène-2-yl)-3-(4-méthyl-pipérazine-1-yl-méthyl)-cyclohexyle d'acide 4-méthoxybenzoïque ;
• ester de 4-hydroxy-4-(6-méthoxynaphtalène-2-yl)-3-(4-méthyl-pipérazine-1-yl-méthyl)-cyclohexyle d'acide 2,6-dichlorobenzoïque ;
• ester de 4-hydroxy-4-(6-méthoxynaphtalène-2-yl)-3-(4-méthyl-pipérazine-1-yl-méthyl)-cyclohexyle d'acide 2,6-fluorobenzoïque ;
• ester de 4-hydroxy-4-(6-méthoxynaphtalène-2-yl)-3-(4-méthyl-pipérazine-1-yl-méthyl)-cyclohexyle d'acide 2-chloro-6-fluorobenzoïque ;
• ester de 4-hydroxy-4-(6-méthoxynaphtalène-2-yl)-3-(4-méthyl-pipérazine-1-yl-méthyl)-cyclohexyle d'acide biphényl-4-carboxylique ;
• 4-(2-chlorobenzyloxy)-2-(4-méthyl-pipérazine-1-yl-méthyl)-1-naphtalène-2-yl-cyclohexanol ;
• 4-(3-chlorobenzyloxy)-2-(4-méthyl-pipérazine-1-yl-méthyl)-1-naphtalène-2-yl-cyclohexanol ;
• 4-(4-chlorobenzyloxy)-2-(4-méthyl-pipérazine-1-yl-méthyl)-1-naphtalène-2-yl-cyclohexanol ;
• 4-(2-fluorobenzyloxy)-2-(4-méthyl-pipérazine-1-yl-méthyl)-1-naphtalène-2-yl-cyclohexanol ;
• 4-(3-fluorobenzyloxy)-2-(4-méthyl-pipérazine-1-yl-méthyl)-1-naphtalène-2-yl-cyclohexanol ;
• 4-(4-fluorobenzyloxy)-2-(4-méthyl-pipérazine-1-yl-méthyl)-1-naphtalène-2-yl-cyclohexanol ;
• 4-(2-méthyl-benzyloxy)-2-(4-méthyl-pipérazine-1-yl-méthyl)-1-naphtalène-2-yl-cyclohexanol ;
• 4-(3-méthyl-benzyloxy)-2-(4-méthyl-pipérazine-1-yl-méthyl)-1-naphtalène-2-yl-cyclohexanol ;
• 4-(4-méthyl-benzyloxy)-2-(4-méthyl-pipérazine-1-yl-méthyl)-1-naphtalène-2-yl-cyclohexanol ;
• 4-(2-méthoxybenzyloxy)-2-(4-méthyl-pipérazine-1-yl-méthyl)-1-naphtalène-2-yl-cyclohexanol ;
• 4-(3-méthoxybenzyloxy)-2-(4-méthyl-pipérazine-1-yl-méthyl)-1-naphtalène-2-yl-cyclohexanol ;
• 4-(4-méthoxybenzyloxy)-2-(4-méthyl-pipérazine-1-yl-méthyl)-1-naphtalène-2-yl-cyclohexanol ;
• 4-(2,6-dichlorobenzyloxy)-2-(4-méthyl-pipérazine-1-yl-méthyl)-1-naphtalène-2-yl-cyclohexanol ;
• 4-(2,6-difluorobenzyloxy)-2-(4-méthyl-pipérazine-1-yl-méthyl)-1-naphtalène-2-yl-cyclohexanol ;
• 4-(2-chloro-6-fluorobenzyloxy)-2-(4-méthyl-pipérazine-1-yl-méthyl)-1-naphtalène-2-yl-cyclohexanol ;
• 4-(2-chlorobenzyloxy)-1-(6-méthoxynaphtalène-2-yl)-2-(4-méthyl-pipérazine-1-ylméthyl)-cyclohexanol ;
• 4-(3-chlorobenzyloxy)-1-(6-méthoxynaphtalène-2-yl)-2-(4-méthyl-pipérazine-1-ylméthyl)-cyclohexanol ;
• 4-(4-chlorobenzyloxy)-1-(6-méthoxynaphtalène-2-yl)-2-(4-méthyl-pipérazine-1-ylméthyl)-cyclohexanol ;
• 4-(2-fluorobenzyloxy)-1-(6-méthoxynaphtalène-2-yl)-2-(4-méthyl-pipérazine-1-ylméthyl)-cyclohexanol ;
• 4-(3-fluorobenzyloxy)-1-(6-méthoxynaphtalène-2-yl)-2-(4-méthyl-pipérazine-1-ylméthyl)-cyclohexanol ;
• 4-(4-fluorobenzyloxy)-1-(6-méthoxynaphtalène-2-yl)-2-(4-méthyl-pipérazine-1-ylméthyl)-cyclohexanol ;
• 4-(2-méthyl-benzyloxy)-1-(6-méthoxynaphtalène-2-yl)-2-(4-méthyl-pipérazine-1-ylméthyl)-cyclohexanol ;
• 4-(3-méthyl-benzyloxy)-1-(6-méthoxynaphtalène-2-yl)-2-(4-méthyl-pipérazine-1-ylméthyl)-cyclohexanol ;
• 4-(4-méthyl-benzyloxy)-1-(6-méthoxynaphtalène-2-yl)-2-(4-méthyl-pipérazine-1-ylméthyl)-cyclohexanol ;
• 4-(2-méthoxybenzyloxy)-1-(6-méthoxynaphtalène-2-yl)-2-(4-méthyl-pipérazine-1-ylméthyl)-cyclohexanol ;
• 4-(3-méthoxybenzyloxy)-1-(6-méthoxynaphtalène-2-yl)-2-(4-méthyl-pipérazine-1-ylméthyl)-cyclohexanol ;
• 4-(4-méthoxybenzyloxy)-1-(6-méthoxynaphtalène-2-yl)-2-(4-méthyl-pipérazine-1-ylméthyl)-cyclohexanol ;
• 4-(2,6-dichlorobenzyloxy)-1-(6-méthoxynaphtalène-2-yl)-2-(4-méthyl-pipérazine-1-ylméthyl)-cyclohexanol ;
• 4-(2,6-difluorobenzyloxy)-1-(6-méthoxynaphtalène-2-yl)-2-(4-méthyl-pipérazine-1-ylméthyl)-cyclohexanol ;
• 4-(2-chloro-6-fluorobenzyloxy)-1-(6-méthoxynaphtalène-2-yl)-2-(4-méthyl-pipérazine-1-ylméthyl)-cyclohexanol ;
• 2-diméthylaminométhyl-4-(4-fluorobenzyloxy)-1-naphtalène-2-yl-cyclohexanol ;
• ester de 3-diméthylaminométhyl-4-hydroxy-4-(6-méthoxy-naphtalène-2-yl)-cyclohexyl d'acide 4-fluorobenzoïque ;
• ester de 3-diméthylaminométhyl-4-hydroxy-4-naphtalène-2-yl-cyclohexyl d'acide 4-fluorobenzoïque ;
le cas échéant sous forme de leurs racémates, de leurs stéréo-isomères purs ou sous forme de mélanges des stéréo-isomères, dans un rapport de mélange quelconque, sous la forme représentée ou sous forme de leurs acides ou de leurs bases ou sous forme de leurs sels, ou sous forme de leurs produits de solvatation.

9. Médicament contenant au moins un dérivé de cyclohexylméthylamine C-substitué suivant l'une des revendications 1 à 8, ainsi que, le cas échéant, des additifs et/ou des substances auxiliaires convenables et/ou le cas échéant, d'autres substances actives.

10. Médicament suivant la revendication 9,
**caractérisé en ce qu'**un dérivé de cyclohexylméthylamine C-substitué suivant l'une des revendications 1 à 8 qu'il contient, est présent sous forme de diastéréo-isomère pur et/ou d'énantiomère pur, sous forme de racémate ou de mélange non équimolaire ou équimolaire des diastéréo-isomères et/ou des énantiomères.

11. Utilisation d'un dérivé de cyclohexylméthylamine C-substitué suivant l'une des revendications 1 à 8 pour la préparation d'un médicament destiné au traitement de la douleur, en particulier d'une douleur neuropathique
ou chronique.

12. Utilisation suivant la revendication 11,
**caractérisée en ce qu'**un dérivé de cyclohexylméthylamine C-substitué suivant l'une des revendications 1 à 8 qu'il contient, est présent sous forme de diastéréo-isomère pur et/ou d'énantiomère pur, sous forme de racémate ou sous forme de mélange non équimolaire ou équimolaire des diastéréo-isomères et/ou des énantiomères.
